(19) Europäisches Patentamt
European Patent Office
Office européen des brevets

(11) EP 4 763 846 A1

(12) **EUROPEAN PATENT APPLICATION**
published in accordance with Art. 153(4) EPC

(43) Date of publication:
**24.06.2026 Bulletin 2026/26**

(21) Application number: **24855720.9**

(22) Date of filing: **15.08.2024**

(51) International Patent Classification (IPC):
***C07D 471/04*** *(2006.01)* ***C07D 487/02*** *(2006.01)*
***C07D 519/00*** *(2006.01)* ***A61K 31/519*** *(2006.01)*
***A61P 31/00*** *(2006.01)* ***A61P 35/00*** *(2006.01)*

(52) Cooperative Patent Classification (CPC):
**A61K 31/519; A61P 31/00; A61P 35/00; C07D 471/04; C07D 487/02; C07D 519/00**

(86) International application number:
**PCT/CN2024/112501**

(87) International publication number:
**WO 2025/039995 (27.02.2025 Gazette 2025/09)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **18.08.2023 CN 202311048960**

(71) Applicant: **Miracure Biotechnology Limited
Haidian, Beijing 100192 (CN)**

(72) Inventors:
- **LI, Xiaolin
Beijing 102629 (CN)**
- **QI, Longwu
Beijing 102629 (CN)**
- **LU, Peichao
Beijing 102629 (CN)**

(74) Representative: **v. Bezold & Partner
Patentanwälte - PartG mbB
Ridlerstraße 57
80339 München (DE)**

(54) **PYRIMIDINE DERIVATIVE OR SALT THEREOF USED AS TLR7/8 AGONIST, PHARMACEUTICAL COMPOSITION THEREOF, AND USE THEREOF**

(57) A pyrimidine derivative represented by formula (I-1), formula (I-2), formula (II-1) or formula (II-2), or a stereoisomer, tautomer, isotopic derivative, hydrate, solvate, prodrug, and pharmaceutically acceptable salt thereof. The pyrimidine derivative can be used as an effective TLR7 and/or TLR8 agonist, and has the characteristic of good selectivity, high activity, and good safety. Also provided are a preparation method for the pyrimidine derivative, and a pharmaceutical composition thereof. The compound or the pharmaceutical composition thereof can be used for preparing drugs for preventing or treating the following diseases: infectious diseases, respiratory system diseases, immune-related disease, viral diseases or tumors.

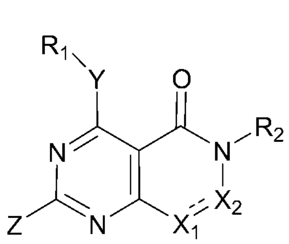

(I-1)

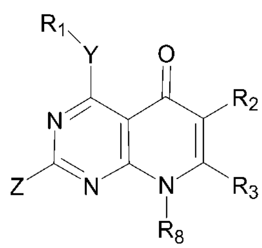

(I-2)

EP 4 763 846 A1

## Description

### CROSS-REFERENCE TO RELATED APPLICATIONS

This application claims priority to Patent Application No. 202311048960.0, filed on August 18, 2023, and entitled "PYRIMIDINE DERIVATIVE OR SALT THEREOF USED AS TLR7/8 AGONIST, PHARMACEUTICAL COMPOSITION THEREOF, AND USE THEREOF", which is incorporated herein by reference in its entirety.

### TECHNICAL FIELD

This application relates to the technical field of Toll-like receptor (TLR) agonists, and in particular, to a pyrimidine derivative or salt thereof used as a TLR7/8 agonist, a pharmaceutical composition and use thereof, as well as methods for preventing or treating an infectious disease, a respiratory disease, an immune-related disease, a viral disease or a tumor by using the pyrimidine derivative used as TLR7 and/or TLR8 agonist or a salt thereof.

### BACKGROUND

Toll-like receptors, as a conservatively evolved receptor family, include at least 13 members, ten of which (namely, TLR1-TLR10) have been identified in humans, where TLR1, TLR2, TLR4, TLR5, TLR6 and TLR10 are expressed on the cell surface and can rapidly identify metabolites of bacteria, and TLR3, TLR7, TLR8 and TLR9 are expressed intracellularly and are mainly for detection and recognition of viral nucleic acids. TLR3 may identify a double-stranded RNA, TLR7 and TLR8 may identify single-stranded RNAs, and TLR9 may identify unmethylated CpG oligodeoxynucleotide to mediate responses to bacterial DNAs and certain viruses.

The TLRs can specifically identify pathogen-associated molecular patterns (PAMPs), and are critical in both innate immunity and adaptive immunity and serve as a bridge linking the innate immunity and the adaptive immunity. After identifying and binding to viral single-stranded RNAs or synthetic small-molecule purine compounds, TLR7 recruits specific adapter proteins, activates a series of signal cascade reactions, and initiates a high-level systemic adaptive immune response, to kill virus-infected cells, thereby completely eliminating the virus. TLR7 agonists have been clinically used to treat chronic viral infectious diseases, e.g. hepatitis B and hepatitis C. In addition, when used as adjuvants for influenza vaccines, TLR7 agonists can induce faster and more effective immune protection. TLR7 agonists can not only directly stimulate pDCs to secrete IFN-$\alpha$, but also enhance co-stimulation and antigen-presenting capacities of the pDCs. Activated pDCs promote proliferation of $CD4^+$ T cells, which in turn activates $CD8^+$ T cells to kill tumor cells. Therefore, the function of the TLR7 agonists as immune adjuvants in identifying and eliminating tumor cells in the body has also gradually gained increasing attention. Under stimulation of antigens and TLR8 agonists, the cDCs get mature and secrete co-stimulatory cytokines such as CD80, CD86 and pro-inflammatory cytokines, including IL-12, TNF-$\alpha$, IL-6, and the like. The cDCs migrating to dLNs activate T cells and stimulate T cells and NK cells to secrete IFN-$\gamma$, thereby further amplifying the immune cascade response.

The TLR7/8 agonists rely on a MyD-NF-$\kappa$B signaling pathway to trigger innate and adaptive immune responses. As immunomodulators, the TLR7/8 agonists can activate and promote differentiation and development of immune cells, regulate a direction of the immune response, and therefore, can exert effects in the fields of respiratory diseases, inflammatory diseases, anti-allergy, anti-virus, anti-tumor and skin injury repair. Extensive researches have been or are being conducted on the TLR7/8 agonists in preclinical and clinical trials.

TLR7 and TLR8 receptors display different expression levels in the cDCs and pDCs, as well as in other somatic cells and immune cells. The same type of immune cells express various levels of TLR7 (or TLR8) receptors when localized in different organs and tissues, and are subject to regulation depending on the cellular/immune status. During TLR signaling regulation, tolerance to agonists is observed, and the TLR7 and TLR8 signaling pathways interconnect with one another. Dosage and administration time of agonist molecules have a significant impact on clinical efficacy. Clinically, this often results in no therapeutic effect, or is accompanied by severe adverse reactions and even paradoxical effects (e.g., promoting rather than inhibiting tumor progression).

More than 20 nuclear scaffolds have been found by now, and approximately 1500 molecules have been synthesized via combined substitution and modification of two main distinct branches, including a large number of small-molecule TLR7/8 agonists with varying activity levels. Many of these molecules exert stimulatory effects on the immune system in clinical practice (RSC Med. Chem., 2021, 12, 1065-1120). The use of R837 and R848, which have been approved for marketing, is limited to external application and treatment of skin-related diseases, mainly due to severe off-target side effects or the lack of clinical efficacy.

While researchers are attempting to develop new small-molecule TLR7 and/or TLR8 agonists, substantial efforts have been focused on optimizing pharmacokinetic profiles to achieve local accumulation or targeted delivery and reduce systemic immune responses, e.g. development of slow-release hydrosols, nano-scaled particles (e.g. peptides, polymers, cyclodextrins, and carrier proteins), prodrugs modified with branched chains like long-chain fatty acids or phosphoric acids, or a combination therapy with other drugs. The same strategies are also applied to the development of TLR7 and/or TLR8 to be used as vaccine adjuvants (Advanced Drug Delivery Reviews 175 (2021) 113803**).**

## SUMMARY

An objective of this application is to provide a pyrimidine derivative, which may be used as potent TLR7 and/or TLR8 receptor agonists, and featured with excellent selectivity, high activity and high safety, and therefore, can be used for preparing a medicament for preventing or treating the following diseases: an infectious disease, a respiratory disease, an immune-related disease, a viral disease or a tumor. This application also provides a method for preparing the pyrimidine derivative, and a pharmaceutical composition thereof.

To achieve the foregoing objective, according to a first aspect, this application provides a pyrimidine derivative, which is a compound of Formula (I-1) or (I-2), or a stereoisomer, a tautomer, an isotopic derivative, a halogenated derivative, a hydrate, a solvate, a prodrug, or a pharmaceutically acceptable salt thereof;

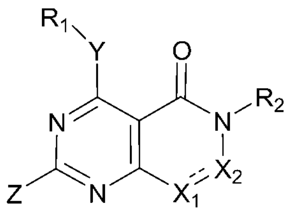

Formula (I-1)

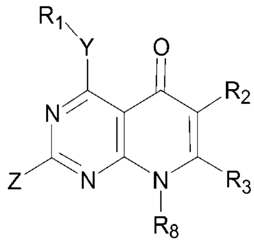

Formula (I-2)

where:

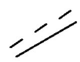

represents a single bond or a double bond;

$X_1$ and $X_2$ are both C-$R_3$, or one of them is C-$R_3$ and the other is N;

Z is $-NH_2$, -OH, -NH-alkyl, -O-alkyl, -NH-C(O)-alkyl, -O-C(O)-alkyl, -NH-C(O)-OH, or -O-C(O)-OH;

Y is -O-, -S-, or $-NR_4-$, where $R_4$ is H or an optionally substituted alkyl group;

$R_1$ is an alkyl group or an aryl group optionally substituted with one or more substituents selected from oxo, halogen, an amino group, a hydroxyl group, a nitro group, a cyano group, a thiol group, $-C(O)-NH_2$, -C(O)-OH, a heteroaryl group (optionally substituted with one or more of halogen, an amino group, a hydroxyl group, a nitro group, a cyano group, a thiol group, an alkoxyl group, and an alkyl group), an aryl group (optionally substituted with one or more of halogen, an amino group, a hydroxyl group, a nitro group, a cyano group, a thiol group, an alkoxyl group, and an alkyl group), a heterocycloalkyl group (optionally substituted with one or more of halogen, an amino group, a hydroxyl group, a nitro group, a cyano group, a thiol group, an alkoxyl group, and an alkyl group), a cycloalkyl group (optionally substituted with one or more of halogen, an amino group, a hydroxyl group, a nitro group, a cyano group, a thiol group, an alkoxyl group, and an alkyl group), an optionally substituted alkyl group, an optionally substituted alkyloxy group, an optionally substituted alkylthio group, an optionally substituted alkylamino group, an optionally substituted alkylacyl group, an optionally substituted alkylsulfinyl group, an optionally substituted alkylsulfonyl group, an optionally substituted -C(O)-O-alkyl, optionally substituted -O-C(O)-alkyl, optionally substituted -C(O)-NH-alkyl, optionally substituted -NH-C(O)-alkyl, optionally substituted -S(O)-O-alkyl, optionally substituted -O-S(O)-alkyl, optionally substituted $-S(O)_2-O$-alkyl, optionally substituted $-O-S(O)_2$-alkyl, optionally substituted -S(O)-NH-alkyl, optionally substituted -NH-S(O)-alkyl, optionally substituted $-S(O)_2-NH$-alkyl, and optionally substituted $-NH-S(O)_2$-alkyl;

$R_2$ is

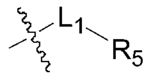 or 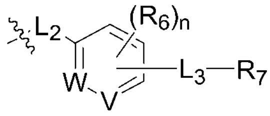 ;

and $-L_3-R_7$ may be substituted at a para-, meta- or ortho-position of $L_2$;

$L_1$, $L_2$ and $L_3$ are each a bond or an optionally substituted linear alkylene group, where one or more carbon atoms in the linear alkylene group may be substituted with oxygen, sulfur and nitrogen heteroatoms, and the substituent is selected from one or more of an optionally substituted alkyl group, halogen, an amino group, a hydroxyl group, a nitro group, a cyano group, a thiol group, an optionally substituted alkyloxy group, an optionally substituted alkylthio group and an optionally substituted alkylamino group;

$R_5$ is a cycloalkyl group or a heterocycloalkyl group optionally substituted with one or more substituents selected from oxo, halogen, an amino group, a hydroxyl group, a nitro group, a cyano group, a thiol group, an optionally substituted alkyl group, an optionally substituted alkyloxy group, an optionally substituted alkylthio group, and an optionally substituted alkylamino group;

each of W and V may be N or CH; and W or V may be substituted with $R_6$ or $-L_3-R_7$ when being CH;

$R_3$ and $R_6$ are each independently H, an optionally substituted alkyl group, an optionally substituted alkyloxy group, an optionally substituted alkylthio group, an optionally substituted alkylamino group, halogen, an amino group, a hydroxyl group, a nitro group, a cyano group, and a thiol group;

$R_7$ is H, -OH, $-N(R_9R_{10})$, $-N(R_9)NH_2$, $-NO(R_9R_{10})$, an optionally substituted heterocycloalkyl group, an optionally substituted cycloalkyl group, an optionally substituted aryl group, an optionally substituted heteroaryl group, optionally substituted heterocycloalkyl-$NR_9$-, optionally substituted cycloalkyl-$NR_9$-, optionally substituted aryl-$NR_9$-, optionally substituted heteroaryl-$NR_9$-, $-OC(O)-R_9$, $-C(O)O-R_9$, $-N(R_9)-C(O)-R_{10}$ or $-C(O)-N(R_9R_{10})$; and the substituent is selected from one or more of an optionally substituted alkyl group, an optionally substituted alkylamino group and oxo;

$R_8$ is H or an optionally substituted alkyl group;

$R_9$ and $R_{10}$ are each independently H, an optionally substituted alkyl group, an optionally substituted cycloalkyl group, an optionally substituted heterocycloalkyl group, an optionally substituted aryl group, and an optionally substituted heteroaryl group; and

the substituent of the optionally substituted alkyl group is selected from one or more of halogen, an amino group, a hydroxyl group, a nitro group, a cyano group, a thiol group, a carboxyl group, an ester group and an amide group;

where n = 0, 1, 2, 3 or 4.

In a group of embodiments, one of $X_1$ and $X_2$ is $C-R_3$ and the other is N.

In a group of embodiments, $R_8$ is H or an optionally substituted C1-8 alkyl group;

In a group of embodiments, $R_1$ is a C1-8 alkyl group optionally substituted with one or more substituents selected from an optionally substituted C1-8 alkyloxy group, an optionally substituted C1-8 alkylthio group, an optionally substituted C1-8 alkylamino group, an optionally substituted C1-8 alkylacyl group, an optionally substituted C1-8 alkylsulfinyl group, and an optionally substituted C1-8 alkylsulfonyl group; preferably, $R_1$ is a C2-6 alkyl group optionally substituted with an optionally substituted C1-6 alkyloxy group, an optionally substituted C1-6 alkylthio group, and an optionally substituted C1-6 alkylsulfonyl group; and preferably, $R_1$ is an optionally substituted methyl group, ethyl group, n-propyl group, isopropyl group, n-butyl group, isobutyl group, tert-butyl group, n-pentyl group, 2-pentyl group, n-hexyl group, 2-hexyl group or 3-hexyl group.

Further, $R_1$ is an n-butyl group, an isobutyl group, a tert-butyl group, an n-pentyl group, a 2-pentyl group, a 3-hexyl group, a methoxyethyl group, a methylthioethyl group, a methylsulfonylethyl group, a methoxypropyl group, a methylthiopropyl group, a methylsulfonylpropyl group,

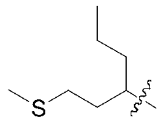 or 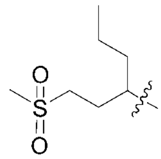.

Preferably, $R_1$ is an n-butyl group,

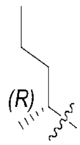, 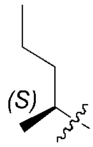,

a methylthiopropyl group, a methylsulfonylpropyl group,

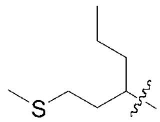 or 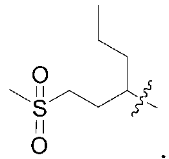.

In a group of embodiments, W and V are both N, W and V are both C, or one of W and V is N.

In a group of embodiments, the heterocycloalkyl group is a 3- to 12-membered monocyclic, bicyclic or polycyclic heterocycloalkyl group; preferably, the heterocycloalkyl group is a 3- to 12-membered monocyclic, bicyclic or polycyclic heterocycloalkyl group containing 1, 2, 3 or 4 heteroatoms selected from N, O, and S; more preferably, the heteroatom in the heterocycloalkyl group is linked to $L_1$ or $L_3$; and more preferably, N atom in the heterocycloalkyl group is linked to $L_1$ or $L_3$.

Further, the heterocycloalkyl group is a 3- to 7-membered monocyclic heterocycloalkyl group containing 1 or 2 heteroatoms selected from N, O, and S, or is a 7- to 12-membered fused-ring or spiro heterocycloalkyl group containing 1, 2, 3 or 4 heteroatoms selected from N, O, and S; and preferably, the heterocycloalkyl group is a 4- to 6-membered monocyclic heterocycloalkyl group containing 1 or 2 heteroatoms selected from N and O, or is a 7- to 11-membered spiro heterocycloalkyl group containing 2, 3 or 4 heteroatoms selected from N and O.

Further, the heterocycloalkyl group may be one of the following groups:

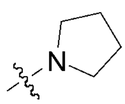, 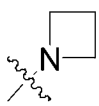, 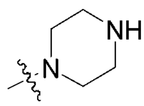, 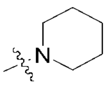, 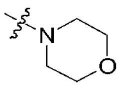, 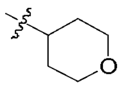, 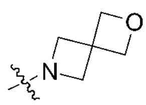 and 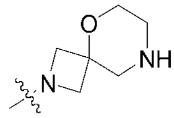.

In a group of embodiments, the aryl group is a C6-10 aryl group, preferably a phenyl group.

In a group of embodiments, $L_1$, $L_2$ and $L_3$ are each a bond or an optionally substituted linear alkylene group containing 1 to 6 chain atoms, 1 or 2 carbon atoms in the alkylene group may be substituted with oxygen, sulfur and nitrogen heteroatoms, and the substituent is selected from the C1-8 alkyl group, halogen, an amino group, a hydroxyl group, a nitro group, a cyano group and a thiol group.

Further, $L_1$ is an optionally substituted linear alkylene group containing 1 to 4 chain atoms, more preferably is an optionally substituted linear alkylene group containing 2 or 3 chain atoms, and more preferably, is -O-propylene or $-CH_2CH_2CH_2-$.

$L_2$ is an optionally substituted linear alkylene group containing 1 to 4 chain atoms, preferably is an optionally substituted linear alkylene group containing 2 or 3 chain atoms, and more preferably, is $-CH_2-$, $-CH_2CH_2-$, -O-, $-O-CH_2-CH_2-$ and $-O-CH_2-$.

$L_3$ is a bond or an optionally substituted linear alkylene group containing 1 to 4 chain atoms, preferably is a bond or an optionally substituted linear alkylene group containing 2 or 3 chain atoms, more preferably is a bond, $-CH_2-$, $-CH_2CH_2-$, $-CH_2CH_2CH_2-$, $-O-CH_2-CH_2-$, $-O-CH_2-$ and $-CH(CH_3)CH_2-$, and more preferably, is

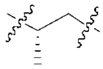 or 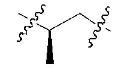.

In a group of embodiments, $R_9$ and $R_{10}$ are each independently H or a $C_{1-8}$ alkyl group.

In a group of embodiments, $R_7$ is -OH, -N($R_9R_{10}$), -N($R_9$)$NH_2$, -NO($R_9R_{10}$), an optionally substituted 3- to 12-membered heterocycloalkyl group containing 1, 2, 3 or 4 heteroatoms selected from N, O, and S, an optionally substituted C6-10 aryl group, optionally substituted 3- to 12-membered heterocycloalkyl-$NR_9$- containing 1, 2, 3 or 4 heteroatoms selected from N, O, and S, or optionally substituted C6-10 aryl-$NR_9$-; the substituent is selected from one or more of an optionally substituted C1-8 alkyl group, an optionally substituted C1-8 alkylamino group, an optionally substituted di(C1-8) alkylamino group and oxo; and $R_9$ and $R_{10}$ are each independently H or a C1-8 alkyl group; and preferably, the heterocycloalkyl group is a 4- to 6-membered monocyclic heterocycloalkyl group containing 1 or 2 heteroatoms selected from N and O, or is a 7- to 11-membered spiro heterocycloalkyl group containing 2, 3 or 4 heteroatoms selected from N and O; more preferably, the heteroatom in the heterocycloalkyl group is linked to $L_3$; and more preferably, the N atom in the heterocycloalkyl group is linked to $L_3$.

In a group of embodiments, $R_2$ is selected from one of the following optionally substituted groups:

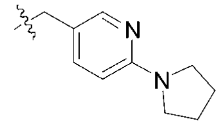, 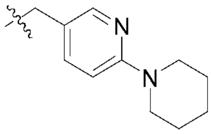, 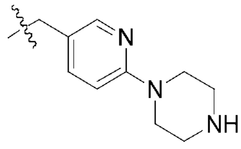, 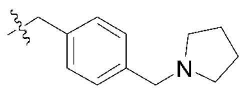,

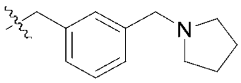, 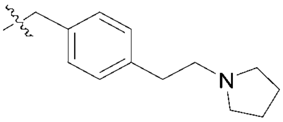, 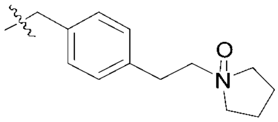, 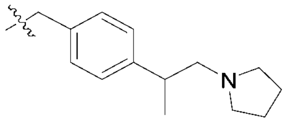,

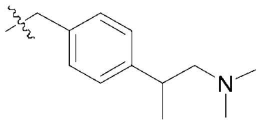, 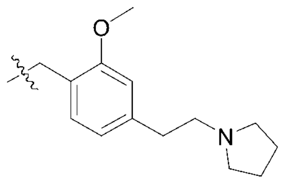, 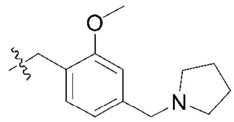, 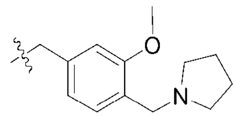,

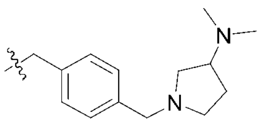, 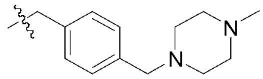, 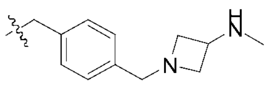, 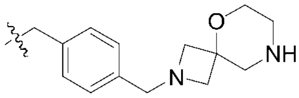,

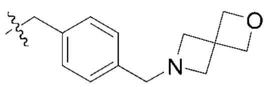, 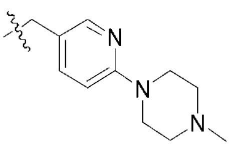, 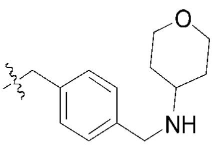, 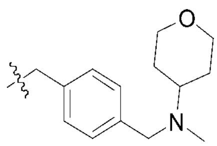, 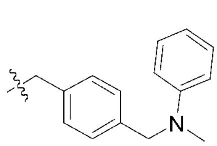,

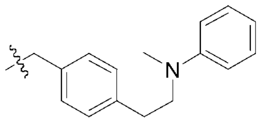, 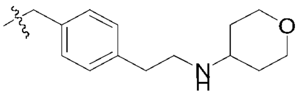, 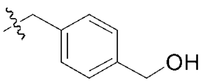, 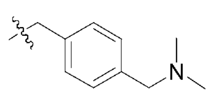,

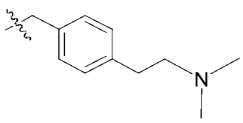, 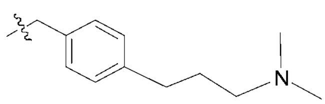, 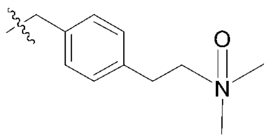, 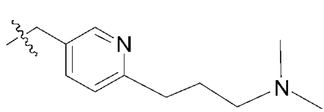,

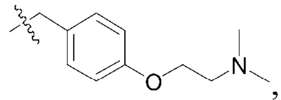, 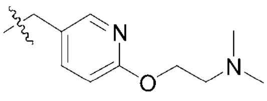, 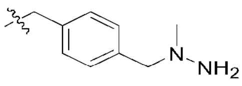, 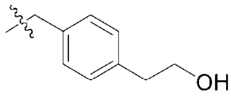, 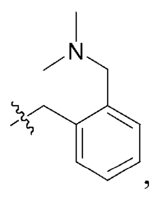,

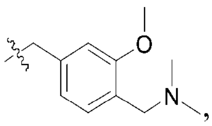, 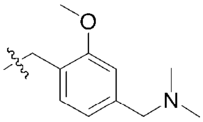, 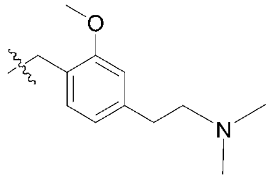, 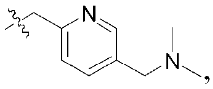, 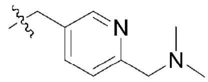,

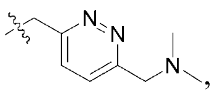, 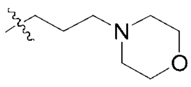, 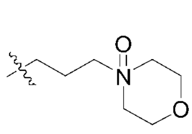, 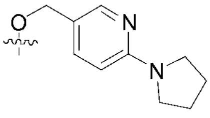, 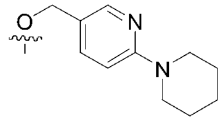,

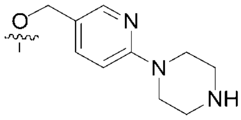, 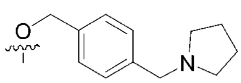, 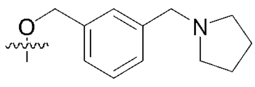, 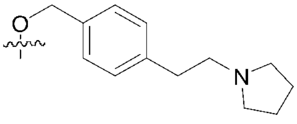,

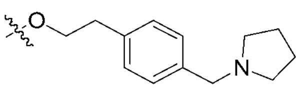, 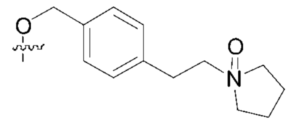, 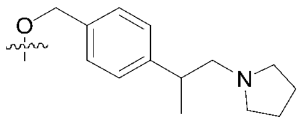, 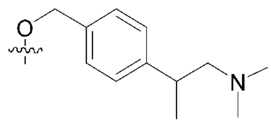,

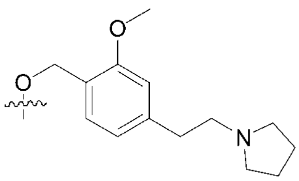, 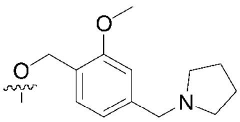, 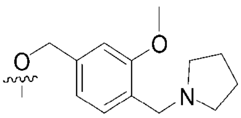, 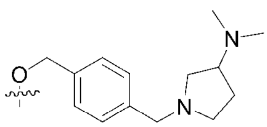,

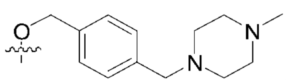, 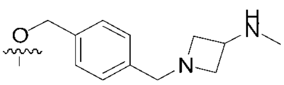, 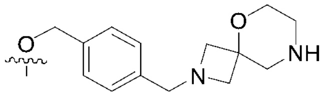, 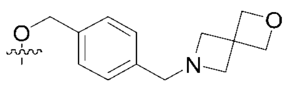,

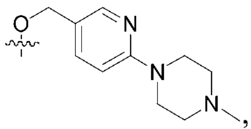, 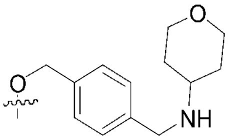, 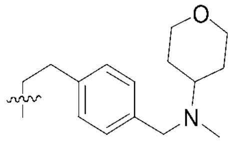, 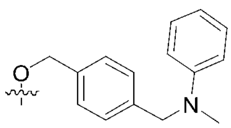,

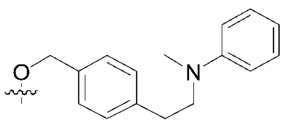, 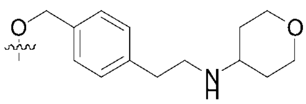, 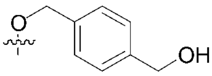, 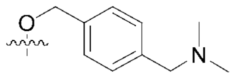,

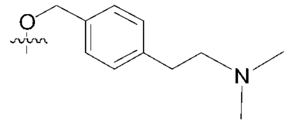,
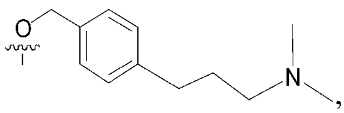,
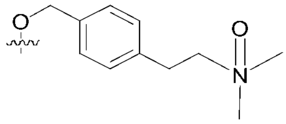,

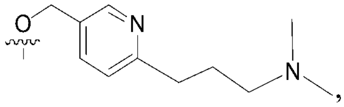,
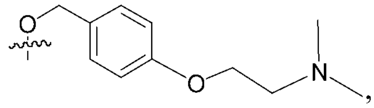,
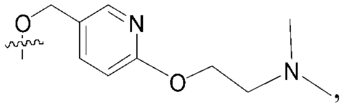,

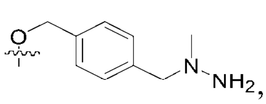,
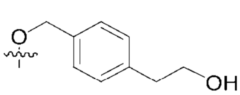,
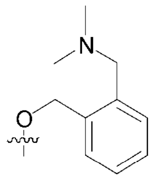,
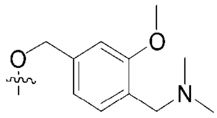,
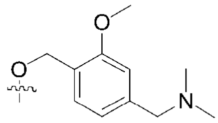,

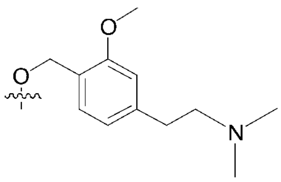,
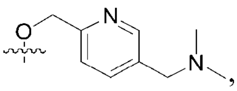,
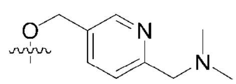,
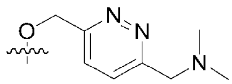,

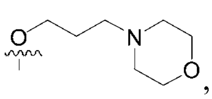,
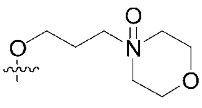,
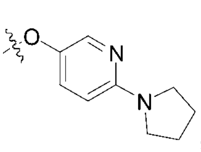,
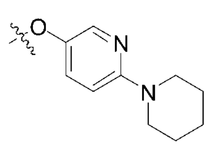,
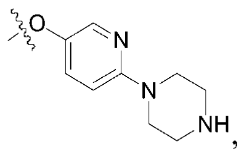,

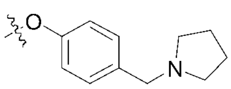,
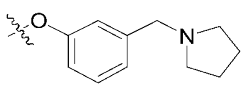,
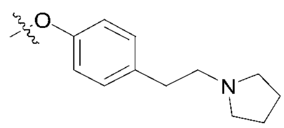,
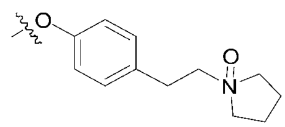,

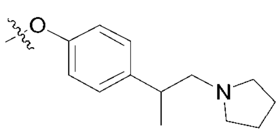,
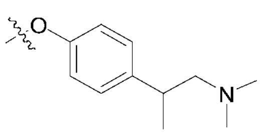,
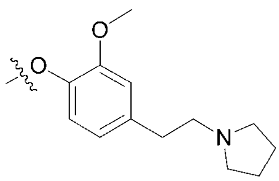,
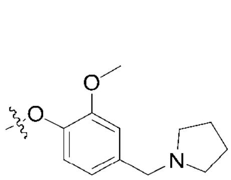,

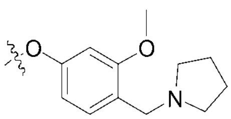,
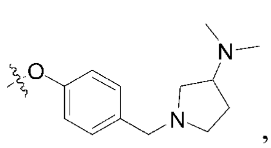,
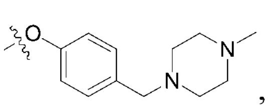,
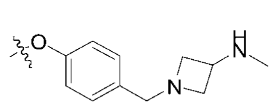,

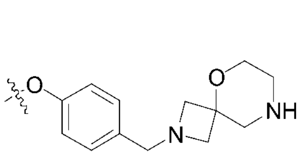,
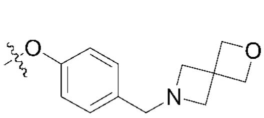,
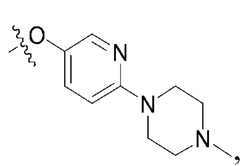,
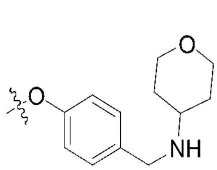,

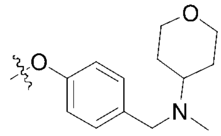, 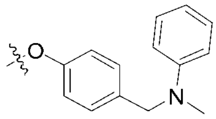, 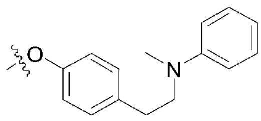, 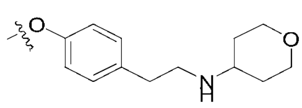,

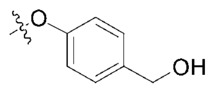, 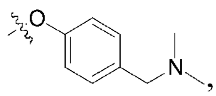, 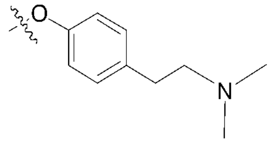, 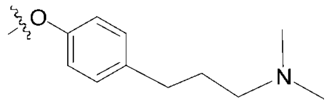,

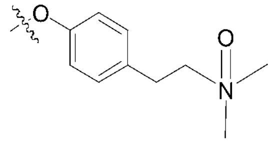, 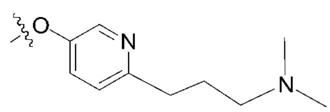, 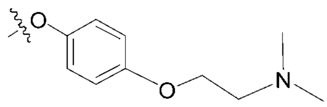,

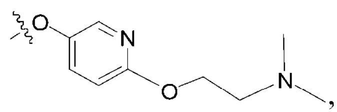, 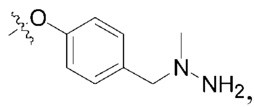, 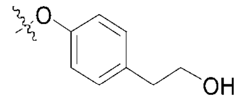, 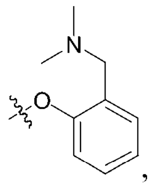, 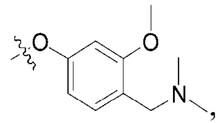,

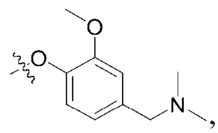, 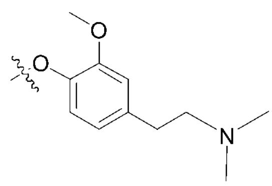, 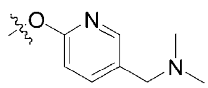, 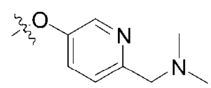, 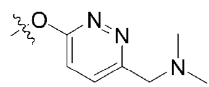,

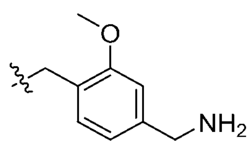, 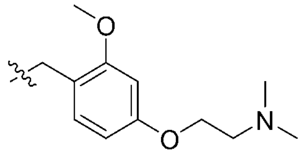, 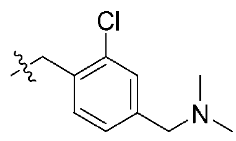, 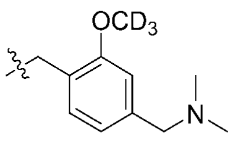,

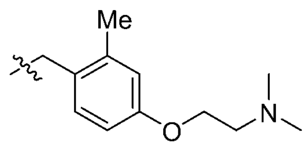, 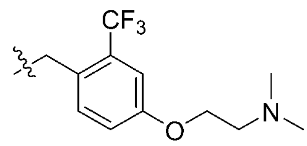, 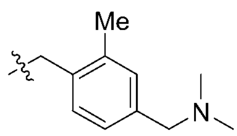, 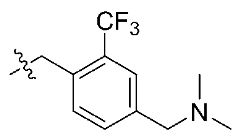,

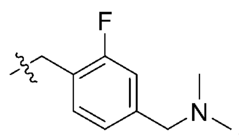, 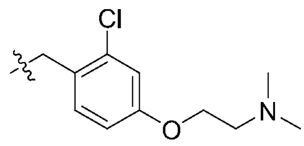, 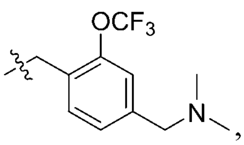, 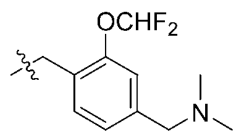,

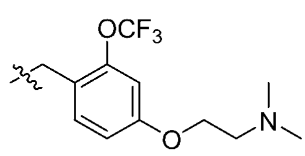, 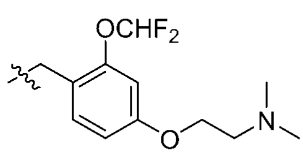, 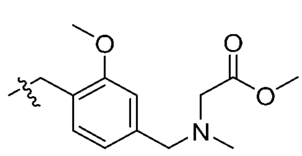, 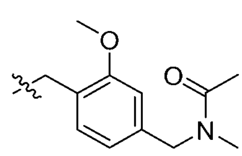

and

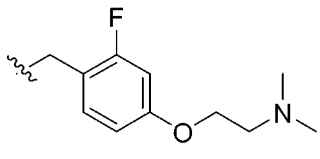;

further, $R_2$ is optionally substituted

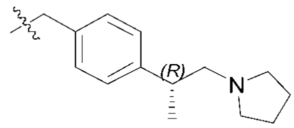, 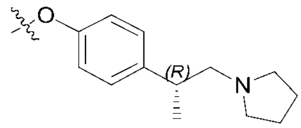,

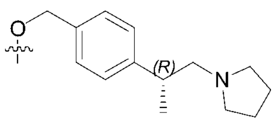, 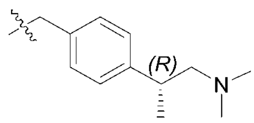, 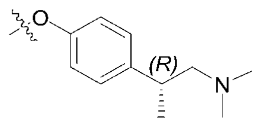, 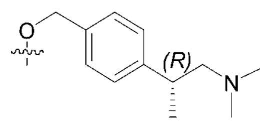,

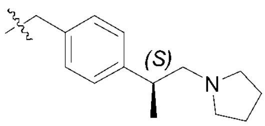, 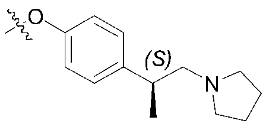, 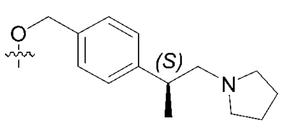, 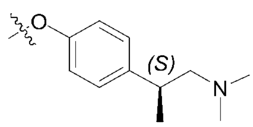,

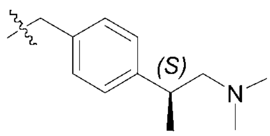 and 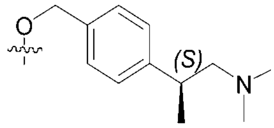

; and

the substituent is selected from one or more of an alkyl group, an alkyloxy group, an alkylthio group, an alkylamino group, halogen, an amino group, a hydroxyl group, a nitro group, a cyano group and a sulfhydryl group.

In a group of preferred embodiments, the compound of Formula (I-1) or (I-2) specifically has one of the following formulas:

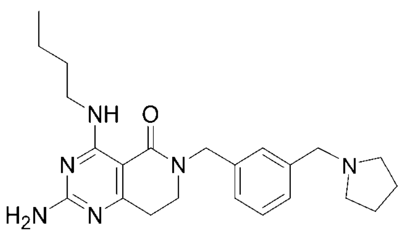

18 ,

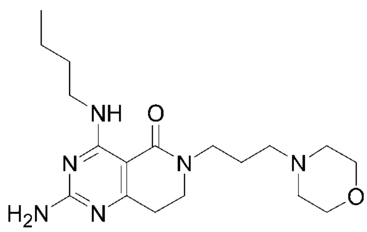

19 ,

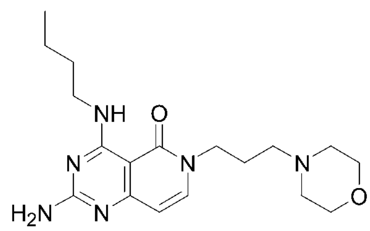

27 ,

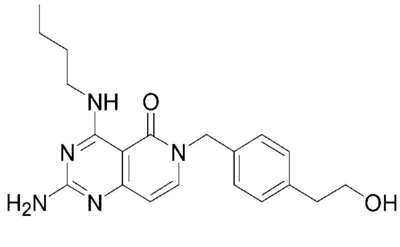

39 ,

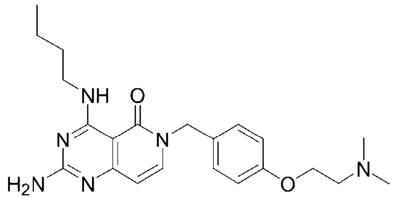

42 ,

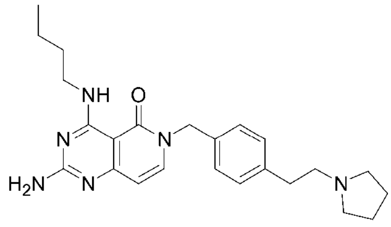

43 ,

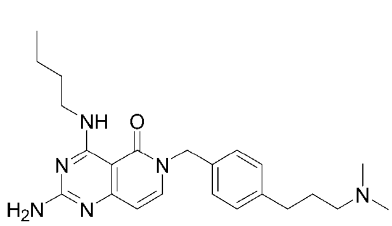

55 ,

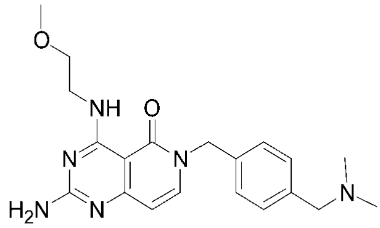

56 ,

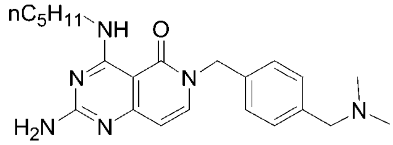

57 ,

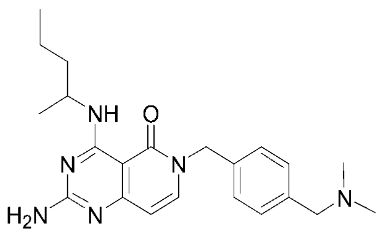

58 ,

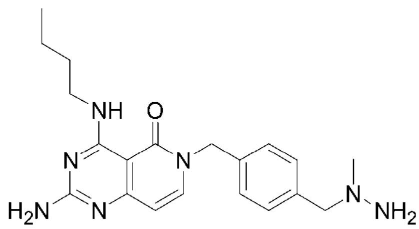

60 ,

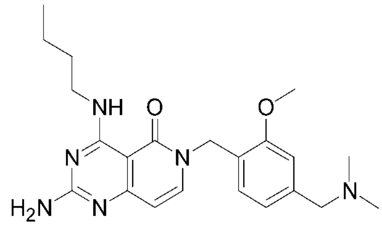

61 ,

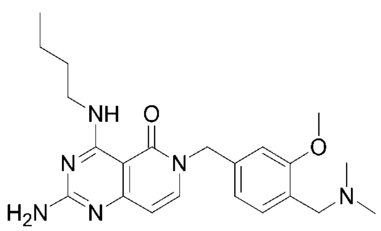

62 ,

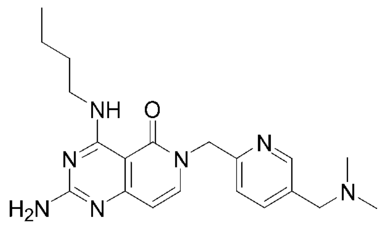

64 ,

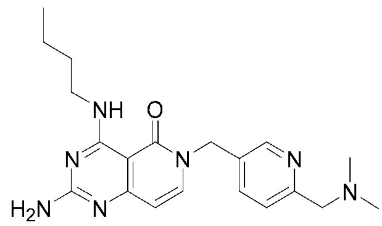

65 ,

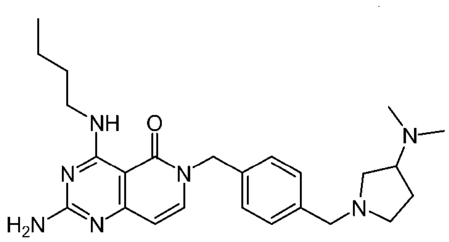

66 ,

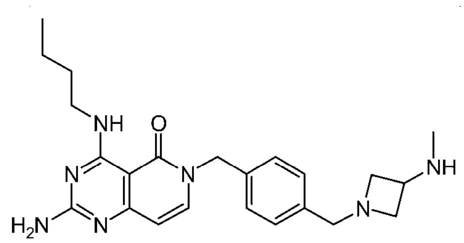

68 ,

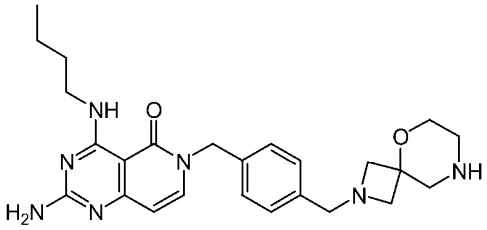

69 ,

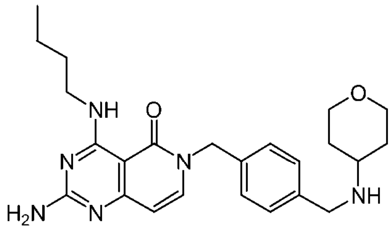

75 ,

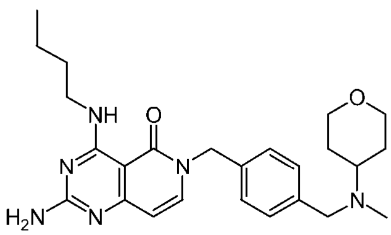

76 ,

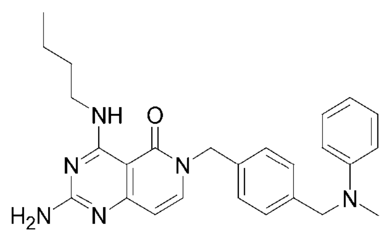

77 ,

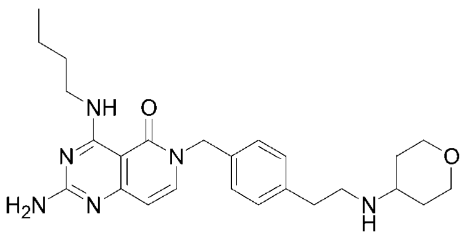

78 ,

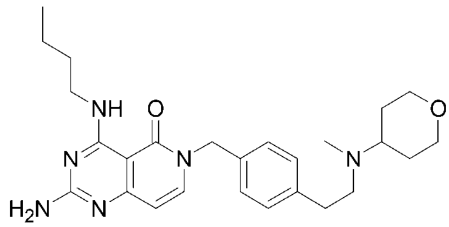

79 ,

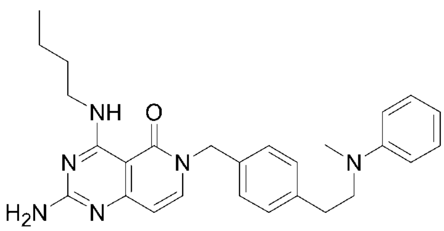

80 ,

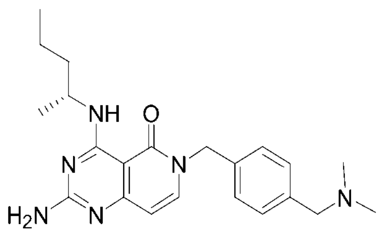

89 ,

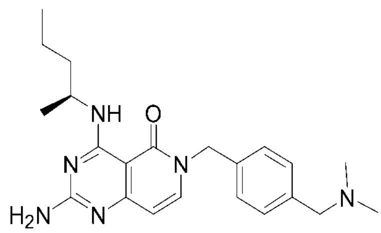

90 ,

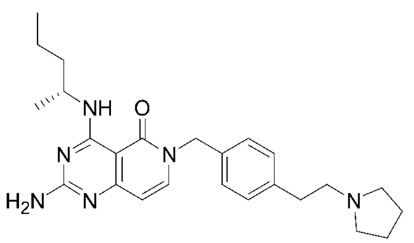

91 ,

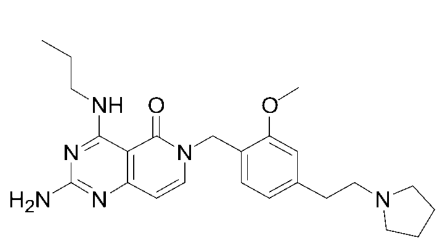

92 ,

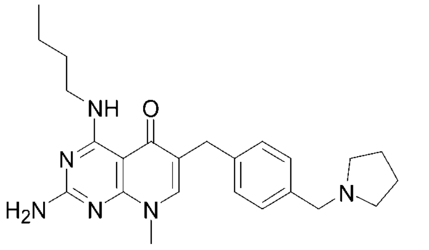

94 ,

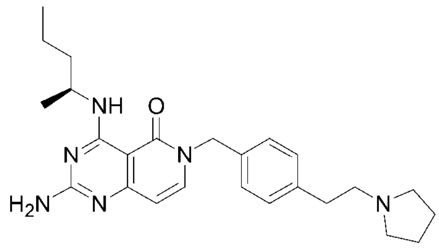

95 ,

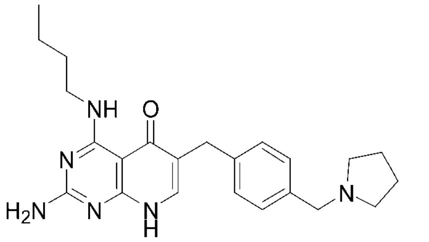

96 ,

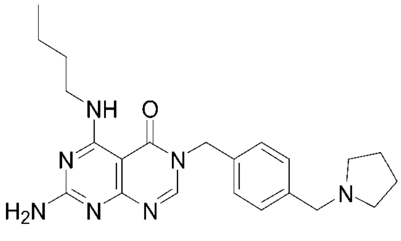

102 ,

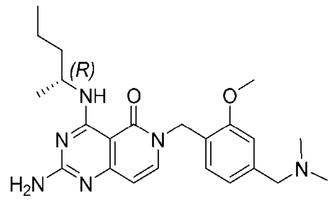

103 ,

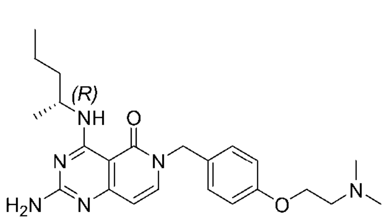

106 ,

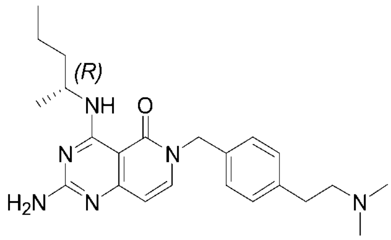

124 ,

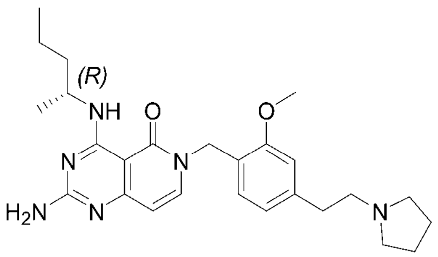

125 ,

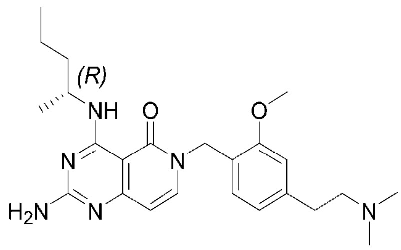

126 ,

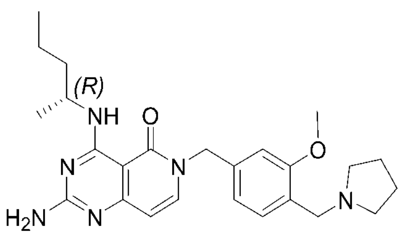

134 ,

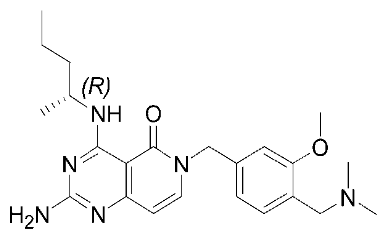

135 ,

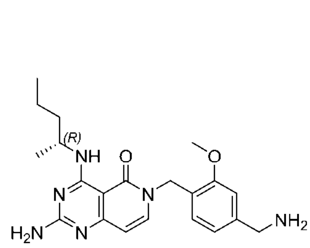

137 ,

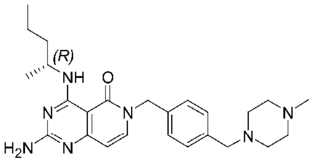
140 ,

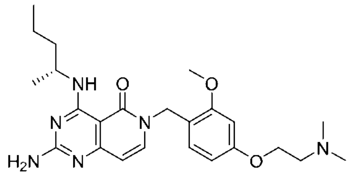
142 ,

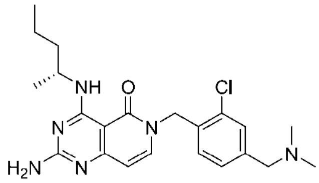
143 ,

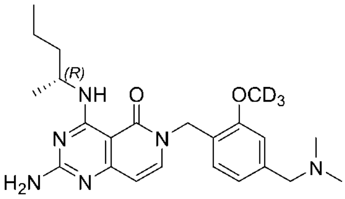
144 ,

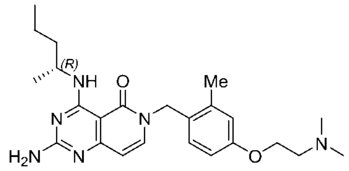
145 ,

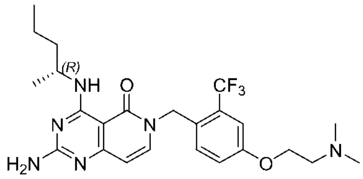
146 ,

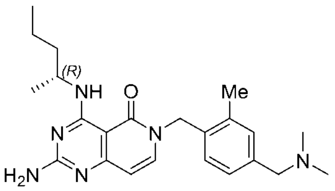
147 ,

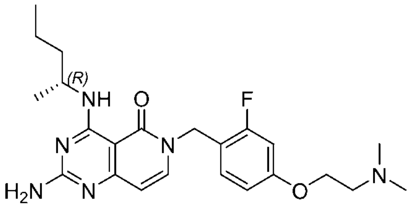
148 ,

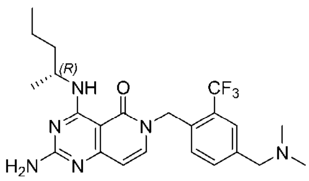
149 ,

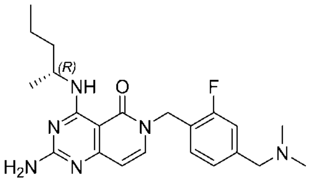
150 ,

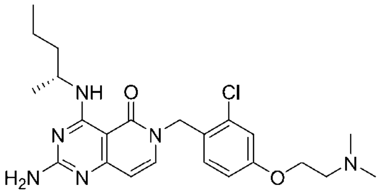
151 ,

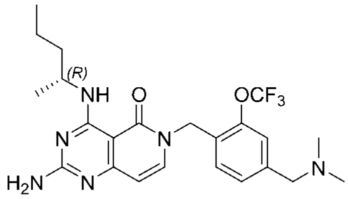
152 ,

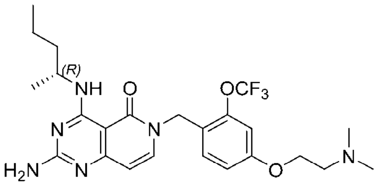
153 ,

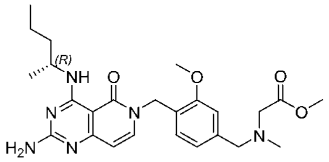
154 ,

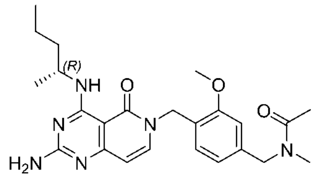
155 ,

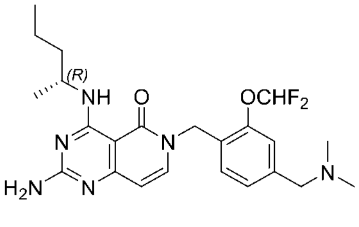
156 and

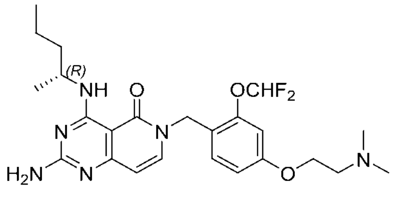
157 .

According to a second aspect, this application provides a pyrimidine derivative, which is a compound of Formula

(I-1), or a stereoisomer, a tautomer, an isotopic derivative, a halogenated derivative, a hydrate, a solvate, a prodrug, or a pharmaceutically acceptable salt thereof;

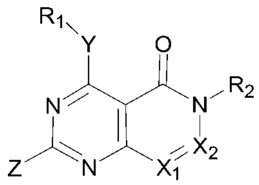

Formula (I-1)

where $X_1$ and $X_2$ are both selected from C-$R_3$;

Z is $NH_2$- or -OH;

Y is -O-, -S-, or -$NR_4$-, where $R_4$ is H or an optionally substituted alkyl group;

$R_1$ is an alkyl group optionally substituted with one or more substituents selected from oxo, halogen, an amino group, a hydroxyl group, a nitro group, a cyano group, a thiol group, an optionally substituted alkyl group, an optionally substituted alkyloxy group, an optionally substituted alkylthio group, an optionally substituted alkylamino group, an optionally substituted alkylacyl group, an optionally substituted alkylsulfinyl group, and an optionally substituted alkylsulfonyl group;

$R_2$ is

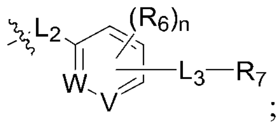

;

and -$L_3$-$R_7$ may be substituted at a para-, meta- or ortho-position of $L_2$;

$L_2$ and $L_3$ are each a bond or an optionally substituted linear alkylene group, where one or more carbon atoms in the linear alkylene group may be substituted with oxygen, sulfur and nitrogen heteroatoms, and the substituent is selected from one or more of an optionally substituted alkyl group, halogen, an amino group, a hydroxyl group, a nitro group, a cyano group, a thiol group, an optionally substituted alkyloxy group, an optionally substituted alkylthio group and an optionally substituted alkylamino group;

each of W and V may be N or CH; and W or V may be substituted with $R_6$ or -$L_3$-$R_7$ when being CH;

$R_3$ and $R_6$ are each independently H, an optionally substituted alkyl group, an optionally substituted alkyloxy group, an optionally substituted alkylthio group, an optionally substituted alkylamino group, halogen, an amino group, a hydroxyl group, a nitro group, a cyano group, and a thiol group;

$R_7$ is H, -OH, -N($R_9R_{10}$), -N($R_9$)$NH_2$, -NO($R_9R_{10}$), an optionally substituted heterocycloalkyl group, an optionally substituted cycloalkyl group, an optionally substituted aryl group, an optionally substituted heteroaryl group, optionally substituted heterocycloalkyl-$NR_9$-, optionally substituted cycloalkyl-$NR_9$-, optionally substituted aryl-$NR_9$-, optionally substituted heteroaryl-$NR_9$-, -OC(O)-$R_9$, -C(O)O-$R_9$, -N($R_9$)-C(O)-$R_{10}$ or -C(O)-N($R_9R_{10}$); and the substituent is selected from one or more of an optionally substituted alkyl group, an optionally substituted alkylamino group and oxo;

$R_9$ and $R_{10}$ are each independently H, an optionally substituted alkyl group, an optionally substituted cycloalkyl group, an optionally substituted heterocycloalkyl group, an optionally substituted aryl group, and an optionally substituted heteroaryl group; and

the substituent of the optionally substituted alkyl group is selected from one or more of halogen, an amino group, a hydroxyl group, a nitro group, a cyano group, a thiol group, a carboxyl group, an ester group and an amide group;

where n = 0, 1, 2, 3 or 4.

In a group of embodiments, $R_1$ is a C1-8 alkyl group optionally substituted with one or more substituents selected from an optionally substituted C1-8 alkylthio group and an optionally substituted C1-8 alkylsulfonyl group; or preferably, $R_1$ is a C2-6 alkyl group optionally substituted with a substituent selected from an optionally substituted C1-6 alkylthio group and an optionally substituted C1-6 alkylsulfonyl group.

Further, $R_1$ is an n-butyl group, an n-pentyl group, a 2-pentyl group, an n-hexyl group, a 2-hexyl group, a 3-hexyl group, a methoxyethyl group, a methylthioethyl group, a methylsulfonylethyl group, a methoxypropyl group, a methylthiopropyl group, a methylsulfonylpropyl group,

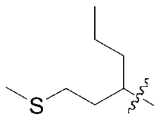 or 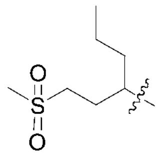 ;

or preferably, $R_1$ is an n-butyl group,

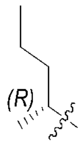, 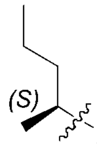,

a methylthiopropyl group, a methylsulfonylpropyl group,

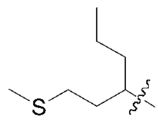

or

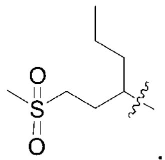.

In a group of embodiments, W and V are both N, W and V are both C, or one of W and V is N.

In a group of embodiments, the heterocycloalkyl group is a 3- to 12-membered monocyclic, bicyclic or polycyclic heterocycloalkyl group; preferably, the heterocycloalkyl group is a 3- to 12-membered monocyclic, bicyclic or polycyclic heterocycloalkyl group containing 1, 2, 3 or 4 heteroatoms selected from N, O, and S; more preferably, the heteroatom in the heterocycloalkyl group is linked to $L_1$ or $L_3$; and more preferably, N atom in the heterocycloalkyl group is linked to $L_1$ or $L_3$.

Further, the heterocycloalkyl group is a 3- to 7-membered monocyclic heterocycloalkyl group containing 1 or 2 heteroatoms selected from N, O, and S, or is a 7- to 12-membered fused-ring or spiro heterocycloalkyl group containing 1, 2, 3 or 4 heteroatoms selected from N, O, and S; and preferably, the heterocycloalkyl group is a 4- to 6-membered monocyclic heterocycloalkyl group containing 1 or 2 heteroatoms selected from N and O, or is a 7- to 11-membered spiro heterocycloalkyl group containing 2, 3 or 4 heteroatoms selected from N and O.

Further, the heterocycloalkyl group is one of the following groups:

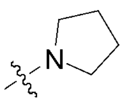, 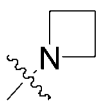, 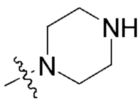, 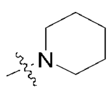, 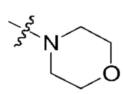, 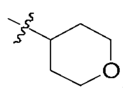, 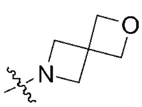 and 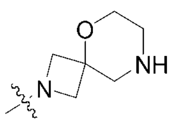.

In a group of embodiments, $L_2$ and $L_3$ are each a bond or an optionally substituted linear alkylene group containing 1 to 6 chain atoms, 1 or 2 carbon atoms in the linear alkylene group may be substituted with a heteroatom of oxygen, and the substituent is selected from the C1-8 alkyl group, halogen, an amino group, a hydroxyl group, a nitro group, a cyano group and a thiol group.

Further, $L_2$ is an optionally substituted linear alkylene group containing 1 to 4 chain atoms, preferably is an optionally substituted linear alkylene group containing 2 or 3 chain atoms, and more preferably, is $-CH_2-$, $-CH_2CH_2-$, -O-, $-O-CH_2-CH_2-$ and $-O-CH_2-$.

$L_3$ is a bond or an optionally substituted linear alkylene group containing 1 to 4 chain atoms, preferably is a bond or an optionally substituted linear alkylene group containing 2 or 3 chain atoms, more preferably is a bond, $-CH_2-$, $-CH_2CH_2-$, $-CH_2CH_2CH_2-$, $-O-CH_2-CH_2-$, $-O-CH_2-$ and $-CH(CH_3)CH_2-$, and more preferably, is

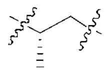 or 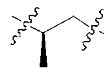.

In a group of embodiments, $R_7$ is -OH, $-N(R_9R_{10})$, $-N(R_9)NH_2$, $-NO(R_9R_{10})$, an optionally substituted 3- to 12-membered heterocycloalkyl group containing 1, 2, 3 or 4 heteroatoms selected from N, O, and S, an optionally substituted C6-10 aryl group, optionally substituted 3- to 12-membered heterocycloalkyl-$NR_9$- containing 1, 2, 3 or 4 heteroatoms selected from N, O, and S, or optionally substituted C6-10 aryl-$NR_9$-; the substituent is selected from one or more of an optionally substituted C1-8 alkyl group, an optionally substituted C1-8 alkylamino group, an optionally substituted di(C1-8) alkylamino group and oxo; and $R_9$ and $R_{10}$ are each independently H or a C1-8 alkyl group; and preferably, the heterocycloalkyl group is a 4- to 6-membered monocyclic heterocycloalkyl group containing 1 or 2 heteroatoms selected from N and O, or is a 7- to 11-membered spiro heterocycloalkyl group containing 2, 3 or 4 heteroatoms selected from N and O; more preferably, the heteroatom in the heterocycloalkyl group is linked to $L_3$; and more preferably, the N atom in the heterocycloalkyl group is linked to $L_3$.

Further, $R_7$ is $-NO(R_9R_{10})$ or an optionally oxo-substituted 3- to 12-membered heterocycloalkyl group containing 1, 2, 3 or 4 heteroatoms selected from N, O, and S.

In a group of embodiments, $R_2$ is selected from one of the following optionally substituted groups:

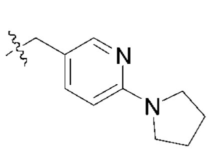, 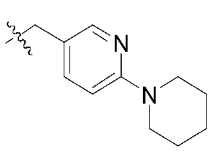, 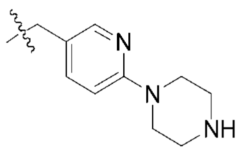, 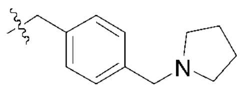,

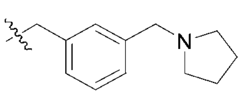, 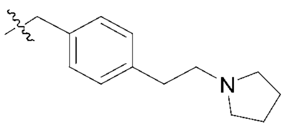, 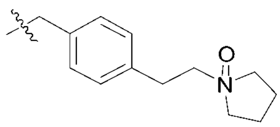, 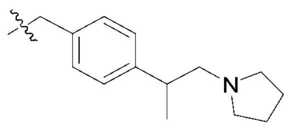,

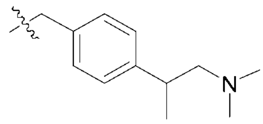, 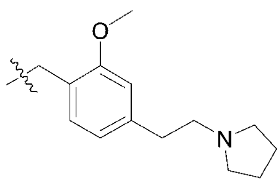, 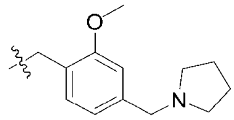, 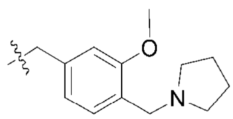,

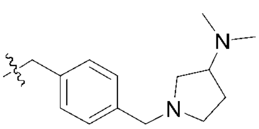, 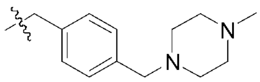, 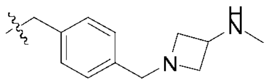, 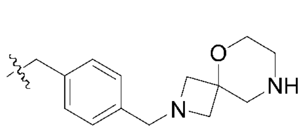,

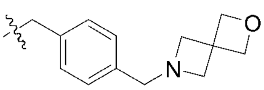, 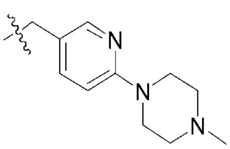, 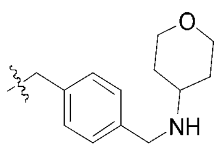, 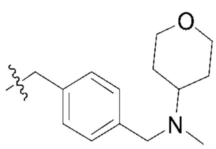, 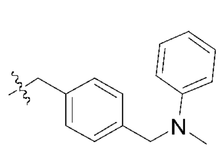,

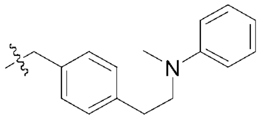, 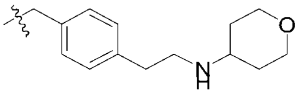, 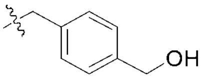, 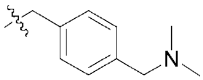,

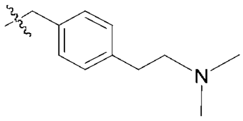, 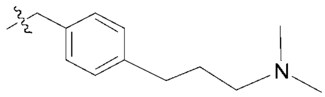, 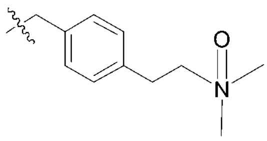, 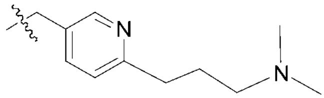,

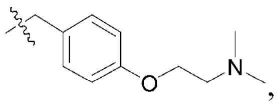, 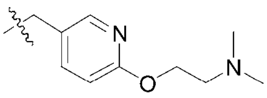, 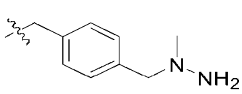, 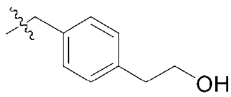, 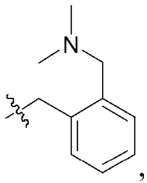,

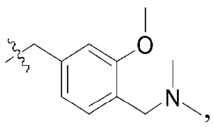, 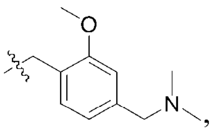, 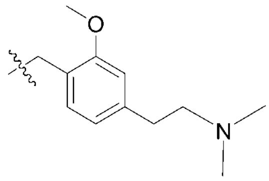, 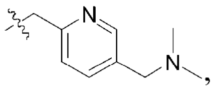, 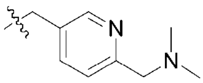,

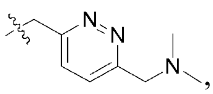, 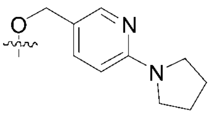, 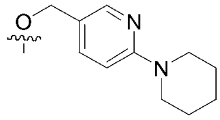, 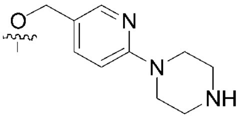, 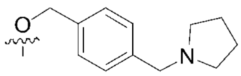,

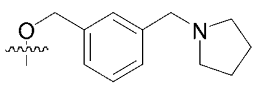, 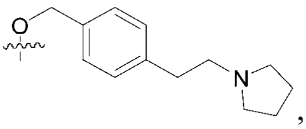, 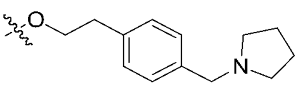, 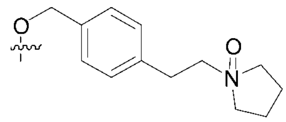,

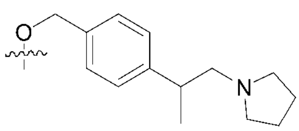, 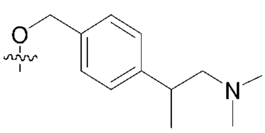, 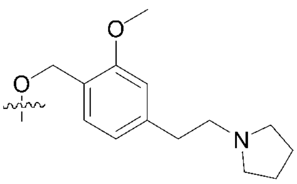, 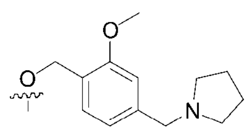,

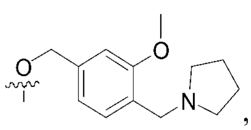, 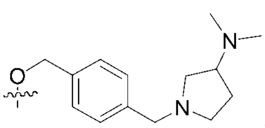, 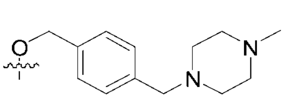, 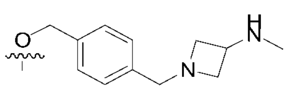,

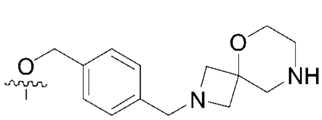, 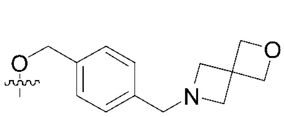, 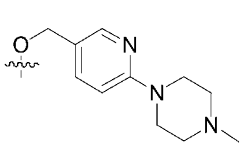, 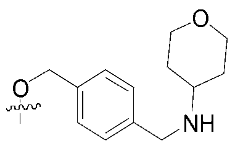,

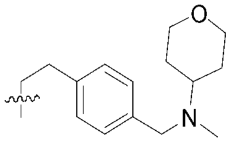, 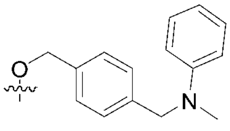, 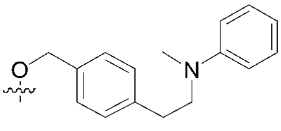, 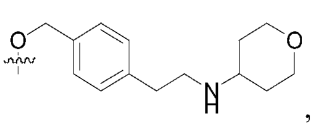,

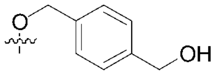, 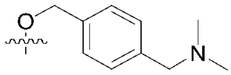, 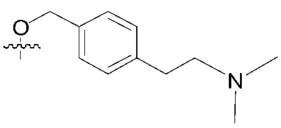, 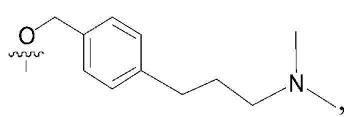,

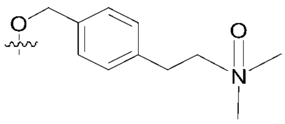, 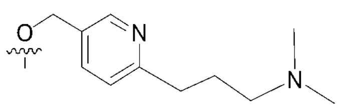, 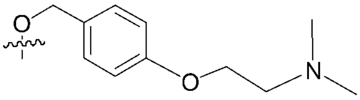,

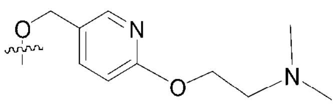, 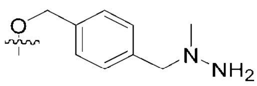, 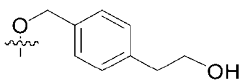, 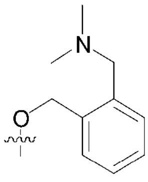,

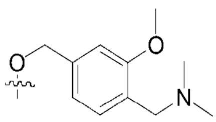, 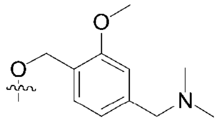, 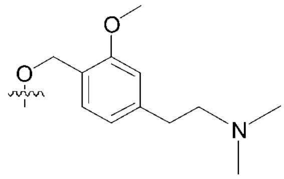, 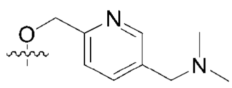,

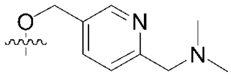, 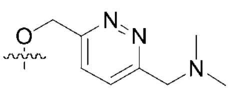, 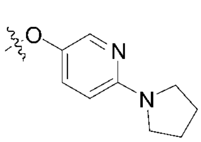, 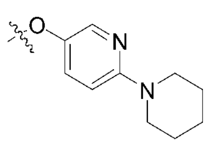, 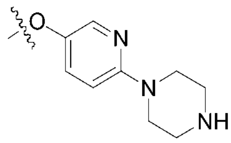,

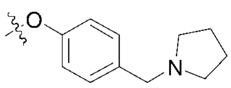, 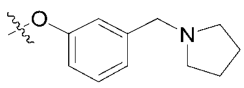, 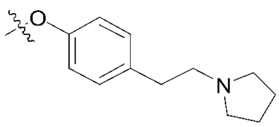, 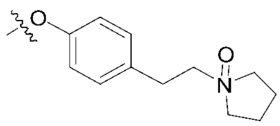,

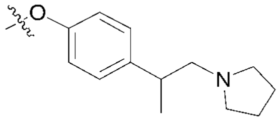, 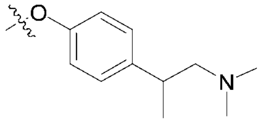, 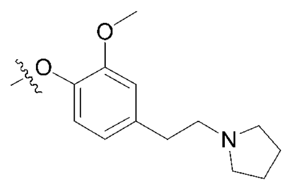, 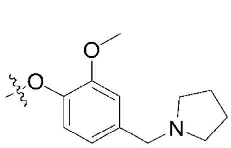,

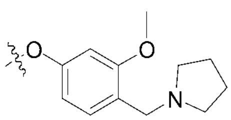, 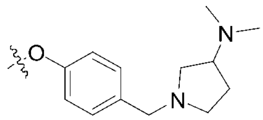, 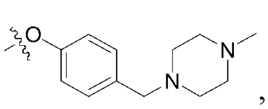, 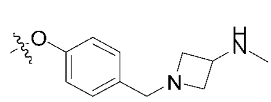,

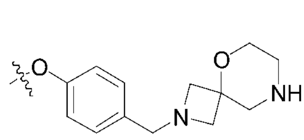, 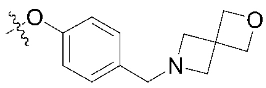, 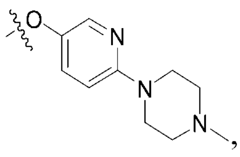, 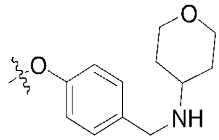,

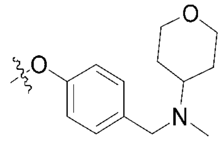, 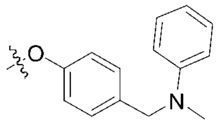, 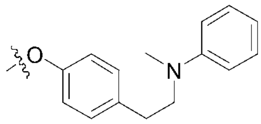, 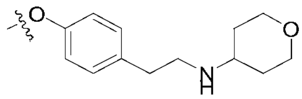,

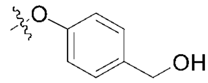, 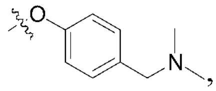, 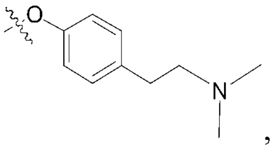, 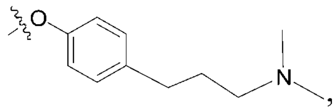,

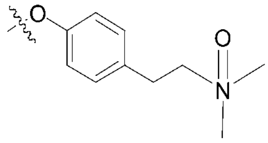, 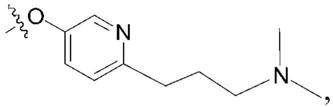, 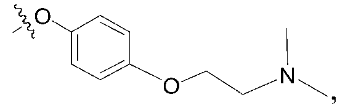,

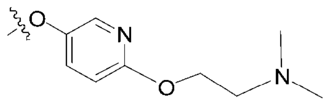, 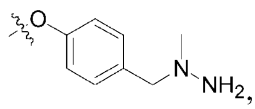, 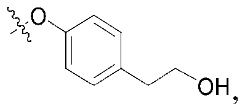, 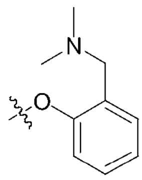, 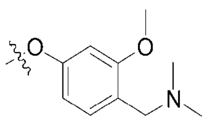,

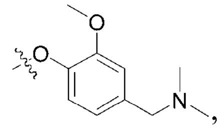, 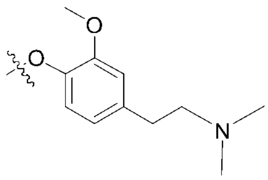, 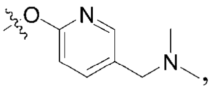, 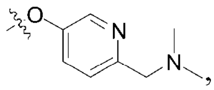, 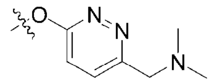,

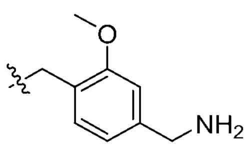, 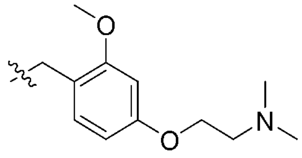, 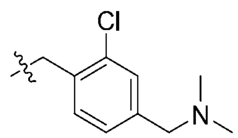, 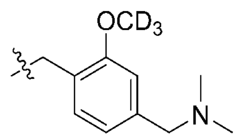,

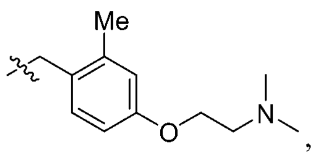, 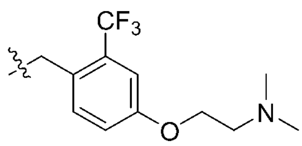, 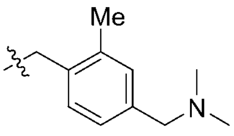, 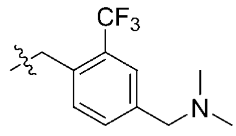,

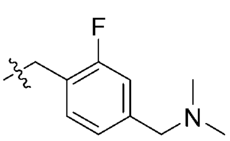, 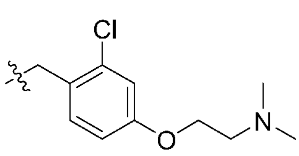, 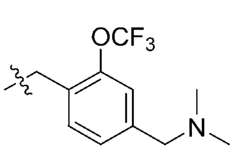, 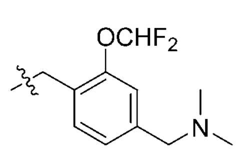,

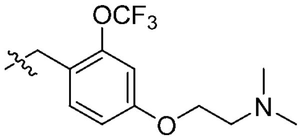, 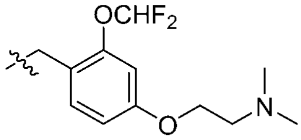, 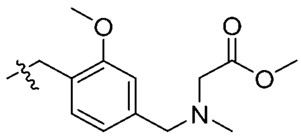, 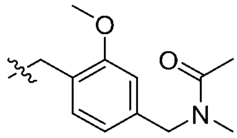

and

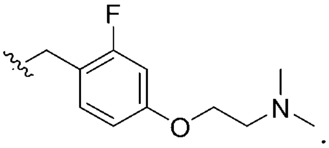.

Further, $R_2$ is optionally substituted

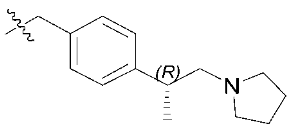, 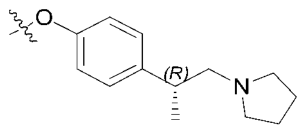,

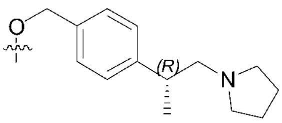, 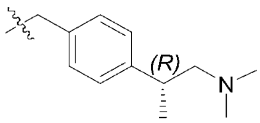, 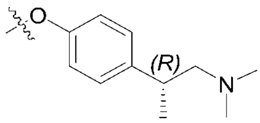, 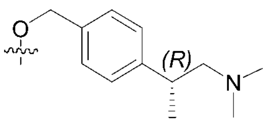,

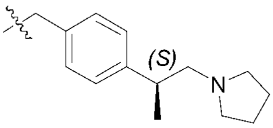, 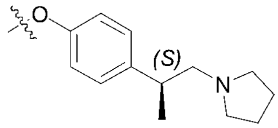, 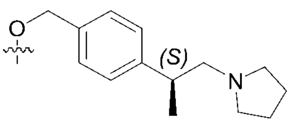, 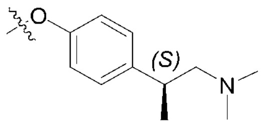,

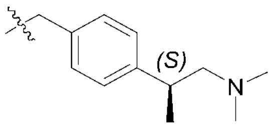 and 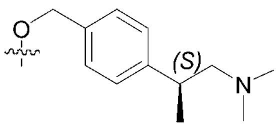;

and

the substituent is selected from one or more of an alkyl group, an alkyloxy group, an alkylthio group, an alkylamino group, halogen, an amino group, a hydroxyl group, a nitro group, a cyano group, a sulfhydryl group, a carboxyl group, an ester group, an amide group and the like.

In a group of preferred embodiments, the compound of Formula (I-1) specifically has one of the following formulas:

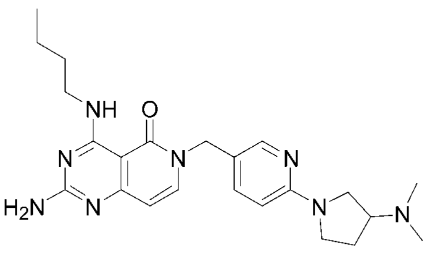

71 ,

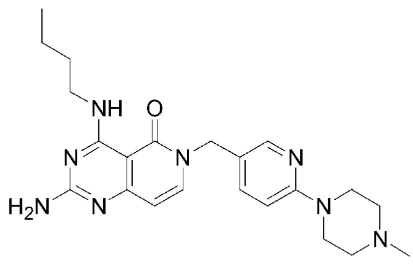

72 ,

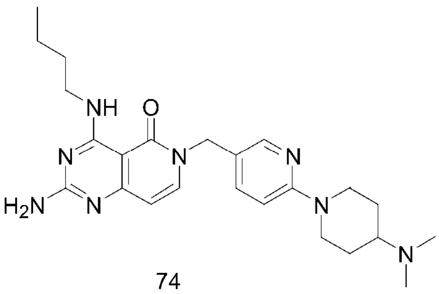

74 ,

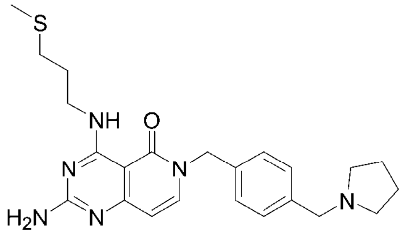

85 ,

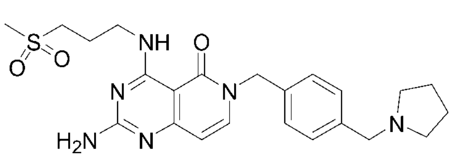

86 ,

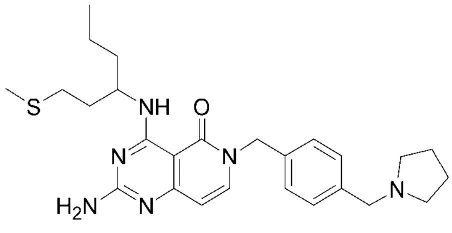

87 ,

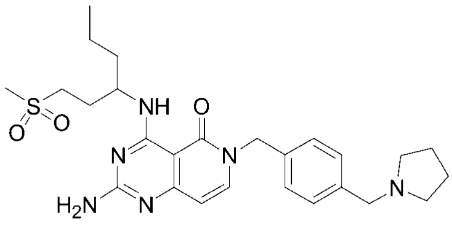

88 ,

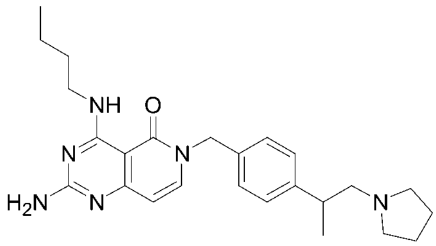

81 ,

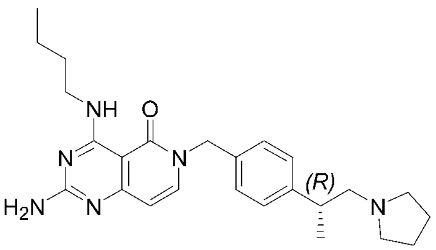

127 ,

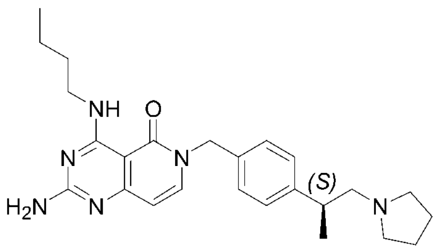

128 ,

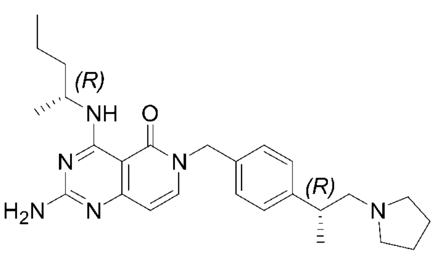

129 ,

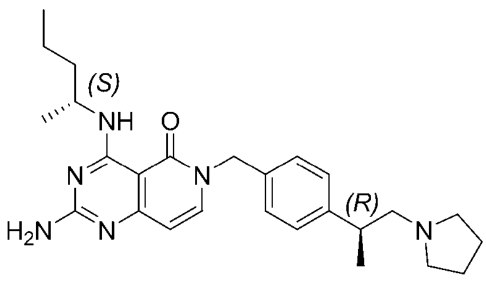

130 ,

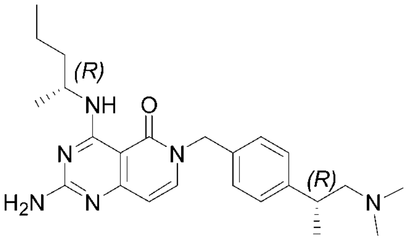

132 ,

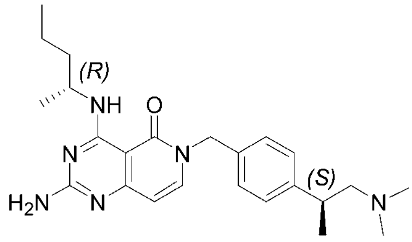

133 ,

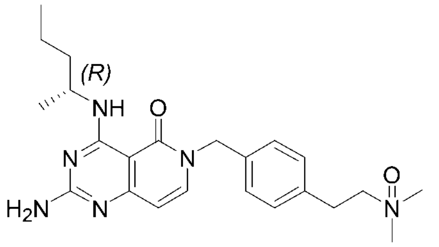

138 and

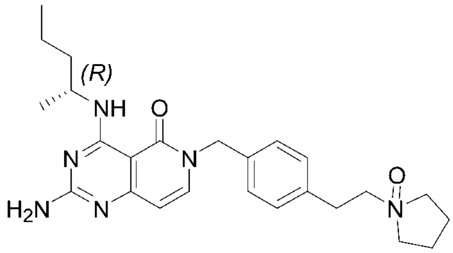

139 .

According to a third aspect, this application provides a pyrimidine derivative, which is a compound of Formula (II-1) or (II-2), or a stereoisomer, a tautomer, an isotopic derivative, a halogenated derivative, a hydrate, a solvate, a prodrug, or a pharmaceutically acceptable salt thereof;

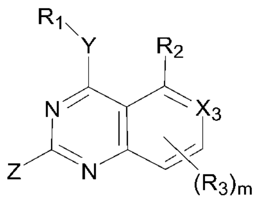

Formula (II-1)

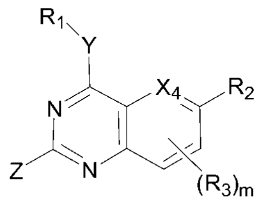

Formula (II-2)

where:

$X_3$ is N or CH, $X_4$ is CH, N or NO, and $X_3$ or $X_4$ can be substituted with $R_3$ when being CH;

Z is $-NH_2$, -OH, -NH-alkyl, -O-alkyl, -NH-C(O)-alkyl, -O-C(O)-alkyl, -NH-C(O)-OH, or -O-C(O)-OH;

Y is -O-, -S-, or $-NR_4-$, where $R_4$ is H or an optionally substituted alkyl group;

$R_1$ is an alkyl group or an aryl group optionally substituted with one or more substituents selected from oxo, halogen, an amino group, a hydroxyl group, a nitro group, a cyano group, a thiol group, $-C(O)-NH_2$, -C(O)-OH, a heteroaryl group (optionally substituted with one or more of halogen, an amino group, a hydroxyl group, a nitro group, a cyano group, a thiol group, an alkoxyl group, and an alkyl group), an aryl group (optionally substituted with one or more of halogen, an amino group, a hydroxyl group, a nitro group, a cyano group, a thiol group, an alkoxyl group, and an alkyl group), a heterocycloalkyl group (optionally substituted with one or more of halogen, an amino group, a hydroxyl group, a nitro group, a cyano group, a thiol group, an alkoxyl group, and an alkyl group), a cycloalkyl group (optionally substituted with one or more of halogen, an amino group, a hydroxyl group, a nitro group, a cyano group, a thiol group, an alkoxyl group, and an alkyl group), an optionally substituted alkyl group, an optionally substituted alkyloxy group, an optionally substituted alkylthio group, an optionally substituted alkylamino group, an optionally substituted alkylacyl group, an optionally substituted alkylsulfinyl group, an optionally substituted alkylsulfonyl group, an optionally substituted -C(O)-O-alkyl, optionally substituted -O-C(O)-alkyl, optionally substituted -C(O)-NH-alkyl, optionally substituted -NH-C(O)-alkyl, optionally substituted -S(O)-O-alkyl, optionally substituted -O-S(O)-alkyl, optionally substituted $-S(O)_2-O-$alkyl, optionally substituted $-O-S(O)_2-$alkyl, optionally substituted -S(O)-NH-alkyl, optionally substituted -NH-S(O)-alkyl, optionally substituted $-S(O)_2-NH-$alkyl, and optionally substituted $-NH-S(O)_2-$alkyl;

$R_2$ is

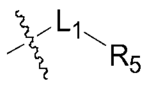 or 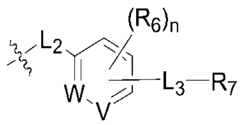 ;

and $-L_3-R_7$ may be substituted at a para-, meta- or ortho-position of $L_2$;

$L_1$, $L_2$ and $L_3$ are each a bond or an optionally substituted linear alkylene group, where one or more carbon atoms in the linear alkylene group may be substituted with oxygen, sulfur and nitrogen heteroatoms, and the substituent is selected from one or more of an optionally substituted alkyl group, halogen, an amino group, a hydroxyl group, a nitro group, a cyano group, a thiol group, an optionally substituted alkyloxy group, an optionally substituted alkylthio group and an optionally substituted alkylamino group;

$R_5$ is a cycloalkyl group or a heterocycloalkyl group optionally substituted with one or more substituents selected from oxo, halogen, an amino group, a hydroxyl group, a nitro group, a cyano group, a thiol group, an optionally substituted alkyl group, an optionally substituted alkyloxy group, an optionally substituted alkylthio group, and an optionally substituted alkylamino group;

each of W and V may be N or CH; and W or V may be substituted with $R_6$ or $-L_3-R_7$ when being CH;

$R_3$ and $R_6$ are independently H, an optionally substituted alkyl group, an optionally substituted alkyloxy group, an optionally substituted alkylthio group, an optionally substituted alkylamino group, halogen, an amino group, a hydroxyl group, a nitro group, a cyano group, and a thiol group;

$R_7$ is H, -OH, $-N(R_9R_{10})$, $-N(R_9)NH_2$, $-NO(R_9R_{10})$, an optionally substituted heterocycloalkyl group, an optionally substituted cycloalkyl group, an optionally substituted aryl group, an optionally substituted heteroaryl group, optionally substituted heterocycloalkyl-$NR_9$-, optionally substituted cycloalkyl-$NR_9$-, optionally substituted aryl-$NR_9$-, optionally substituted heteroaryl-$NR_9$-, $-OC(O)-R_9$, $-C(O)O-R_9$, $-N(R_9)-C(O)-R_{10}$ or $-C(O)-N(R_9R_{10})$; and the substituent is selected from one or more of an optionally substituted alkyl group, an optionally substituted alkylamino group and oxo;

$R_9$ and $R_{10}$ are each independently H, an optionally substituted alkyl group, an optionally substituted cycloalkyl group, an optionally substituted heterocycloalkyl group, an optionally substituted aryl group, and an optionally substituted heteroaryl group; and

the substituent of the optionally substituted alkyl group is selected from one or more of halogen, an amino group, a hydroxyl group, a nitro group, a cyano group, a thiol group, a carboxyl group, an ester group and an amide group;

where m = 0, 1, 2 or 3; and n = 0, 1, 2, 3 or 4.

In a group of embodiments, $R_1$ is a C1-8 alkyl group optionally substituted with one or more substituents selected from an optionally substituted C1-8 alkyloxy group, an optionally substituted C1-8 alkylthio group, an optionally substituted C1-8 alkylamino group, an optionally substituted C1-8 alkylacyl group, an optionally substituted C1-8 alkylsulfinyl group, and an optionally substituted C1-8 alkylsulfonyl group; preferably, R1 is a C2-6 alkyl group optionally substituted with an optionally substituted C1-6 alkyloxy group, an optionally substituted C1-6 alkylthio group, and an optionally substituted C1-6 alkylsulfonyl group.

Further, $R_1$ is an optionally substituted methyl group, ethyl group, n-propyl group, isopropyl group, n-butyl group, isobutyl group, tert-butyl group, n-pentyl group, 2-pentyl group, n-hexyl group, 2-hexyl group or 3-hexyl group;

Preferably, $R_1$ is an n-butyl group, an isobutyl group, a tert-butyl group, an n-pentyl group, a 2-pentyl group, a 3-hexyl group, a methoxyethyl group, a methylthioethyl group, a methylsulfonylethyl group, a methoxypropyl group, a methylthiopropyl group, a methylsulfonylpropyl group,

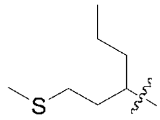 or 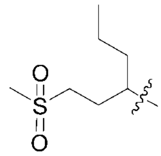 ;

or

more preferably, $R_1$ is an n-butyl group,

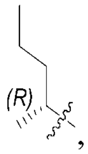, 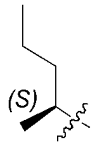,

a methylthiopropyl group, a methylsulfonylpropyl group,

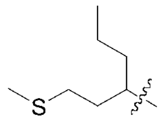 or 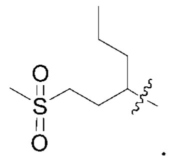 .

In a group of embodiments, W and V are both N, W and V are both C, or one of W and V is N.

In a group of embodiments, the heterocycloalkyl group is a 3- to 12-membered monocyclic, bicyclic or polycyclic heterocycloalkyl group; preferably, the heterocycloalkyl group is a 3- to 12-membered monocyclic, bicyclic or polycyclic heterocycloalkyl group containing 1, 2, 3 or 4 heteroatoms selected from N, O, and S; more preferably, the heteroatom in the heterocycloalkyl group is linked to $L_1$ or $L_3$; and more preferably, N atom in the heterocycloalkyl group is linked to $L_1$ or $L_3$.

Further, the heterocycloalkyl group is a 3- to 7-membered monocyclic heterocycloalkyl group containing 1 or 2 heteroatoms selected from N, O, and S, or is a 7- to 12-membered fused-ring or spiro heterocycloalkyl group containing 1, 2, 3 or 4 heteroatoms selected from N, O, and S; and preferably, the heterocycloalkyl group is a 4- to 6-membered monocyclic heterocycloalkyl group containing 1 or 2 heteroatoms selected from N and O, or is a 7- to 11-membered spiro heterocycloalkyl group containing 2, 3 or 4 heteroatoms selected from N and O.

Further, the heterocycloalkyl group may be one of the following groups:

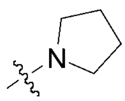, 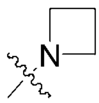, 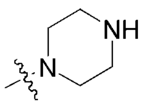, 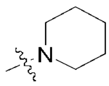, 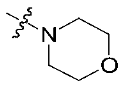, 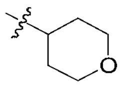, 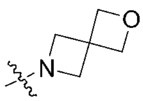 and 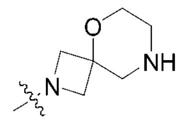 .

In a group of embodiments, the aryl group is a C6-10 aryl group, preferably a phenyl group.

In a group of embodiments, $L_1$, $L_2$ and $L_3$ are each a bond or an optionally substituted linear alkylene group containing 1 to 6 chain atoms, 1 or 2 carbon atoms in the alkylene group may be substituted with oxygen and sulfur heteroatoms, and the substituent is selected from the C1-8 alkyl group, halogen, an amino group, a hydroxyl group, a nitro group, a cyano group and a thiol group.

Further, $L_1$ is an optionally substituted linear alkylene group containing 1 to 4 chain atoms, more preferably is an optionally substituted linear alkylene group containing 2 or 3 chain atoms, and more preferably, is -O-propylene or $-CH_2CH_2CH_2-$.

$L_2$ is an optionally substituted linear alkylene group containing 1 to 4 chain atoms, preferably is an optionally substituted linear alkylene group containing 2 or 3 chain atoms, and more preferably, is $-CH_2-$, $-CH_2CH_2-$, -O-, $-O-CH_2-CH_2-$ and $-O-CH_2-$; and

$L_3$ is a bond or an optionally substituted linear alkylene group containing 1 to 4 chain atoms, preferably is a bond or an optionally substituted linear alkylene group containing 2 or 3 chain atoms, more preferably is $-CH_2-$, $-CH_2CH_2-$, $-CH_2CH_2CH_2-$, $-O-CH_2-CH_2-$, $-O-CH_2-$ and $-CH(CH_3)CH_2-$, and more preferably, is

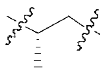 or 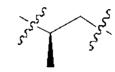 .

In a group of embodiments, $R_7$ is -OH, $-N(R_9R_{10})$, $-N(R_9)NH_2$, $-NO(R_9R_{10})$, an optionally substituted 3- to 12-membered heterocycloalkyl group containing 1, 2, 3 or 4 heteroatoms selected from N, O, and S, an optionally substituted C6-10 aryl group, optionally substituted 3- to 12-membered heterocycloalkyl-$NR_9$- containing 1, 2, 3 or 4 heteroatoms selected from N, O, and S, or optionally substituted C6-10 aryl-$NR_9$-; the substituent is selected from one or more of an optionally substituted C1-8 alkyl group, an optionally substituted C1-8 alkylamino group, an optionally substituted di(Cl-8) alkylamino group and oxo; and $R_9$ and $R_{10}$ are each independently H or a C1-8 alkyl group. Preferably, the heterocycloalkyl group is a 4- to 6-membered monocyclic heterocycloalkyl group containing 1 or 2 heteroatoms selected from N and O, or is a 7- to 11-membered spiro heterocycloalkyl group containing 2, 3 or 4 heteroatoms selected from N and O; more preferably, the heteroatom in the heterocycloalkyl group is linked to $L_3$; and more preferably, the N atom in the heterocycloalkyl group is linked to $L_3$.

In a group of embodiments, $R_2$ is selected from one of the following optionally substituted groups:

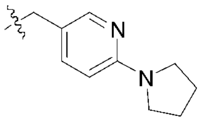, 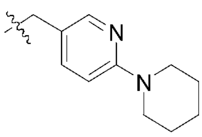, 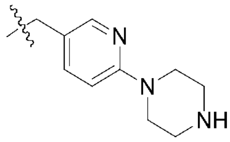, 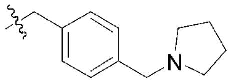, 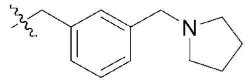,

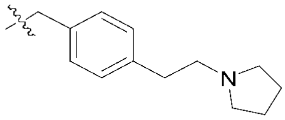, 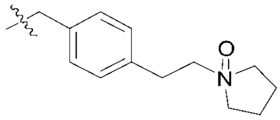, 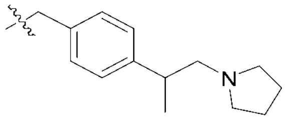, 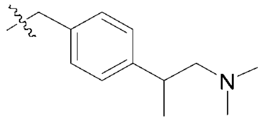,

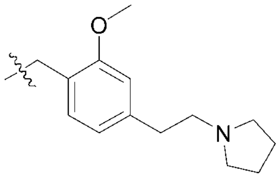, 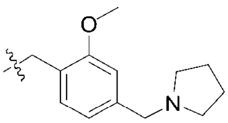, 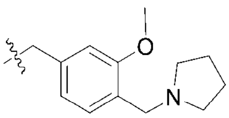, 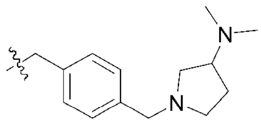,

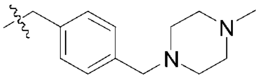, 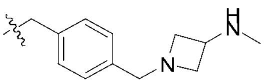, 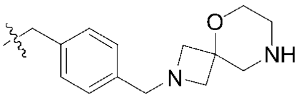, 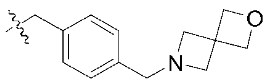,

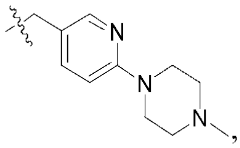, 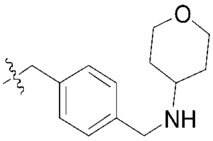, 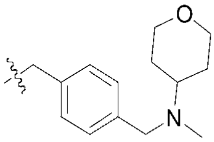, 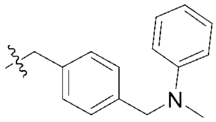, 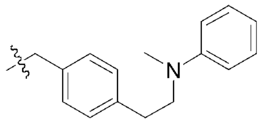,

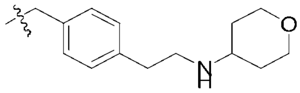, 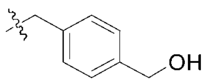, 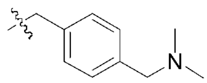, 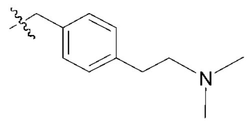,

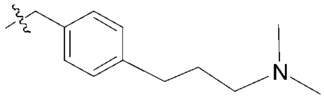, 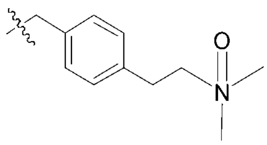, 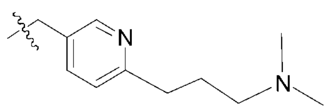, 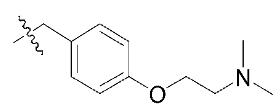,

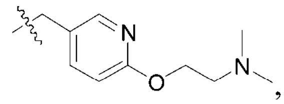, 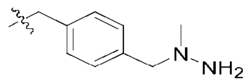, 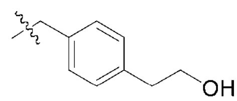, 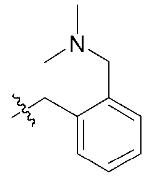, 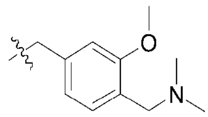,

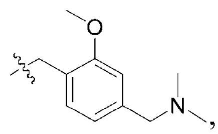, 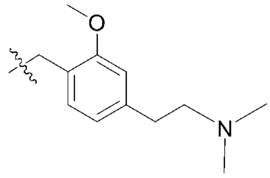, 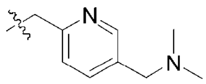, 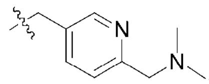, 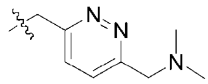,

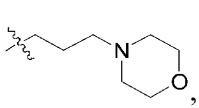, 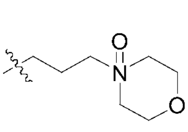, 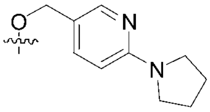, 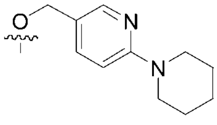, 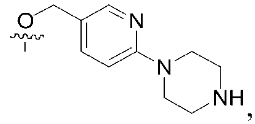,

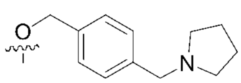, 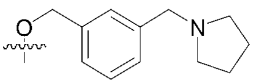, 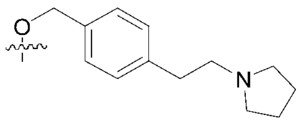, 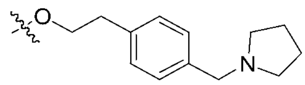,

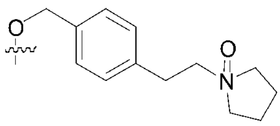, 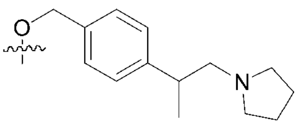, 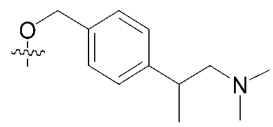, 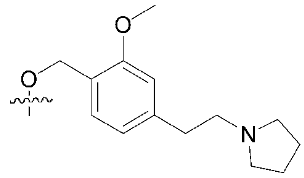,

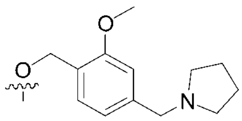, 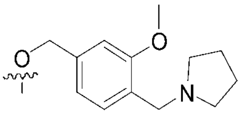, 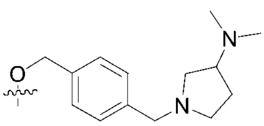, 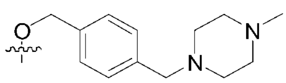,

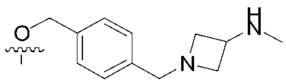, 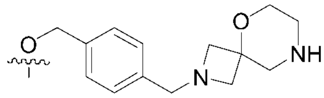, 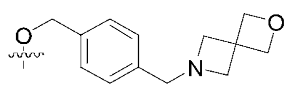, 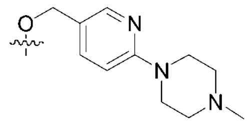,

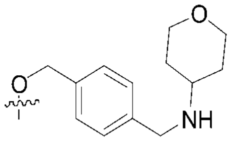, 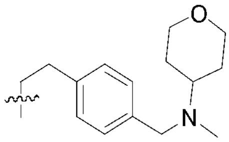, 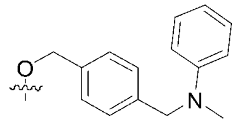, 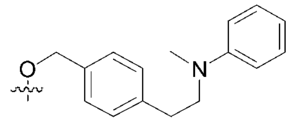,

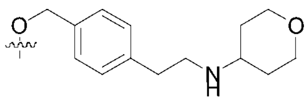, 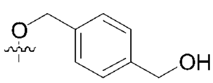, 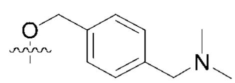, 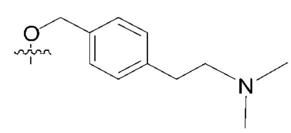,

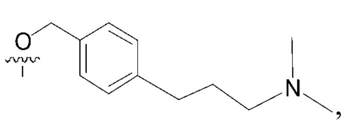, 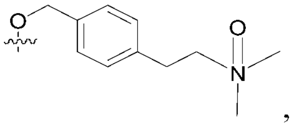, 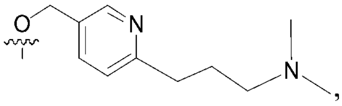,

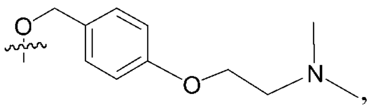, 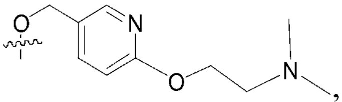, 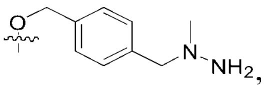,

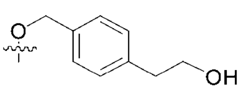, 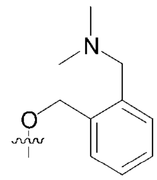, 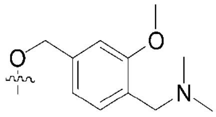, 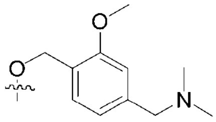, 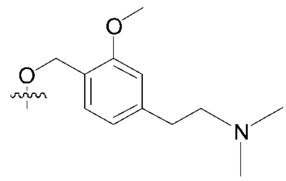,

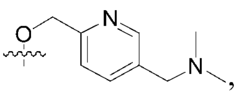, 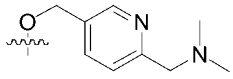, 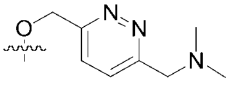, 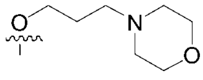, 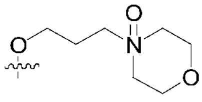,

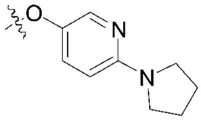, 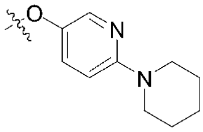, 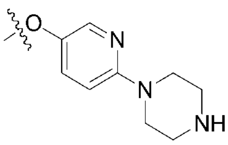, 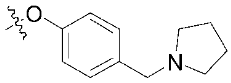, 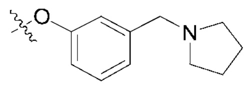,

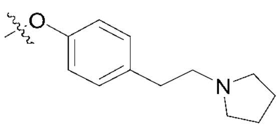, 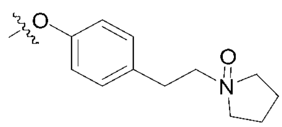, 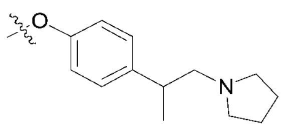, 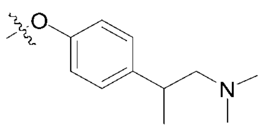,

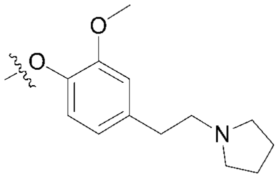, 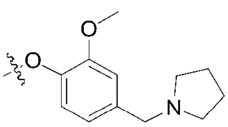, 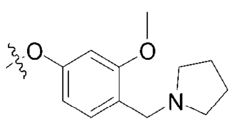, 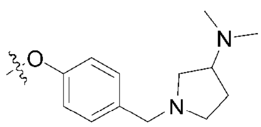,

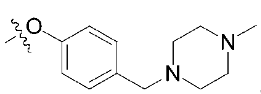, 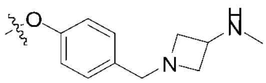, 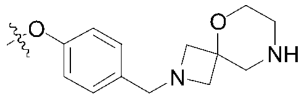, 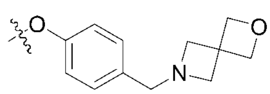,

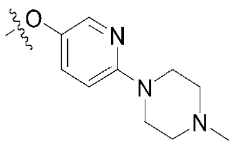, 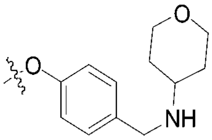, 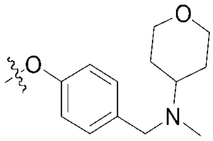, 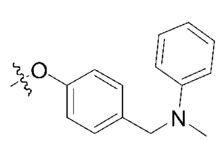, 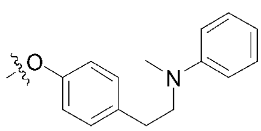,

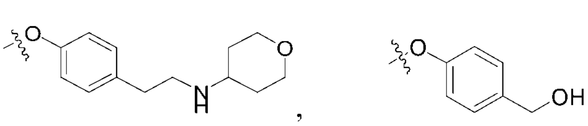, 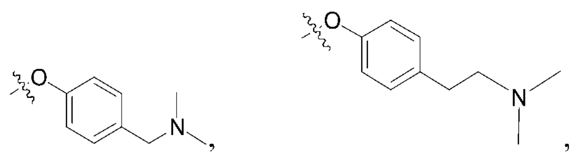,

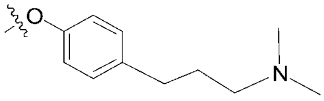, 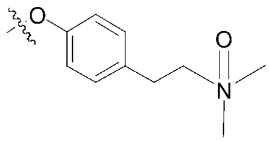, 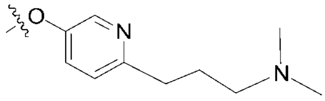,

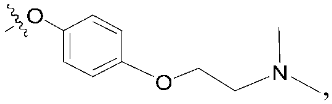, 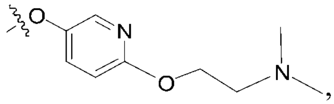, 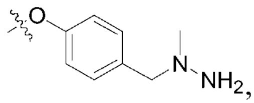, 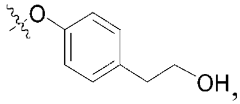,

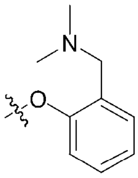, 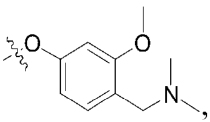, 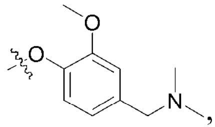, 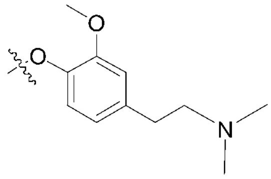, 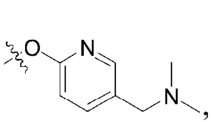,

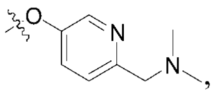, 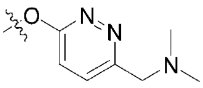, 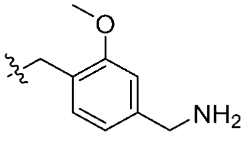, 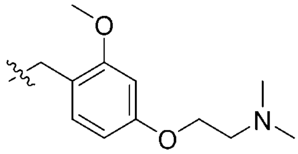,

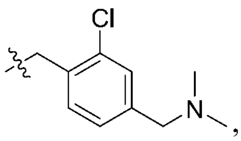, 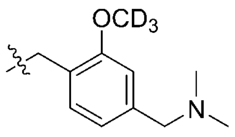, 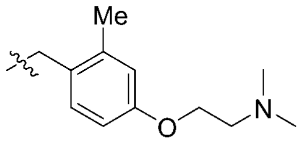, 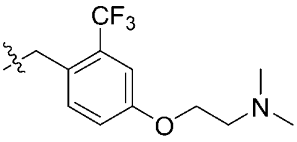,

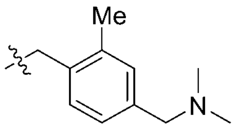, 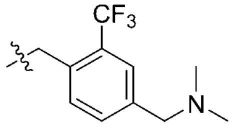, 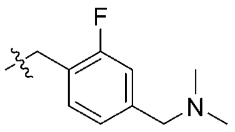, 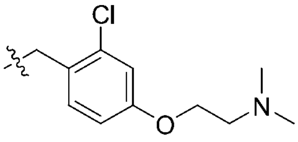,

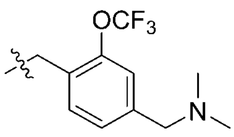, 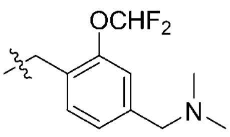, 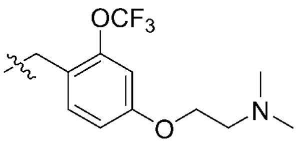, 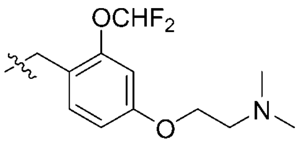,

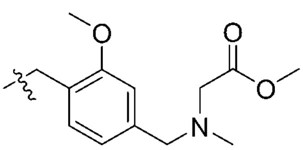, 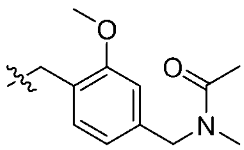 and 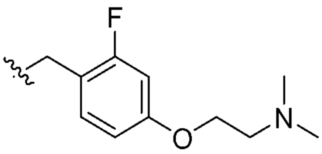.

Further, $R_2$ is optionally substituted

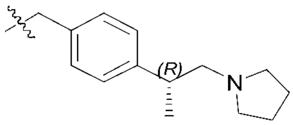, 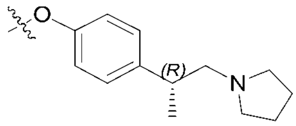,

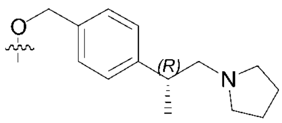, 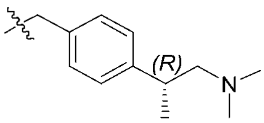, 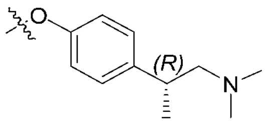, 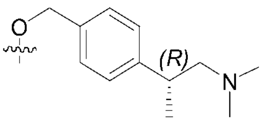,

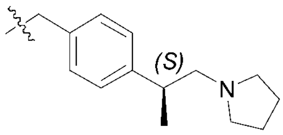, 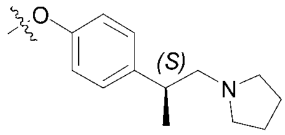, 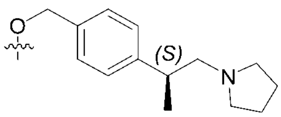, 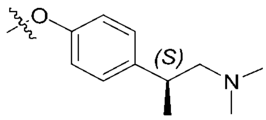,

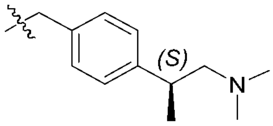 and 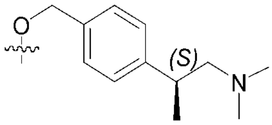 ; and

the substituent is selected from one or more of an alkyl group, an alkyloxy group, an alkylthio group, an alkylamino group, halogen, an amino group, a hydroxyl group, a nitro group, a cyano group, a sulfhydryl group, a carboxyl group, an ester group, an amide group and the like.

In a group of preferred embodiments, the compound of Formula (II-1) or (II-2) may specifically have the following formulas:

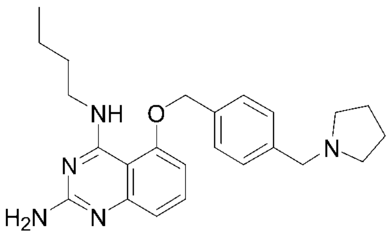

12 ,

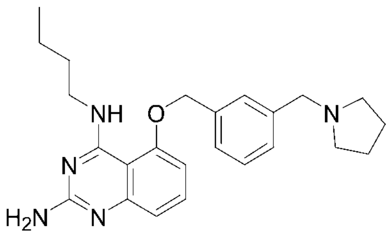

13 ,

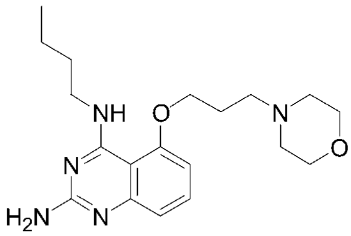

14 ,

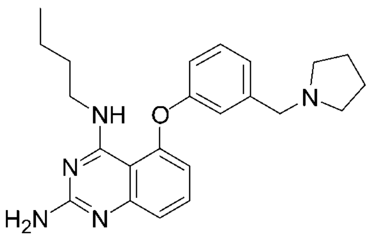

16 ,

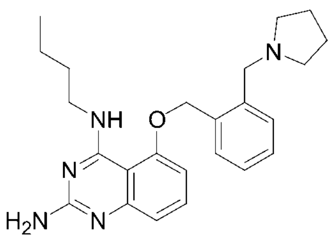

44 ,

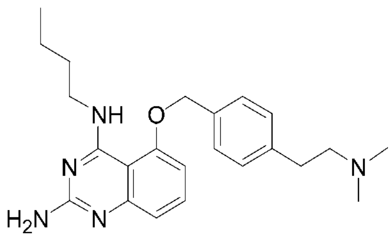

46 ,

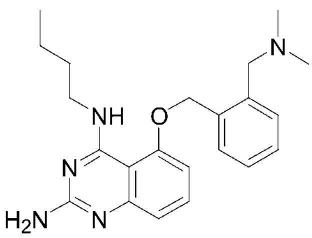

47 ,

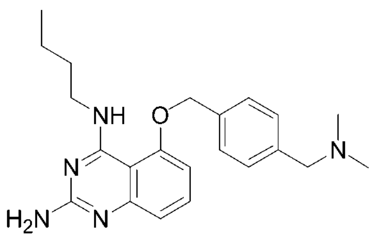

48 ,

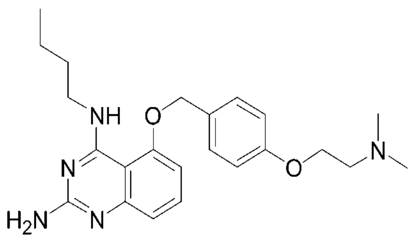

49 ,

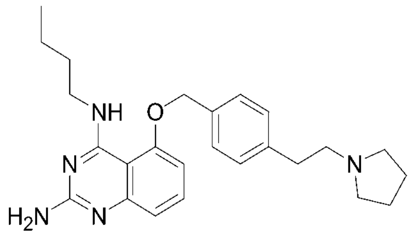

50 ,

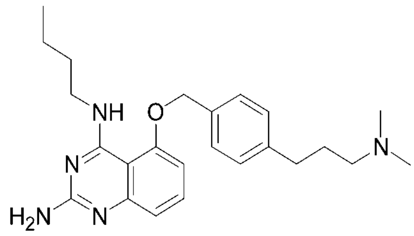

59 ,

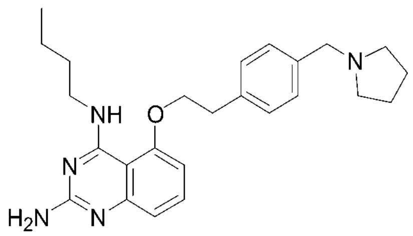

82 ,

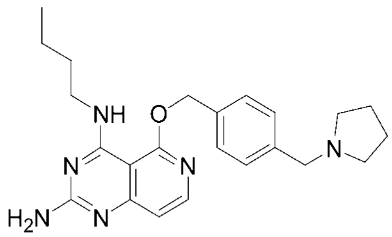

20 ,

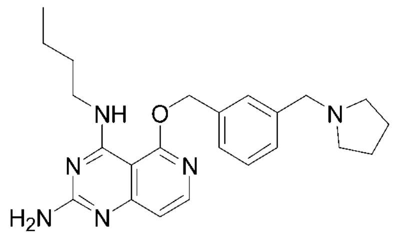

21 ,

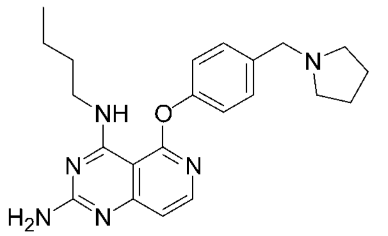

23 ,

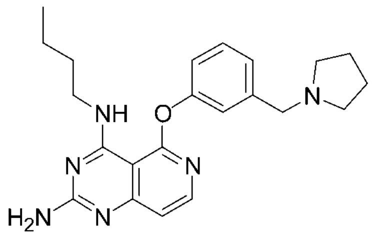

24 ,

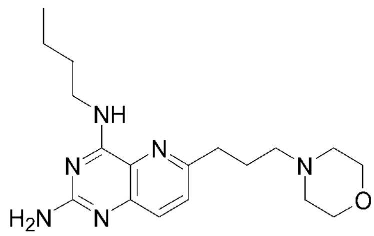

4 ,

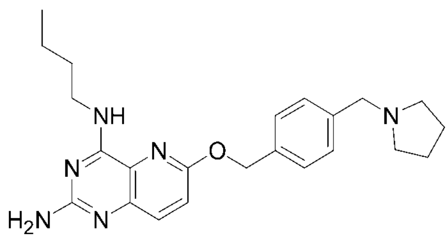

5 ,

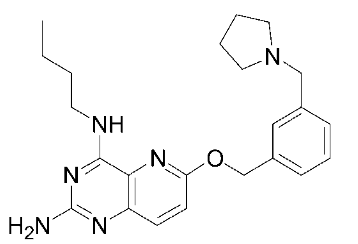

6 ,

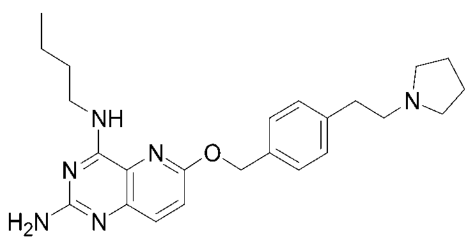

7 ,

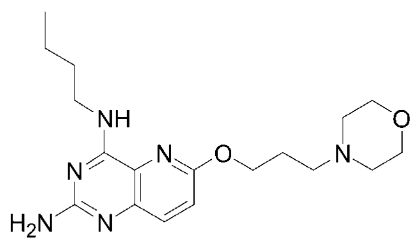

8 ,

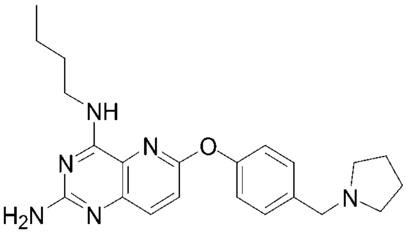

9 ,

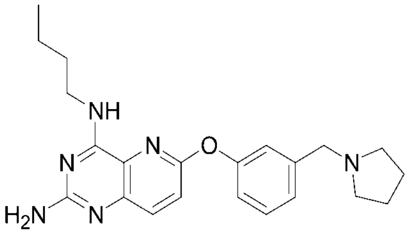

10 ,

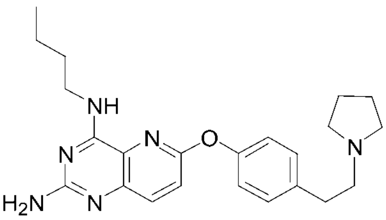

11 and

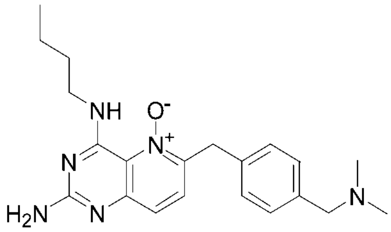

28 .

For a purpose of brevity, terms "Formula (I-1)", "Formula (I-2)", "Formula (II-1)", "Formula (II-2)", "compound in this application" and "compounds in this application" described hereinafter also include their stereoisomers, tautomers, isotopic derivatives, halogenated derivatives, hydrates, solvates, prodrugs and pharmaceutically acceptable salts.

This application also provides a preparation method for the compound denoted as Formula (I-1), which can be prepared via one of the following synthetic routes:

Route 1:

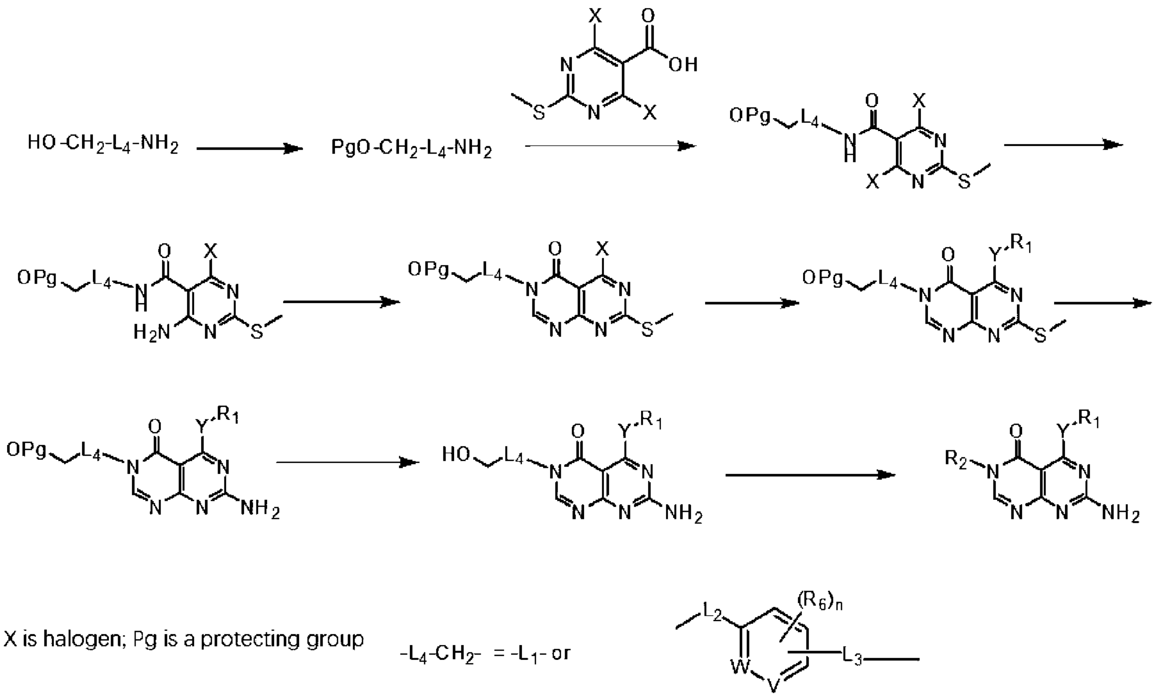


;

Route 2:

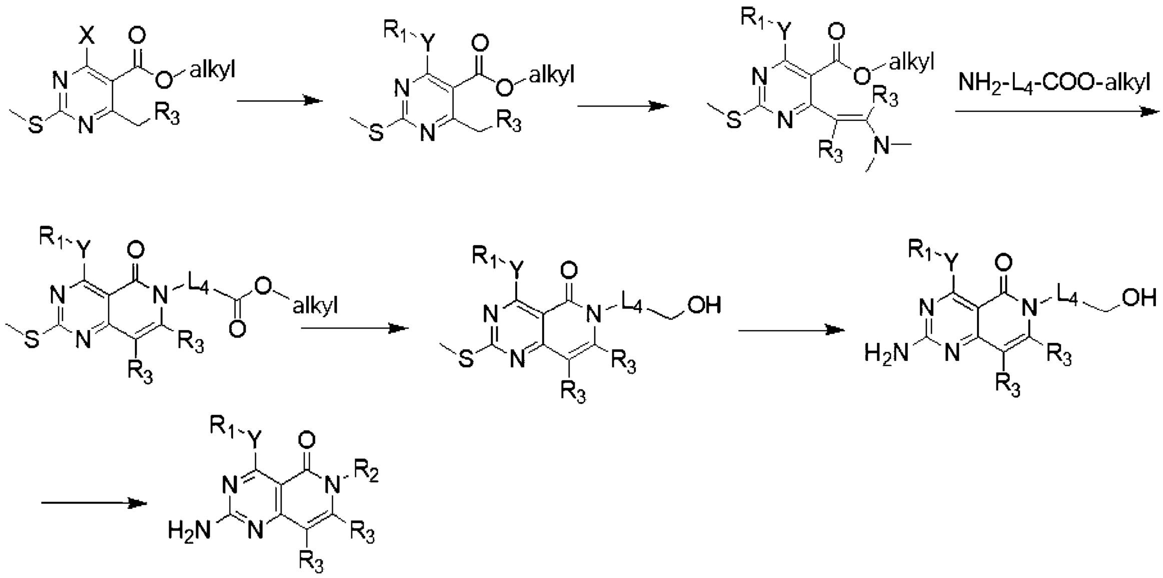


X is halogen -L4-CH2- = -L1- or 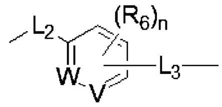 ;

Route 3:

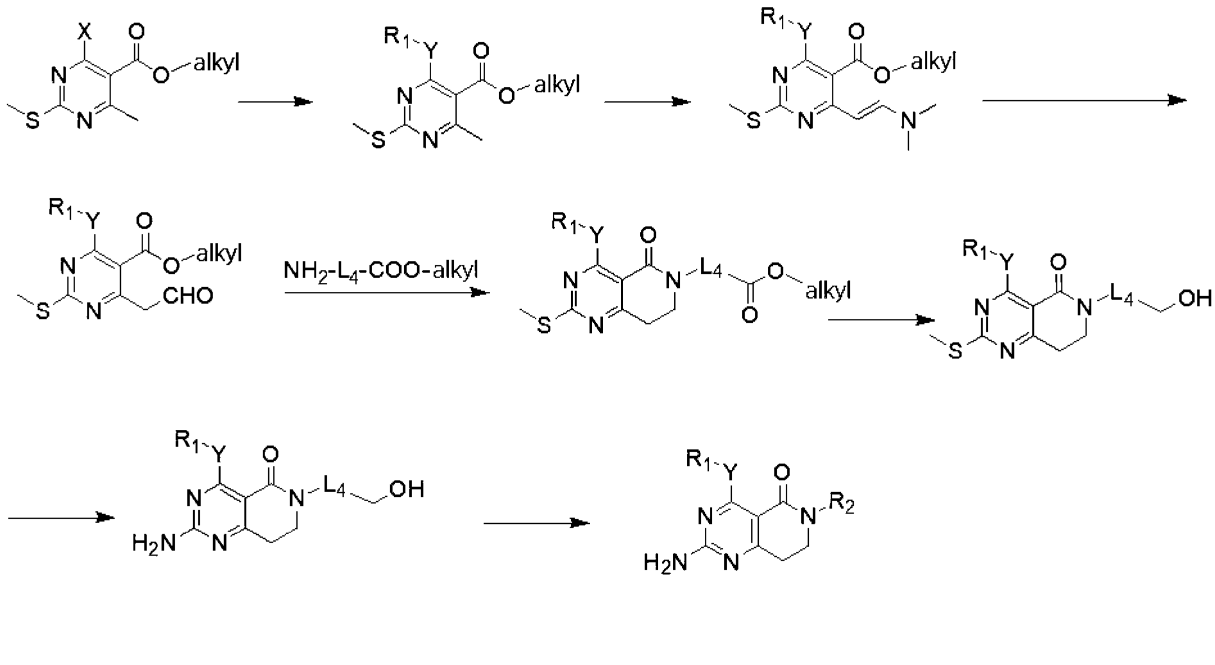


-L4-CH2- = -L1- or 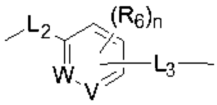 X is halogen ;

Route 4:

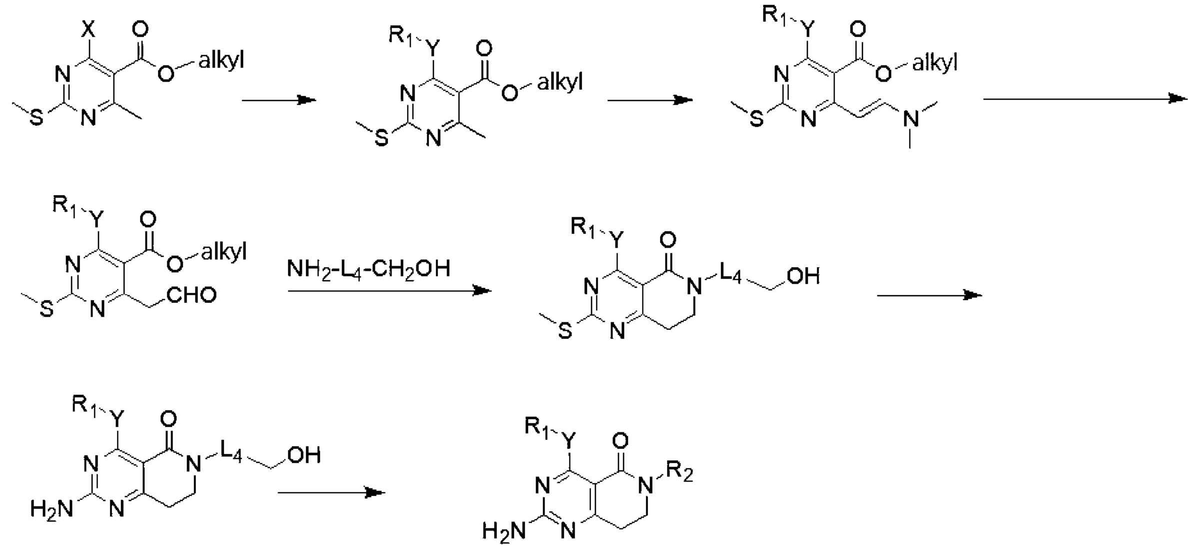


$-L_4-CH_2-$ = $-L_1-$ or 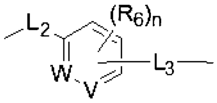 X is halogen ;

Definitions of all other groups are the same as those described above.

This application also provides a preparation method for the compound denoted as Formula (II-1), which can be prepared via one of the following synthetic routes:

Route 5:

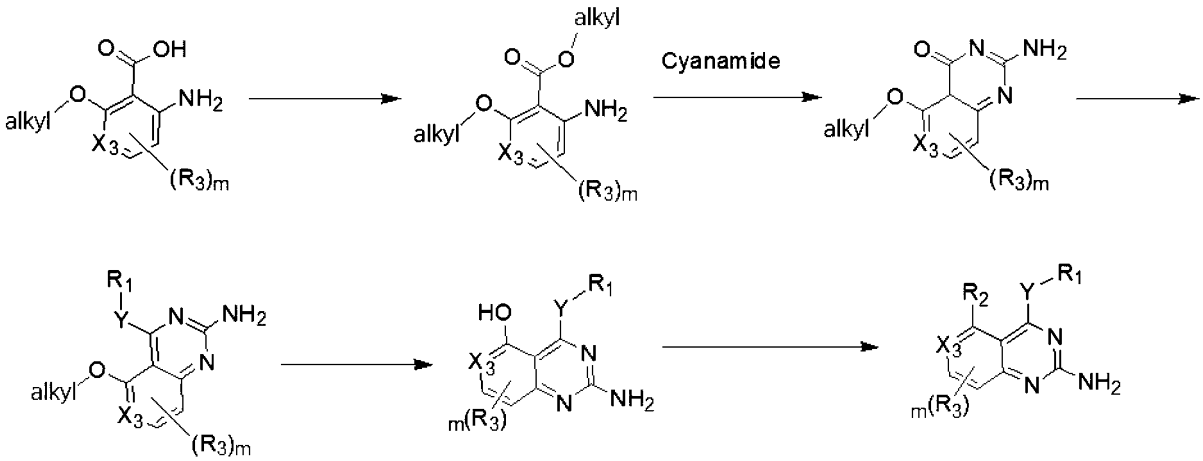


;

Route 6:

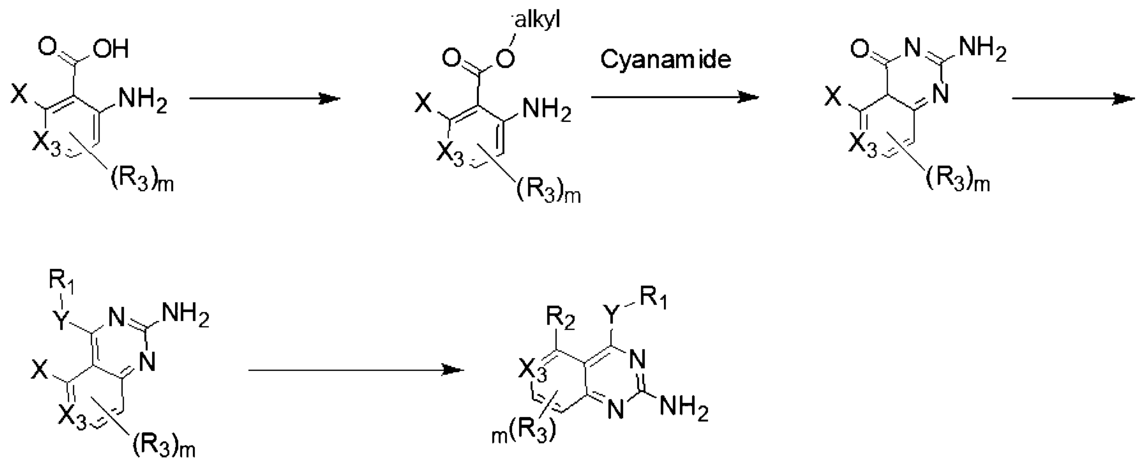


X is halogen

;

Definitions of all other groups are the same as those described above.

In an embodiment, a linking group between $L_1$ or $L_2$ and a ring bearing $X_3$ is -O-.

This application also provides a preparation method for the compound denoted as Formula (II-2), which can be prepared via the following synthetic route:

Route 7:

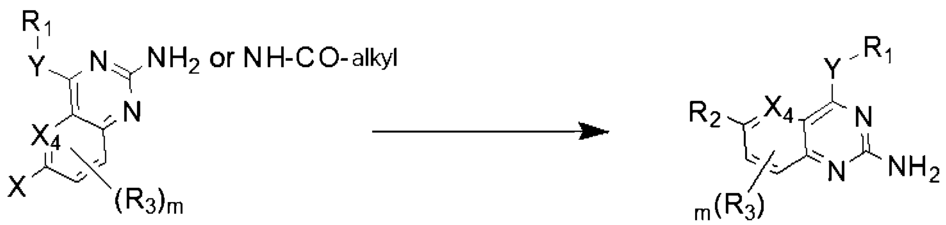

X is halogen

Definitions of all other groups are the same as those described above.

In an embodiment, a linking group between $L_1$ or $L_2$ and a ring bearing $X_4$ is -O-.

This application also provides a preparation method for the compound denoted as Formula (II-2), which can be prepared via the following synthetic route:

Route 8:

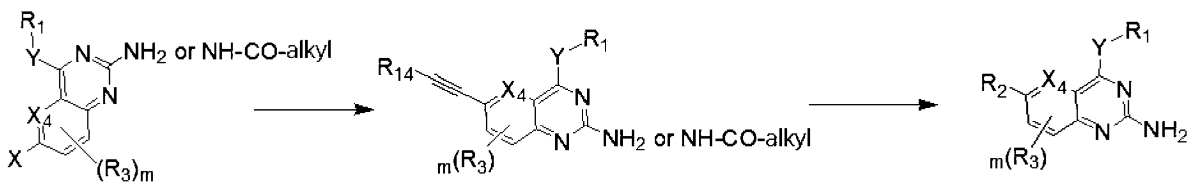

X is halogen ; $R_2$- = $-CH_2CH_2-R_{14}-$

;

Definitions of all other groups are the same as those described above.

This application also provides a pharmaceutical composition, containing the compound in this application as an active ingredient and further containing a pharmaceutically acceptable excipient, where the pharmaceutical composition

further includes a pharmaceutically acceptable carrier or excipient.

The administration routes for the compound in this application or the pharmaceutical composition thereof include, but are not limited to, oral, rectal, transmucosal, topical, transdermal, inhalation, parenteral, sublingual, intravaginal, intranasal, intramuscular, subcutaneous and intravenous administration routes. The administration dose is 0.1 - 0.2 mg/kg, preferably 0.1 mg/kg, once daily. Further, a concentration of the compound in this application in the composition is 0.1 - 0.2 mg/kg, preferably 0.1 mg/kg.

The pharmaceutical composition further includes at least one additional therapeutic agent, where the therapeutic agent is selected from a chemotherapeutic agent, immunotherapeutics, an anti-angiogenic agent, a cytokine, a hormone, a polynucleotide, an antibody, and an immunologically active fragment. When such medicament contains multiple active ingredients, the active ingredients may be administered simultaneously, sequentially or separately at discretion of a physician

The antibodies are anti-Her2, anti-PD-1, anti-PD-L1 and/or anti-TIM-3 antibodies; and preferably, the antibodies are anti-PD-L1 antibody and anti-Her2 antibody.

The compound in this application or the pharmaceutical composition thereof can be used as the TLR receptor agonist, and used for preparing a medicament for preventing or treating a disease or disorder associated with TLR activity; and the term "disease or disorder associated with the TLR activity" refers to any disease state related to a Toll-like receptor. Preferably, the compound in this application or the pharmaceutical composition thereof acts as TLR7 and/or TLR8 receptor agonists, and are preferably used for mammals.

This application further provides use of the compound in this application or the pharmaceutical composition thereof in preparation of a medicament for preventing or treating an infectious disease, a respiratory disease, an immune-related disease, a viral disease or a cell proliferative disease.

Preferably, the foregoing disease is asthma, a tumor, HIV or HBV.

The respiratory diseases include, but are not limited to, asthma, a chronic obstructive pulmonary disease and an acute respiratory distress syndrome. The immune-related diseases include, but are not limited to, systemic lupus erythematosus, Sjögren's syndrome, Wegener's granulomatosis, sarcoidosis, Reiter's syndrome, Behçet's syndrome, rheumatoid arthritis, inflammatory bowel disease, polymyositis, vasculitis, ankylosing spondylitis and psoriatic arthritis.

The viral diseases include, but are not limited to, Ebola virus disease, anthrax, condyloma acuminatum, verruca vulgaris, plantar wart, respiratory syncytial virus, hepatitis B, hepatitis C, dengue virus, herpes simplex virus (e.g., HSV-1 and HSV-2), molluscum contagiosum, cowpox, smallpox, lentivirus, human immunodeficiency virus (HIV), human papillomavirus (HPV), cytomegalovirus, varicella-zoster virus, rhinovirus, enterovirus, adenovirus, influenza, parainfluenza, mumps virus, measles virus, papovavirus, flavivirus, retrovirus, arenavirus (e.g., LCM, Junin virus, Machupo virus, Guanarito virus and Lassa fever) or filovirus (e.g., Ebola virus or Marburg virus).

The cell proliferative disease is a tumor, including, but not limited to, a human sarcoma and carcinoma, for example, lymphoma, osteosarcoma, fibrosarcoma, myosarcoma, liposarcoma, chondrosarcoma, osteogenic sarcoma, chordoma, angiosarcoma, endothelial sarcoma, lymphangiosarcoma, lymphangioendotheliosarcoma, synovioma, mesothelioma, Ewing's tumor, leiomyosarcoma, rhabdomyosarcoma, uterine tumor, neuroma, gastrointestinal carcinoma, colon cancer, rectal cancer, squamous cell carcinoma, basal cell carcinoma, adenocarcinoma, papillary carcinoma, medullary carcinoma, bronchogenic carcinoma, hepatoma, liver cancer, cholangiocarcinoma, choriocarcinoma, seminoma, embryonal carcinoma, nephroblastoma, cervical cancer, ovarian cancer, testicular tumor, renal cancer, lung cancer, epithelial carcinoma, glioma, astrocytoma, medulloblastoma, craniopharyngioma, ependymoma, pinealoma, hemangioblastoma, acoustic neuroma, oligodendroglioma, meningioma, melanoma, neuroblastoma, retinoblastoma, leukemia, polycythemia vera, multiple myeloma and a heavy chain disease. The adenocarcinoma may be thyroid carcinoma, pancreatic carcinoma, sweat gland carcinoma, sebaceous gland carcinoma, breast carcinoma, papillary adenocarcinoma, cystadenocarcinoma or prostate carcinoma; and the lung cancer may be small cell lung cancer. The leukemia is acute lymphoblastic leukemia, acute myeloblastic leukemia, acute promyelocytic leukemia, acute myelomonocytic leukemia, acute monocytic leukemia, acute erythroleukemia, chronic myelogenous leukemia, chronic lymphocytic leukemia or chronic granulocytic leukemia. The lymphoma is Hodgkin's disease or non-Hodgkin's disease.

Preferably, the tumor is a cold tumor; or more preferably, the tumor is colon cancer, bladder cancer, melanoma, meningioma, lung cancer, liver cancer or pancreatic cancer.

This application also provides use of the compound in this application or a pharmaceutical composition thereof in preparation of a medicament for preventing tumor recurrence or preferably a medicament capable of inducing durable systemic immune memory to prevent recurrence.

This application also provides use of the compound or pharmaceutical composition in this application as payloads in preparation of a conjugate (ADC) and as payloads in preparation of a conjugate (SMDC).

This application also provides use of the compound or pharmaceutical composition in this application in preparation of a pharmaceutical preparation; and preferably, the pharmaceutical preparation increases the number of genes beneficial to immunotherapy in various pathways such as inflammation- and cytokine-mediated pathways, T cell activation/apoptosis signaling pathway, and B cell activation pathway. More preferably, the pharmaceutical preparation

increases the number of genes beneficial to tumor immunotherapy in the foregoing pathways. Further, the pharmaceutical preparation increases the number of $CD8^+$ T cells, a ratio of $CD8^+$ T cells to Treg cells, and expression of PDL-1. Further, the pharmaceutical preparation increases a level of interleukin-2 and/or a level of interferon-y, and/or decreases a level of interleukin-10.

This application also provides use of the compound in this application or a pharmaceutical composition thereof in preparation of an agent for down-regulating a Wnt signaling pathway. Preferably, a protein expression level of S-catenin is down-regulated.

This application also provides use of the compound in this application or the pharmaceutical composition thereof in preparation of a pharmaceutical preparation for modulating secretion levels of various cytokines in macrophages or preferably modulating mRNA expression levels of IL-1β, IL-6, IL-12β, TNF, IFN-β1, CXCL1, CXCL10, IL-10 and other genes.

This application further provides a method for enhancing a positive immune response of an organism, comprising administering a therapeutically effective dose of the compound in this application or the pharmaceutical composition thereof to a system or an individual in need, thereby modulating TLR7 and/or TLR8.

This application further provides a method for enhancing chemotherapeutic efficacy, comprising administering a therapeutically effective dose of a chemotherapeutic agent to a system or an individual in need, and simultaneously or subsequently administering a therapeutically effective dose of the compound in this application or a pharmaceutical composition thereof.

This application further provides a method for improving immunotherapy, comprising administering a therapeutically effective dose of the compound in this application or a pharmaceutical composition thereof to a system or an individual in need, and simultaneously or subsequently injecting chimeric antigen receptor T cells (CAR-T) into the system or the individual.

This application also provides use of the compound in this application or a pharmaceutical composition thereof in preparation of a vaccine adjuvant.

This application has the following beneficial effects:

1. The compound in this application has excellent TLR7 and/or TLR8 receptor agonistic activity and can be used for preparing a medicament for preventing or treating an infectious disease, a respiratory disease, an immune-related disease, a viral disease or a cell proliferative disease.

2. The compound in this application exhibits potent immunostimulatory activity in the peripheral blood mononuclear cell system in the immune system, and induces human peripheral blood mononuclear cells to secrete significantly higher levels of interferon-γ and various other pro-inflammatory cytokines and chemokines compared with control D18 and R848. The compound in this application exhibits great capability of activating human peripheral blood mononuclear cells to kill cancer cells.

3. The compound in this application has shorter half-life, and the shorter half-life can reduce a risk of inducing systemic immune responses by the compound in vivo. The compound in this application is distributed at a high concentration in the liver tissue.

4. The compound in this application has higher relative safety and a superior tumor-suppressive effect.

## BRIEF DESCRIPTION OF DRAWINGS

FIG. 1 shows an effect of a compound in this application on a cytokine secretion level of human peripheral blood mononuclear cells.

FIG. 2 shows a pharmacokinetic profile of a compound in this application in mice.

FIG. 3 shows an inhibitory effect of a compound in this application on CT26 tumor in mice.

## DETAILED DESCRIPTION

The specific embodiments of this application are described in detail hereinafter. It should be understood that, the specific embodiments described herein are only intended to illustrate and explain this application, rather than limiting this application.

The endpoints and any value of ranges disclosed herein are not limited to precise ranges or values, and these ranges or values should be understood to include values close to these ranges or values. For numerical ranges, combination can be made among the endpoint values of each range, between the endpoint values of each range and individual point values, and among individual point values to obtain one or more new numerical ranges, and these numerical ranges should be regarded as being specifically disclosed herein.

Before detailed description of this application, it should be understood that the terms used herein are intended to describe a specific embodiment other than limit the scope of this application, where the scope of this application is limited only by the appended claims. To more completely understand this application described herein, the following terms are used, and the definitions of terms are shown below. Unless otherwise defined, all technical and scientific terms used herein have the same meanings as those understood by persons of ordinary skills in the art to which this application belongs.

Definitions

Unless otherwise specified, the following terms mentioned in this application have the following definitions.

In this application, for specific compound numbers, "M030xxx", "030xxx", "M30xxx" and "30xxx" may all be abbreviated as "xxx" and refer to the same compound as "xxx". "M0300xx", "0300xx", "M300xx" and "300xx" may all be abbreviated as "xx" and refer to the same compound as "xx". "M03000x", "03000x", "M3000x" and "3000x" may all be abbreviated as "x" and refer to the same compound as "x". For example, M030012 and 12 refer to the same compound, namely Compound 12; 30102 and 102 refer to the same compound, namely Compound 102; and M30004 and 4 refer to the same compound, namely Compound 4.

In this application,

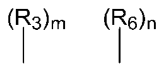

on a ring group indicates that m $R_3$ and n $R_6$ can be linked at any possible position on the ring group.

In this application,

" 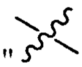 "

in some substituents represents a junction site.

Unless otherwise specified, "optionally substituted" means that hydrogen on a group to be substituted is not substituted or that one or more substitutable sites of the group to be substituted are independently substituted by the substituent. The substituent is one or more independently selected from deuterium, oxo, halogen, an amino group, a hydroxyl group, a nitro group, a cyano group, a sulfhydryl group, an optionally substituted alkyl group, an optionally substituted alkyloxy group, an optionally substituted alkylthio group, an optionally substituted alkylamino group and an optionally substituted dialkylamino group. A substituent on the alkyl group is one or more selected from oxo, deuterium, halogen, an amino group, a hydroxyl group, a nitro group, a cyano group or a sulfhydryl group. Selecting "oxo" as the substituent means that two hydrogen atoms at the same substitution position on the carbon atom are substituted with oxygen atoms, or a heteroatom is linked to an oxygen atom, to form, for example, a nitrogen oxide by linking a nitrogen atom to an oxygen atom or form a sulfinyl group or a sulfonyl group by linking a sulfur atom to an oxygen atom. "Optional oxo" means that the hydrogen on the substituted group is not substituted, or two hydrogen atoms on carbon at the same substitution site are substituted by oxygen atoms, or a heteroatom is bonded to oxygen, to form, for example, a nitrogen oxide with nitrogen bonded to oxygen or a sulfinyl group or sulfonyl group with sulfur bonded to oxygen. The term "independently" means that when the number of substituents is greater than one, these substituents may be the same or different.

The term "aryl group" represents a monocyclic or bicyclic aromatic carbon ring system containing 6-10 carbon atoms, and examples of aryl groups include a phenyl group and a naphthalene group.

The term "heteroaryl group" represents an aromatic monocyclic or multi-cyclic system containing a 5- to 10-membered structure, or preferably, a 5- to 8-membered structure, or more preferably, a 5- to 6-membered structure, where one or more ring atoms are heteroatoms and other atoms are carbon atoms, heteroatoms are independently selected from O, N or S, and the number of heteroatoms is preferably 1, 2, 3 or 4. The heteroaryl group can be 5- to 6-membered aromatic monocyclic structure containing 1 or 2 atoms selected from N or S atoms, and can be 5- to 6-membered aromatic monocyclic structure containing 1 or 2 N atoms. Examples of heteroaryl groups include, but are not limited to, a furanyl group, a thienyl group, an oxazolyl group, a thiazolyl group, an isooxazolyl group, an oxadiazolyl group, a thiadiazolyl group, a pyrrolyl group, a pyrazolyl group, an imidazol group, a triazolyl group, a tetrazolyl group, a pyridinyl group, a pyrimidyl group, a pyrazinyl group, a pyridazinyl group, a thiodiazolyl group, a triazinyl group, a phtharazinyl group, a

quinolinyl group, an isoquinolinyl group, a pteridinyl group, a purinyl group, an indolyl group, an isoindolyl group, an inazolyl group, a benzo-furanyl group, a benzo-thiophenyl group, a benzo-pyridinyl group, a benzo-pyrimidinyl group, a benzo-pyrazinyl group, a benzo-imidazol group, a benzo-phtharazinyl group, a pyrrolo[2,3-b]pyridinyl group, an imidazo[1,2-a]pyridinyl group, a pyrazolo[1,5-a]pyridinyl group, a pyrazolo[1,5-a]pyrimidinyl group, an imidazo[1,2-b]pyridazinyl group, a [1,2,4]triazolo[4,3-b]pyridazinyl group, a [1,2,4]triazolo[1,5-a]pyrimidinyl group and a [1,2,4]triazolo[1,5-a] pyridyl group.

The term "cycloalkyl group" denotes a saturated monocyclic, bicyclic or tricyclic system containing 3-12 carbon atoms, where the monocyclic, bicyclic or tricyclic system does not include an aromatic ring including a bridged ring group, a spiro ring group, a fused ring group, and the like. The bridged ring group means that any two rings share two indirectly linked ring atoms. Preferably, 3-10 carbon atoms (C3-10 cycloalkyl group) are included, and further preferably, 3-8 carbon atoms (C3-8 cycloalkyl group), 3-6 carbon atoms (C3-6 cycloalkyl group), 4-6 carbon atoms (C4-6 cycloalkyl group), and 5-6 carbon atoms (C5-6 cycloalkyl group) are included. Examples include, but are not limited to, a cyclopropyl group, a cyclobutyl group, a cyclopentyl group, a cyclohexyl group, a methylcyclopropyl group, a 2-ethyl-cyclopentyl group, and a dimethylcyclobutyl group.

The term "heterocycloalkyl group" refers to a saturated monocyclic, bicyclic or tricyclic system containing heteroatoms, where the monocyclic, bicyclic or tricyclic system does not include an aromatic ring including a bridged ring group, a spiro ring group, a fused ring group, and the like. The bridged ring group means that any two rings share two indirectly linked ring atoms. The heterocycloalkyl group contains 3-12 ring atoms, where one or more ring atoms are selected from N, O or S atoms, and other ring atoms are C atoms. Preferably, 3-8 ring atoms (3- to 8-membered heterocycloalkyl group), 3-7 ring atoms (3- to 7-membered heterocycloalkyl group) or 3-6 ring atoms (3- to 6-membered heterocycloalkyl group), or 4-6 ring atoms (4- to 6-membered heterocycloalkyl group), or 5-6 ring atoms (5- to 6-membered heterocycloalkyl group) are included. The heterocycloalkyl group may be a 7- to 12-membered fused or spiro heterocycloalkyl group, or a 7- to 9-membered spiro heterocycloalkyl group. The number of heteroatoms is preferably, 1-4, or more preferably, 1-3 (that is, 1, 2 or 3). The heterocycloalkyl group may be a 4- to 6-membered monocyclic heterocycloalkyl group containing 1 or 2 N or O atoms, or preferably, is a 4-, 5- or 6-membered monocyclic heterocycloalkyl group containing 1 or 2 N atoms, a 4-, 5- or 6-membered monocyclic heterocycloalkyl group containing 1 or 2 O atoms, a 4-, 5- or 6-membered monocyclic heterocycloalkyl group containing 1 N atom and 1 O atom, or a 7-, 8-, 9-, 10- or 11-membered spirocycloalkyl group containing 1, 2, 3 or 4 N or O atoms. Examples of heterocycloalkyl groups include a pyrrolidyl group, an imidazolidinyl group, a tetrahydrofuranyl group, a piperidinyl group, a piperazinyl group, a pyranyl group, an azacyclopropyl group, an oxacyclopropyl group, a thiacyclopropyl group, an azacyclobutyl group, an oxacyclobutyl group, a thiacyclobutyl group, oxacyclohexane, a morpholinyl group, a thiomorpholinyl group, a dioxane group, a dithiacyclohexyl group, an oxazolidinyl group, a thiazolidinyl group, a pyrazolidinyl group, imidazolidine,

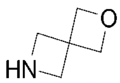, 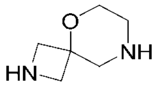,

and the like.

The term "alkyl group" refers to a monovalent saturated aliphatic hydrocarbonyl group, and is preferably a straight chain or branch chain group (the number of carbon atoms is 1-10, and is specifically 1, 2, 3, 4, 5, 6, 7, 8, 9 or 10) that contains 1-10 carbon atoms (C1-10 alkyl group), or more preferably, contains 1-8 carbon atoms (that is, C1-8 alkyl group, where the number of carbon atoms is 1-8, and is specifically 1, 2, 3, 4, 5, 6, 7 or 8), or contains 1-6 carbon atoms (that is, C1-6 alkyl group, where the number of carbon atoms is 1-6, and is specifically 1, 2, 3, 4, 5, or 6). Examples include, but are not limited to, a methyl group, an ethyl group, an n-propyl group, an isopropyl group, an n-butyl group, an isobutyl group, a tert-butyl group, a sec-butyl group, an n-amyl group, a neopentyl group, a 1,1-dimethylpropyl group, 1,2-dimethylpropyl group, 2,2-dimethylpropyl group, 1-ethylpropyl group, 2-methylbutyl group, 3-methylbutyl group, an n-hexyl group, an n-heptyl group, an n-octyl group, 3-hexyl group, and 2-pentyl group.

The term "alkyloxy group", "alkylthio group", "alkylacyl group", "alkylsulfonyl group" or "alkylsulfinyl group" refers to -O-alkyl, -S-alkyl, -C(O)-alkyl, -$S(O)_2$-alkyl or -S(O)-alkyl, respectively. The term "alkylamino group" refers to -NH-alkyl or N-dialkyl. The term "-N(O)alkyl" refers to nitrogen oxide with the alkylamino group, where the alkyl group is as defined above. Representative examples include, but are not limited to, a methoxy group, an ethoxy group, a propoxy group, an isopropoxy group, a butoxy group, a 1-methylpropoxy group, a 2-methylpropoxy group, a tert-butoxy group, a methylthio group, an ethylthio group, a propylthio group, an isopropylthio group, a butylthio group, a 1-methylpropylthio group, a 2-methylpropylthio group, a tert-butylthio group, a methylamino group, an ethylamino group, a propylamino group, a dimethylamino group, a diethylamino group, a dipropylamino group, methylethylamino group, a methanesulfonyl group and dimethyl-N(O)-.

The term "linear alkylene group" refers to a divalent saturated linear aliphatic hydrocarbonyl group. One or more carbon atoms in the linear alkylene group may be substituted with an oxygen, sulfur or nitrogen heteroatom. The linear alkylene group preferably contains 1-10 chain atoms (the number of chain atoms is 1-10, and is specifically 1, 2, 3, 4, 5, 6, 7,

8, 9 or 10), or more preferably, contains 1-8 chain atoms (that is, the number of chain atoms is 1-8, and is specifically 1, 2, 3, 4, 5, 6, 7 or 8), or contains 1-6 chain atoms (that is, the number of chain atoms is 1-6, and is specifically 1, 2, 3, 4, 5, or 6). One or more carbon atoms in the linear alkylene group may be substituted with an oxygen, sulfur or nitrogen heteroatom. Examples include, but are not limited to, -O-, $-CH_2-$, $-O-CH_2-$, $-CH_2-O-$, $-NH-CH_2-$, $-CH_2-NH-$, $-O-CH_2CH_2-$, $-CH_2CH_2-O-$, $-NH-CH_2CH_2-$, $-CH_2CH_2-NH-$, $-CH_2CH_2CH_2-$, $-CH_2CH_2-$, $-OCH_2CH_2CH_2-$, $-CH_2CH_2CH_2-O-$, $-NHCH_2CH_2CH_2-$, $-CH_2CH_2CH_2-NH-$ and $-CH(CH_3)CH_2-$.

The term "halogen" refers to F, Cl, Br, and I.

Herein, number ranges related to the number of substituents, the number of carbon atoms, and the number of ring atoms represent enumerations of all integers within the range, and the range is only used as a simplified representation. For example, "1-4 substituents" represent 1, 2, 3, or 4 substituents; and "3-8 ring atoms" represent 3, 4, 5, 6, 7, or 8 ring atoms. Therefore, the number ranges related to the number of substituents, the number of carbon atoms, and the number of ring atoms also cover any one of its sub-ranges, and each sub-range is also construed as being disclosed herein.

The active compound in this application is interpreted as including the compound in this application and a stereoisomer, a tautomer, an isotopic derivative, a halogenated derivative, a hydrate, a solvate, a prodrug and a pharmaceutically acceptable salt thereof. The stereoisomer, the tautomer, the isotope derivative, the hydrate, the solvate, the prodrug, and the pharmaceutically acceptable salt of the compound in this application are obtained through conventional technical methods in the art, and exert the same or similar effects *in vitro* and *in vivo* through the same mechanism of action as the compound.

The term "stereoisomer" refers to an isomer produced due to different spatial distribution of atoms in a molecule, including a configurational isomer and a conformational isomer. The configurational isomer further includes a geometric isomer (or a cis-trans isomer) and an optical isomer (including an enantiomer and a diastereomer). The geometric isomer can be present in the compound. The optical isomers refer to substances that have identical molecular structures, similar physical and chemical properties, but have different optical activities. When having an R or S configuration, the compound in this application may contain asymmetrically substituted carbon atoms, where the terms "R" and "S" have the same definitions as those in IUPAC 1974 Recommendations for Section E, Fundamental Stereochemistry, Pure Appl. Chem. (1976)45, 13-10. Compounds with asymmetrically substituted carbon atoms (with equal numbers of R and S configurations) are racemic at those carbon atoms. Atoms possessing an excess of one configuration (relative to the other configuration) result in a higher abundance of the configuration, preferably with an excess of about 85%-90%, more preferably about 95%-99%, or more preferably about 99%. Correspondingly, this application includes a racemic mixture, relative and absolute optical isomers and a mixture of relative and absolute optical isomers.

The term "tautomer" refers to a structural isomer that has different energy levels and can be converted into each other via a low energy barrier. If tautomerism possibly exists (for example, in a solution), chemical equilibrium of the tautomers can be achieved. For example, proton tautomers (also referred to as proton-transfer tautomers) are converted into each other through proton migration, for example, keto-enol isomerization and imine-enamine isomerization. Valence tautomers are converted into each other through recombination of some bonding electrons.

The term "isotope derivative" means that the compound in this application may be isotopically traced or enriched and contain one or more atoms whose atomic weight or mass differs from that of most atoms found in nature. Isotopes can be radioactive or non-radioactive isotopes. Isotopes of, for example, hydrogen, carbon, phosphorus, sulfur, fluorine, chlorine, and iodine atoms include, but are not limited to, $^{2}H$,$^{3}H$,$^{13}C$,$^{14}C$,$^{15}N$,$^{18}O$,$^{32}P$,$^{35}S$,$^{18}F$,$^{36}Cl$ and $^{125}I$. Compounds containing other isotopes of these and/or other atoms fall within the scope of this application. The isotopically labeled compound in this application may be prepared in a general method familiar to persons of ordinary skills in the art.

The term "hydrate" refers to an adduct formed by one or more water molecules and the compound in this application.

The term "solvate" refers to an adduct formed by one or more solvent molecules and the compound in this application.

The term "prodrug" is a derivative of a designed active drug and can improve some definite, undesirable physical or biological properties. The physical property is usually related solubility (excessively high or insufficient lipid or water solubility) or stability, while problematic biological properties include excessively fast metabolism or poor bioavailability, which may be related to physicochemical properties.

The term "pharmaceutically acceptable salt" refers to salt that is suitable for contact with the tissues of mammals, especially humans, within a reasonable medical judgment range without excessive toxicity, irritation, allergic reactions or the like and that is commensurate with a reasonable benefit-risk ratio. If the compound is alkaline, the pharmaceutically acceptable salts include salts prepared from inorganic acids and salts prepared from organic acids. If the compound is acidic, the pharmaceutically acceptable salts include salts prepared from inorganic bases and/or organic bases.

The term "pharmaceutically acceptable adjuvant", "pharmaceutically acceptable carrier" or "pharmaceutically acceptable excipient" includes, but is not limited to, any adjuvant, carrier, excipient, glidant, sweetener, diluent, preservative, dye/colorant, flavoring agent, surfactant, wetting agent, dispersant, suspending agent, stabilizer, isotonic agent, solvent, emulsifier, or disintegrant approved for use in humans or livestock by a relevant regulatory authority.

The pharmaceutical composition in this application can be prepared into a tablet, a pill, a capsule, powder, a granule, an ointment, an emulsion, a suspending agent, a solution, a suppository, an injection, an inhalant, and a gel.

The term "treatment" used herein refers to any administration of a therapeutic agent according to a therapeutic regimen, where the therapeutic regimen achieves desired effects, that is, effects of partially or completely relieving, improving, remitting, inhibiting, delaying, and reducing severity and/or incidence of one or more symptoms or conditions of a particular disease, disorder, and/or condition; and in some embodiments, administration of a therapeutic agent according to a therapeutic regimen is associated with achievement of desired effects. Such treatment may be prescribed for a subject who do not have a relevant disease, disorder, and/or symptom, and/or a subject having only an early sign of the disease, disorder and/or condition. Alternatively or additionally, such treatment may be prescribed for a subject having one or more established signs of the relevant disease, disorder, and/or condition. In some embodiments, the treatment may be prescribed for a subject who has been diagnosed with the relevant disease, disorder, and/or condition. In some embodiments, the treatment may be prescribed for a subject for whom one or more predisposing factors have been identified, where the predisposing factors are statistically associated with an increased risk of developing the relevant disease, disorder, and/or condition.

The composition in this application can be synthesized in various methods known to persons skilled in the field of organic synthesis. The following specific examples provide some exemplary synthetic methods of specific compounds. Apparently, with reference to the exemplary solution in this application, persons skilled in the art can easily design another synthetic route for the related compound by appropriately adjusting a reactant, a reaction condition and a protecting group.

This application is further described below with reference to examples, but these examples are not intended to limit the scope of this application. Unless otherwise stated, all reactants used in the examples are commercially available; and instruments and devices used in synthesis experiments and product analyses and detection are all conventional instruments and devices commonly used in organic syntheses.

**Preparation of Intermediate 30092-6**

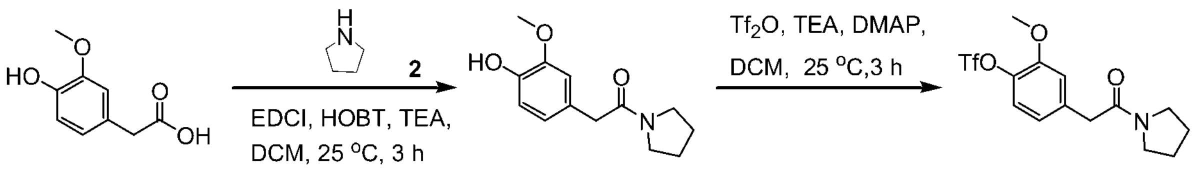


30092-1 30092-3 30092-4

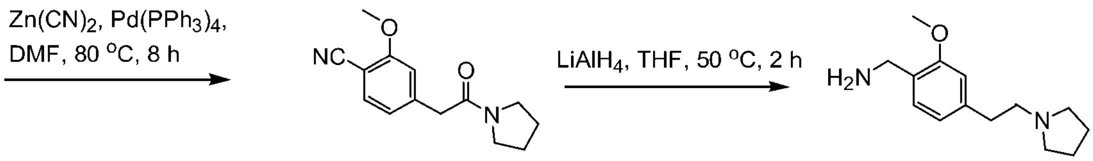


30092-5 30092-6

**Step 1: General Method for Preparation of Compound 30092-3**

At room temperature under a nitrogen atmosphere, triethylamine (3.34g, 33 mmol) was added to a DMF (30 mL) solution of Compound 30092-1 (3g, 17 mmol), EDCI (4.74g, 25 mmol), HOBt (3.34g, 25 mmol) and pyrrolidine (1.41g, 20 mmol) during stirring. The reaction mixture was stirred at room temperature for 3 hours. The reaction mixture was concentrated under reduced pressure and extracted with ethyl acetate (30 mL x 2). The organic phase was concentrated under reduced pressure and then directly purified by flash silica gel column chromatography (MeOH/DCM = 0%-5%), to obtain Compound 30092-3 (2.6g, 60% yield) as a brown solid.

**Step 2: General Method for Preparation of Compound 30092-4**

At room temperature under a nitrogen atmosphere, $Tf_2O$ (3.13g, 11 mmol) was added to a DCM (30 mL) solution of Compound 30092-3 (2.6g, 11 mmol), TEA (1.35g, 13 mmol) and DMAP (0.14g, 1 mmol) during stirring. The reaction

mixture was stirred at room temperature for 3 hours. The reaction mixture was concentrated under reduced pressure and extracted with DCM (30 mL x 2). The organic phase was concentrated under reduced pressure and then directly purified by flash silica gel column chromatography (MeOH/DCM = 0%-10%), to obtain Compound 30092-4 (2.6g, 64% yield) as a yellow oil.

**Step 3: General Method for Preparation of Compound 30092-5**

At room temperature under a nitrogen atmosphere, a mixture of Compound 30092-4 (2.6g, 7.1 mmol), $Zn(CN)_2$ (0.50g, 4 mmol), and $Pd(PPh_3)_4$ (0.82g, 1 mmol) in DCM (15 mL) was stirred at 80°C for 8 hours. The reaction mixture was concentrated under reduced pressure and extracted with EA (30 mL x 2). The organic phase was concentrated under reduced pressure and then directly purified by flash silica gel column chromatography (MeOH/DCM = 0%-10%), to obtain Compound 30092-5 (1.2g, 69.2% yield) as a yellow oil.

**Step 4: General Method for Preparation of Compound 30092-6**

At room temperature under a nitrogen atmosphere, $LiAlH_4$ (0.93g, 2.5 mmol) was added dropwise to a THF (50 mL) solution of Compound 30092-5 (1.2g, 5 mmol) during stirring. The reaction mixture was stirred at 50°C for 2 hours. The reaction was subjected to quenching by adding $H_2O$, followed by filtration. The filtrate was concentrated under reduced pressure to obtain crude product 30092-6 (0.9g, 67.4% yield) as a yellow oil.

**Preparation of Intermediate 30124-2**

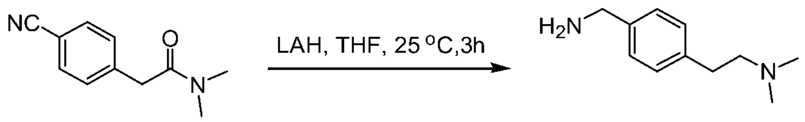


**30124-1** **30124-2**

At room temperature under a nitrogen atmosphere, $LiAlH_4$ (323mg, 8.5 mmol) was added dropwise to a THF (10 mL) solution of Compound 30124-1 (320mg, 1.7 mmol) during stirring. The reaction mixture was stirred at 25°C for 3 hours. The reaction was subjected to quenching by adding $H_2O$ (10 mL), followed by filtration. The filtrate was concentrated under reduced pressure, and the crude product was purified by flash reverse-phase (C18) silica gel column chromatography (ACN/ $H_2O$ = 1%-10%) to afford Compound 30124-2 (300 mg, 1.68 mmol, 99% yield) as a white solid.

**Preparation methods of intermediates 30125-INT and 30126-INT are the same as that of 30092-6.**

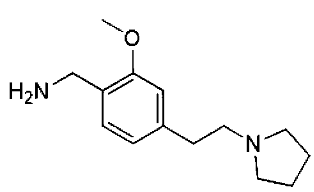

**30125-INT,** i.e.,**30092-6**

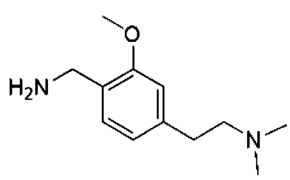

**30126-INT**

**Preparation of Intermediate 30144-3**

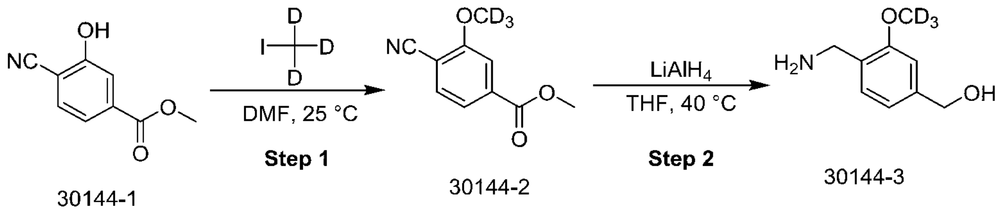


30144-1 30144-2 30144-3

**Step 1: General Method for Preparation of Compound 30144-2**

At room temperature under a nitrogen atmosphere, NaH (0.34g, 16.8 mmol) was added to a DMF (8 mL) solution of Compound 30144-1 (1.0g, 5.6 mmol) during stirring. The reaction mixture was stirred at 25°C for half an hour. A solution of $ICD_3$ (2.44g, 16.8 mmol) in DMF (2 mL) was added dropwise, and the reaction mixture was stirred at 25°C for 1 hour. The reaction was subjected to quenching with a saturated aqueous solution (20 mL) of $NH_4Cl$, followed by extraction with ethyl acetate (20 mL x 3). The organic phase was washed with saturated brine (20 mL x 3) and dried over anhydrous $Na_2SO_4$, to obtain crude compound 30144-2 (1g, 88% yield) to be directly used for the next step. $[M+H]^+$: 195.1.

**Step 2: General Method for Preparation of Compound 30144-3**

At 40°C under a nitrogen atmosphere, a solution of 30144-2 (0.36g, 1.86 mmol) in THF (10 mL) was added dropwise to a solution of $LiAlH_4$ (7.4 mL, 1 M). The reaction mixture was stirred at 40°C for 3 hours. The reaction was subjected to quenching by adding $Na_2SO_4 \cdot 10H_2O$, followed by filtration. The filtrate was concentrated under reduced pressure to obtain crude product 30144-3 (0.3g, 76% yield) as a colorless oil. The crude product was directly used for the next step. $[M+H]^+$ 171.1.

**Preparation of Intermediate 30145-3**

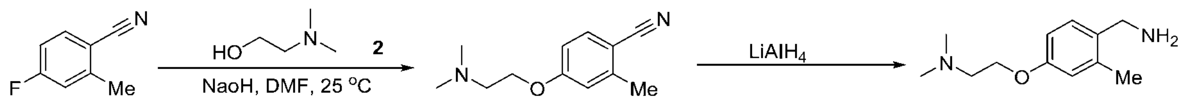


**30145-1** **30145-2** **30145-3**

**Step 1: General Method for Preparation of Compound 30145-2**

At room temperature under a nitrogen atmosphere, 2 (0.66g, 7.4 mmol) and NaOH (0.59g, 14.8 mmol) were added to a DMF (10 mL) solution of Compound 30145-1 (1.0g, 7.4 mmol) during stirring. The reaction mixture was stirred at 25°C for 12 hours. The reaction was subjected to quenching with water (30 mL) at 0°C, followed by extraction with ethyl acetate (20 mL x 3). The organic phase was washed with saturated brine (50 mL x 3), dried over anhydrous $Na_2SO_4$, and filtered. The filtrate was concentrated under reduced pressure to obtain crude compound 30145-2 (0.8g, 50% yield) as a yellow oil, which was directly used for the next step. $[M+H]^+$: 205.2.

**Step 2: General Method for Preparation of Compound 30145-3**

At 40°C under a nitrogen atmosphere, a solution of 30145-2 (0.5g, 2.44 mmol) in THF (5 mL) was added dropwise to a solution of $LiAlH_4$ (4.9 mL, 1 M) in THF (5 mL). The reaction mixture was stirred at 40°C and then cooled to room temperature. Then the reaction was subjected to quenching by adding $Na_2SO_4 \cdot 10H_2O$, followed by filtration. The filtrate was concentrated under reduced pressure to obtain crude product 30145-3 (0.5g, 93% yield) as a yellow oil. The crude product was directly used for the next step. $[M+H]^+$ 209.2.

**Intermediate 30146-3 was prepared with reference to the preparation of intermediate 30145-3.**

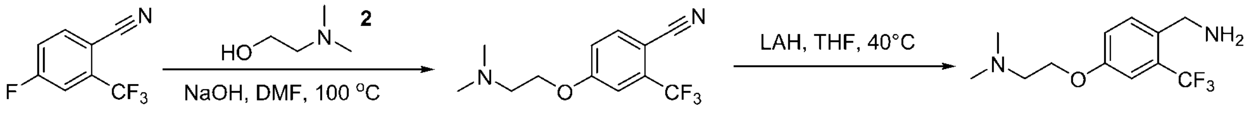


30146-1 Step 1 30146-2 Step 2 30146-3

**Preparation of Intermediate 30147-2**

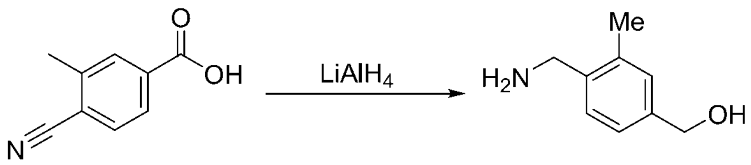


30147-1 Step 1 30147-2

**Step 1: General Method for Preparation of Compound 30147-2**

At 40°C under a nitrogen atmosphere, a solution of 30147-1 (0.5g, 3.10 mmol) in THF (10 mL) was added dropwise to a solution of $LiAlH_4$ (6.2 mL, 1 M). The reaction mixture was stirred at 40°C for 2 hours. The reaction was subjected to quenching by adding $Na_2SO_4 \cdot 10H_2O$, followed by filtration. The filtrate was concentrated under reduced pressure, and the resulting crude product was separated by reverse-phase column chromatography (0%-30% $ACN/H_2O$ (0.1% $NH_3 \cdot H_2O$)) to obtain 30147-2 (0.15g, 28.8% yield) as a white solid. $[M+H]^+$ 152.1.

**Intermediate 30148-3 was prepared with reference to the preparation of intermediate 30145-3.**

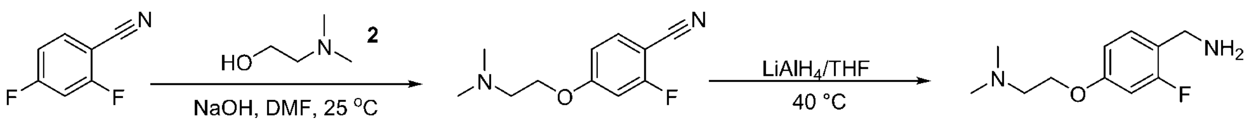


30148-1 Step 1 30148-2 Step 2 30148-3

**Preparation of Intermediate 30149-4**

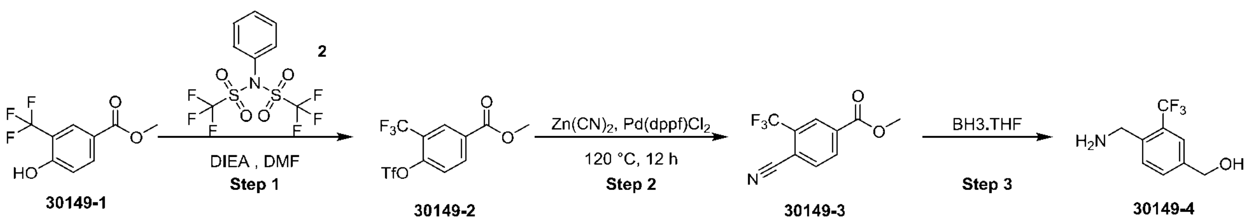


30149-1 30149-2 30149-3 30149-4

**Step 1: General Method for Preparation of Compound 30149-2**

At room temperature under a nitrogen atmosphere, DIEA (2.64g, 20.4 mmol) and 2 (2.92g, 8.16 mmol) were added to a DMF (15 mL) solution of Compound 30149-1 (1.5g, 6.8 mmol) during stirring. The reaction mixture was stirred at room temperature for 1 hour. The reaction was subjected to quenching with 30 mL of water, followed by extraction with ethyl acetate (50 mL x 3). The organic phase was washed with saturated brine (50 mL x 3), concentrated under reduced pressure and then directly purified by flash silica gel column chromatography (THF/PE = 0%-1%), to obtain Compound 30149-2 (2g, 84% yield) as a yellow oil.

### Step 2: General Method for Preparation of Compound 30149-3

At room temperature under a nitrogen atmosphere, TEA (0.43g, 4.25 mmol), $Zn(CN)_2$ (199.46 mg, 1.69872 mmol) and $PdCl_2$(dppf) (103.58 mg, 0.14156 mmol) were added to a DMF (2 mL) solution of 30149-2 (0.5g, 1.4 mmol) during stirring. The reaction mixture was stirred at 120°C for 12 hours. The reaction was subjected to quenching with 10 mL of water, followed by extraction with ethyl acetate (15 mL x 3). The organic phase was washed with saturated brine (15 mL x 3) and concentrated under reduced pressure, and a resulting crude product was directly purified by flash silica gel column chromatography (THF/PE = 0%-5%), to obtain Compound 30149-3 (150 mg, 44% yield) as a white solid. $[M+H]^+$ 230.1.

### Step 3: General Method for Preparation of Compound 30149-4

Under a nitrogen atmosphere, 30149-3 (100 mg, 0.4345 mmol) was dissolved in $BH_3$ (2M) in THF (5 mL). The reaction mixture was stirred at 60°C for 12 hours. The reaction mixture was concentrated under reduced pressure to obtain crude product 30144-3 (90 mg, 95% yield) as a yellow oil. The crude product was directly used for the next step. $[M+H]^+$ 206.1.

### Preparation of Intermediates INT-I and INT-II

Reference: J. Med. Chem. 2021, 64, 7507-7532. The preparation was carried out according to the following scheme.

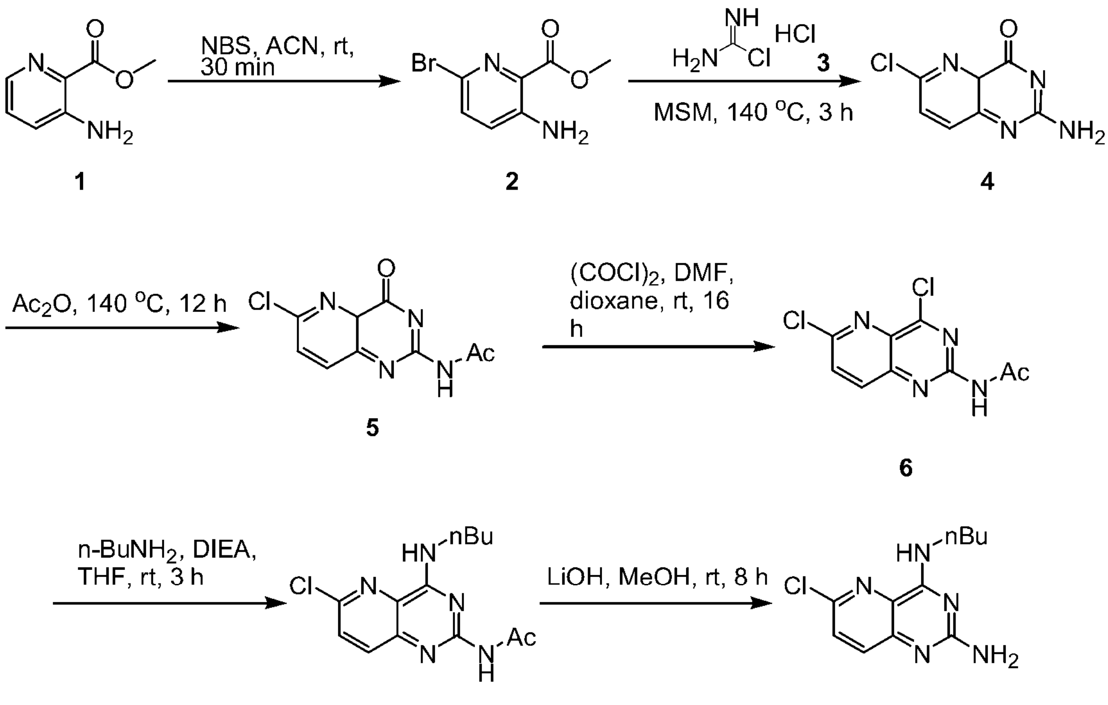


1 2 4

5 6

7, INT-I 8, INT-II

### Example 1: Preparation of Compound 30066

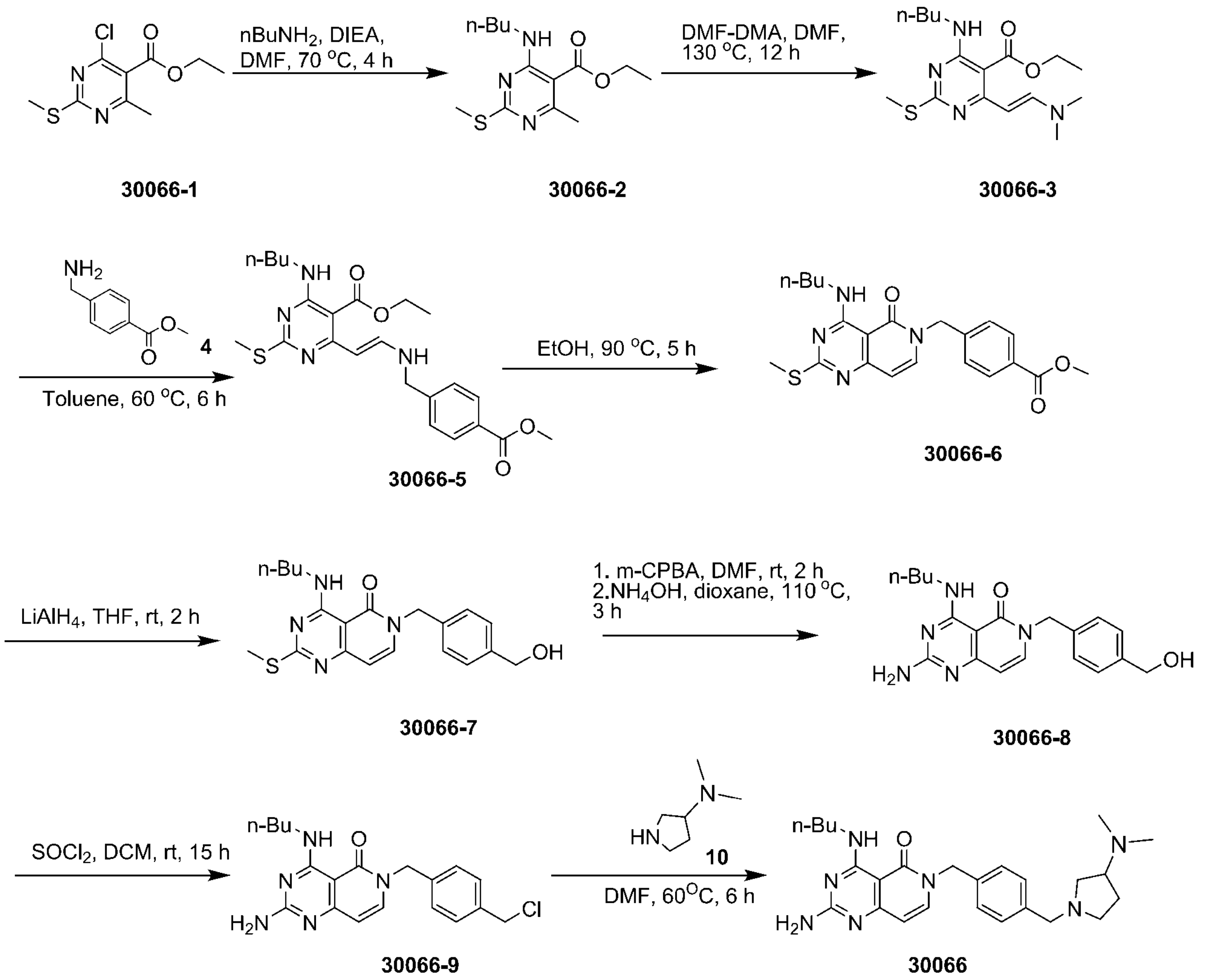


**Step 1: General Method for Preparation of Compound 30066-2**

A solution of Compound 30066-1 (10g, 0.041 mol), $^{n}BuNH_2$ (4.44g, 0.061 mol) and N,N-diisopropylethylamine (DIEA, 15.7g, 0.122 mol) in DMF (100 mL) was heated to 70°C and stirred for 4 hours. The reaction was subjected to quenching with $H_2O$ (200 mL), followed by extraction with ethyl acetate (100 mL x 3). The solvent was removed from the reaction mixture under reduced pressure to obtain the crude product. The crude product was directly purified by flash silica gel column chromatography (PE/EA = 9/1), to obtain Compound 30066-2 (10g, 86.17%) as a yellow oil.

**Step 2: General Method for Preparation of Compound 30066-3**

A solution of Compound 30066-2 (10g, 0.035 mol) and N,N-dimethylformamide dimethyl acetal (DMF-DMA) (21.03g, 0.177 mol) in DMF (100 mL) was heated to 130°C and stirred for 12 hours. The solution was evaporated directly to obtain a brown solid, Compound 30066-3 (10g of crude product).

**Step 3: General Method for Preparation of Compound 30066-5**

A solution of Compound 30066-3 (1g, 0.003 mol) and Compound 30066-4 (0.99g, 0.006 mol) in toluene (20 mL) was heated to 60°C and stirred for 6 hours. The solution was evaporated under reduced pressure. A crude product was directly purified by flash silica gel column chromatography (PE/EA = 9/1), to obtain Compound 30066-5 (1g, 63.49%) as a yellow oil.

**Step 4: General Method for Preparation of Compound 30066-6**

A solution of Compound 30066-5 (250 mg, 0.739 mol) in EtOH (5 mL) was heated to 90°C and stirred for 5 hours.

The solvent was removed under reduced pressure. A crude product was directly purified by flash silica gel column chromatography (DCM/MeOH = 10/1), to obtain Compound 30066-6 (200 mg, 64.99%) as a yellow oil.

**Step 5: General Method for Preparation of Compound 30066-7**

A mixture of Compound 30066-6 (200 mg, 0.485 mmol) and $LiAlH_4$ (55 mg, 1.454 mmol) in THF (5 mL) was stirred at room temperature for 2 hours. The reaction was subjected to quenching with $H_2O$ (10 mL), followed by extraction with EA (15 mL x 3). The solution was evaporated to obtain Compound 30066-7 (160 mg, crude product) as a yellow oil.

**Step 6: General Method for Preparation of Compound 30066-8**

A solution of Compound 30066-7 (400 mg, 1.040 mmol) and m-CPBA (413 mg, 2.393 mmol) in DMF (4 mL) was stirred at room temperature for 2 hours. The reaction was subjected to quenching with $H_2O$ (20 mL), followed by extraction with EA (20 mL x 3). The **organic** layer was washed with brine (20 mL x 5), dried over $Na_2SO_4$, and concentrated, to obtain a crude product. The crude product was redissolved in dioxane (10 mL) and then $NH_4OH$ (365 mg, 10.403 mmol) was added. The resulting solution was stirred and heated at 110°C for 3 hours in a sealed tube. The reaction was subjected to quenching with $H_2O$ (200 mL), followed by extraction with EA (100 mL x 3). The crude product was evaporated and directly purified by flash silica gel column chromatography (DCM/MeOH = 10/1) to obtain crude product 30066-8 (200 mg, 53.85%) as a yellow solid. The crude product was further purified by preparative HPLC (column: Phenomenex Luna C18, 250 mm x 100 mm x 10 μm; mobile phase: [A: $H_2O$ (0.1% trifluoroacetic acid), B: acetonitrile]; B%: 20%-60%, 30 minutes) to obtain Compound 30066-8 (10 mg, 0.027 mmol) as a white solid.

LCMS: $[M+H]^+$ = 354.2; Rt = 0.956 min.

$^1$H NMR: (400 MHz, $d_6$-DMSO)

δ 9.457 - 9.431 (t, *J* = 10.4 Hz, 1H), δ 7.691 (s, 1H), 7.265 - 7.217 (m, 4H), 6.696 (s, 2H), 6.102 - 6.084 (d, *J* = 7.2 Hz, 1H), 5.145 (s, 1H), 5.032 (s, 2H), 4.458 - 4.449 (d, *J* = 3.6 Hz, 2H), 3.440 - 3.391 (m, 2H), 2.497 (m, 2H), 1.376 - 1.302 (m, 2H), 0.929 - 0.892 (m, 3H).

**Step 7: General Method for Preparation of Compound 30066-9**

A solution of Compound 30066-8 (170 mg, 0.481 mmol) and $SOCl_2$ (114 mg, 0.962 mmol) in DCM (2 mL) was stirred at room temperature for 15 hours. The reaction was subjected to quenching with $H_2O$ (200 mL), followed by extraction with EA (100 mL x 3). The solvent was removed under reduced pressure to obtain a yellow solid, Compound 30066-9 (180 mg, 85.53%).

**Step 8: General Method for Preparation of Compound 30066**

DIEA (36 mg, 0.282 mmol) was added to a solution of Compound 30066-9 (35 mg, 0.094 mmol) and Compound 30066-10 (16 mg, 0.141 mmol) in DMF (3 mL), and the mixture was stirred at 60°C for 6 hours. After filtration, the solution was directly purified by preparative HPLC (column: Phenomenex Luna C18, 250 mm x 100 mm x 10 μm; mobile phase: [A: $H_2O$ (0.1% formic acid), B: acetonitrile]; B%: 40%-80%, 40 min), to obtain Compound 30066 (10 mg, 22.95%) as a white solid.

LCMS: $[M+H]^+$ = 450.50; Rt = 1.557 min.

$^1$H NMR: (400 MHz, $d_6$-DMSO) δ 9.403 - 9.376 (t, *J* = 10.8 Hz, 1H), 8.235 (s, 1H), 7.260 - 7.193 (m, 4H), 6.616 (s, 2H), 6.084 - 6.066 (d, *J* = 7.2 Hz, 1H), 5.024 (s, 2H), 3.559 - 3.437 (m, 4H), 2.735 - 2.666 (m, 1H), 2.636 - 2.596 (m, 1H), 2.557 - 2.536 (m, 1H), 2.422 - 2.329 (m, 1H), 2.248 - 2.209 (m, 1H), 2.077 (s, 6H), 1.834 - 1.780 (m, 1H), 1.605 - 1.501 (m, 3H), 1.356 - 1.301 (m, 2H), 0.927 - 0.891 (t, *J* = 14.4 Hz, 3H).

The following compounds were prepared with reference to the synthetic method of Compound 30066.

| | |
|---|---|
| 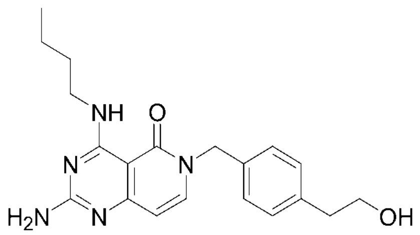<br>39 | $[M+H]^+$ = 368.3; Rt = 1.424 min. **$^1$H NMR:** (400 MHz, $d_6$-DMSO) $\delta$ 9.395 (t, *J* = 5.6 Hz, 1H), 7.671 (d, *J* = 7.6 Hz, 1H), 7.176 (s, 4H), 6.608 (s, 2H), 6.067 (d, *J* = 7.6 Hz, 1H), 5.001 (s, 2H), 4.616 (s, 1H), 3.548 (t, *J* = 7.2 Hz, 2H), 3.431 - 3.382 (m, 2H), 2.676 (s, 2H), 1.527 (dd, *J* = 14.8, 7.2 Hz, 2H), 1.344 (dd, *J* = 15.2, 7.2 Hz, 2H), 0.911 (t, *J* = 7.2 Hz, 3H). |
| 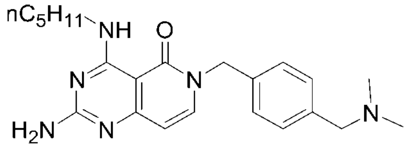<br>57 | $[M+H]^+$ = 395.40; Rt = 1.691 min. **$^1$H NMR:** (400 MHz, MeOD) $\delta$ 7.583 - 7.564 (d, *J* = 7.6 Hz, 1H), 7.335 - 7.287 (m, 4H), 6.192 - 6.173 (d, *J* = 7.6 Hz, 1H), 5.111 (s, 2H), 3.566 (s, 2H), 3.487 - 3.469 (m, 2H), 2.300 (s, 6H), 1.636 (s, 2H), 1.414 - 1.381 (d, *J* = 13.2 Hz, 4H), 0.952 - 0.917 (m, 3H). |
| 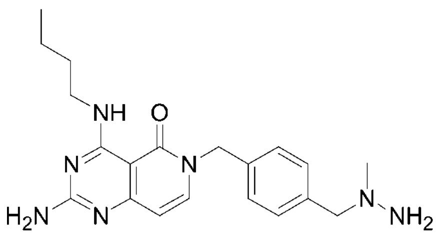<br>60 | $[M+H]^+$ = 382.1; Rt = 0.772 min. **$^1$H NMR:,** (400 MHz, $CD_3OD$) $\delta$ 9.709 (s, 1H), 7.664 (d, *J* = 7.6 Hz, 1H), 7.389 - 7.298 (m, 4H), 6.221 (d, *J* = 7.6 Hz, 1H), 5.132 (s, 2H), 3.806 (s, 2H), 3.554 - 3.499 (m, 2H), 2.539 (s, 3H), 1.656 - 1.601 (m, 2H), 1.461 - 1.422 (m, 2H), 0.976 (t, *J* = 4.8 Hz, 3H). |
| 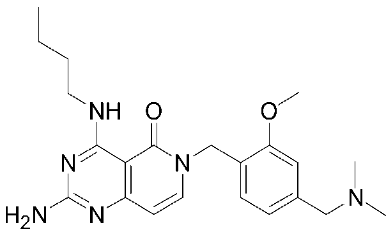<br>61 | $[M+H]^+$ = 411.1; Rt = 0.823 min. **$^1$H NMR:** (400 MHz, $CD_3OD$) $\delta$ 7.537 (d, *J* = 7.6 Hz, 1H), 7.121 (d, *J* = 7.6 Hz, 1H), 7.002 (s, 1H), 6.857 (d, *J* = 7.6 Hz, 1H), 6.150 (d, *J* = 7.6 Hz, 1H), 5.049 (s, 2H), 3.880 (s, 3H), 3.534 - 3.445 (m, 4H), 2.241 (s, 6H), 1.672 - 1.578 (m, 2H), 1.489 - 1.401 (m, 2H), 0.973 (t, *J* = 7.2 Hz, 3H). |
| 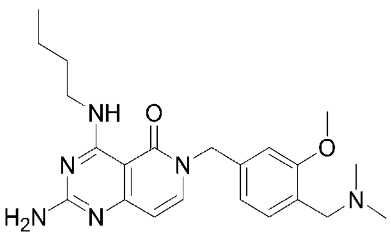<br>62 | $[M+H]^+$ = 411.1; Rt = 0.800 min. **$^1$H NMR:** (400 MHz, $d_6$-DMSO) $\delta$ 9.409 - 9.396 (d, *J* = 5.2 Hz, 1H), 8.205 (s, 2H), 7.671 (s, 1H), 7.213 (s, 1H), 6.765 (s, 1H), 6.746 (s, 1H), 6.616 (s, 2H), 6.087 - 6.068 (d, *J* = 7.6 Hz, 1H), 5.025 (s, 2H), 3.747 (s, 3H), 3.420 (s, 2H), 2.135 (s, 6H), 1.558 - 1.503 (m, *J* = 22.0 Hz, 2H), 1.359 - 1.322 (m, *J* = 14.8 Hz, 2H), 0.928 - 0.891 (d, *J* = 14.8 Hz, 3H). |

(continued)

| | |
|---|---|
| 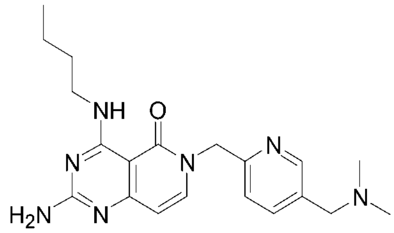<br>64 | $[M+H]^+$ = 382.4; Rt = 1.415 min. **$^1$H NMR:** (400 MHz, $CD_3OD$) $\delta$ 8.438 (d, $J$ = 2.0 Hz, 1H), 7.757 (dd, $J$ = 8.0, 2.0 Hz, 1H), 7.633 (d, $J$ = 7.6 Hz, 1H), 7.290 (d, $J$ = 8.0 Hz, 1H), 6.212 (d, $J$ = 7.6 Hz, 1H), 5.190 (s, 2H), 3.517 - 3.451 (m, 4H), 2.235 (s, 6H), 1.643 - 1.561 (m, 2H), 1.450 - 1.372 (m, 2H), 0.958 (t, $J$= 7.3 Hz, 3H). |
| 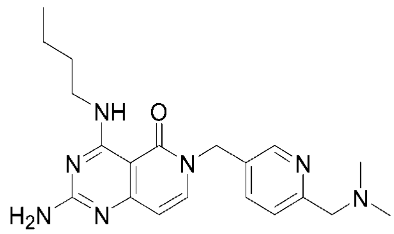<br>65 | $[M+H]^+$ = 382.35; Rt = 1.375 min. **$^1$H NMR:** (400 MHz, MeOD) $\delta$ 8.664 (d, $J$ = 1.6 Hz, 1H), 8.389 (s, 1.26H), 7.851 (dd, $J$ = 4.8, 5.6 Hz, 1H), 7.728 (d, $J$ = 7.6 Hz, 1H), 7.446 (d, $J$ = 8.0 Hz, 1H), 6.231 (d, $J$ = 7.2 Hz, 1H), 5.172 (s, 2H), 4.27 (s, 2H), 3.535 - 3.473 (m, 2H), 2.778 (s, 6H), 1.662 - 1.589 (m, 2H), 1.461 - 1.405 (m, 2H), 0.971 (t, $J$ = 7.4 Hz, 3H). |
| 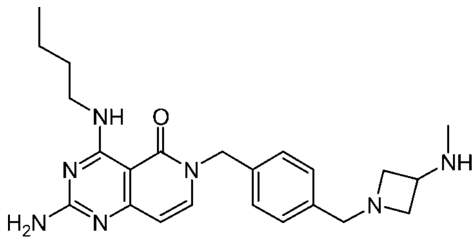<br>68 | $[M+H]^+$ = 422.8; Rt = 1.403 min. **$^1$H NMR:** (400 MHz, $CD_3OD$) $\delta$ 9.679 (br s, 1H), 7.637 (dd, $J$ = 9.2, 5.2 Hz, 1H), 7.324 (s, 4H), 6.211 (dd, $J$ = 7.6, 3.6 Hz, 1H), 5.113 (s, 2H), 3.831 (s, 2H), 3.707 (s, 3H), 3.550 - 3.500 (m, 2H), 3.358 (s, 2H), 2.511 (s, 3H), 1.689 - 1.589 (m, 2H), 1.490 - 1.400 (m, 2H), 0.978 (t, $J$ = 7.6 Hz, 3H). |
| 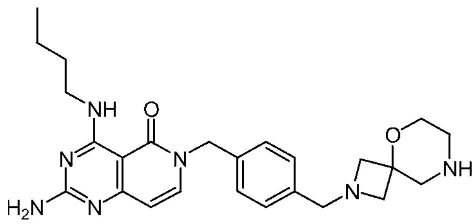<br>69 | $[M+H]^+$ = 464.45; Rt = 1.392 min. **$^1$H NMR:** (400 MHz, $d_6$-DMSO) $\delta$ 9.399 - 9.373 (t, $J$ = 10.4 Hz, 1H), 8.258 (s, 1H), 7.687 - 7.668 (d, $J$ = 7.6 Hz, 2H), 6.616 (s, 2H), 7.239 - 7.186 (m, 4H), 6.612 (s, 2H), 6.081 - 6.063 (d, $J$ = 7.2 Hz, 1H), 5.016 (s, 2H), 3.554 (s, 4H), 3.248 - 3.229 (d, $J$ = 7.6 Hz, 4H), 2.773 - 2.756 (d, $J$= 6.8 Hz, 4H), 2.588 - 2.577 (t, $J$ = 4.4 Hz, 2H), 1.572 - 1.500 (m, 2H), 1.393 - 1.301 (m, 2H), 0.928 - 0.891 (t, $J$ = 14.8 Hz, 3H). |
| 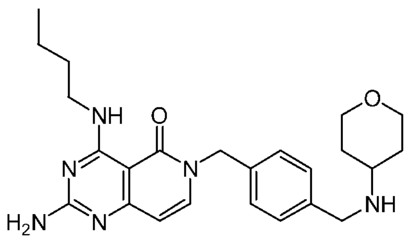<br>75 | $[M+H]^+$ = 437.45; Rt = 1.494 min. **$^1$H NMR:** (400 MHz, MeOD) $\delta$ 7.608 (d, $J$ = 7.6 Hz, 1H), 7.423 (dd, $J$ = 13.2 Hz, 8.0 Hz, 4H), 6.191 (d, $J$ = 7.6 Hz, 1H), 5.131 (s, 2H), 4.143 (s, 2H), 4.052 - 3.973 (m, 2H), 3.517 - 3.389 (m, 5H), 2.047- 2.020 (m, 2H), 1.684 - 1.551 (m, 4H), 1.465 - 1.409 (m, 2H), 0.974 (t, $J$ = 7.4 Hz, 3H). |

(continued)

| | |
|---|---|
| 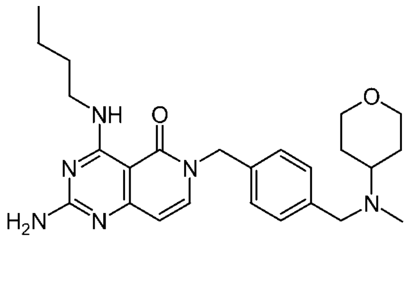 76 | $[M+H]^+$ = 451.85; Rt = 1.617 min. **$^1$H NMR:** (400 MHz, MeOD) $\delta$ 7.703 (d, *J* = 7.6 Hz, 1H), 7.424 (dd, *J* = 12.0, 8.0 Hz, 4H), 6.245 (d, *J* = 7.6 Hz, 1H), 5.160 (s, 2H), 4.15 - 4.011 (m, 4H), 3.535 - 3.508 (m, 2H), 3.442 - 3.387 (m, 2H), 3.313 - 3.297 (m, 1H), 2.549 (s, 3H), 1.995 - 1.961 (m, 2H), 1.826 - 1.784 (m, 2H), 1.671 -1.598 (m, 2H), 1.484 - 1.409 (m, 2H), 0.974 (t, *J*= 7.2 Hz, 3H). |
| 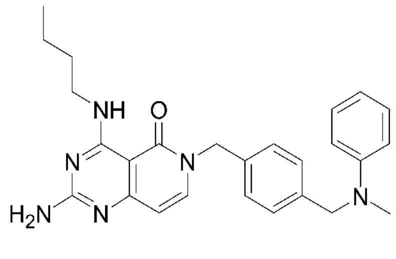 77 | $[M+H]^+$ = 443.45; Rt = 1.927 min. **$^1$H NMR:** (400 MHz, MeOD) $\delta$ 7.711 (d, *J* = 7.6 Hz, 1H), 7.240 (dd, *J* = 12.4, 8.0 Hz, 4H), 7.127 (dd, *J* = 1.6, 7.2 Hz, 2H), 6.722 (d, *J* = 8.4 Hz, 2H), 6.627 (t, *J* = 7.6 Hz, 1H), 6.223 (d, *J* = 7.6 Hz, 1H), 5.109 (s, 2H), 4.511 (s, 2H), 3.559 - 3.524 (m, 2H), 2.972 (s, 3H), 1.682 - 1.603 (m, 2H), 1.470 - 1.414 (m, 2H), 0.977 (t, *J* = 7.2 Hz, 3H). |
| 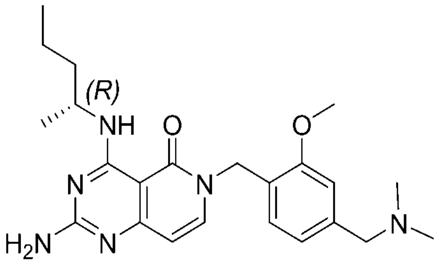 103 | 4.339-4.290 $[M+H]^+$ = 425.45; Rt = 1.699 min. **$^1$H NMR:** (400 MHz, $CD_3OD$) $\delta$ 7.632-7.613 (d, *J*= 7.6 Hz, 1H), 7.235-7.216 (d,*J* = 7.6 Hz, 1H), 7.102 (s, 1H), 6.985-6.966 (d, *J* = 7.6 Hz, 1H), 6.198-6.179 (d, *J* = 7.6 Hz, 1H), 5.119-5.033 (m, 2H), 4.339-4.290 (m, 2H), 3.988 (s, 3H), 3.916 (s, 2H), 2.627 (s, 6H), 1.593-1.487 (m, 2H), 1.419-1.293 (m, 2H), 1.226-1.210 (d, *J* = 6.4 Hz, 3H), 0.956-0.920 (t, *J* = 14.4 Hz, 3H). |
| 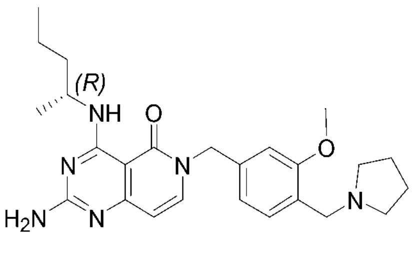 134 | $[M+H]^+$ = 451.10; Rt = 0.731 min. **$^1$H NMR:** (400 MHz, $CD_3OD$) $\delta$ 8.512 (s, 1H), 7.623 (dd, *J* = 7.6, 1.2 Hz, 1H), 7.378 (d, *J* = 7.6 Hz, 1H), 7.115 (s, 1H), 6.946 (d, *J* = 7.6 Hz, 1H), 6.208 (dd, *J* = 7.6, 1.2 Hz, 1H), 5.131 (s, 2H), 4.31 (s, 3H), 3.920 (d, *J* = 1.2 Hz, 3H), 3.295 - 3.222 (m, 4H), 2.067 (s, 4H), 1.554 (dd, *J* = 13.6, 6.4 Hz, 2H), 1.466 - 1.367 (m, 2H), 1.229 (d, *J* = 6.4 Hz, 3H), 0.946 (dd, *J* = 7.6, 6.8 Hz, 3H). |
| 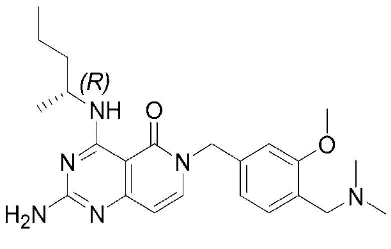 135 | $[M+H]^+$ = 425.40; Rt = 1.700 min. **$^1$H NMR:** (400 MHz, *$CD_3OD$*) $\delta$ 8.534 (s, 6H), 7.693 (d, *J* = 6.8 Hz, 1H), 7.369 (s, 1H), 7.112 (s, 1H), 6.951 (d, *J* = 7.2 Hz, 1H), 6.258 (d, *J* = 7.2 Hz, 1H), 5.153 (s, 2H), 4.366 - 4.272 (m, 1H), 4.225 (s, 2H), 3.913 (s, 3H), 2.774 (s, 6H), 1.649 - 1.516 (m, 2H), 1.472 - 1.357 (m, 2H), 1.234 (d, *J* = 6.4 Hz, 3H), 0.946 (t, *J* = 7.2 Hz, 3H). |

(continued)

| | |
|---|---|
| 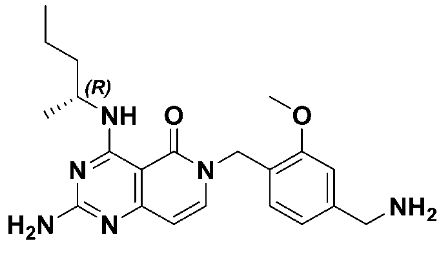 137 | $[M+H]^+$ 397.2.; Rt = 1.048 min. $^1H$ NMR (400 MHz, DMSO-d6) δ 9.32 - 9.30 (m, 1H), 8.33 (s, 1H), 7.57 - 7.55 (m, 1H), 7.16 (s, 1H), 6.92 (s, 2H), 6.60 (s, 2H), 6.08 - 6.06 (m, 1H), 5.00 - 4.91 (m, 2H), 4.21 - 4.19 (m, 1H), 3.87 - 3.84 (m, 5H), 1.52 - 1.31 (m, 4H), 1.14 - 1.13 (m, 3H), 0.89 - 0.86 (m, 3H). |
| 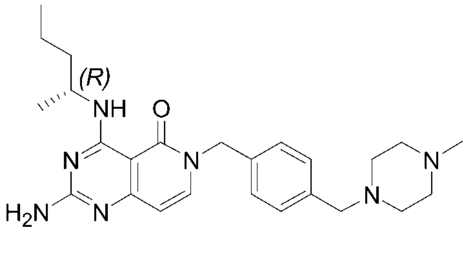 140 | $[M+H]^+$ = 450.25; Rt = 0.724 min. **$^1H$ NMR:** (400 MHz, *$CD_3OD$*) δ 8.111 (s, 0.44H), 7.612 (d, *J* = 7.6 Hz, 1H), 7.315 (q, *J* = 8.0 Hz, 4H), 6.196 (d, *J* = 7.6 Hz, 1H), 5.102 (q, *J* = 14.8 Hz, 2H), 4.304 (dd, *J* = 12.8, 6.0 Hz, 1H), 3.586 (s, 2H), 3.197 - 3.112 (m, 2H), 2.853 (s, 2H), 2.654 (s, 4H), 2.555 (s, 3H), 2.387 (s, 1H), 1.567 (dd, *J* = 14.0, 7.2 Hz, 2H), 1.456 - 1.393 (m, 2H), 1.234 (d, *J* = 6.4 Hz, 3H), 0.962 (d, *J* = 7.2 Hz, 3H). |
| 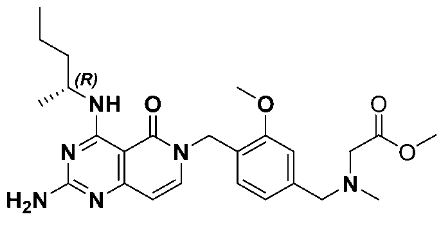 154 | $[M+H]^+$ 483.2.; Rt = 1.073 min.**$^1H$ NMR** (400 MHz, DMSO-*$d_6$*) δ 9.34 - 9.32 (m, 1H), 7.58 - 7.56 (m, 1H), 6.98 (s, 1H), 6.83 - 6.81 (m, 2H), 6.61 (s, 2H), 6.09 - 6.07 (m, 1H), 5.00 - 4.90 (m, 2H), 4.23 - 4.19 (m, 1H), 3.83 (s, 1H), 3.61 - 3.58 (m, 5H), 3.28 (s, 2H), 2.26 (s, 3H), 1.49 - 1.30 (m, 4H), 1.13 (s, 3H), 0.90 - 0.86 (m, 3H). |

**Example 2: Preparation of Compound 30018**

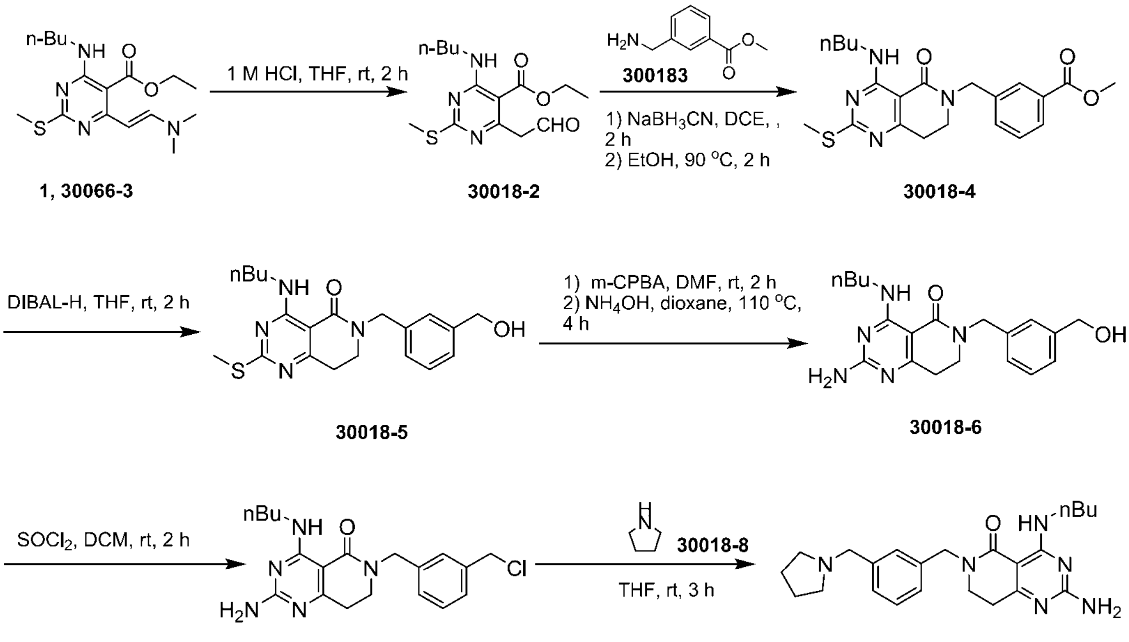


1, 30066-3 30018-2 30018-4 30018-5 30018-6 30018-7 M30018

### Step 1: General Method for Preparation of Compound 30018-2

At room temperature under a nitrogen atmosphere, HCl (1.6 mL, 1 M) was added dropwise to a THF (5 mL) solution of Compound 30066-3 (200 mg, 0.591 mmol). The reaction mixture was stirred at room temperature for 2 hours. The mixture was diluted with water (20 mL), extracted with EA (20 mL x 3), and evaporated under vacuum to obtain Compound 30018-2 (200 mg, crude product) as a yellow oil.

### Step 2: General Method for Preparation of Compound 30018-4

A DCE (10 mL) solution of Compound 30018-2 (770 mg, 2.47 mmol), Compound 30018-3 (490 mg, 2.97 mmol) and $NaBH_3CN$ (311 mg, 4.95 mmol) was stirred at room temperature for 2 hours. The mixture was extracted with EA (10 mL x 3), washed with water (10 mL x 3), dried over $Na_2SO_4$, and evaporated under vacuum to obtain the crude product of Compound 30018-4. A solution of the crude product of Compound 30018-4 in EtOH (20 mL) was heated and stirred at 90°C for 2 hours. The solvent was removed under reduced pressure. The crude product was purified by flash column chromatography (PE/EA = 3:1) to obtain Compound 30018-4 (270 mg, 85% purity, 22.39% yield) as a yellow oil.

### Step 3: General Method for Preparation of Compound 30018-5

A mixed solution of Compound 30018-4 (350 mg, 0.84 mmol) and DIBAL-H (240 mg, 1.69 mmol) in THF (5 mL) was stirred at room temperature for 2 hours. The mixture was diluted with water (10 mL), extracted with EA (10 mL x 3), washed with saturated brine (50 mL), dried over $Na_2SO_4$, and evaporated under vacuum. The crude product was purified by flash column chromatography (PE/EA = 1:1) to obtain Compound 30018-5 (175g, 85% purity, 45.59% yield) as a yellow oil.

### Other steps were performed to prepare Compound 30018 with reference to the synthetic method of Compound 30066.

$[M+H]^+$ =409.2; Rt = 0.756 min. **$^1$H NMR:** (400 MHz, $d_6$-DMSO) δ 9.034 (t, *J* = 5.6 Hz, 1H), 8.193 (s, 0.1H), 7.215 (ddd, *J* = 24.4, 15.6, 7.6 Hz, 4H), 6.627 (s, 2H), 4.575 (s, 2H), 3.556 (s, 2H), 3.421 - 3.195 (m, 4H), 2.643 (t, *J* = 6.8 Hz, 2H), 2.414 (s, 4H), 1.678 (dt, *J* = 6.4, 3.2 Hz, 4H), 1.512 (dt, *J* = 14.8, 7.2 Hz, 2H), 1.343 (dq, *J* = 14.4, 7.2 Hz, 2H), 0.908 (t, *J* = 7.4 Hz, 3H).

### Example 3: Preparation of Compound 30042

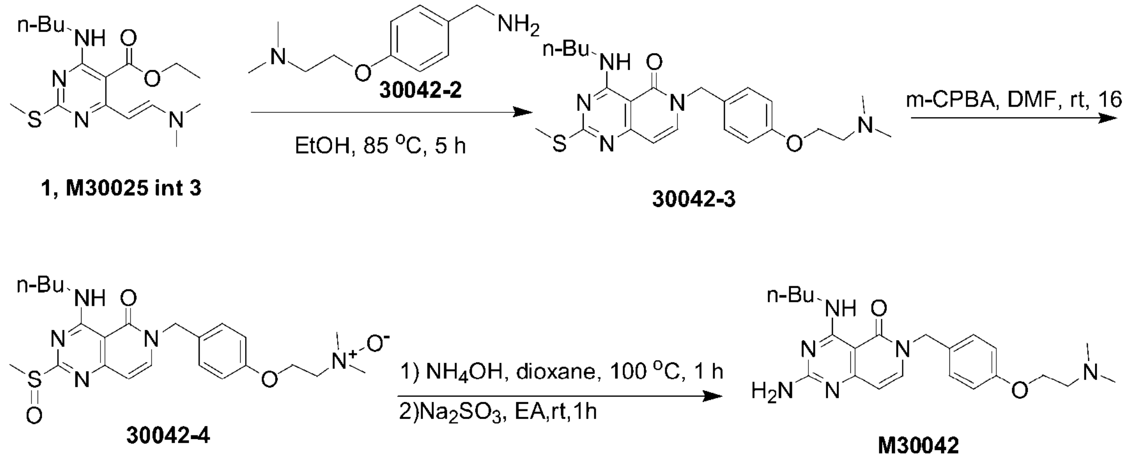


1, M30025 int 3

30042-2

30042-3

30042-4

M30042

**Step 1: General Method for Preparation of Compound 30042-3**

A mixture of Compound 30025-3 (300 mg, 0.89 mmol) and Compound 30042-2 (207 mg, 1.06 mmol) in EtOH (6 mL) was heated to 85°C and stirred for 5 hours. The reaction was subjected to quenching with $H_2O$ (200 mL), followed by extraction with ethyl acetate (100 mL x 3). The mixture was evaporated under vacuum to obtain Compound 30042-3 (360 mg, crude product) as a brown oil.

**Step 2: General Method for Preparation of Compound 30042-4**

A mixture of Compound 30042-3 (360 mg, 0.82 mmol) and m-CPBA (414 mg, 2.04 mmol, 85%) in DMF (3 mL) was stirred at room temperature for 16 hours. The mixture was diluted with water (20 mL), extracted with EA (1 mL x 2), and evaporated under vacuum to obtain Compound 30042-4 (280 mg, crude product) as a brown oil.

**Step 3: General Method for Preparation of Compound 30042**

A mixture of Compound 30042-4 (250 mg, 0.53 mmol) and $NH_4OH$ (2 mL) in 1,4-dioxane (1 mL) was heated to 100°C and stirred for 1 hour. The mixture was extracted with ethyl acetate (5 mL x 2) and concentrated under reduced pressure. The residue was dissolved in EA (2 mL) and then a saturated solution (2 mL) of $Na_2SO_3$ was added. The resulting mixture was stirred vigorously for 1 hour, and then the organic phase was separated, concentrated under reduced pressure and purified by preparative HPLC (column: Phenomenex Luna C18, 250 mm x 100 mm x 10 μm; mobile phase: [A: $H_2O$ (0.1% trifluoroacetic acid), B: acetonitrile]; B%: 10%-50%, 40 min), to obtain Compound 30042 (12.35 mg, 6% yield) as a white solid.

LCMS: $[M+H]^+$ = 411.1; Rt = 0.886 min.

$^1$H NMR: (400 MHz, $CDCl_3$) δ 9.797 (br s, 1H), 7.293 (d, *J* = 7.6 Hz, 1H), 7.212 (d, *J* = 8.4 Hz, 2H), 6.899 (d, *J* = 8.4 Hz, 2H), 6.314 (d, *J* = 7.6 Hz, 1H), 5.026 (s, 2H), 4.189 (t, *J* = 5.2 Hz, 2H), 3.516 (dd, *J* = 12.8, 7.2 Hz, 2H), 2.982 - 2.901 (m, 2H), 2.507 (s, 6H), 1.678 - 1.613 (m, 2H), 1.456 - 1.404 (m, 2H), 0.965 (t, *J* = 7.2 Hz, 3H).

The following compounds were prepared with reference to the synthetic method of Compound 30042. Compound 30019 was obtained by reduction of Compound 30027 ($Pd(OH)_2$/C, $H_2$, dioxane, EtOH, rt, 15 hours).

| | |
|---|---|
| 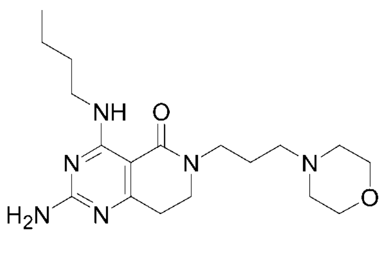<br>19 | $[M+H]^+$ = 363.2; Rt = 1.034 min. **$^1$H NMR:** (400 MHz, $d_4$-MeOD) δ 3.879 (s, 4H), 3.555 (d, *J* = 8.0 Hz, 4H), 3.452 (d, *J* = 8.0 Hz, 2H), 3.150 - 3.030 (m, 6H), 2.839 (s, 2H), 2.030 - 2.012 (dd, *J* = 8.0 Hz, 2H), 1.517 (d, *J* = 8.4 Hz, 2H), 1.420 (d, *J* = 8.4 Hz, 2H), 0.982 - 0.945 (t, *J* = 7.6 Hz, 3H). |

(continued)

| | |
|---|---|
| 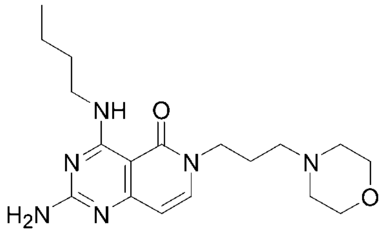<br>27 | $[M+H]^+$ = 361.2; Rt = 0.655 min. **$^1$H NMR:** (400 MHz, $d_6$-DMSO) δ 10.183 (s, 1H), 8.045 - 8.027 (dd, *J* = 8.0 Hz, 2H), 6.433 - 6.415 (dd, *J* = 8.0 Hz, 2H), 4.042 - 4.008 (m, 4H), 3.635 - 3.512 (m, 4H), 3.404 (s, 2H), 3.047 (d, *J* = 12.0 Hz, 4H), 2.063 (s, 1H), 1.556 (t, *J* = 7.6 Hz, 2H), 1.340 (t, *J* = 6.4 Hz, 2H), 0.929 - 0.911 (t, *J* = 7.6 Hz, 3H). |
| 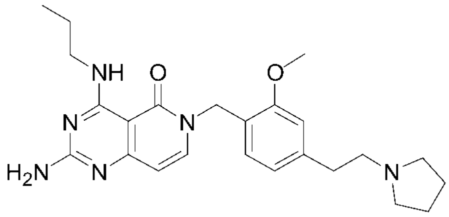<br>92 | $[M+H]^+$ = 451.5; Rt = 1.807 min. **$^1$H NMR:** (400 MHz, MeOD) δ 7.534 (d, *J* = 7.6 Hz, 1H), 7.133 (d, *J* = 7.6 Hz, 1H), 6.917 (s, 1H), 6.824 (d, *J* = 6.8 Hz, 1H), 6.144 (d, *J* = 7.6 Hz, 1H), 5.032 (s, 2H), 3.871 (s, 3H), 3.496 (dd, *J* = 8.8, 5.2 Hz, 2H), 2.992 - 2.857 (m, 8H), 1.913 (d, *J* = 2.8 Hz, 4H), 1.666 - 1.594 (m, 2H), 1.449 (dd, *J* = 15.2, 7.5 Hz, 2H), 0.987 (t, *J* = 7.6 Hz, 3H). |
| 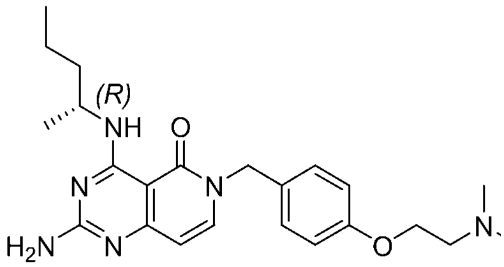<br>106 | $[M+H]^+$ = 425.25; Rt = 1.671 min. **$^1$H NMR:** (400 MHz, $CD_3OD$) δ 8.027-8.008 (d, *J* = 7.6 Hz, 1H), 7.390-7.369 (d, *J* = 8.4 Hz, 2H), 7.029-7.007 (d, *J* = 8.8 Hz, 2H), 6.391-6.372 (d, *J* = 7.6 Hz, 1H), 5.192-5.101 (m, 2H), 4.452-4.382 (m, 1H), 4.346-4.321 (t, *J* = 10 Hz, 2H), 3.593-3.569 (t, *J* = 9.6 Hz, 2H), 2.967 (s, 6H), 1.669-1.553 (m, 2H), 1.454-1.386 (m, 2H), 1.289-1.273 (d, *J* = 6.4 Hz, 3H), 0.978-0.941 (t, *J*= 14.8 Hz, 3H). |
| 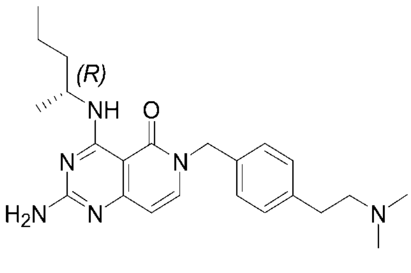<br>124 | $[M+H]^+$ = 409.4; Rt = 1.697 min. **$^1$H NMR:** (400 MHz, $CD_3OD$) δ 7.553 (d, *J* = 7.6 Hz, 1H), 7.255-7.201 (m, 4H), 6.177 (d, *J* = 7.6 Hz, 1H), 5.115-5.015 (m, 2H), 4.336-4.286 (m, 1H), 2.813-2.777 (m, 2H), 2.598 (s, 2H), 2.330 (s, 6H), 1.605-1.509 (m, 2H), 1.471-1.401 (m, 2H), 1.234 (d, *J* = 6.4 Hz, 3H), 0.966 (t, *J* = 14.4 Hz, 3H). |
| 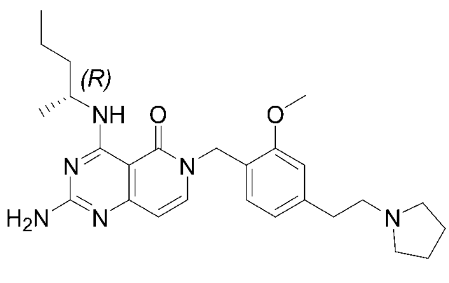<br>125 | $[M+H]^+$ = 465.5; Rt = 1.826 min. **$^1$H NMR:** (400 MHz, MeOD) δ 8.532 (s, 1H), 7.575 (d, *J* = 7.6 Hz, 1H), 7.184 (d, *J* = 7.6 Hz, 1H), 6.956 (s, 1H), 6.861 (d, *J* = 7.6 Hz, 1H), 6.155 (d, *J* = 7.6 Hz, 1H), 5.038 (d, *J* = 4.4 Hz, 2H), 4.345 - 4.263 (m, 1H), 3.897 (s, 3H), 3.358 (d, *J* = 6.4 Hz, 2H), 3.290 - 3.243 (m, 4H), 3.048 - 2.987 (m, 2H), 2.045 (s, 4H), 1.543 (dd, *J* = 13.6, 6.4 Hz, 2H), 1.418 (dt, *J* = 13.6, 7.2 Hz, 2H), 1.224 (d, *J* = 6.4 Hz, 3H), 0.946 (t, *J* = 7.2 Hz, 3H). |

(continued)

| | |
|---|---|
| 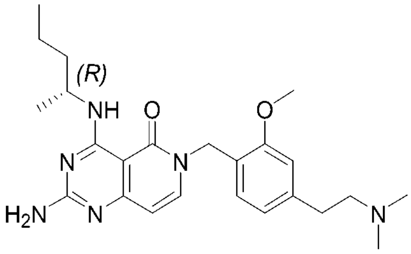 126 | [M+H] + = 439.5; Rt = 1.728 min.**1H NMR:** (400 MHz, MeOD) δ 8.002 (d, *J* = 7.6 Hz, 1H), 7.333 (d, *J* = 7.6 Hz, 1H), 6.987 (s, 1H), 6.905 (d, *J* = 7.6 Hz, 1H), 6.357 (d, *J* = 7.6 Hz, 1H), 5.123 (d, *J* = 2.8 Hz, 2H), 4.444 - 4.369 (m, 1H), 3.897 (s, 3H), 3.383 (dd, *J* = 9.6, 6.8 Hz, 2H), 3.056 (dd, *J* = 9.6, 6.8 Hz, 2H), 2.934 (s, 6H), 1.601 (dd, *J* = 13.4, 8.0 Hz, 2H), 1.440 - 1.372 (m, 2H), 1.273 (d, *J* = 6.4 Hz, 3H), 0.957 (t, *J* = 7.2 Hz, 3H). |
| 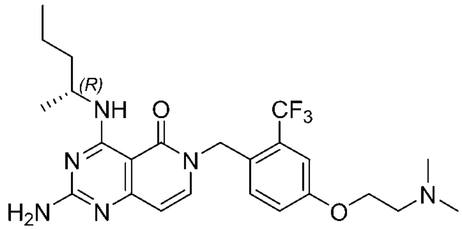 146 | [M+H] + = 493.3; Rt = 1.000 min. **1H NMR** 1H NMR (400 MHz, DMSO-$d_6$) δ 9.22 (d, *J* = 8.2 Hz, 1H), 7.57 (d, *J* = 7.6 Hz, 1H), 7.28 (d, *J* = 2.6 Hz, 1H), 7.20 (dd, *J* = 8.8, 2.4 Hz, 1H), 6.86 (d, *J* = 8.8 Hz, 1H), 6.66 (s, 2H), 6.13 (d, *J* = 7.6 Hz, 1H), 5.16 (q, *J* = 16.0 Hz, 2H), 4.28 - 4.17 (m, 1H), 4.10 (t, *J* = 5.8 Hz, 2H), 2.60 (t, *J* = 5.8 Hz, 2H), 2.19 (s, 6H), 1.46 (tt, *J* = 13.6, 6.8 Hz, 2H), 1.34 - 1.26 (m, 2H), 1.14 (d, *J* = 6.6 Hz, 3H), 0.87 (t, *J* = 7.4 Hz, 3H). |
| 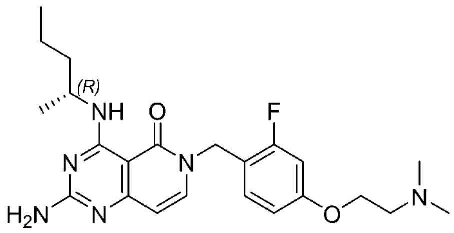 148 | [M+H] + = 443.3; Rt = 1.109 min. **1H NMR** (400 MHz, DMSO-$d_6$) δ 9.32 (d, *J* = 8.2 Hz, 1H), 7.65 (d, *J* = 7.6 Hz, 1H), 7.22 - 7.08 (m, 1H), 6.96 (dd, *J* = 11.4, 2.4 Hz, 1H), 6.73 (td, *J* = 8.6, 2.4 Hz, 1H), 6.56 (s, 2H), 6.03 (d, *J* = 7.6 Hz, 1H), 4.92 (q, *J* = 14.8 Hz, 2H), 4.21 (dt, *J* = 13.8, 6.8 Hz, 1H), 4.08 (t, *J* = 5.6 Hz, 2H), 2.68 - 2.62 (m, 2H), 2.22 (s, 6H), 1.49 (ddd, *J* = 14.6, 8.9, 5.8 Hz, 2H), 1.32 (dt, *J* = 13.8, 6.8 Hz, 2H), 1.14 (d, *J* = 6.6 Hz, 3H), 0.88 (t, *J* = 7.4 Hz, 3H). |

The following compounds were prepared with reference to the synthetic method of Compound 30042.

| | |
|---|---|
| 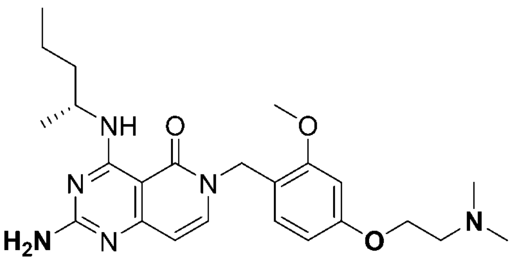 142 | [M+H] + = 425.25; Rt = 1.671 min. **1H NMR** (400 MHz, DMSO-$d_6$) δ 9.35 (d, *J*= 8.2 Hz, 1H), 8.17 (s, 2H), 7.52 (d, *J* = 7.6 Hz, 1H), 6.94 (d, *J* = 8.4 Hz, 1H), 6.63 - 6.53 (m, 3H), 6.49 (dd, *J* = 8.4, 2.4 Hz, 1H), 6.04 (d, *J* = 7.6 Hz, 1H), 4.88 (q, *J* = 14.8 Hz, 2H), 4.24 - 4.18 (m, 1H), 4.05 (t, *J* = 6.0 Hz, 2H), 3.81 (s, 3H), 2.67 (t, *J* = 5.6 Hz, 2H), 2.25 (s, 6H), 1.52 - 1.45 (m, 2H), 1.37 - 1.27 (m, 2H), 1.17 - 1.15 (m, 3H), 0.88 (t, *J* = 7.2 Hz, 3H). |
| 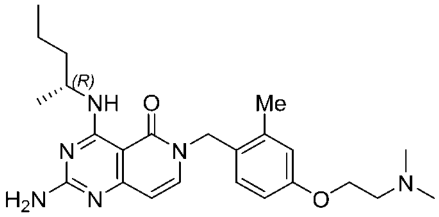 145 | 4.452-4.382 [M+H] + = 440.3; Rt = 1.084 min. **1H NMR**(400 MHz, $CD_3OD$) δ 8.027-8.008 (d, *J* = 7.6 Hz, 1H), 7.390-7.369 (d, *J* = 8.4 Hz, 2H), 7.029-7.007 (d, *J* = 8.8 Hz, 2H), 6.391-6.372 (d, *J* = 7.6 Hz, 1H), 5.192-5.101 (m, 2H), 4.452-4.382 (m, 1H), 4.346-4.321 (t, *J* = 10 Hz, 2H), 3.593-3.569 (t, *J* = 9.6 Hz, 2H), 2.967 (s, 6H), 1.669-1.553 (m, 2H), 1.454-1.386 (m, 2H), 1.289-1.273 (d, *J* = 6.4 Hz, 3H), 0.978-0.941 (t, *J*= 14.8 Hz, 3H). |

(continued)

| | |
|---|---|
| 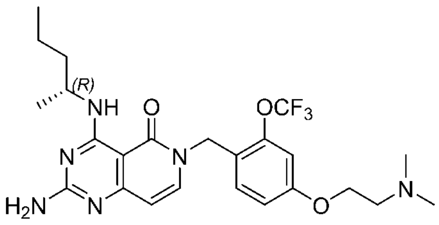<br>153 | [M+H] + = 509.3 |
| 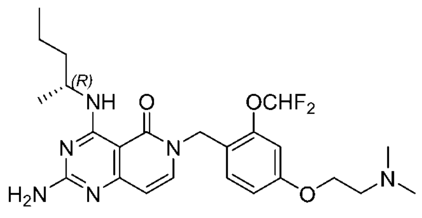<br>157 | [M+H] + = 491.3 |

**Example 4: Preparation of Compound 30043**

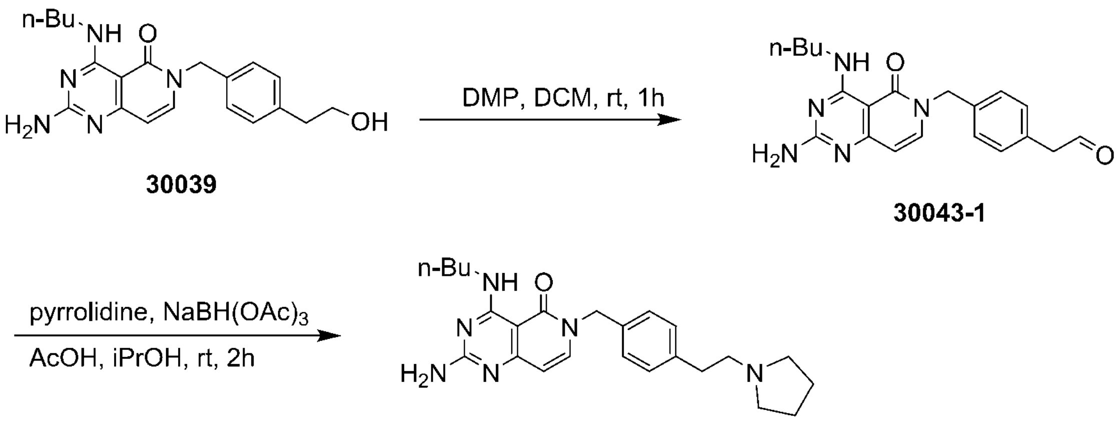


30039

30043-1

30043

**Step 1: General Method for Preparation of Compound 30043-1**

A solution of Compound 30039 (80 mg, 0.22 mmol) and compound DMP (185 mg, 0.44 mmol) in DCM (3 mL) was stirred at room temperature for 1 hour. The solution was concentrated under reduced pressure. A crude product was directly purified by flash silica gel column chromatography (DCM/MeOH = 95/5), to obtain Compound 30043-1 (80 mg, 91% yield) as a yellow oil.

**Step 2: General Method for Preparation of Compound 30043**

Sodium triacetoxyborohydride (43 mg, 0.21 mmol) was added to an isopropanol (2 mL) solution of Compound 30043-1 (50 mg, 0.14 mmol), pyrrolidine (12 mg, 0.16 mmol) and AcOH (12 mg, 0.21 mmol) at room temperature. The mixture was stirred for 2 hours at room temperature. After filtration, the solution was directly purified by preparative HPLC (column: Phenomenex Luna C18, 250 mm × 100 mm × 10 μm; mobile phase: [A: $H_2O$ (0.1% formic acid), B: acetonitrile]; B%: 10%-50%, 40 min), to obtain Compound 30043 (7.16 mg, 15% yield) as a white solid.

**LCMS:** [M+H] + = 421.1; Rt = 0.878 min.

**$^1$H NMR:** (400 MHz, $CD_3OD$) $\delta$ 7.562 (d, *J* = 7.6 Hz, 1H), 7.289 - 7.221 (m, 4H), 6.171 (d, *J* = 7.6 Hz, 1H), 5.077 (s, 2H), 3.522 - 3.467 (m, 2H), 3.001 - 2.931 (m, 2H), 2.93 - 2.84 (m, 6H), 1.954 - 1.883 (m, 4H), 1.656 - 1.597 (m, 2H), 1.480 -

1.412 (m, 2H), 0.978 (t, *J* = 7.2 Hz, 3H).

The following compounds were prepared with reference to the synthetic method of Compound 30043.

| | |
|---|---|
| 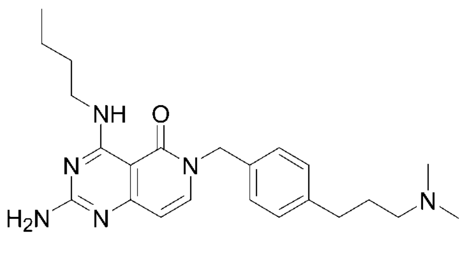<br>55 | [M+H]+ = 409.40; Rt = 1.669 min. **$^1$H NMR:** (400 MHz, MeOD) $\delta$ 7.565 (d, *J* = 7.2 Hz, 1H), 7.237 (dd, *J* = 8.0 Hz, 10.8 Hz, 4H), 6.174 (d, *J* = 7.2 Hz, 1H), 5.073 (s, 2H), 3.531 - 3.492 (m, 2H), 2.891 - 2.844 (m, 2H), 2.662 (m, 8H), 1.961 - 1.914 (m, 2H), 1.661 - 1.613 (m, 2H), 1.451 (m, 2H), 0.980 (t, *J* = 7.2 Hz, 3H). |
| 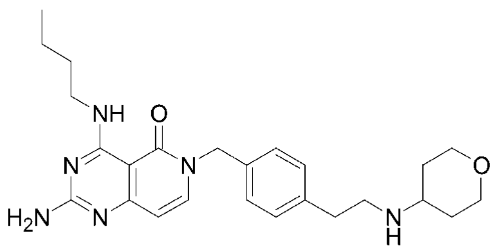<br>78 | [M+H] + = 451.40; Rt = 1.529 min. **$^1$H NMR:,** (400 MHz, MeOD) $\delta$ 7.610 (d, *J* = 7.6 Hz, 1H), 7.301 (q, *J* = 8.0 Hz, 4H), 6.189 (d, *J* = 7.6 Hz, 1H), 5.095 (s, 2H), 4.002 (dd, *J* = 11.6, 4.4 Hz, 2H), 3.503 (dd, *J* = 8.8, 5.2 Hz, 2H), 3.413 (dd, *J* = 12.0, 10.4 Hz, 2H), 3.233 (m, *J* = 21.2 Hz, 3H), 2.958 (m, 2H), 1.977 (m, 2H), 1.668 (m, 4H), 1.452 (dd, *J* = 15.2, 7.2 Hz, 2H), 0.978 (t, *J* = 7.2 Hz, 3H). |
| 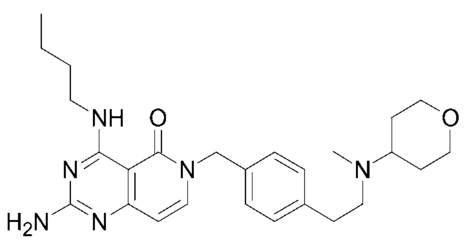<br>79 | [M+H]+ = 465.40; Rt = 1.619 min. **$^1$H NMR:** (400 MHz, MeOD) $\delta$ 7.639 (d, *J* = 7.6 Hz, 1H), 7.309 (s, 4H), 6.204 (d, *J* = 7.2 Hz, 1H), 5.076 (s, 2H), 4.032 (d, *J* = 8.0 Hz, 2H), 3.474 (dd, *J* = 14.8, 8.0 Hz, 3H), 3.376 (t, *J* = 11.2 Hz, 4H), 3.010 (m, 2H), 2.854 (s, 3H), 1.930 (m, 2H), 1.654 (m, *J* = 7.6 Hz, 2H), 1.600 (dd, *J* = 14.8, 7.2 Hz, 2H), 1.425 (dd, *J* = 14.8, 7.2 Hz, 2H), 0.979 (s, 3H). |
| 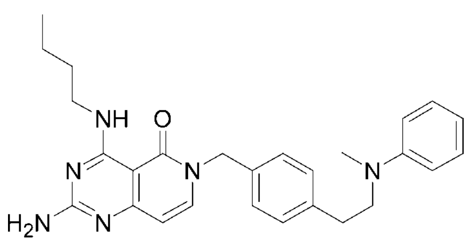<br>80 | [M+H]+ = 457.45; Rt = 1.973 min. **$^1$H** NMR: (400 MHz, MeOD) $\delta$ 7.565 (d, *J* = 7.6 Hz, 1H), 7.242 (m, *J* = 8.0 Hz, 4H), 7.123 (m, 2H), 6.707 (d, *J* = 8.0 Hz, 2H), 6.606 (t, *J* = 7.2 Hz, 1H), 6.184 (d, *J* = 7.6 Hz, 1H), 5.076 (s, 2H), 3.516 (m, 4H), 2.843 (s, 3H), 2.810 (m, 2H), 1.626 (dd, *J* = 14.8, 7.2 Hz, 2H), 1.452 (dd, *J* = 15.2, 7.6 Hz, 2H), 0.982 (t, *J* = *7.2* Hz, 3H). |
| 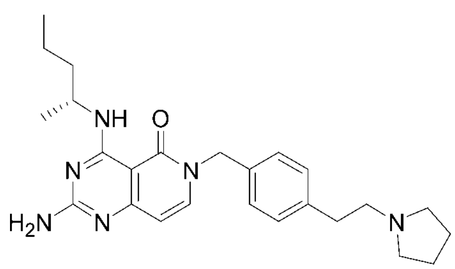<br>91 | **SFC:** Rt = 10.788 min, 214 nm > 99.9% purity, 254 nm > 99.9% purity. **LCMS:** [M+H]+ = 435.2; Rt = 0.867 min. **$^1$H NMR:** (400 MHz, $CD_3OD$) $\delta$ 9.492 (d, *J* = 7.6 Hz, 1H), 7.545 (d, *J* = 7.6 Hz, 1H), 7.267 - 7.186 (m, 4H), 6.168 (d, *J* = 7.6 Hz, 1H), 5.130 - 5.003 (m, 2H), 4.351 - 4.255 (m, 1H), 2.856 - 2.798 (m, 2H), 2.779 - 2.614 (m, 6H), 1.874 - 1.801 (m, 4H), 1.609 - 1.525 (m, 2H), 1.464 - 1.375 (m, 2H), 1.226 (d, *J* = 6.4 Hz, 3H), 0.948 (t, *J* = 7.2 Hz, 3H). |

(continued)

| | |
|---|---|
| 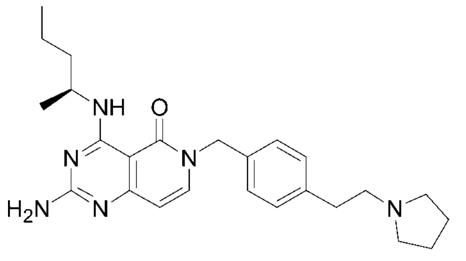<br>95 | **SFC:** Rt = 13.657 min, 214 nm = 99.6% purity, 254 nm = 99.6% purity. $[M+H]^+$ = 435.2; Rt = 0.879 min. **$^1$H NMR:** (400 MHz, $CD_3OD$) $\delta$ 9.490 (d, *J* = 8.0 Hz, 1H), 7.544 (d, *J* = 7.6 Hz, 1H), 7.257 - 7.200 (m, 4H), 6.168 (d, *J* = 7.6 Hz, 1H), 5.125 - 4.996 (m, 2H), 4.341 - 4.245 (m, 1H), 2.861 - 2.804 (m, 2H), 2.789 - 2.712 (m, 2H), 2.702 - 2.628 (m, 4H), 1.867 - 1.801 (m, 4H), 1.611 - 1.511 (m, 2H), 1.468 - 1.374 (m, 2H), 1.225 (d, *J* = 6.4 Hz, 3H), 0.947 (t, *J* = 7.2 Hz, 3H). |

**Example 5: Preparation of Compounds 30058, 30089 and 30090**

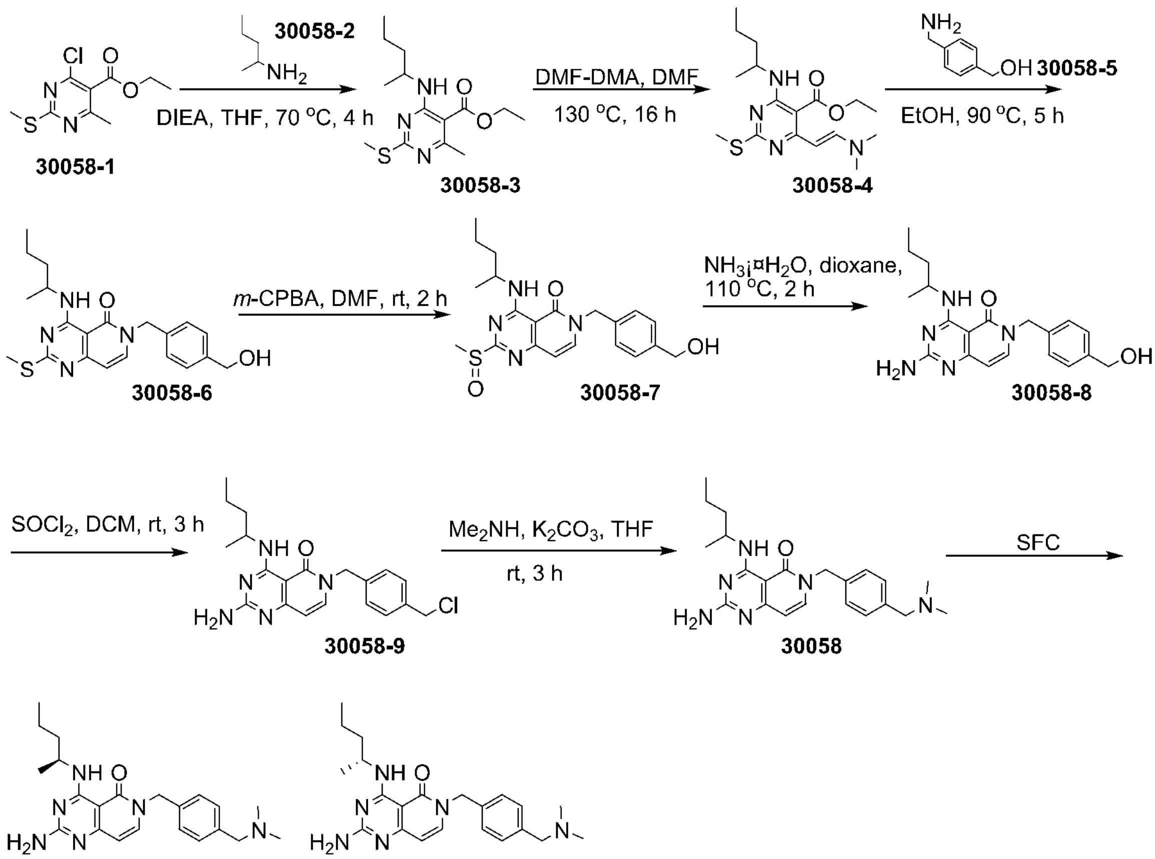


**Step 1: General Method for Preparation of Compound 30058-3**

At room temperature under a nitrogen atmosphere, N,N-diisopropylethylamine (DIEA, 786 mg, 6.080 mmol) and Compound 30058-2 (212 mg, 2.432 mmol) were added dropwise to a THF (15 mL) solution of Compound 30058-1 (500 mg, 2.027 mmol). The reaction mixture was heated and stirred at 70°C for 4 hours. The solvent was removed under reduced pressure to obtain the crude product. The crude product was purified by flash column chromatography (MeOH/DCM = 2%) to obtain Compound 30058-3 (440 mg, 1.479 mmol, 72.96% yield) as a yellow solid.

**Step 2: General Method for Preparation of Compound 30058-4**

At room temperature under a nitrogen atmosphere, N,N-dimethylformamide dimethyl acetal (DMF-DMA, 384.65 mg, 3.228 mmol) was added dropwise to a DMF (5 mL) solution of compound 30058-3 (240 mg, 0.807 mmol). The reaction

mixture was heated and stirred at 130°C for 16 hours. The reaction was subjected to quenching with $H_2O$ (2 mL), followed by addition of DCM (100 mL) for dilution, and then the resulting mixture was washed with water and brine. The organic layer was collected, dried over $Na_2SO_4$, filtered and concentrated. The crude product was purified by flash column chromatography (MeOH/DCM = 3%) to obtain Compound 30058-4 (218 mg, 0.618 mmol, 76.58% yield) as a yellow solid.

**Step 3: General Method for Preparation of Compound 30058-6**

At room temperature under a nitrogen atmosphere, Compound 30058-5 (177 mg, 1.288 mmol) was added to an EtOH (5 mL) solution of Compound 30058-4 (218 mg, 0.618 mmol). The reaction mixture was heated and stirred at 90°C for 5 hours. The solvent was removed under reduced pressure to obtain a residue. The crude product was purified by flash column chromatography (MeOH/DCM = 10%) to obtain Compound 30058-6 (200 mg, 0502 mmol, 81.23% yield) as a yellow solid.

**Step 4: General Method for Preparation of Compound 30058-7**

A solution of Compound 30058-6 (200 mg, 0.502 mmol) and m-CPBA (260 mg, 1.506 mmol) in DMF (4 mL) was stirred at room temperature for 2 hours. The reaction solution was subjected to quenching with water (20 mL), followed by extraction with EA (20 mL × 3). The organic layer was collected, washed with water (20 mL × 5), dried over $Na_2SO_4$, and concentrated. The crude product was purified by flash column chromatography (MeOH/DCM = 6%) to obtain Compound 30058-7 (150 mg, 0.362 mmol, 72.11% yield) as a yellow solid.

**Step 5: General Method for Preparation of Compound 58-8**

A solution of Compound 30058-7 (150 mg, 0.362 mmol) in 1,4-dioxane/$NH_3 \cdot H_2O$ (1:2, 5 mL) was heated to 110°C and stirred for 2 hours. The solvent was removed under reduced pressure. The crude product was purified by flash column chromatography (MeOH/DCM = 10%) to obtain Compound 30058-8 (100 mg, 0.272 mmol, 75.14% yield) as a yellow solid.

**Step 6: General Method for Preparation of Compound 30058-9**

At room temperature under a nitrogen atmosphere, $SOCl_2$ (49 mg, 0.408 mmol) was added dropwise to a DCM (5 mL) solution of Compound 30058-8 (100 mg, 0.272 mmol). The reaction mixture was stirred at room temperature for 3 hours. The solvent was removed under reduced pressure. The crude product was purified by flash column chromatography (MeOH/DCM = 3%) to obtain Compound 30058-9 (70 mg, 0.181 mmol, 66.54% yield) as a yellow solid.

**Step 7: General Method for Preparation of Compound 30058**

A mixture of Compound 30058-9 (70 mg, 0.181 mmol), $Me_2NH$ (41 mg, 0.907 mmol) and $K_2CO_3$ (75 mg, 0.544 mmol) in THF (5 mL) was stirred at room temperature under a nitrogen atmosphere for 3 hours. The solvent was removed under reduced pressure. Then the crude product was purified by preparative HPLC (column: Phenomenex Luna C18, 250 mm × 100 mm × 10 μm; mobile phase: [A: $H_2O$ (0.1% trifluoroacetic acid), B: acetonitrile]; B%: 40%-80%, 40 min), to obtain Compound 30058 (25 mg, 0.062 mmol, 34.23% yield) as a white solid.

LCMS: $[M+H]^+$ = 395.45; Rt = 1.655 min.

$^1$H NMR: (400 MHz, MeOD) δ 8.420 (s, 1H), 7.748 - 7.729 (d, J = 7.6 Hz, 1H), 7.480 - 7.420 (dd, J = 8.4, 15.6 Hz, 4H), 6.278 - 6.259 (d, J = 7.6 Hz, 1H), 5.221 - 5.124 (dd, J = 15.2, 23.6 Hz, 2H), 4.353 - 4.317 (m, 1H), 4.200 (s, 2H), 2.764 (s, 6H), 1.606 - 1.516 (m, 2H), 1.443 - 1.374 (m, 2H), 1.241 - 1.225 (d, J = 6.4 Hz, 3H), 0.962 - 0.926 (t, J = 7.2 Hz, 3H).

**Step 8: General Method for Preparation of Compounds 30089 and 30090**

Compound 30058 was separated by supercritical fluid chromatography (SFC) to obtain Compounds 30089 and 30090.

Column: DAICEL IG 4.6 mm I.D.*250 mL 5 μm;

Mobile phase: $CO_2$/MeOH [0.1% $NH_3$ (7 M Solution in MeOH)];

Temperature: 4°C;

Flow rate: 2.5 mL/min;

Device: SHIMADZU LC-30AD SFC

SFC: Compound 30089, Rt = 5.929 min.

LCMS: $[M+H]^+$ = 395.55; Rt = 1.659 min.

$^1H$ NMR: (400 MHz, MeOD) δ 8.420 (s, 1H), 7.708 (s, 1H), 7.455-7.416 (t, *J* = 15.6 Hz, 4H), 6.267-6.248 (d, *J* = 7.6 Hz, 1H), 5.157-5.119 (m, 2H), 4.347- 4.316 (m, 1H), 4.187 (s, 2H), 2.755 (s, 6H), 1.586-1.533 (m, 2H), 1.425-1.392 (m, 2H), 1.239-1.223 (m, 3H), 0.962-0.926 (t, *J* = 14.4 Hz, 3H).

SFC: Compound 30090, Rt = 6.597 min.

LCMS: $[M+H]^+$ = 395.90; Rt = 1.668 min.

$^1H$ NMR: (400 MHz, MeOD) δ 8.452 (s, 1H), 7.7689 (s, 1H), 7.450-7.409 (t, *J* = 16.4 Hz, 4H), 6.257-6.238 (d, *J* = 7.6 Hz, 1H), 5.173-5.113 (m, 2H), 4.344-4.311 (m, 1H), 4.165 (s, 2H), 2.739(s, 6H), 1.586-1.533 (m, 2H), 1.425-1.392 (m, 2H), 1.239-1.223 (m, 3H), 0.962-0.926 (t, *J* = 14.4 Hz, 3H).

Compound 30056 was prepared with reference to the synthetic method of Compound 30058.

| | |
|---|---|
| 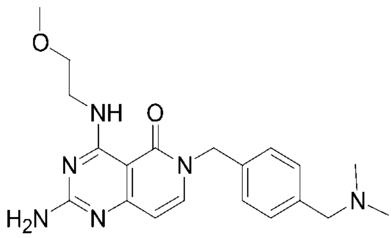 56 | $[M+H]^+$ = 384.40; Rt = 1.343 min. $^1H$ NMR: (400 MHz, $d_6$-DMSO) δ 9.460 (t, *J* = 5.6 Hz, 1H), 8.231 (s, 0.62H), 7.688 (d, *J* = 7.6 Hz, 1H), 7.228 (q, *J* = 8.0 Hz, 4H), 6.643 (s, 2H), 6.080 (d, *J* = 7.6 Hz, 1H), 5.030 (s, 2H), 3.606 - 3.566 (m, 2H), 3.503 - 3.477 (m, 2H), 3.33 - 3.32 (m, 2H), 3.280 (s, 3H), 2.110 (s, 6H). |

**Example 6: Preparation of Compound 30089**

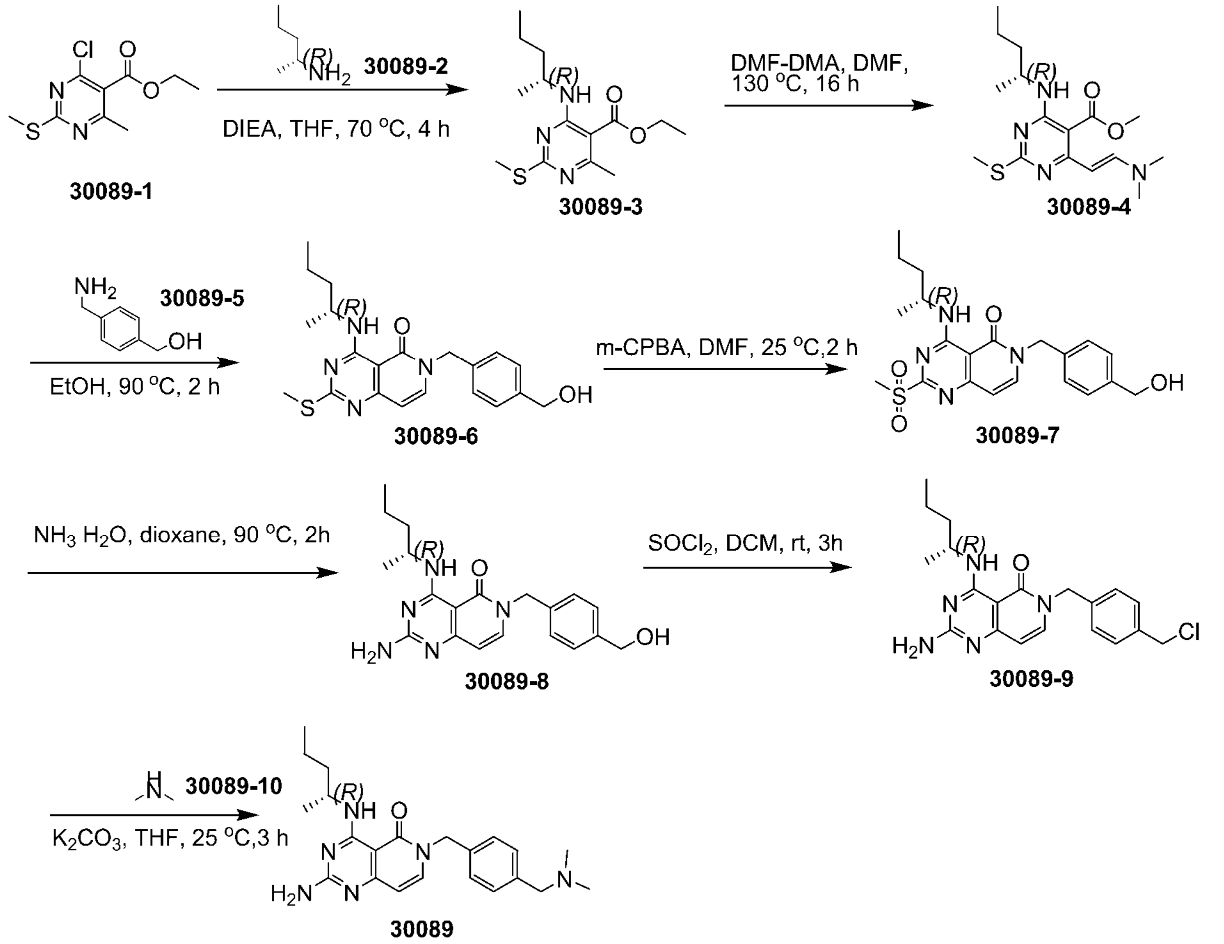


The synthetic method was the same as that in Example 5, and chiral amine reagent 30089-2 was used. The final product was purified by preparative HPLC under the following conditions: Column: Phenomenex Luna C18, 250 mm × 100 mm × 10 μm; mobile phase A: $H_2O$ (0.1% FA); mobile phase B: ACN; gradient elution: 10% B to 25% B over 30 minutes. Compound 30089 (20 mg, 0.05 mmol, 40.12% yield) was obtained as a white solid.

LCMS: $[M+H]^+$ = 395.4; Rt = 1.655 min.

$^1H$ NMR: (400 MHz, MeOD) δ 8.415 (s, 1H), 7.666 (s, 1H), 7.444-7.408 (t, *J* = 14.4 Hz, 4H), 6.243-6.224 (d, *J* = 7.6 Hz, 1H), 5.147-5.110 (m, 2H), 4.357-4.288 (m, 1H), 4.158 (s, 2H), 2.737 (s, 6H), 1.601-1.511 (m, 2H), 1.444-1.359 (m, 2H), 1.235-1.219 (m, 3H), 0.962-0.925 (t, *J* = 14.8 Hz, 3H).

The following compounds were prepared with reference to the synthetic method of Compound 30089 in Example 6.

| | |
|---|---|
| 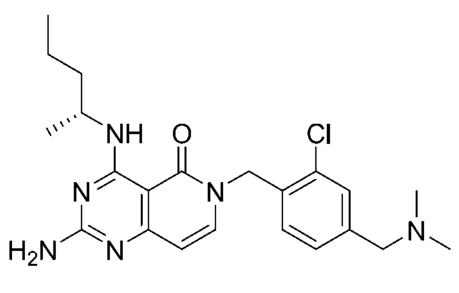 143 | $[M+H]^+$ = 463.2; Rt = 1.502 min. $^1H$ NMR: (400 MHz, $d_6$-DMSO) ) δ 9.25 (d, *J* = 8 Hz, 1H), 7.63 (d, *J* = 8 Hz, 1H), 7.44 (s, 1H), 7.23 (d, *J* = 8 Hz, 1H), 6.90 - 6.72 (m, 3H), 6.15 (d, *J* = 8 Hz, 1H), 5.16 - 5.05 (m, 2H), 4.23 - 4.20 (m, 1H), 3.48 (s, 2H), 2.20 (s, 6H), 1.51 - 1.29 (m, 4H), 1.14 (d, *J* = 8 Hz, 1H), 0.88 - 0.85 (m, 3H). |

(continued)

| | |
|---|---|
| 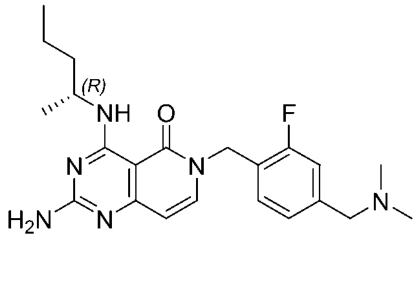 150 | $[M+H]^+$ = 413.3; Rt =0.930 min. $^1$H NMR: (400 MHz, DMSO-$d_6$) δ 9.25 (d, *J* = 8.2 Hz, 1H), 7.63 (d, *J* = 7.6 Hz, 1H), 7.18 - 7.01 (m, 3H), 6.61 (s, 2H), 6.10 (d, *J* = 7.6 Hz, 1H), 5.07 (q, *J* = 15.2 Hz, 2H), 4.21 (dt, *J* = 13.8, 6.8 Hz, 1H), 3.36 (s, 2H), 2.12 (s, 6H), 1.48 (dt, *J* = 8.8, 6.4 Hz, 2H), 1.31 (dt, *J* = 13.6, 6.8 Hz, 2H), 1.14 (d, *J* = 6.6 Hz, 3H), 0.88 (t, *J* = 7.4 Hz, 3H). |
| 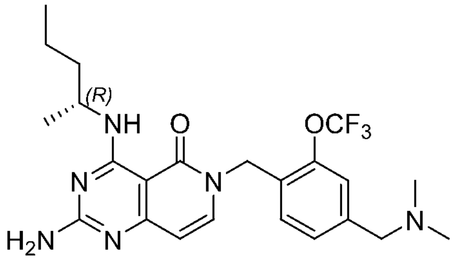 152 | $[M+H]^+$ = 479.5; |
| 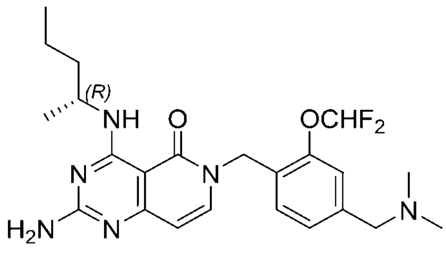 156 | $[M+H]^+$ = 461.2 |

Intermediate 30144-3 was prepared into the following compounds with reference to the synthetic method of Compound 30089 in Example 6.

| | |
|---|---|
| 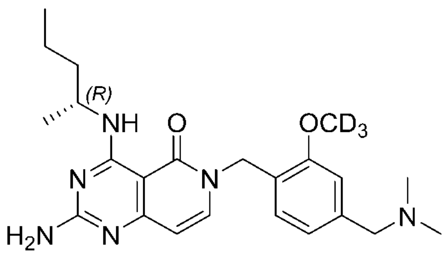 144 | $[M+H]^+$ = 428.3; Rt = 0.939 min. $^1$H NMR: (400 MHz, $d_6$-DMSO) ) δ 9.32 (d, *J* = 8.2 Hz, 1H), 7.56 (d, *J* = 7.6 Hz, 1H), 6.94 (s, 1H), 6.83 (dd, *J* = 20.8, 7.6 Hz, 2H), 6.58 (s, 2H), 6.07 (d, *J* = 7.6 Hz, 1H), 4.95 (q, *J* = 15.4 Hz, 2H), 4.27 - 4.14 (m, 1H), 3.34 (s, 2H), 2.13 (s, 6H), 1.54 - 1.41 (m, 2H), 1.31 (td, *J* = 13.8, 6.8 Hz, 2H), 1.14 (d, *J* = 6.6 Hz, 3H), 0.88 (t, *J* = 7.4 Hz, 3H). |

Intermediate 30147-2 was prepared into the following compounds with reference to the synthetic method of Compound 30089 in Example 6.

| | |
|---|---|
| 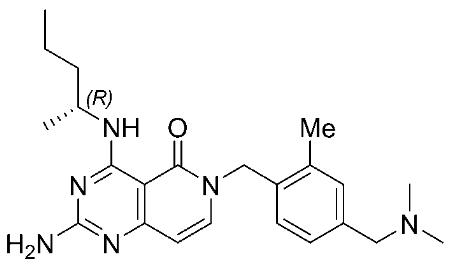 147 | [M+H] + = 409.3; Rt = 0.844 min. **1H NMR** (400 MHz, DMSO-$d_6$) δ 9.34 (d, *J* = 8.2 Hz, 1H), 7.50 (d, *J* = 7.6 Hz, 1H), 7.13 (s, 1H), 7.04 (d, *J* = 7.8 Hz, 1H), 6.74 (d, *J* = 7.8 Hz, 1H), 6.61 (s, 2H), 6.10 (d, *J* = 7.6 Hz, 1H), 5.02 (q, *J* = 15.6 Hz, 2H), 4.26 - 4.17 (m, 1H), 3.32 (s, 2H), 2.29 (s, 3H), 2.11 (s, 6H), 1.48 (dt, *J* = 13.4, 6.6 Hz, 2H), 1.31 (td, *J* = 13.8, 6.8 Hz, 2H), 1.15 (d, *J* = 6.6 Hz, 3H), 0.88 (t, *J* = 7.4 Hz, 3H). |

Intermediate 30149-4 was prepared into the following compounds with reference to the synthetic method of Compound 30089 in Example 6.

| | |
|---|---|
| 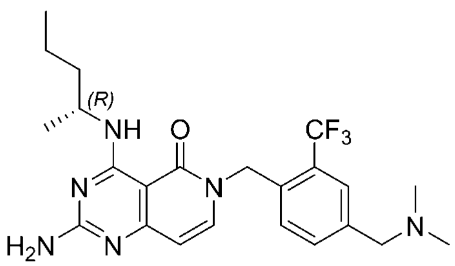 149 | [M+H] + = 463.2; Rt = 0.881 min. **1H** NMR (400 MHz, DMSO-$d_6$) δ 9.19 (d, *J* = 8.2 Hz, 1H), 7.68 (s, 1H), 7.63 (d, *J* = 7.6 Hz, 1H), 7.51 (d, *J* = 8.0 Hz, 1H), 6.87 (d, *J* = 8.0 Hz, 1H), 6.66 (s, 2H), 6.15 (d, *J* = 7.6 Hz, 1H), 5.24 (q, *J* = 16.4 Hz, 2H), 4.26 - 4.17 (m, 1H), 3.43 (s, 2H), 2.14 (s, 6H), 1.46 (d, *J* = 6.6 Hz, 2H), 1.30 (dd, *J* = 8.8, 4.5 Hz, 2H), 1.13 (d, *J* = 6.6 Hz, 3H), 0.87 (t, *J* = 7.4 Hz, 3H). |

**Example 7: Preparation of Compound 30102**

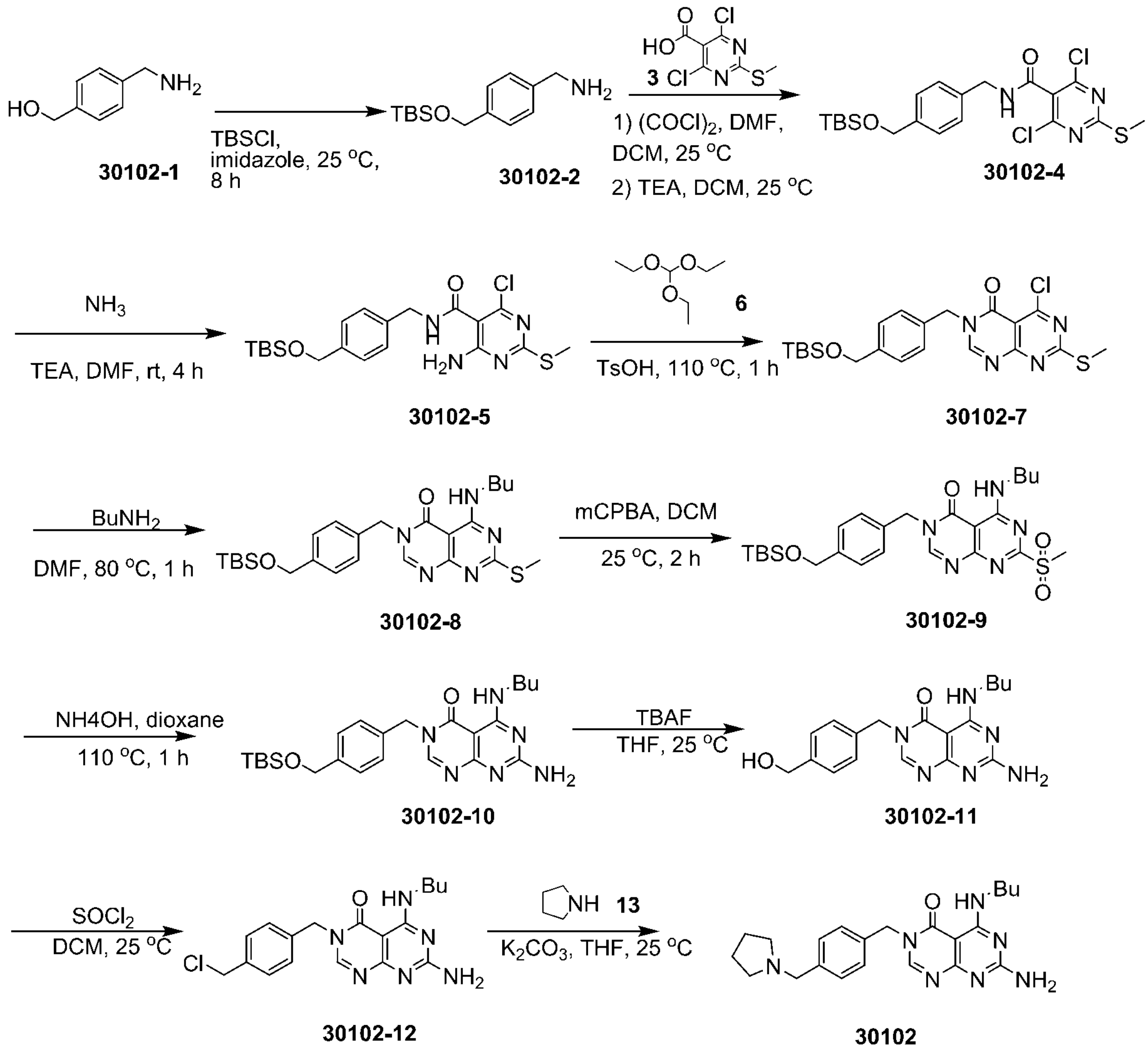


30102-1 30102-2 30102-4 30102-5 30102-7 30102-8 30102-9 30102-10 30102-11 30102-12 30102

**Step 1: General Method for Preparation of Compound 30102-2**

A solution of Compound 30102-1 (1g, 7 mmol), imidazole (0.74g, 11 mmol) and compound TBSCl (1.32g, 9 mmol) in DCM (20 mL) was stirred at room temperature under a nitrogen atmosphere for 8 hours. The mixture was diluted with water (20 mL), extracted with DCM (20 mL × 3), and evaporated under vacuum, and the crude product was purified by flash column chromatography (MeOH/DCM = 0%-5%) to obtain Compound 30102-2 (1g, 49.3% yield) as a yellow oil.

**Step 2: General Method for Preparation of Compound 30102-4**

At 0°C under a nitrogen atmosphere, oxalyl chloride (510 mg, 4.02 mmol) was added dropwise to a solution of Compound 102-3 (800 mg, 3.35 mmol) in DCM/DMF (100/1, 15 mL), and the reaction mixture was stirred at room temperature for 1 hour. The solvent was removed under reduced pressure to obtain a yellow solid. At room temperature under a nitrogen atmosphere, TEA (966 mg, 9.54 mmol) was added dropwise to a DCM (20 mL) solution of the obtained yellow solid and Compound 30102-2 (800 mg, 3.18 mmol). The reaction mixture was stirred at room temperature for 1 hour. The mixture was diluted with water (20 mL), extracted with EA (20 mL × 3), and evaporated under vacuum, and the crude product was purified by flash column chromatography (MeOH/DCM = 0%-5%) to obtain Compound 30102-4 (0.8g, 47.9% yield) as a yellow solid.

**Step 3: General Method for Preparation of Compound 30102-5**

At room temperature under a nitrogen atmosphere, a MeOH (0.7 mL, 5 mmol) solution containing Compound 30102-4 (800 mg, 3.18 mmol) and $NH_3$ and a DMF (15 mL) solution of EA (0.39g, 4 mmol) were stirred for 4 hours. The mixture was diluted with water (20 mL), extracted with EA (20 mL × 3), and evaporated under vacuum to obtain crude

product 30102-5 (1g, 56.3% yield) as a yellow oil.

**Step 4: General Method for Preparation of Compound 30102-7**

At room temperature under a nitrogen atmosphere, a reaction mixture of Compound 30102-5 (1g, 2.2 mmol), $TsOH \cdot H_2O$ (0.04g, 0.2 mol) and triethyl orthoformate (10 mL) was stirred at 110°C for 1 hour. The mixture was evaporated under vacuum to obtain crude product 30102-7 (800 mg, 63.6% yield) as a yellow oil.

**Step 5: General Method for Preparation of Compound 30102-8**

At room temperature under a nitrogen atmosphere, DIEA (330 mg, 3 mmol) was added to a DMF (10 mL) solution of Compound 30102-7 (0.8g, 2 mmol) and 1-butylamine (150 mg, 2 mmol). The reaction mixture was stirred at 80°C for 1 hour. The mixture was extracted with EA (20 mL × 2), and evaporated under vacuum, and the crude product was purified by flash column chromatography (EA/PE = 0%-25%) to obtain Compound 30102-8 (0.65g, 70.6% yield) as a white solid.

**Step 6: General Method for Preparation of Compound 30102-9**

A solution of Compound 30102-8 (700 mg, 1.40 mmol) and m-CPBA (363 mg, 2.10 mmol) in DCM (15 mL) was stirred at 25°C for 2 hours. The solution was evaporated under vacuum to obtain crude product 30102-9 (700 mg, 75.2% yield).

**Step 7: General Method for Preparation of Compound 30102-10**

The crude product 30102-9 (700 mg) was redissolved in dioxane (2 mL) and then $NH_4OH$ (4mL) was added. The resulting solution was stirred and heated at 110°C for 1 hour in a sealed tube. The solution was evaporated under vacuum to obtain crude product 30102-10 (450 mg, 62% yield) as a white solid.

**Step 7: General Method for Preparation of Compound 30102-11**

At room temperature under a nitrogen atmosphere, a solution of 1 M TBAF in THF (0.5 mL, 0.21 mmol) was added dropwise to a THF (3 mL) solution of Compound 30102-10 (100 mg, 0.21 mmol). The reaction mixture was stirred at room temperature for 1 hour. DCM (10 mL) was added, and the resulting mixture was filtered. The solvent was removed under reduced pressure to obtain crude product 30102-11 (40 mg, 85% yield) as a yellow solid.

Other steps were performed to prepare Compound 30102 with reference to the synthetic method of Compound 30093.

**LCMS:** $[M+H]^+$ = 408.4; Rt = 1.619 min.

**$^1H$** NMR: (400 MHz, $CD_3OD$) $\delta$ 8.419 (s, 1H), 7.396 (s, 4H), 5.125 (s, 2H), 3.883 (s, 2H), 3.505 (t, J = 7.2 Hz, 2H), 2.811 (s, 4H), 1.891 (s, 4H), 1.611 (dd, J = 14.8, 7.2 Hz, 2H), 1.425 (dd, J = 15.2, 7.2 Hz, 2H), 0.969 (t, J = 7.2 Hz, 3H).

**Example 8: Preparation of Compound 30085**

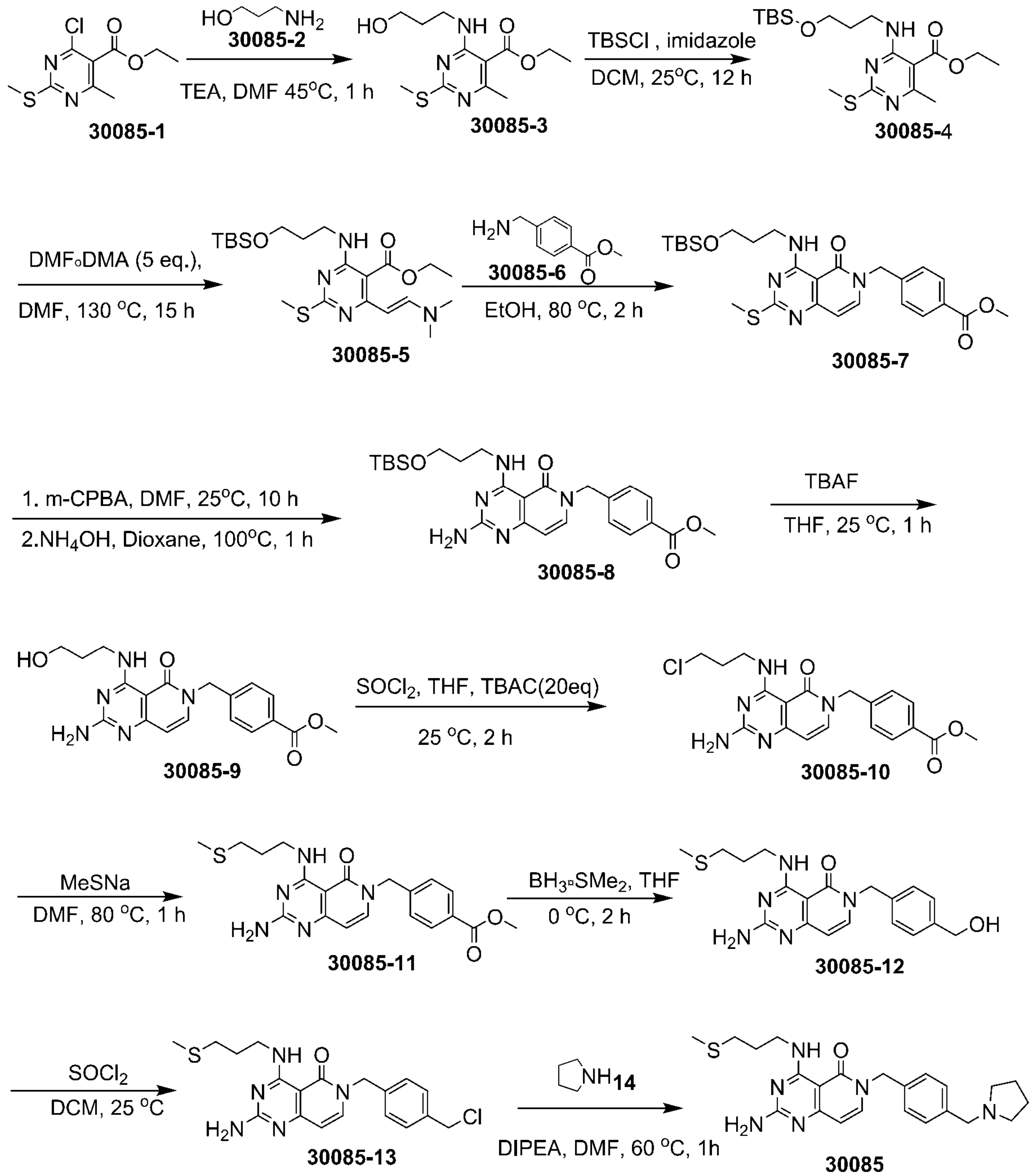


30085-1, 30085-3, 30085-4, 30085-5, 30085-7, 30085-8, 30085-9, 30085-10, 30085-11, 30085-12, 30085-13, 30085

**Step 1: General Method for Preparation of Compound 30085-3**

A mixture of Compound 30085-1 (4g, 16.21 mmol), TEA (1.64g, 16.21 mmol) and Compound 30085-2 (1.22g, 16.21 mmol) in DMF (40 mL) was heated to 45°C and stirred for 1 hour. The reaction was subjected to quenching with $H_2O$ (50 mL), followed by extraction with ethyl acetate (50 mL × 2). The mixture was evaporated under vacuum to obtain Compound 30085-3 (4.3g, 93.04% yield) as a white solid.

**Step 2: General Method for Preparation of Compound 30085-4**

At room temperature under a nitrogen atmosphere, TBSCl (2.73g, 18.11 mmol) and imidazole (2.06g, 30.18 mmol) were added to a DCM (45 mL) solution of Compound 30085-3 (4.3g, 15.09 mmol). The reaction mixture was stirred at room temperature for 12 hours. The mixture was diluted with water (50 mL), extracted with DCM (50 mL × 2), and evaporated under vacuum, and the crude product was purified by flash column chromatography (EtOH/PE = 0%-20%) to obtain Compound 30085-4 (4.5g, 74.74% yield) as a yellow solid.

**Step 3: General Method for Preparation of Compound 30085-5**

A solution of Compound 30085-4 (4.5g, 11.28 mmol) and N,N-dimethylformamide dimethyl acetal (DMF·DMA, 6.72g, 56.39 mmol) in DMF (50 mL) was heated to 130°C and stirred for 15 hours. The solution was evaporated directly to obtain a yellow solid, Compound 30085-5 (80% yield, 4.5g of crude product).

**Step 4: General Method for Preparation of Compound 30085-7**

A solution of Compound 30085-5 (4.5g, 9.89 mmol) and Compound 30085-6 (2.0g, 9.89 mmol) in EtOH (50 mL) was heated to 80°C and stirred for 2 hours. The solution was evaporated under reduced pressure. A crude product was directly purified by flash silica gel column chromatography (EA/PE = 0% - 25%), to obtain Compound 30085-7 (4.5g, 86.18% yield) as a yellow solid.

**Step 5: General Method for Preparation of Compound 30085-8**

A solution of Compound 30085-7 (4.5g, 8.51 mmol) and m-CPBA (4.32g, 21.28 mmol) in DMF (50 mL) was stirred at room temperature for 10 hours. The solution was concentrated by evaporation under reduced pressure to obtain a crude product. The crude product was redissolved in dioxane (40 mL) and then $NH_4OH$ (20mL) was added. The resulting solution was stirred and heated at 100°C for 1 hour. The solution was concentrated by evaporation under reduced pressure. The solution was subjected to quenching by adding $H_2O$ (50 mL), followed by extraction with EA (50 mL × 2). The crude product was evaporated and directly purified by flash silica gel column chromatography (MeOH/DCM = 0% - 5%) to obtain Compound 30085-8 (3.0g, 70.89% yield) as a yellow solid.

**Step 6: General Method for Preparation of Compound 30085-9**

At room temperature under a nitrogen atmosphere, TBAF (15 mL) was added dropwise to a THF (20 mL) solution of Compound 30085-8 (3.0g, 6.03 mmol). The reaction mixture was stirred at room temperature for 1 hour. The reaction was subjected to quenching by adding $H_2O$ (50 mL), followed by extraction with EA (50 mL × 2). The solvent was removed under reduced pressure to obtain crude product 30085-9 (2.0g, 86.59% yield) as a yellow solid.

**Step 7: General Method for Preparation of Compound 30085-10**

At room temperature under a nitrogen atmosphere, TBAC (21.74g, 78.2 mmol) was added dropwise to a $SOCl_2$/THF (1/4, 20 mL) solution of Compound 30085-9 (1.5g, 3.91 mmol). The reaction mixture was stirred at room temperature for 2 hours. The solution was concentrated under reduced pressure and subjected to quenching by adding $H_2O$ (80 mL), followed by extraction with EA (80 mL × 3). The solvent was removed under reduced pressure to obtain crude product 30085-10 (1.3g, 82.89% yield) as a yellow solid.

**Step 8: General Method for Preparation of Compound 30085-11**

At room temperature under a nitrogen atmosphere, MeSNa (340 mg, 4.86 mmol) was added to a DMF (20 mL) solution of Compound 30085-10 (1.3g, 3.24 mmol). The reaction mixture was stirred at 80°C for 1 hour. The reaction was subjected to quenching by adding $H_2O$ (30 mL), followed by extraction with EA (30 mL × 3). The solvent was removed under reduced pressure to obtain crude product 30085-11 (800 mg, 59.76%) as a yellow solid.

**Step 9: General Method for Preparation of Compound 30085-12**

At 0°C under a nitrogen atmosphere, $BH_3 \cdot SMe_2$ (551 mg, 7.25 mmol) was added to a THF (10 mL) solution of Compound 30085-11 (600 mg, 1.45 mmol). The reaction mixture was stirred at 0°C for 2 hours. The reaction was subjected to quenching by adding $H_2O$ (20 mL), followed by extraction with EA (30 mL × 2). The solvent was removed under reduced pressure to obtain a crude product. The crude product was directly purified by flash silica gel column chromatography (MeOH/DCM = 0% - 5%), to obtain Compound 30085-12 (350 mg, 62.69% yield) as a yellow solid.

**Step 10: General Method for Preparation of Compound 30085-13**

A solution of Compound 30085-12 (100 mg, 0.26 mmol) and $SOCl_2$ (2 mL) in DCM (2 mL) was stirred at room temperature for 1 hour. The solution was concentrated by evaporation under reduced pressure to obtain crude product 30085-13 (80 mg, 76.35% yield).

**Step 11: General Method for Preparation of Compound 30085**

A solution of Compound 30085-13 (30 mg, 0.07 mmol), Compound 30085-14 (14 mg, 0.18 mmol) and DIPEA (23 mg, 0.18 mmol) in DMF (2 mL) was stirred at 60°C for 1 hour. The reaction was subjected to quenching by adding $H_2O$ (5 mL), followed by extraction with EA (5 mL × 2). The solution was concentrated by evaporation under reduced pressure. The solution was purified by preparative HPLC (column: Phenomenex Luna C18, 250 mm × 100 mm × 10 μm; mobile phase: [A: $H_2O$ (0.1% trifluoroacetic acid), B: acetonitrile]; B%: 40%-80%, 40 min), to obtain Compound M030085 (5.84 mg, 0.013 mmol, 19.04% yield) as a white solid.

LCMS: $[M+H]^+$ = 439.40; Rt = 1.627 min.

$^1$H NMR: (400 MHz, MeOD) $\delta$ 7.569 (d, *J* = 7.6 Hz, 1H), 7.359 (d, *J* = 8.0 Hz, 2H), 7.315 (d, *J* = 8.0 Hz, 2H), 6.185 (d, *J* = 7.6 Hz, 1H), 5.110 (s, 2H), 3.775 (s, 2H), 3.611 (t, *J* = 6.8 Hz, 2H), 2.698 (s, 4H), 2.569 (t, *J* = 7.2 Hz, 2H), 2.090 (s, 3H), 1.969 - 1.934 (m, 2H), 1.852 (s, 4H).

Compound 30087 was prepared with reference to the synthetic method of Compound 30085.

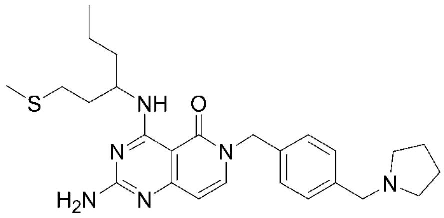

87

$[M+H]^+$ = 481.40; Rt = 1.895 min. **$^1$H NMR:** (400 MHz, MeOD) $\delta$ 7.567 (d, *J* = 7.6 Hz, 1H), 7.312 (d, *J* = 13.6 Hz, 4H), 6.189 (d, *J* = 7.6 Hz, 1H), 5.104 (s, 2H), 3.673 (s, 2H), 2.543 (dd, *J* = 18.0, 9.6 Hz, 7H), 2.065 (s, 3H), 1.813 - 1.793 (m, 6H), 1.613 - 1.515 (m, 2H), 1.441 - 1.367 (m, 2H), 0.942 (t, *J* = 7.2 Hz, 3H).

**Example 9: Preparation of Compound 30086**

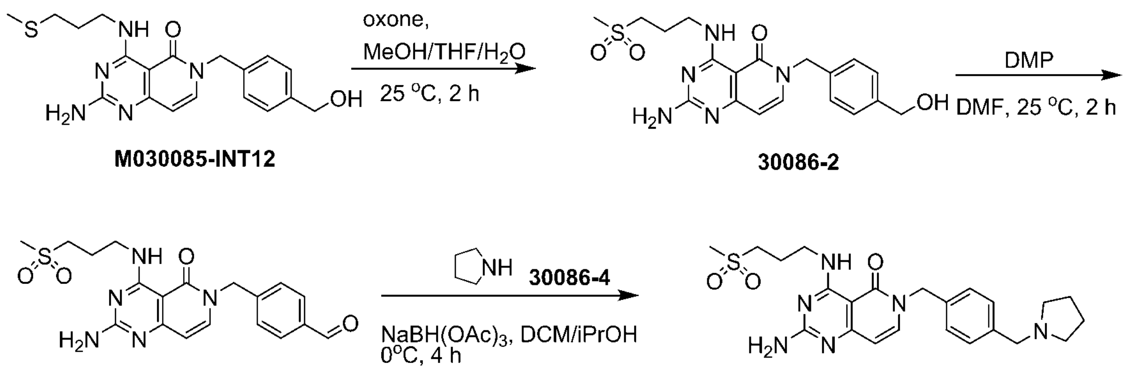


M030085-INT12 30086-2 30086-3 30086

**Step 1: General Method for Preparation of Compound 30086-2**

At room temperature under a nitrogen atmosphere, oxone (191 mg, 0.31 mmol) was added to a MeOH/THF/$H_2O$ (1:1:1, 6 mL) solution of Compound 30085-12 (100 mg, 0.26 mmol) during stirring. The reaction mixture was stirred at room temperature for 2 hours. The solution was concentrated by evaporation under reduced pressure and purified by preparative HPLC (column: Phenomenex Luna C18, 250 mm × 100 mm × 10 μm; mobile phase: [A: $H_2O$ (0.1% trifluoroacetic acid), B: acetonitrile]; B%: 10%-50%, 40 min), to obtain Compound 30086- 2 (50 mg, 43.87% yield) as a white solid.

**Step 2: General Method for Preparation of Compound 30086-3**

At room temperature under a nitrogen atmosphere, Dess-Martin periodinane (203 mg, 0.48 mmol) was added to

a DMF (5 mL) solution of Compound 30086-2 (40 mg, 0.10 mmol) during stirring. The reaction mixture was stirred at room temperature for 2 hours. The solution was concentrated by evaporation under reduced pressure and purified by preparative HPLC (column: Phenomenex Luna C18, 250 mm × 100 mm × 10 μm; mobile phase: [A: $H_2O$ (0.1% trifluoroacetic acid), B: acetonitrile]; B%: 10%-50%, 40 min), to obtain Compound 30086-3 (30 mg, 67.85% yield) as a white solid.

**Step 3: General Method for Preparation of Compound 30086**

At 0°C under a nitrogen atmosphere, Compound 30086-4, pyrrolidine (16 mg, 0.22 mmol) and $NaBH(OAc)_3$ (31 mg, 0.14 mmol) were added to a solution of Compound 30086-3 (30 mg, 0.072 mmol) in DCM/iPrOH (1:1, 4 mL) during stirring. The reaction mixture was stirred at 0°C for 4 hours. The solution was concentrated by evaporation under reduced pressure and purified by preparative HPLC (column: Phenomenex Luna C18, 250 mm × 100 mm × 10 μm; mobile phase: [A: $H_2O$ (0.1% trifluoroacetic acid), B: acetonitrile]; B%: 10%-30%, 30 min), to obtain Compound M030086 (12 mg, 34.63% yield) as a white solid.

**LCMS:** $[M+H]^+$ = 471.40; Rt = 1.342 min.

**$^1$H NMR:** (400 MHz, MeOD) δ 7.649 (d, *J* = 7.2 Hz, 1H), 7.485 (d, *J* = 8.0 Hz, 2H), 7.432 (d, *J* = 8.0 Hz, 2H), 6.227 (d, *J* = 7.6 Hz, 1H), 5.150 (s, 2H), 4.291 (s, 2H), 3.704 (t, *J* = 13.6 Hz, 2H), 3.306-3.185 (m, 6H), 2.970 (s, 3H), 2.172-2.116 (m, 2H), 2.066-2.001 (m, 4H).

**Example 10: Preparation of Compound 30088**

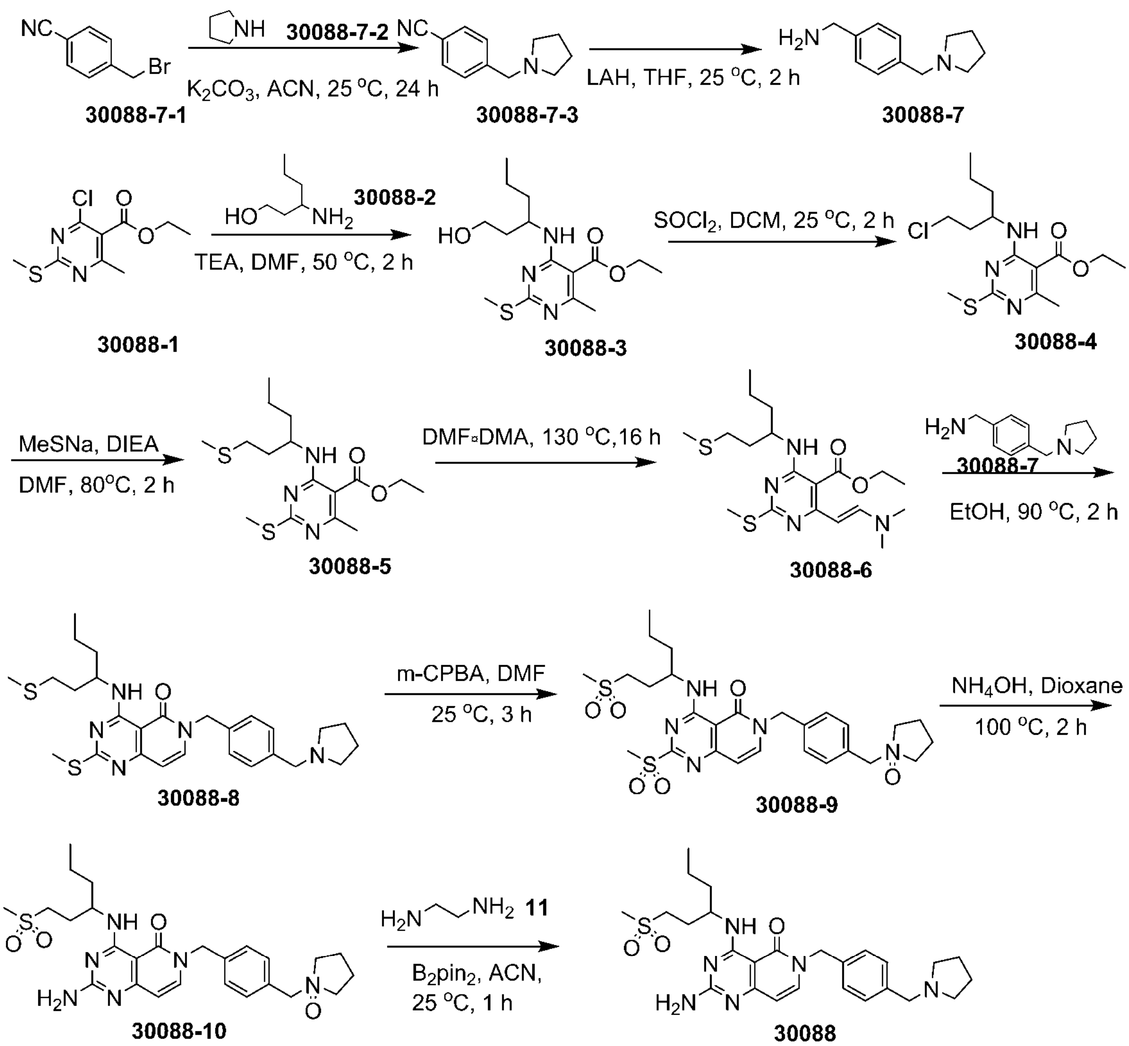


30088-7-1, 30088-7-3, 30088-7, 30088-1, 30088-3, 30088-4, 30088-5, 30088-6, 30088-8, 30088-9, 30088-10, 30088

**Step 1: General Method for Preparation of Compound 30088-7-3**

At room temperature under a nitrogen atmosphere, $K_2CO_3$ (1548 mg, 15.30 mmol) and pyrrolidine (726 mg, 10.20 mmol) were added to an ACN (30 mL) solution of Compound 30088-7-1 (1000 mg, 5.10 mmol) during stirring. The reaction mixture was stirred at room temperature for 24 hours. The solution was concentrated by evaporation under reduced pressure and directly purified by flash silica gel column chromatography (MeOH/DCM = 0% - 8%) to obtain Compound 30088-7-3 (800 mg, 4.30 mmol, 84.31% yield) as a white solid.

**Step 2: General Method for Preparation of Compound 30088-7**

At 0°C under a nitrogen atmosphere, LAH (489 mg, 12.90 mmol) was added to a THF (20 mL) solution of Compound 30088-7-3 (800 mg, 4.30 mmol). The reaction mixture was stirred at room temperature for 2 hours. The reaction was subjected to quenching by adding $H_2O$ (10 mL), followed by dilution with EA (100 mL), and then the resulting mixture was washed with water and saturated brine, dried over $Na_2SO_4$ and concentrated. The crude product was purified by flash column chromatography (MeOH/DCM = 0% - 30%) to obtain Compound 30088-7 (850 mg, 4.47 mmol, 93.60% yield) as a white solid.

**Step 3: General Method for Preparation of Compound 30088-3**

A solution of Compound 30088-1 (1g, 4.1 mmol), Compound 30088-2 (0.96g, 8.2 mmol) and TEA (1.24g, 12.3 mmol) in DMF (15 mL) was stirred and heated at 50°C for 2 hours. The solvent was removed from the reaction mixture under reduced pressure to obtain the crude product. The crude product was directly purified by flash silica gel column chromatography (EA/PE = 0% - 15%), to obtain Compound 30088-3 (1.1g, 3.4 mmol, 81.93% yield) as a yellow solid.

**Step 4: General Method for Preparation of Compound 30088-4**

At room temperature under a nitrogen atmosphere, $SOCl_2$ (273 mg, 2.29 mmol) was added dropwise to a DCM (15 mL) solution of Compound 30088-3 (500 mg, 1.53 mmol) during stirring. The reaction mixture was stirred at room temperature for 2 hours. The reaction mixture was concentrated under reduced pressure to obtain crude product 30088-4 (500 mg, 1.45 mmol, 94.77% yield) as a yellow oil.

**Step 5: General Method for Preparation of Compound 30088-5**

At room temperature under a nitrogen atmosphere, DIEA (561 mg, 4.34 mmol) and MeSNa (203 mg, 2.89 mmol) were added to a DMF (15 mL) solution of Compound 30088-4 (500 mg, 1.45 mmol). The reaction mixture was stirred at 80°C for 2 hours. The solvent was removed from the reaction mixture under reduced pressure to obtain the crude product. The crude product was directly purified by flash silica gel column chromatography (EA/PE = 0% - 15%), to obtain Compound 30088-5 (250 mg, 0.70 mmol, 49.30% yield) as a yellow oil.

**Step 6: General Method for Preparation of Compound 30088-6**

A solution of Compound 30088-5 (50 mg, 0.14 mmol) and N,N-dimethylformamide dimethyl acetal (DMF-DMA, 50 mg, 0.42 mmol) in DMF (3 mL) was heated to 130°C and stirred for 16 hours. The solution was evaporated directly. The crude product was directly purified by flash silica gel column chromatography (EA/PE = 0% - 20%), to obtain Compound 30088-6 (50 mg, 0.12 mmol, 85.71% yield) as a yellow oil.

**Step 7: General Method for Preparation of Compound 30088-8**

A mixture of Compound 30088-6 (50 mg, 0.12 mmol) and Compound 30088-7 (72 mg, 0.39 mmol) in EtOH (3 mL) was heated to 90°C and stirred for 2 hours. The solution was evaporated directly. The crude product was purified by flash column chromatography (MeOH/DCM = 0% - 5%) to obtain Compound 30088-8 (30 mg, 0.06 mmol, 48.29% yield) as a yellow solid.

**Step 8: General Method for Preparation of Compound 30088-9**

At room temperature under a nitrogen atmosphere, m-CPBA (81 mg, 0.48 mmol) was added to a DMF (3 mL) solution of Compound 30088-8 (30 mg, 0.06 mmol) during stirring. The reaction mixture was stirred at room temperature for 3 hours. The solution was concentrated by evaporation under reduced pressure and purified by preparative HPLC

(column: Phenomenex Luna C18, 250 mm × 100 mm × 10 μm; mobile phase: [A: $H_2O$ (0.1% trifluoroacetic acid), B: acetonitrile]; B%: 10%-40%, 30 min), to obtain Compound 30088-9 (15 mg, 0.024 mmol, 41.13% yield) as a white solid.

**Step 9: General Method for Preparation of Compound 30088-10**

A solution of Compound 30088-9 (15 mg, 0.024 mmol) in 1,4-dioxane/$NH_3 \cdot H_2O$ (1:2, 3 mL) was heated to 100°C and stirred under reflux for 2 hours. The solution was concentrated by evaporation under reduced pressure and purified by preparative HPLC (column: Phenomenex Luna C18, 250 mm × 100 mm × 10 μm; mobile phase: [A: $H_2O$ (0.1% FA), B: acetonitrile]; B%: 5%-25%, 30 min), to obtain Compound 30088-10 (10 mg, 0.018 mmol, 71.15% yield) as a white solid.

**Step 10: General Method for Preparation of Compound M030088**

At room temperature under a nitrogen atmosphere, 30088-11 (23 mg, 0.36 mmol) was added to an ACN (3 mL) solution of Compound 30088-10 (10 mg, 0.018 mmol) and pinacol diboronate ($B_2Pin_2$, 6 mg, 0.023 mmol) during stirring. The reaction mixture was stirred at room temperature for 1 hour. The solution was concentrated by evaporation under reduced pressure and purified by preparative HPLC (column: Phenomenex Luna C18, 250 mm × 100 mm × 10 μm; mobile phase: [A: $H_2O$ (0.1% FA), B: acetonitrile]; B%: 5%-30%, 30 min), to obtain Compound M030088 (3.5 mg, 0.007 mmol, 34.92% yield) as a white solid.

**LCMS:** $[M+H]^+$ = 513.50; Rt = 1.574 min.

**$^1H$ NMR:** (400 MHz, MeOD) δ 7.634-7.615 (d, *J* = 7.6 Hz, 1H), 7.475-7.403 (m, 4H), 6.222 (d, *J* = 7.6 Hz, 1H), 5.198-5.085 (m 2H), 4.453 (s, 1H), 4.232 (s, 1H), 3.204-3.180 (m, 6H), 2.943 (s, 3H), 2.188-2.114 (m, 1H), 2.027-1.975 (m, 5H), 1.663-1.568 (m, 2H), 4.423-1.366 (m, 2H), 0.969 (t, *J* = 14.8 Hz, 3H).

**Example 11: Preparation of Compound 30081**

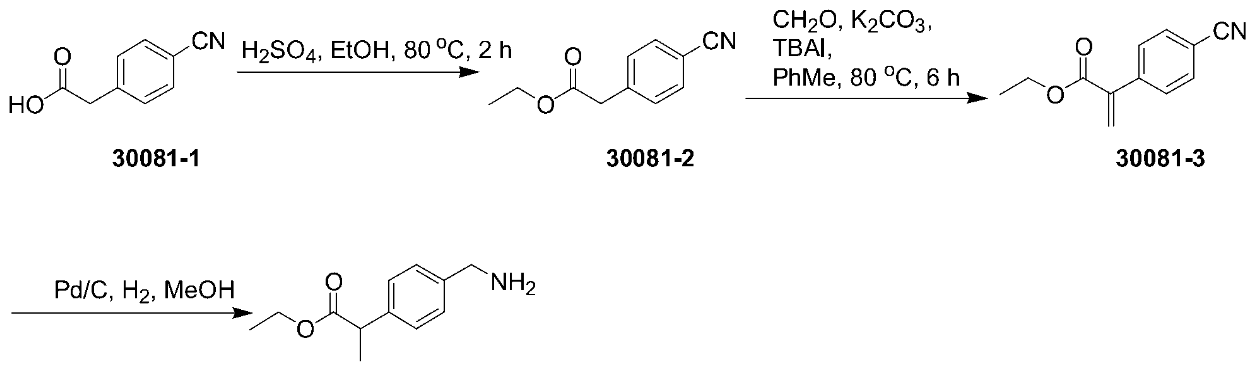


30081-1 30081-2 30081-3

30081-4

**Step 1: General Method for Intermediate 30081-2**

Concentrated sulfuric acid (180 mg, 1.8 mmol) was added to a solution of Compound 30081-1 (3g, 18.6 mmol) in EtOH (20 mL), and then a resulting solution was heated to 80°C and stirred under reflux for 2 hours. The resulting solution was concentrated by evaporation under reduced pressure to obtain Compound 30081-2 (3.2g, 16.9 mmol, 91% yield, 90% purity) as a yellow solid. $^1H$ NMR: (400 MHz, CDCl3) δ 7.632 (d, J = 8.4 Hz, 2H), 7.414 (d, J = 8.4 Hz, 2H), 4.171 (q, J = 7.2 Hz, 2H), 3.685 (s, 2H), 1.262 (t, J = 7.2 Hz, 3H).

**Step 2: General Method for Intermediate 30081-3**

Paraformaldehyde (3.04g, 33.8 mmol), $K_2CO_3$ (4.67g, 33.8 mmol) and TBAI (2.50g, 6.7 mmol) were added to a solution of Compound 30081-2 (3.2g, 16.9 mmol) in toluene (30 mL), and then a resulting mixture was heated to 80°C and stirred under reflux for 6 hours. The resulting solution was concentrated by evaporation under reduced pressure. The crude product was purified by flash column chromatography (EtAc/PE = 0% - 30%) to obtain Compound 30081-3 (0.8g, 4.0 mmol, 24% yield, 90% purity) as a white solid. $^1H$ NMR: (400 MHz, CDCl3) δ 7.656 (d, J = 8.4 Hz, 2H), 7.543 (d, J = 8.4 Hz, 2H), 6.502 (s, 1H), 5.984 (s, 1H), 4.301 (q, J = 7.2 Hz, 2H), 1.344 (t, J = 7.2 Hz, 3H).

**Step 3: General Method for Intermediate 30081-4**

A mixture of Compound 30081-3 (600 mg, 2.98 mmol), MeOH (10 mL) and Pd/C (318 mg, 2.98 mmol) was stirred at 25°C under a hydrogen atmosphere for 4 hours. The resulting solution was filtered and then concentrated by evaporation under reduced pressure. The crude product was purified by flash reversed-phase C18 silica gel column chromatography (acetonitrile aqueous solution = 10%-40%, 0.1% HCl) to obtain Compound 30081-4 (500 mg, 2.41 mmol, 81% yield, 90% purity) as a white solid. LC-MS: $(M+H)^+$, 208.25; Rt = 1.343 min.

Compound 30081 was prepared with reference to the synthetic method of Compound 30043 by using a raw material of Compound 30081-4.

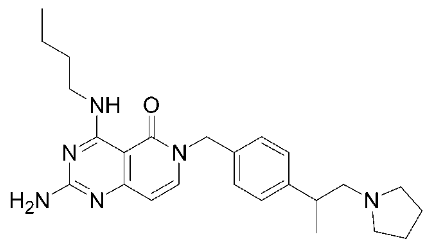

81

$[M+H]^+$, 435.45; Rt = 1.790 min.

**$^1$H NMR** (400 MHz, $CD_3OD$) $\delta$ 8.534 (s, 1H), 7.582 (d, *J* = 7.6 Hz, 1H), 7.366 - 7.242 (m, 4H), 6.183 (d, *J* = 7.6 Hz, 1H), 5.094 (s, 2H), 4.592 (s, 1H), 3.544 - 3.470 (m, 2H), 3.168 - 2.992 (m, 3H), 2.901 (s, 3H), 1.876 (s, 4H), 1.623 (dd, *J* = 14.8, 7.2 Hz, 2H), 1.456 (dd, *J* = 15.2, 7.2 Hz, 2H), 1.285 (d, *J* = 6.4 Hz, 4H), 0.987 (t, *J* = 7.2 Hz, 3H).

**Example 12: Preparation of Compounds 30129, 30130, 30138 and 30139**

The intermediate was prepared according to the following scheme with reference to the preparation method of Compound 30081.

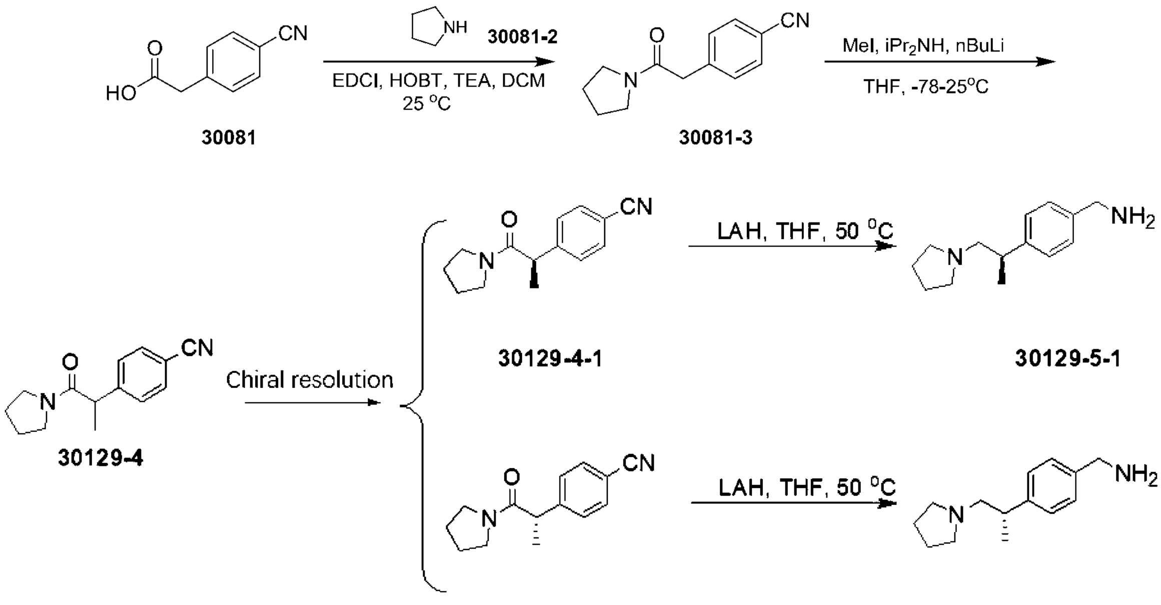


30081 30081-3 30129-4 30129-4-1 30129-5-1 30129-4-2 30129-5-2

Compounds 30129, 30130, 30138 and 30139 were prepared with reference to the synthetic method of Compound 30042.

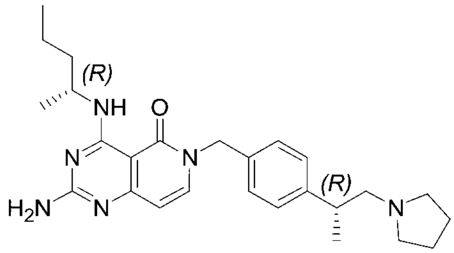

129

[M+H] $^+$ = 449.25; Rt = 0.874 min.

**$^1$H NMR:** (400 MHz, $CD_3OD$) δ 8.054 (d, *J* = 7.6 Hz, 1H), 7.395 (q, *J* = 8.0 Hz, 4H), 6.398 (d, *J* = 7.6 Hz, 1H), 5.310 - 5.162 (m, 2H), 4.464 - 4.352 (m, 1H), 3.616 (s, 1H), 3.525 - 3.474 (m, 1H), 3.401 (dd, *J* = 13.2, 6.0 Hz, 2H), 3.219 (dd, *J* = 16.4, 6.8 Hz, 2H), 3.168 - 3.120 (m, 1H), 3.023 (d, *J* = 8.0 Hz, 1H), 2.222 - 2.034 (m, 2H), 1.941 (ddd, *J* = 15.2, 9.6, 6.8 Hz, 2H), 1.676 - 1.566 (m, 2H), 1.467 - 1.389 (m, 2H), 1.321 (d, *J* = 6.8 Hz, 3H), 1.283 (d, *J* = 6.8 Hz, 3H), 0.965 (t, *J* = 7.2 Hz, 3H).

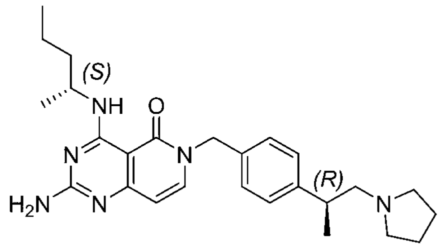

130

**[M+H] $^+$ = 449.2; Rt = 0.906 min.**

**$^1$H NMR:** (400 MHz, MeOD) δ 8.057-8.038 (d, *J* = 7.6 Hz, 1H), 7.418-7.353 (m, 4H), 6.424-6.406 (d, *J* = 7.2 Hz, 1H), 5.248-5.156 (m, 2H), 4.435-4.381 (m, 1H), 3.611-3.583 (m, 1H), 3.542-3.474 (m, 1H), 3.453-3.379 (m, 2H), 2.091-2.061 (m, 2H), 1.950-1.917 (m, 2H), 1.633-1.581 (m, 2H), 1.427-1.383 (m, 2H), 1.329-1.311 (d, *J* = 7.2 Hz, 3H), 1.283-1.267 (d, *J* = 6.4 Hz, 3H), 0.975-0.939 (t, *J* = 14.4 Hz, 3H).

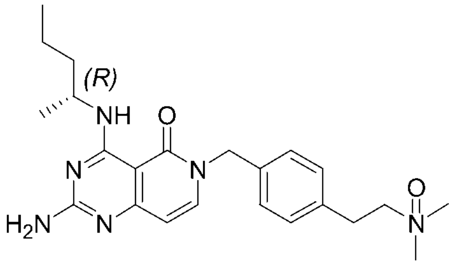

138

[M+H] $^+$ = 425.65; Rt = 1.320 min.

**$^1$H** NMR: (400 MHz, $CD_3OD$) δ 7.567 (d, *J* = 7.2 Hz, 1H), 7.310-7.263 (m, 4H), 6.183 (d, *J* = 7.6 Hz, 1H), 5.124-5.027 (m, 2H), 4.336-4.268 (m, 1H), 3.486-3.444 (m, 2H), 3.198 (s, 6H), 3.180-3.129 (m, 2H), 1.603-1.494 (m, 2H), 1.452-1.381 (m, 2H), 1.233 (d, *J* = 6.4 Hz, 3H), 0.965 (t, *J* = 14.4 Hz, 3H).

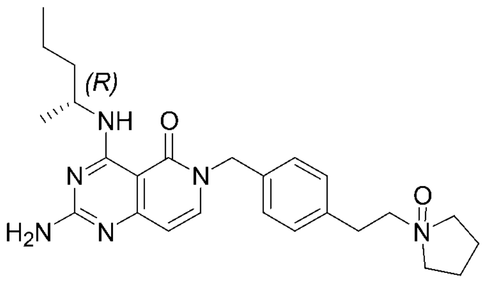

139

[M+H] + = 451.55; Rt = 1.353 min.

**$^1$H NMR:** (400 MHz, $CD_3OD$) δ 7.567-7.549 (d, *J* = 7.2 Hz, 1H), 7.313-7.262 (m, 4H), 6.182-6.163 (d, *J* = 7.6 Hz, 1H), 5.123-5.027 (m 2H), 4.336-4.284 (m, 1H), 4.232 (s, 1H), 3.546-3.504 (m, 2H), 3.413-3.392 (m, 4H), 3.247-3.206 (m, 2H), 2.336-2.319 (d, *J* = 6.8 Hz, 5H), 2.047 s, 2H), 1.603-1.526 (m, 2H), 1.417-1.382 (m, 2H), 1.233-1.217 (d, *J* = 6.4 Hz, 3H), 0.965-0.929 (t, *J* = 14.4 Hz, 3H).

**Example 13: Preparation of Compound 30012**

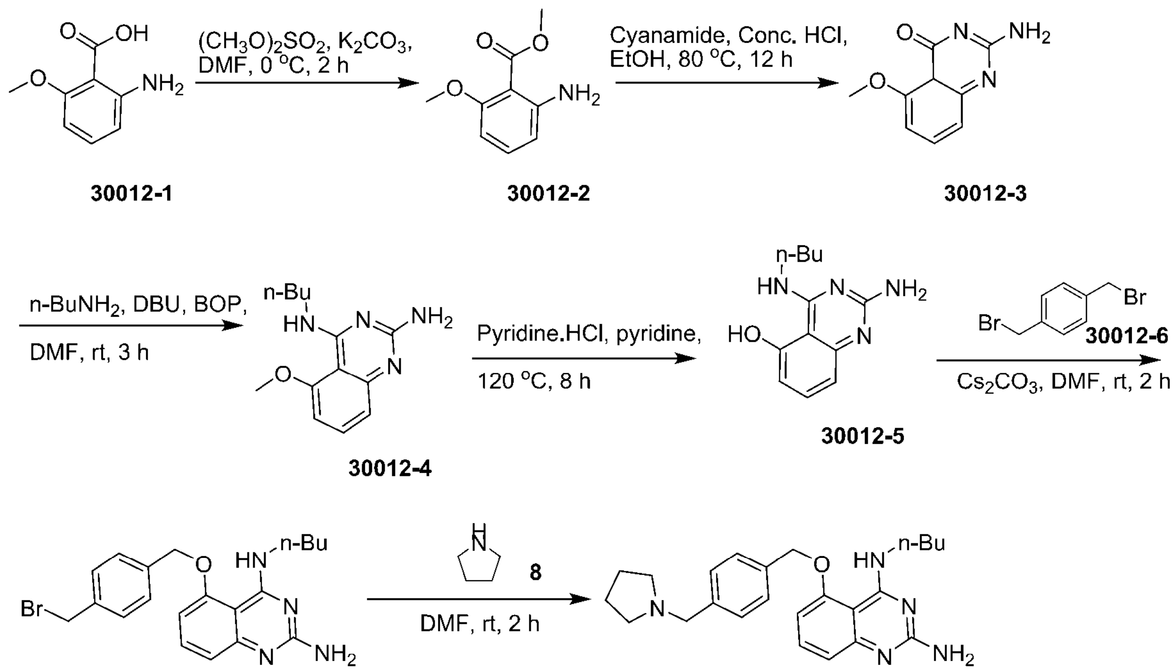


30012-1 30012-2 30012-3 30012-4 30012-5 30012-7 30012

**Step 1: General Method for Preparation of Compound 30012-2**

Under a nitrogen atmosphere, a solution of Compound 30012-1 (5g, 0.030 mol), $K_2CO_3$ (12.40g, 0.090 mol) and dimethyl sulfate (4.53g, 0.036 mol) in DMF (50 mL) was stirred at 0°C for 2 hours. The mixture was diluted with water (100 mL), extracted with EA (50 mL × 3), and evaporated under vacuum, and the crude product was purified by flash column chromatography (PE/EA = 2/1) to obtain Compound 30012-2 (3g, 54.85% yield) as a white oil.

**Step 2: General Method for Preparation of Compound 30012-3**

Under a nitrogen atmosphere, a solution of Compound 30012-2 (3g, 0.017 mol), cyanamide (1.39g, 0.033 mol) and concentrated hydrochloric acid (4.14 mL) in EtOH (30 mL) was stirred at 80°C for 12 hours. The yellow solid was collected by filtration to obtain the crude product, Compound 30012-3 (1.2g, 37.35% yield).

**Step 3: General Method for Preparation of Compound 30012-4**

At room temperature under a nitrogen atmosphere, DBU (2.88g, 0.019 mol), benzotriazol-1-yloxytris(dimethylamino)phosphonium hexafluorophosphate (BOP, 3.34g, 0.008 mol) and n-butylamine (1.38g, 0.019 mol) were added to a solution of Compound 30012-3 (1.2g, 0.006 mol) in DMF (10 mL). The reaction mixture was stirred at room temperature for 3 hours. The mixture was diluted with water (20 mL), extracted with EA (10 mL × 3), and evaporated under vacuum, and the crude product was purified by flash column chromatography (MeOH/DCM = 1/20) to obtain Compound 30012-4 (1g, 63.49% yield) as a yellow oil.

**Step 4: General Method for Preparation of Compound 30012-5**

Under a nitrogen atmosphere, a mixture of Compound 30012-4 (1g, 0.004 mol) and pyridine hydrochloride (2.84g, 0.025 mol) in pyridine (10 mL) was stirred at 120°C for 8 hours. The solution was evaporated under reduced pressure. The crude product was purified by reversed-phase C18 silica gel column chromatography (ACN-$H_2O$ = 35%), to obtain Compound 30012-5 (0.8g, 82.93% yield) as a yellow solid.

**Step 5: General Method for Preparation of Compound 30012-7**

Under a nitrogen atmosphere, a mixture of Compound 30012-5 (200 mg, 0.861 mmol), $Cs_2CO_3$ (842 mg, 2.583 mmol) and Compound 30012-6 (250 mg, 0.947 mmol) in DMF (5 mL) was stirred at room temperature for 2 hours. The mixture was diluted with water (10 mL), extracted with EA (5 mL × 3), and evaporated under vacuum to obtain a crude product, Compound 30012-7 (120 mg) as a yellow oil.

**Step 6: General Method for Preparation of Compound 30012**

Under a nitrogen atmosphere, a mixture of Compound 30012-7 (100 mg, 0.241 mmol) and Compound 30012-8, pyrrolidine (51 mg, 0.722 mmol) in DMF (2 mL) was stirred at room temperature for 2 hours. The mixture was filtered and the solvent was removed under reduced pressure. Then the crude product was purified by preparative HPLC (column: Phenomenex Luna C18, 250 mm × 100 mm × 10 μm; mobile phase: [A: $H_2O$ (0.1% FA), B: acetonitrile]; B%: 10%-50%, 40 min), to obtain Compound M030012 (20 mg, 20.06% yield) as a white solid.

**LCMS:** $[M+H]^+$ = 406.3; Rt = 0.814 min.

**$^1$H NMR:** (400 MHz, $d_6$-DMSO) $\delta$ 8.225 (s, 1H), 7.815 (s, 1H), 7.505 - 7.485 (dd, *J* = 8.0 Hz, 2H), 7.425 - 7.375 (m, 3H), 6.815 (d, *J* = 8.0 Hz, 1H), 6.725 (d, *J* = 10.0 Hz, 1H), 6.351 (s, 1H), 5.215 (s, 2H), 3.615 (s, 2H), 3.355 - 3.305 (dd, *J* = 8.4 Hz, 2H), 2.445 (s, 4H), 1.705 (d, *J* = 8.0 Hz, 4H), 1.123 (t, *J* = 7.6 Hz, 2H), 1.085 (t, *J* = 6.4 Hz, 2H), 0.758 (t, *J* = 6.4 Hz, 3H).

Compounds 30013, 30044, 30047 and 30048 were prepared with reference to the synthetic method of Compound 30012.

| | |
|---|---|
| 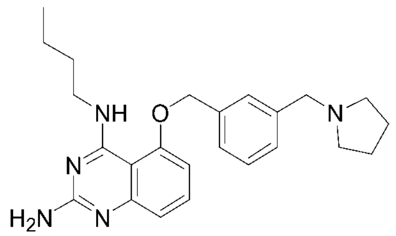 13 | $[M+H]^+$ = 406.3; Rt = 0.766 min. **$^1$H NMR:** (400 MHz, $d_6$-DMSO) $\delta$ 8.235 (s, 2H), 7.528 - 7.431 (m, 6H), 7.265 (s, 2H), 6.903 - 6.875 (dd, *J* = 8.0 Hz, 2H), 5.314 (s, 1H), 6.351 (s, 2H), 3.745 (s, 2H), 3.417 - 3.381 (t, *J* = 8.4 Hz, 2H), 2.565 - 2.501 (dd, *J* = 8.4 Hz, 4H), 1.738 (s, 4H), 1.123 (t, *J* = 7.6 Hz, 2H), 1.085 (t, *J* = 6.4 Hz, 2H), 0.795 - 0.765 (t, *J* = 7.6 Hz, 3H). |
| 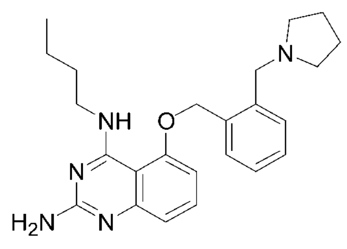 44 | $[M+H]^+$ = 406.3; Rt = 0.880 min. **$^1$H NMR:** (400 MHz, $CD_3OD$) $\delta$ 7.813 - 7.722 (m, 3H), 7.644 (dd, *J* = 5.6, 3.2 Hz, 2H), 7.294 (d, *J* = 8.0 Hz, 1H), 7.071 (d, *J* = 8.0 Hz, 1H), 5.627 (s, 2H), 4.643 (s, 2H), 3.642 - 3.572 (m, 2H), 3.528 (t, *J* = 7.2 Hz, 2H), 3.311 - 3.232 (m, 2H), 2.258 - 2.169 (m, 2H), 2.131 - 2.027 (m, 2H), 1.380 - 1.310 (m, 2H), 1.167 - 1.062 (m, 2H), 0.831 (t, *J* = 7.2 Hz, 3H). |

(continued)

| | |
|---|---|
| 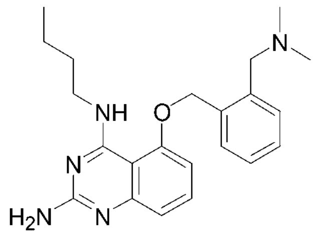 47 | $[M+H]^+$ = 380.1; Rt = 0.846 min. **$^1$H NMR:** (400 MHz, $CD_3OD$) $\delta$ 7.811 - 7.722 (m, 3H), 7.667 (dd, *J* = 5.2, 3.6 Hz, 2H), 7.280 (d, *J* = 8.4 Hz, 1H), 7.067 (d, *J* = 8.4 Hz, 1H), 5.595 (s, 2H), 4.585 (s, 2H), 3.529 (t, *J* = 7.2 Hz, 2H), 2.946 (s, 6H), 1.380 - 1.298 (m, 2H), 1.160 - 1.067 (m, 2H), 0.830 (t, *J* = 7.2 Hz, 3H). |
| 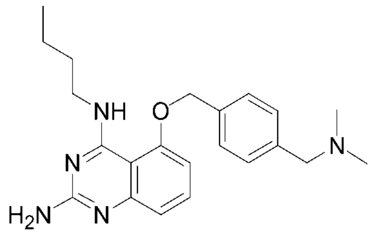 48 | $[M+H]^+$ = 380.2; Rt = 0.808 min. **$^1$H NMR:** (400 MHz, $CD_3OD$) $\delta$ 7.639 (s, 1H), 7.554 (d, *J* = 8.0 Hz, 2H), 7.475 (d, *J* = 8.0 Hz, 2H), 7.063 (d, *J* = 8.4 Hz, 1H), 6.961 (d, *J* = 8.4 Hz, 1H), 5.339 (s, 2H), 3.592 (s, 2H), 3.518 (t, *J* = 7.2 Hz, 2H), 2.316 (s, 6H), 1.480 - 1.380 (m, 2H), 1.227 (dq, *J* = 14.4, 7.2 Hz, 2H), 0.856 (t, *J* = 7.2 Hz, 3H). |

**Example 14: Preparation of Compound 30014**

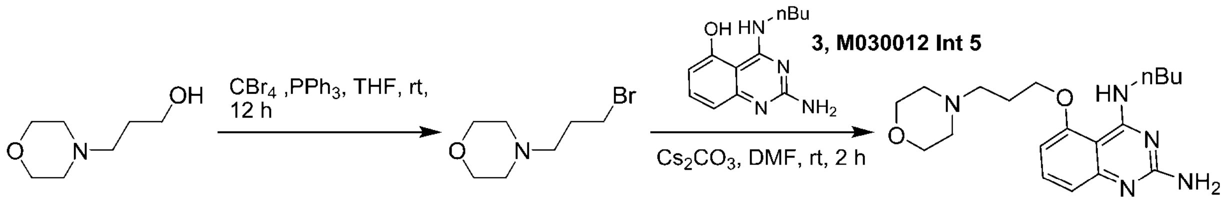


**30014-1** **30014-2** **30014**

**Step 1: General Method for Preparation of Compound 30014-2**

At room temperature under a nitrogen atmosphere, a reaction mixture of Compound 30014-1 (200 mg, 1.379 mmol), $PPh_3$ (387 mg, 1.517 mmol) and $CBr_4$ (490 mg, 1.517 mmol) in THF (50 mL) was stirred for 12 hours. The mixture was diluted with a mixture of $H_2O/Et_2O$ (1:3, 20 mL), and extracted with 1 M HCl (20 mL). The aqueous phase was adjusted to pH 10 with 4 M NaOH, then extracted with EA (20 mL × 3), and evaporated under vacuum to obtain the crude product, Compound 30014-2 (200 mg) as a white oil.

**Step 1: General Method for Preparation of Compound 30014**

Under a nitrogen atmosphere, a mixture of Compound 30014-2 (99 mg, 0.474 mmol), Compound 30012-5 (100 mg, 0.431 mmol) and $Cs_2CO_3$ (421 mg, 1.292 mmol) in DMF (2 mL) was stirred at room temperature for 2 hours. The mixture was filtered and the solvent was removed under reduced pressure. Then the crude product was purified by preparative HPLC (column: Phenomenex Luna C18, 250 mm × 100 mm × 10 μm; mobile phase: [A: $H_2O$ (0.1% FA), B: acetonitrile]; B%: 5%-30%, 20 min), to obtain Compound M030014 (20 mg, 12.01% yield) as a white solid.

**LCMS:** $[M+H]^+$ = 360.3.

**$^1$H NMR:** (400 MHz, $d_6$-DMSO) $\delta$ 8.305 (s, 1H), 7.995 - 7.981 (d, *J* = 8.0 Hz, 1H), 7.415 - 7.395 (dd, *J* = 8.0 Hz, 1H), 6.835 - 6.815 (dd, *J* = 8.0 Hz, 1H), 6.735 (s, 1H), 6.665 - 6.645 (dd, *J* = 8.0 Hz, 1H), 4.225 - 4.195 (dd, *J* = 8.0 Hz, 2H), 3.596(s, 4H), 3.475 (s, 2H), 2.505 - 2.385 (m, 6H), 1.995 (d, *J* = 8.4 Hz, 2H), 1.585 (d, *J* = 8.4 Hz, 2H), 1.415 (d, *J* = 8.4 Hz, 2H) 0.965 - 0.925 (t, *J* = 7.6 Hz, 3H).

**Example 15: Preparation of Compound 30016**

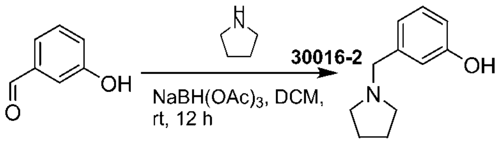


30016-1 30016-3

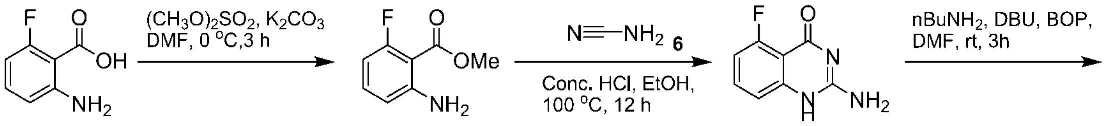


30016-4 30016-5 30016-7

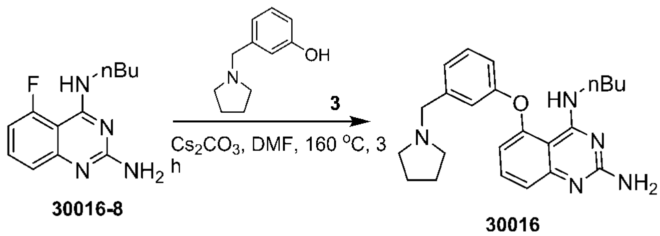


30016-8

30016

**Step 1: General Method for Preparation of Compound 30016-3**

At 0°C under a nitrogen atmosphere, Compound 30016-2, pyrrolidine (1.28g, 0.018 mol) was added dropwise to a solution of Compound 30016-1 (2g, 0.016 mol) in DCM (15 mL), and then $NaBH(OAc)_3$ (5.21g, 0.025 mol) was added. The reaction mixture was then stirred at room temperature for 12 hours. The mixture was diluted with water (30 mL), extracted with DCM (30 mL × 3), dried over $Na_2SO_4$ and concentrated. The crude product 30016-3 (1g, 0.005 mmol, 31.10% yield) was obtained as the white solid.

**Step 2: General Method for Preparation of Compound 30016-5**

Under a nitrogen atmosphere, a solution of Compound 30016-4 (1g, 0.006 mol), $K_2CO_3$ (2.65g, 0.019 mol) and compound dimethyl sulfate (0.97g, 0.008 mol) in DMF (10 mL) was stirred at 0°C for 3 hours. The mixture was diluted with water (50 mL) and extracted with EA (50 mL × 3). The organic layer was collected, washed with saturated brine (50 mL × 5), dried over $Na_2SO_4$ and concentrated. The crude product was purified by flash column chromatography (PE/EA = 2/1) to obtain Compound 30016-5 (1g, 82.81% yield) as a yellow oil.

**Step 3: General Method for Preparation of Compound 30016-7**

Under a nitrogen atmosphere, a solution of Compound 30016-5 (1g, 0.006 mol), cyanamide (0.4g, 0.010 mmol) and concentrated hydrochloric acid (1 mL) in EtOH (30 mL) was stirred at 100°C for 12 hours. The white solid was collected by filtration to obtain the crude product, Compound 30016-7 (0.5g, 43.75% yield).

**Step 4: General Method for Preparation of Compound 30016-8**

Reference was made to Step 3 of preparing Compound 30012.

**Step 5: General Method for Preparation of Compound 16**

Under a nitrogen atmosphere, a mixture of Compound 30016-3 (45 mg, 0.256 mmol), $Cs_2CO_3$ (83 mg, 0.256

mmol) and Compound 30016-8 (50 mg, 0.213 mmol) in DMF (1 mL) was stirred at 160°C for 3 hours. The mixture was diluted with MeOH (5 mL). Then a crude product was purified by preparative HPLC (column: Phenomenex Luna C18, 250 mm × 100 mm × 10 μm; mobile phase: [A: $H_2O$ (0.1% FA), B: acetonitrile]; B%: 10%-50%, 40 min), to obtain Compound M030016 (1.05 mg, 0.003 mmol, 1.3% yield) as a white solid.

**LCMS:** $[M+H]^+$ = 392.1; Rt = 0.852 min.

**$^1$H NMR:,** (400 MHz, MeOD) δ 7.440 (d, *J* = 8.0 Hz, 1H), 7.380 (d, *J* = 8.0 Hz, 1H), 7.250 (d, *J* = 7.6 Hz, 1H), 7.152 (s, 1H), 7.070 (dd, *J* = 8.0, 2.0 Hz, 1H), 7.020 (d, *J* = 8.4 Hz, 1H), 6.414 (d, *J* = 8.0 Hz, 1H), 3.675 (s, 2H), 3.567 (s, 2H), 2.574 (s, 4H), 1.813 (t, *J* = 3.2 Hz, 4H), 1.600 (s, 2H), 1.368 (s, 2H), 0.915 - 0.889 (m, 3H).

**Example 16: Preparation of Compound 30046**

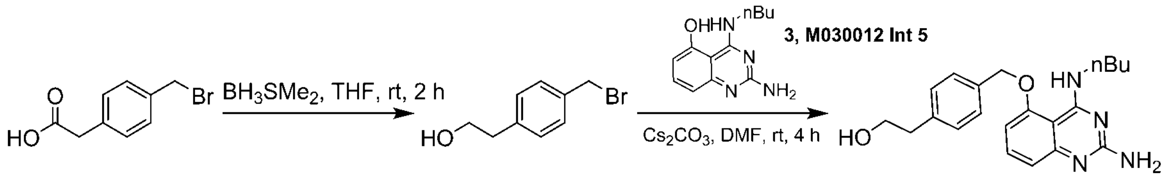


30046-1 30046-2 30046-4

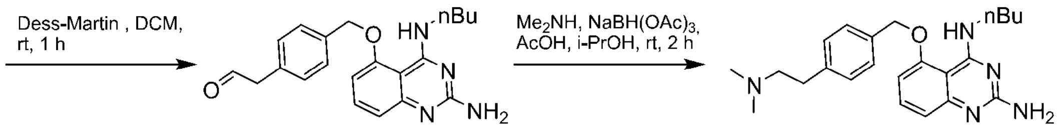


30046-5 30046

**Step 1: General Method for Preparation of Compound 30046-2**

At 0°C under a nitrogen atmosphere, $BH_3 \cdot SMe_2$ (497 mg, 6.548 mmol) was added dropwise to a THF (20 mL) solution of Compound 30046-1 (500 mg, 2.183 mmol). The reaction mixture was stirred at room temperature for 2 hours. The mixture was diluted with water (10 mL), filtered and concentrated to obtain the crude product 30046-2 (350 mg) as a white solid.

**Step 2: General Method for Preparation of Compound 30046-4**

Under a nitrogen atmosphere, a solution of Compound 30046-2 (350 mg, 1.627 mmol), $Cs_2CO_3$ (1591 mg, 4.882 mmol) and Compound 30012-5 (378 mg, 1.627 mmol) in DMF (10 mL) was stirred at room temperature for 4 hours. The mixture was diluted with water (2 mL) and extracted with DCM (100 mL). The organic layer was collected, washed with water and saturated brine, dried over $Na_2SO_4$ and concentrated. The crude product was purified by flash column chromatography (MeOH/DCM = 10%) to obtain Compound 30046-4 (280 mg, 0.76 mmol, 46.96% yield) as a yellow solid.

**Step 3: General Method for Preparation of Compound 30046-5**

At room temperature under a nitrogen atmosphere, Dess-Martin periodinane (116 mg, 0.27 mmol) was added to a DCM (1 mL) solution of Compound 30046-4 (50 mg, 0.14 mmol) during stirring. The reaction mixture was stirred at room temperature for 1 hour. The reaction mixture was concentrated under reduced pressure. The crude product was purified by flash column chromatography (MeOH/DCM = 5%) to obtain Compound 30046-5 (30 mg, 50% yield) as a brown solid.

**Step 4: General Method for Preparation of Compound 30046**

At 0°C under a nitrogen atmosphere, $NaBH(OAc)_3$ (26 mg, 0.12 mmol) was added to a solution of Compound 30046-5 (30 mg, 0.08 mmol), dimethylamine (0.05 mL, 0.10 mmol, 2 M in THF) and AcOH (7 mg, 0.12 mmol) in iPrOH (1 mL) during stirring. The reaction mixture was stirred at room temperature for 2 hours. The reaction mixture was concentrated under reduced pressure and purified by preparative HPLC (column: Phenomenex Luna C18, 250 mm × 100 mm × 10 μm; mobile phase: [A: $H_2O$ (0.1% FA), B: acetonitrile]; B%: 10%-50%, 40 min), to obtain Compound

M030046 (1.60 mg, 4% yield) as a white solid.

**LCMS:** $[M+H]^+$ = 394.4; Rt = 1.761 min.

**$^1$H NMR:** (400 MHz, $CD_3OD$) $\delta$ 7.646 (t, $J$ = 8.4 Hz, 1H), 7.508 (d, $J$ = 8.0 Hz, 2H), 7.370 (d, $J$ = 8.0 Hz, 2H), 7.067 (d, $J$ = 8.4 Hz, 1H), 6.963 (d, $J$ = 8.4 Hz, 1H), 5.308 (s, 2H), 3.512 (t, $J$ = 6.8 Hz, 2H), 2.967 - 2.867 (m, 4H), 2.562 (s, 6H), 1.434 - 1.362 (m, 2H), 1.211 - 1.131 (m, 2H), 0.834 (t, $J$ = 7.2 Hz, 3H).

Compound 50 was prepared with reference to the same synthetic method of Compound 30046.

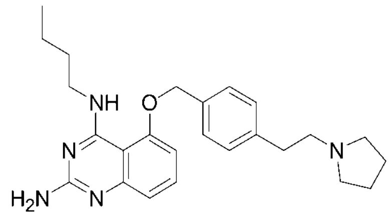

50

LC-MS: $[M+H]^+$ = 420.2; Rt = 0.871 min.

**$^1$H NMR:** (400 MHz, d6-DMSO) $\delta$ 8.330 (s, 1H), 7.763 (s, 1H), 7.443 (m, 2H), 7.359 (m, 1H), 7.315 - 7.295 (d, J = 8.0 Hz, 2H), 6.813 - 6.792 (d, J = 8.4 Hz, 1H), 6.720 - 6.700 (d, J = 8.0 Hz, 1H), 6.242 (s, 2H), 5.186 (s, 2H), 3.340 - 3.294 (m, 6H), 2.793 - 2.756 (t, J = 14.8 Hz, 2H), 2.660 - 2.622 (t, J = 15.2 Hz, 2H), 1.682 (s, 4H), 1.301 - 1.264 (m, 2H), 1.127 - 1.071 (m, 2H), 0.783 - 0.746 (t, J = 14.8 Hz, 3H).

**Example 17: Preparation of Compound 30049**

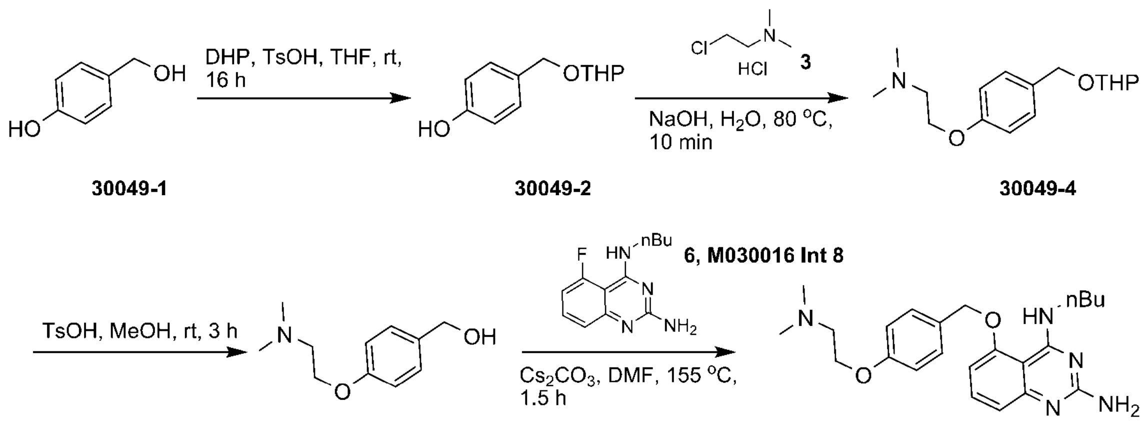


30049-1 30049-2 30049-4 30049-5 30049

**Step 1: General Method for Preparation of Compound 30049-2**

At 0°C under a nitrogen atmosphere, a reaction mixture of Compound 30049-1 (5.0g, 40.30 mmol), 3,4-dihydro-2H-pyran (4.07g, 48.30 mmol) and TsOH-$H_2O$ (80 mg, 0.40 mmol) in THF (25 mL) solution was stirred at room temperature for 16 hours. The mixture was extracted with EA (20 mL × 2), filtered and concentrated to obtain the crude product 30049-2 (9.0g) as a brown oil.

**Step 2: General Method for Preparation of Compound 30049-4**

At 80°C under a nitrogen atmosphere, Compound 30049-3 (2076 mg, 14.40 mmol) was added to a solution of Compound 30049-2 (3000 mg, 14.40 mmol) and sodium hydroxide (1152 mg, 28.80 mmol) in water (12 mL) during stirring. The reaction mixture was stirred at 80°C for 10 minutes. The mixture was extracted with EA (10 mL × 2), filtered and concentrated. The crude product was purified by flash column chromatography (MeOH/DCM = 3%-5%) to obtain Compound 30049-4 (800 mg, 18% yield) as a colorless oil.

**Step 3: General Method for Preparation of Compound 30049-5**

At room temperature under a nitrogen atmosphere, a reaction mixture of Compound 30049-4 (115 mg, 0.41 mmol) and $TsOH \cdot H_2O$ (78 mg, 0.41 mmol) in MeOH (3 mL) was stirred at room temperature for 3 hours. The mixture was diluted with a saturated aqueous solution of $NaHCO_3$, and extracted with EA (5 mL × 2). The organic layer was concentrated to obtain the crude product 30049-5 (80 mg) as a colorless oil.

**Step 4: General Method for Preparation of Compound 30049**

Under a nitrogen atmosphere, a mixture of Compound 30049-5 (40 mg, 0.20 mmol), $Cs_2CO_3$ (67 mg, 0.20 mmol) and Compound 30016-8 (40 mg, 0.17 mmol) in DMF (1 mL) was stirred at 155°C for 1.5 hours. The mixture was extracted with EA (5 mL × 2), and the organic layer was concentrated to obtain a crude product. Then the crude product was purified by preparative HPLC (column: Phenomenex Luna C18, 250 mm × 100 mm × 10 μm; mobile phase: [A: $H_2O$ (0.1% FA), B: acetonitrile]; B%: 10%-50%, 40 min), to obtain Compound M030049 (1.66 mg, 2% yield) as a white solid.

**LCMS:** $[M+H]^+$ = 410.2; Rt = 0.843 min.

**$^1$H NMR:** (400 MHz, $CD_3OD$) $\delta$ 7.547 (t, *J* = 8.4 Hz, 1H), 7.480 (d, *J* = 8.4 Hz, 2H), 7.052 (d, *J* = 8.8 Hz, 2H), 6.932 (dd, *J* = 8.0, 4.8 Hz, 2H), 5.182 (s, 2H), 4.149 (t, *J* = 5.2 Hz, 2H), 3.419 (t, *J* = 6.8 Hz, 2H), 2.817 (t, *J* = 5.2 Hz, 2H), 2.370 (s, 6H), 1.378 - 1.301 (m, 2H), 1.152 - 1.067 (m, 2H), 0.806 (t, *J* = 7.2 Hz, 3H).

**Example 18: Preparation of Compound 30059**

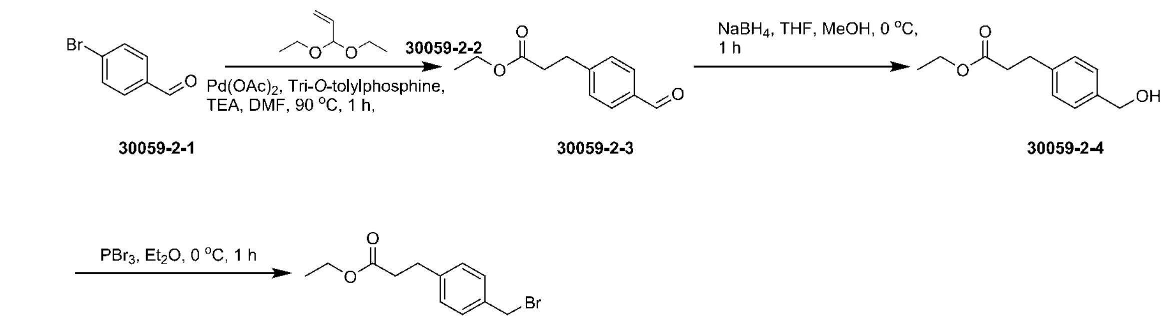


30059-2-1 30059-2-3 30059-2-4 30059-2

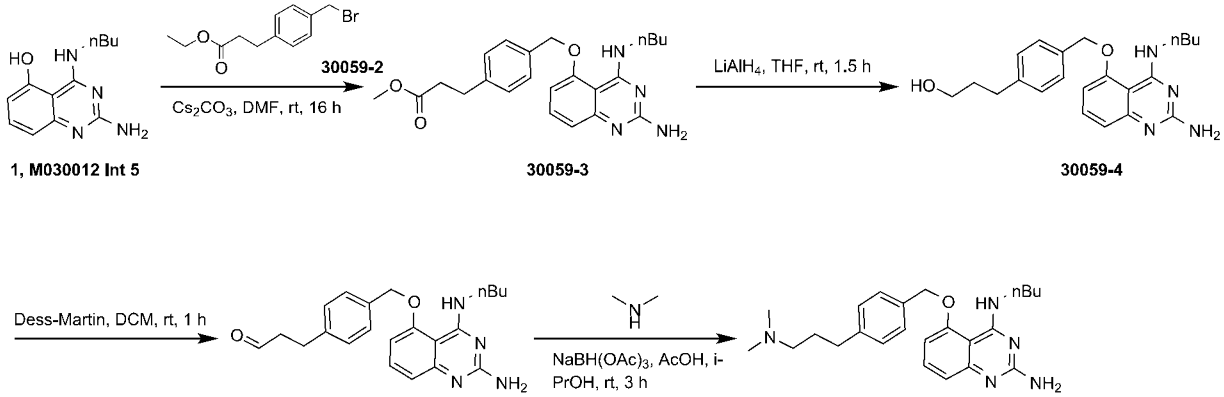


1, M030012 Int 5 30059-3 30059-4 30059-5 30059

**Step 1: General Method for Preparation of Compound 30059-2-3**

Under a nitrogen atmosphere, a reaction mixture of Compound 30059-2-1 (5g, 0.027 mol), Compound 30059-2-2 (10.54g, 0.081 mol), palladium acetate (0.18g, 0.001 mol), tris(o-tolyl)phosphine (0.99g, 0.003 mol) and TEA (5.46g, 0.054 mol) in DMF (20 mL) was stirred at 90°C for 1 hour. The mixture was concentrated, diluted with water (20

mL) and extracted with EA (20 mL × 5). The organic phase was washed with saturated brine, dried over anhydrous $Na_2SO_4$ and evaporated under vacuum. The crude product was purified by flash column chromatography (EA/PE = 50%) to obtain Compound 30059-2-3 (2.6g, 0.0126 mol, 46.67% yield) as a white solid.

**Step 2: General Method for Preparation of Compound 30059-2-4**

At 0°C under a nitrogen atmosphere, $NaBH_4$ (0.71g, 0.019 mol) was added to THF/MeOH (3:1, 10 mL) solution of Compound 30059-2-3 (2.6g, 0.0126 mol) during stirring. The reaction mixture was stirred for 1 hour at 0°C. The mixture was concentrated, diluted with water (10 mL) and extracted with EA (10 mL × 3). The organic phase was washed with saturated brine, dried over anhydrous $Na_2SO_4$ and evaporated under vacuum. The crude product was purified by flash column chromatography (DCM/MeOH = 10/ 1) to obtain Compound 30059-2-4 (1.9g, 0.009 mol, 71.43% yield) as a white solid.

**Step 3: General Method for Preparation of Compound 30059-2**

Under a nitrogen atmosphere, a reaction mixture of Compound 30059-2-4 (1.9g, 0.009 mol) and $PBr_3$ (3.69g, 0.0134 mol) in $Et_2O$ (10 mL) was stirred at 0°C for 1 hour. The mixture was concentrated, diluted with water (10 mL) and extracted with EA (10 mL × 3). The organic phase was washed with saturated brine, dried over anhydrous $Na_2SO_4$ and evaporated under vacuum. The crude product was purified by flash column chromatography (EA/PE = 50%) to obtain Compound 30059-2 (1.3g, 0.004 mol, 47.25% yield) as a white solid.

**Step 4: General Method for Preparation of Compound 30059-3**

Under a nitrogen atmosphere, a solution of Compound 30059-2 (700 mg, 2.583 mmol), $Cs_2CO_3$ (2104 mg, 6.458 mmol) and Compound 30012-5 (500 mg, 2.153 mmol) in DMF (5 mL) was stirred at room temperature for 16 hours. The mixture was concentrated, diluted with water (30 mL) and extracted with EA (30 mL × 3). The organic layer was collected, washed with water and saturated brine, dried over $Na_2SO_4$ and concentrated. The crude product was purified by flash column chromatography (MeOH/DCM = 1/10) to obtain Compound 30059-3 (600 mg, 1.278 mmol, 59.37% yield) as a white solid.

**Step 5: General Method for Preparation of Compound 30059-4**

At 0°C under a nitrogen atmosphere, a solution of Compound 30059-3 (600 mg, 1.420 mmol) in THF (5 mL) was added dropwise to a solution of $LiAlH_4$ (108 mg, 2.840 mmol) in THF (5 mL). The reaction mixture was stirred at room temperature for 1.5 hours. The mixture was concentrated, diluted with water (10 mL) and extracted with EA (10 mL × 3). The organic phase was washed with saturated brine, dried over anhydrous $Na_2SO_4$ and evaporated under vacuum. The crude product was purified by flash column chromatography (DCM/MeOH = 10/1) to obtain Compound 30059-4 (340 mg, 0.804 mmol, 56.63% yield) as a white solid.

**Step 6: General Method for Preparation of Compound 30059-5**

Reference was made to Step 3 of preparing Compound 30046.

**Step 7: General Method for Preparation of Compound 59**

Reference was made to Step 4 of preparing Compound 30046.

**LCMS:** $[M+H]^+$ = 408.1; Rt = 0.835 min.

**$^1$H** NMR (400 MHz, $d_4$-MeOD) $\delta$ 8.536 (s, 1H), 7.601 (t, $J$ = 8.4 Hz, 1H), 7.469 (s, 2H), 7.337 (d, $J$ = 8.0 Hz, 2H), 6.975 (dd, $J$ = 25.8, 8.4 Hz, 2H), 5.266 (s, 2H), 3.457 (t, $J$ = 6.8 Hz, 2H), 2.723 - 2.685 (m, 2H), 2.610 - 2.571 (m, 2H), 2.422 (s, 6H), 1.895 (dt, $J$ = 15.8, 8.0 Hz, 2H), 1.414 - 1.342 (m, 2H), 1.191 - 1.117 (m, 2H), 0.839 - 0.764 (m, 3H).

**Example 19: Preparation of Compound 30004**

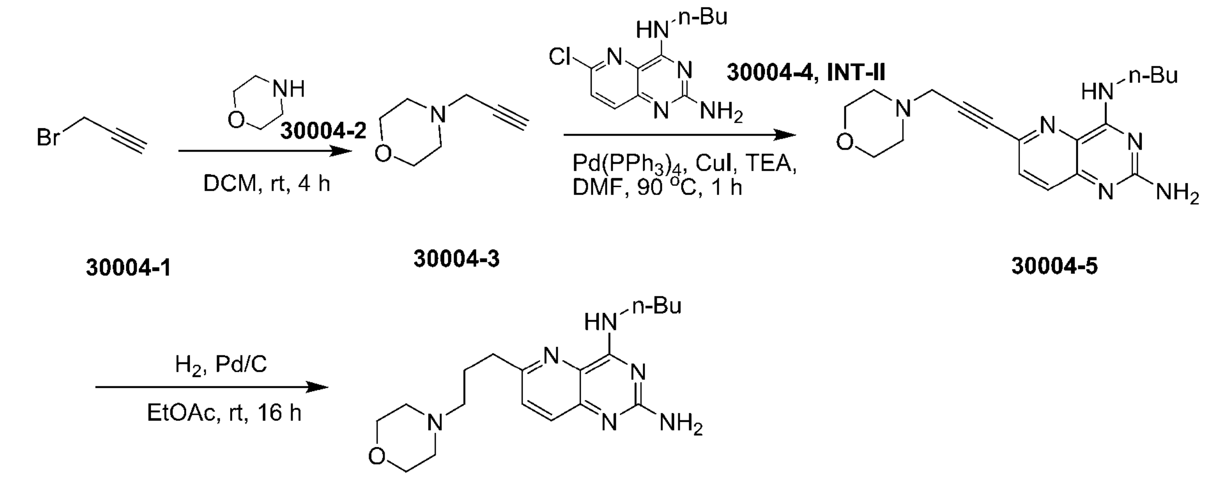


30004-1 30004-3 30004-5

30004

**Step 1: General Method for Preparation of Compound 30004-3**

Under a nitrogen atmosphere, a solution of Compounds 30004-1 (500 mg, 4.20 mmol) and 30004-2 (806 mg, 9.25 mmol) in DCM (5 mL) was stirred at room temperature for 4 hours. The mixture was concentrated and extracted with DCM (10 mL × 2). The organic layer was collected, washed with saturated brine, dried over $Na_2SO_4$ and concentrated to obtain the crude product, Compound 30004-3 (300 mg) as a colorless oil.

**Step 2: General Method for Preparation of Compound 30004-5**

Under a nitrogen atmosphere, a reaction mixture of Compound 30004-4 (i.e., INT-II, 30 mg, 0.12 mmol), Compound 30004-3 (18 mg, 0.14 mmol), $Pd(PPh_3)_4$ (21 mg, 0.02 mmol), copper(I) iodide (3 mg, 0.02 mmol) and triethylamine (36 mg, 0.36 mmol) in DMF (0.5 mL) was stirred at 90°C for 1 hour. The mixture was extracted with EA (10 mL × 2), the organic phase was evaporated under vacuum, and the crude product was purified by flash column chromatography (DCM/MeOH = 20/1) to obtain Compound 30004-5 (40 mg, 90% yield) as a brown solid.

**Step 3: General Method for Preparation of Compound 30004**

Under a hydrogen atmosphere, a solution of Compound 30004-5 (40 mg, 0.12 mmol) and Pd/C (10 mg) in EA (3 mL) was stirred at room temperature for 16 hours. The mixture was filtered, and the organic layer was concentrated and then purified by preparative HPLC (column: Phenomenex Luna C18, 250 mm × 100 mm × 10 μm; mobile phase (A: $H_2O$ (0.1% FA), B: acetonitrile); B%: 15%-55%, 40 min), to obtain Compound M030004 (8.2 mg, 20% yield) as a white solid.

**LC-MS:** $[M+H]^+$ = 345.3; Rt = 0.631 min.

**$^1$H NMR:** (400 MHz, CDCl3) $\delta$ 8.044 (br s, 1H), 7.835 (d, *J* = 8.4 Hz, 1H), 7.438 (d, *J* = 8.4 Hz, 1H), 5.971 (br s, 2H), 3.825 (t, *J* = 4.8 Hz, 4H), 3.660 (dd, *J* = 13.6, 6.8 Hz, 2H), 2.912 (t, *J* = 7.2 Hz, 2H), 2.671 (s, 4H), 2.607 - 2.550 (m, 2H), 2.201 - 2.102 (m, 2H), 1.759 - 1.681 (m, 2H), 1.510 - 1.401 (m, 2H), 0.996 (t, *J* = 7.2 Hz, 3H).

**Example 20: Preparation of Compound 30005**

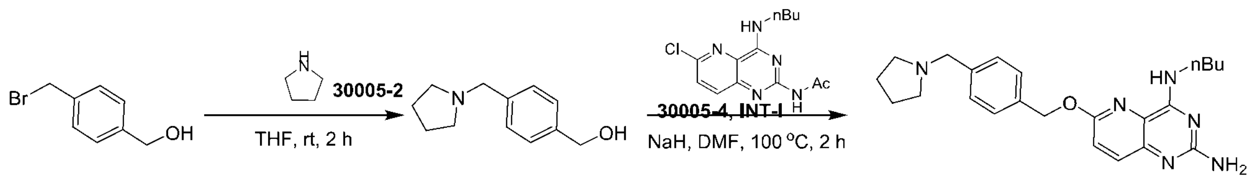


30005-1 30005-3 30005

### Step 1: General Method for Preparation of Compound 30005-3

At 0°C under a nitrogen atmosphere, a THF (5 mL) solution of Compound 30005-1 (500 mg, 2.487 mmol) was added dropwise to a THF (10 mL) solution of pyrrolidine (707 mg, 9.947 mmol) during stirring. The reaction mixture was stirred at room temperature for 2 hours. The reaction mixture was concentrated under reduced pressure and dissolved in 1 M HCl (25 mL). The resulting solution was washed with $Et_2O$ (× 2) and cooled with ice water, and NaOH (2g, 50 mmol) was added. The mixture was extracted with EA, and the organic layer was collected, dried over $Na_2SO_4$ and concentrated to obtain the crude product, Compound 30005-3 (440 mg) as a yellow oil.

### Step 2: General Method for Preparation of Compound 30005

At 0°C under a nitrogen atmosphere, a DMF (1 mL) solution of Compound 30005-4 (i.e., INT-I, 41 mg, 0.214 mmol) and NaH (10 mg, 0.255 mmol) was stirred for 0.5 hours. Then a DMF (1 mL) solution of Compound 30005-3 (30 mg, 0.102 mmol) was added dropwise. The reaction mixture was stirred for 2 hour at 100°C. The mixture was diluted with MeOH, and the organic layer was concentrated and then purified by preparative HPLC (column: Phenomenex Luna C18, 250 mm × 100 mm × 10 μm; mobile phase (A: $H_2O$ (0.1% FA), B: acetonitrile); B%: 5%-50%, 35 min), to obtain Compound 30005 (7.66 mg, 0.02 mmol, 17% yield) as a white solid.

**LCMS:** $[M+H]^+$ = 407.2; Rt = 0.911 min.

**$^1$H NMR:** (400 MHz, CDCl3) δ 7.750 (d, *J* = 9.2 Hz, 1H), 7.418 (s, 4H), 7.117 (d, *J* = 9.2 Hz, 1H), 6.825 (s, 1H), 6.234 (s, 2H), 5.368 (s, 2H), 3.752 (s, 2H), 3.598 (dd, *J* = 13.6, 6.8 Hz, 2H), 2.668 (s, 4H), 1.855 (s, 4H), 1.732 - 1.659 (m, 2H), 1.460 (dd, *J* = 15.2, 7.4 Hz, 2H), 1.003 (t, *J* = 7.2 Hz, 3H).

### Example 21: Preparation of Compound 30006

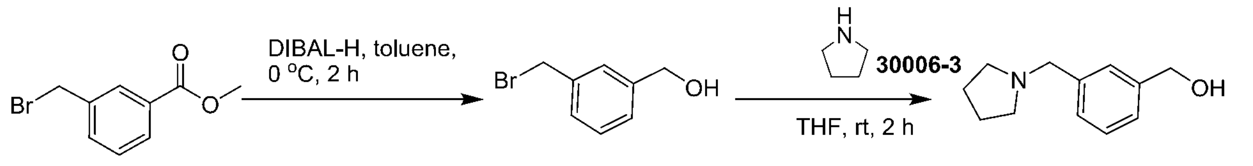


**30006-1** **30006-2** **30006-4**

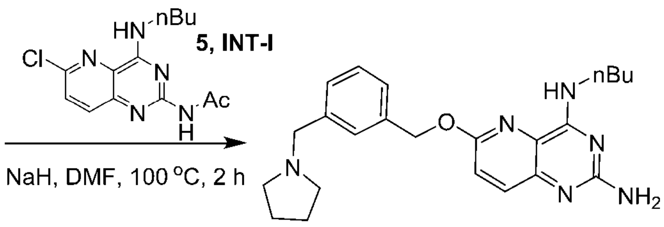


**30006**

### Step 1: General Method for Preparation of Compound 30006-2

Under a nitrogen atmosphere, DIBAL-H (621 mg, 4.365 mmol) was added dropwise to a toluene (5 mL) solution of Compound 30006-1 (500 mg, 2.183 mmol) at 0°C during stirring. The reaction mixture was stirred for 2 hour at 0°C. The mixture was concentrated and extracted with EA, and the organic layer was collected, dried over $Na_2SO_4$ and concentrated to obtain the crude product, Compound 30006-2 (350 mg) as a yellow oil.

Other steps were performed to prepare Compound 30006 with reference to the synthetic method of Compound 30005.

**LCMS:** $[M+H]^+$ = 407.2.

**$^1$H NMR:** 400 MHz, CDCl3) δ 7.850 (d, *J* = 9.2 Hz, 1H), 7.694 (s, 1H), 7.440 - 7.382 (m, 3H), 7.250 - 7.212 (m, 1H), 7.167 (d, *J* = 9.2 Hz, 1H), 5.410 (s, 2H), 3.984 (s, 2H), 3.644 (d, *J* = 6.4 Hz, 2H), 2.950 (s, 4H), 1.987 (s, 4H), 1.706 (t, *J* = 7.2 Hz, 2H), 1.478 - 1.440 (m, 2H), 1.000 (t, *J* = 7.2 Hz, 3H).

**Example 22: Preparation of Compound 30007**

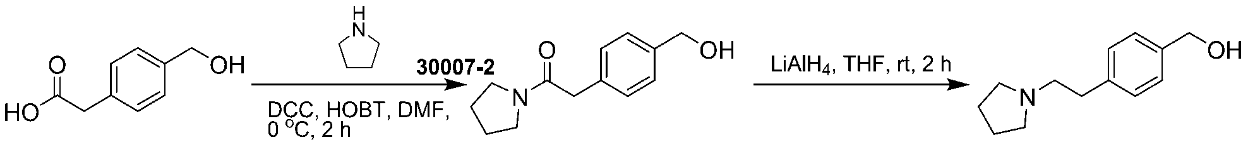


**30007-1** **30007-3** **30007-4**

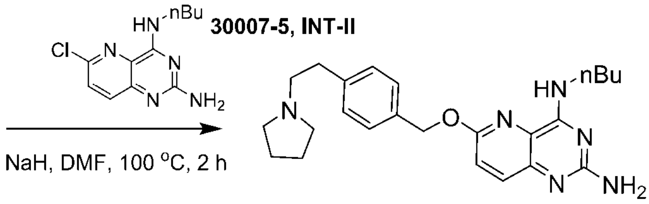


**30007**

**Step 1: General Method for Preparation of Compound 30007-3**

Under a nitrogen atmosphere, a reaction mixture of Compound 30007-1 (500 mg, 3.009 mmol), pyrrolidine (235 mg, 3.310 mmol), DCC (683 mg, 3.310 mmol) and HOBT (447 mg, 3.310 mmol) in DMF (5 mL) was stirred at 0°C for 2 hours. The mixture was concentrated, diluted with water (30 mL), and extracted with EA (30 mL × 3). The organic layer was collected, washed with saturated brine (40 mL × 5), dried over $Na_2SO_4$ and concentrated. The crude product was purified by flash column chromatography (EA/PE = 1/5) to obtain the crude product, Compound 30007-3 (230 mg, 34.5% yield) as a colorless oil.

**Step 2: General Method for Preparation of Compound 30007-4**

Under a nitrogen atmosphere, a reaction mixture of Compound 30007-3 (50 mg, 0.228 mmol) and $LiAlH_4$ (17 mg, 0.456 mmol) in THF (1 mL) was stirred at 25°C for 2 hours. The mixture was concentrated and extracted with EA (30 mL × 3), and the organic layer was collected, dried over $Na_2SO_4$ and concentrated to obtain the crude product, Compound 30007-4 (19 mg, 0.09 mmol, 38.55% yield) as a yellow oil.

Other steps were performed to prepare Compound 30007 with reference to the synthetic method of Compound 30005.

**LCMS:** $[M+H]^+$ = 421.1.

**$^1$H** NMR: (400 MHz, CDCl3) δ 7.850 (d, *J* = 9.2 Hz, 1H), 7.410 (d, *J* = 8.0 Hz, 2H), 7.287 (s, 2H), 7.140 (d, *J* = 9.2 Hz, 1H), 7.046 (s, 1H), 5.349 (s, 2H), 3.646 - 3.595 (m, 2H), 3.180 (s, 8H), 2.099 (s, 4H), 1.724 - 1.687 (m, 2H), 1.460 (dd, *J* = 14.8, 7.2 Hz, 2H), 1.010 (t, *J* = 7.2 Hz, 3H).

**Example 23: Preparation of Compound 30008**

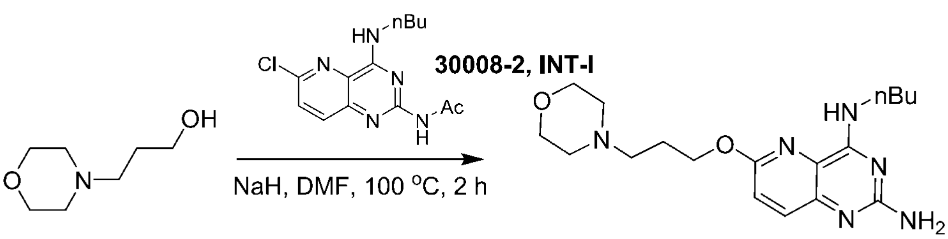


**30008-1** **30008**

Compound 30008 was prepared with reference to the synthetic method of Compound 30007.

**LCMS:** [M+H] $^+$ = 361.3;

**$^1$H NMR:** (400 MHz, CDCl3) δ 7.874 (d, *J* = 9.2 Hz, 1H), 7.479 (s, 1H), 7.080 (d, *J* = 9.2 Hz, 1H), 4.440 (t, *J* = 4.4 Hz, 2H), 3.844 (s, 4H), 3.664 - 3.612 (m, 2H), 2.741 (s, 6H), 2.136 (d, *J* = 5.2 Hz, 2H), 1.700 (dd, *J* = 14.8, 7.6 Hz, 2H), 1.443 (dt, *J* = 14.8, 7.6 Hz, 2H), 1.000 (t, *J* = 7.6 Hz, 3H).

**Example 24: Preparation of Compound 30009**

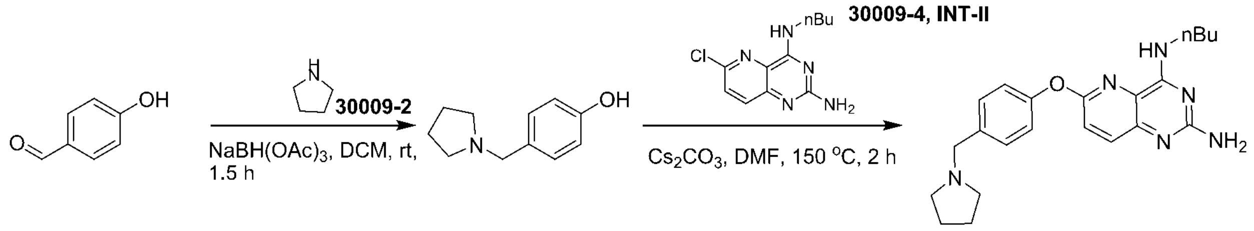


30009-1 30009-3 30009

**Step 1: General Method for Preparation of Compound 30009-3**

At room temperature under a nitrogen atmosphere, $NaBH(OAc)_3$ (1168 mg, 5.509 mmol) was added to a DCM (5 mL) solution of Compound 30009-1 (500 mg, 3.673 mmol) and pyrrolidine (287 mg, 4.040 mmol). The reaction mixture was stirred at room temperature for 1.5 hours. The mixture was extracted with EA, and the organic layer was collected, dried over $Na_2SO_4$ and concentrated to obtain the crude product, Compound 30009-3 (400 mg) as a yellow solid.

**Step 2: General Method for Preparation of Compound 30009**

Under a nitrogen atmosphere, a solution of Compound 30009-3 (42 mg, 0.238 mmol), Compound 30009-4 (that is, INT-II, 50 mg, 0.199 mmol) and $Cs_2CO_3$ (78 mg, 0.238 mmol) in DMF (1 mL) was stirred at 150°C for 2 hours. Then the mixture was diluted with MeOH and purified by preparative HPLC (column: Phenomenex Luna C18, 250 mm × 100 mm × 10 μm; mobile phase: [A: $H_2O$ (0.1% FA), B: acetonitrile]; B%: 10%-40%, 25 min), to obtain Compound M030009 (3.85 mg, 0.01 mmol, 5.1% yield) as a white solid.

**LCMS:** [M+H] $^+$ = 393.3;

**$^1$H NMR:** (400 MHz, CDCl3) δ 7.950 (d, *J* = 8.8 Hz, 1H), 7.605 (d, *J* = 8.0 Hz, 2H), 7.248 (s, 1H), 7.180 (d, *J* = 8.4 Hz, 2H), 7.101 (s, 1H), 4.091 (s, 2H), 3.556 - 3.505 (m, 2H), 3.126 (s, 4H), 2.075 (s, 4H), 1.606 - 1.551 (m, 2H), 1.367 (dd, *J* = 14.8, 7.2 Hz, 2H), 0.935 (t, *J* = 7.2 Hz, 3H).

**Example 25: Preparation of Compound 30010**

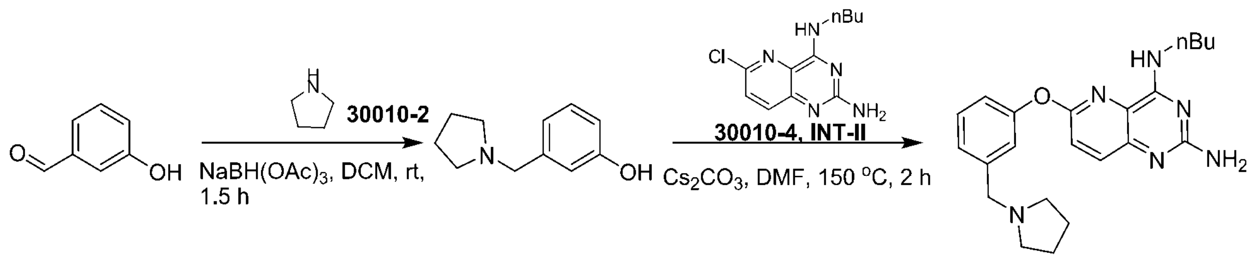


30010-1 30010-3 30010

Compound 30010 was prepared with reference to the synthetic method of Compound 30009.

**LCMS:** [M+H] $^+$ = 393.3

**$^1$H NMR:** (400 MHz, CDCl3) δ 7.710 (d, *J* = 8.8 Hz, 1H), 7.349 (t, *J* = 7.6 Hz, 1H), 7.204 (d, *J* = 7.6 Hz, 1H), 7.159 - 7.123 (m, 2H), 7.065 (d, *J* = 8.0 Hz, 1H), 6.468 (s, 1H), 4.819 (s, 2H), 3.655 (s, 2H), 3.467 (dd, *J* = 13.2, 6.8 Hz, 2H), 2.551 (s, 4H), 1.800 (s, 4H), 1.569 (dd, *J* = 14.8, 7.2 Hz, 2H), 1.372 (dd, *J* = 15.2, 7.2 Hz, 2H), 0.931 (t, *J* = 7.2 Hz, 3H).

**Example 26: Preparation of Compound 30011**

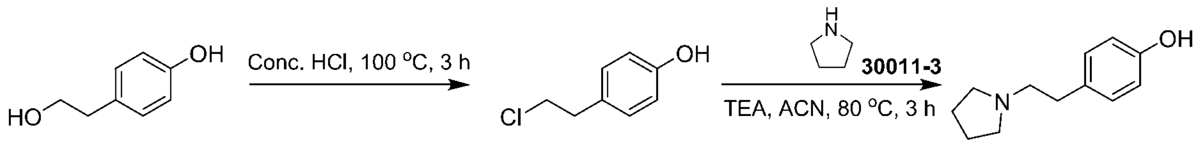


**30011-1** **30011-2** **30011-4**

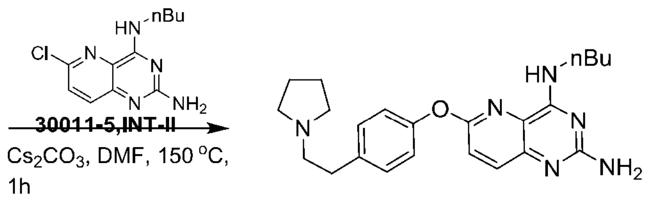


**30011**

**Step 1: General Method for Preparation of Compound 30011-2**

At room temperature under a nitrogen atmosphere, the reaction mixture of Compound 30011-1 (2.0g, 14.50 mmol) and concentrated hydrochloric acid (15 mL) was stirred at 100°C for 3 hours. The mixture was quenched with water and extracted with MTBE (30 mL × 2). The organic layer was collected and concentrated to obtain the crude product, Compound 30011-2 (2.2g, 92%) as a brown oil.

**Step 2: General Method for Preparation of Compound 30011-4**

Under a nitrogen atmosphere, a mixture of Compound 30011-2 (1000 mg, 6.39 mmol), pyrrolidine (707 mg, 9.947 mmol) and triethylamine (1938 mg, 19.16 mmol) in acetonitrile (10 mL) solution was stirred at 80°C for 3 hours. The reaction mixture was concentrated under reduced pressure. The crude product was purified by flash column chromatography (DCM/MeOH = 20/1) to obtain Compound 30011-4 (500 mg, 40% yield) as a brown solid.

**Step 3: General Method for Preparation of Compound 30011**

Compound 30011 was prepared with reference to Step 2 of preparing Compound 30009.

**LCMS:** $[M+H]^+$ = 407.2.

**$^1$H NMR:** (400 MHz, CDCl3) $\delta$ 10.888 (br s, 2H), 7.888 (d, *J* = 8.8 Hz, 1H), 7.301 - 7.269 (m, 2H), 7.203 (d, *J* = 8.8 Hz, 1H), 7.092 (d, *J* = 8.4 Hz, 2H), 7.047 (t, *J* = 5.6 Hz, 1H), 3.510 (dd, *J* = 13.2, 6.8 Hz, 2H), 3.170 - 2.981 (m, 8H), 2.062 - 1.970 (m, 4H), 1.628 - 1.537 (m, 2H), 1.409 - 1.301 (m, 2H), 0.938 (t, *J* = 7.2 Hz, 3H).

**Example 27: Preparation of Compound 30151**

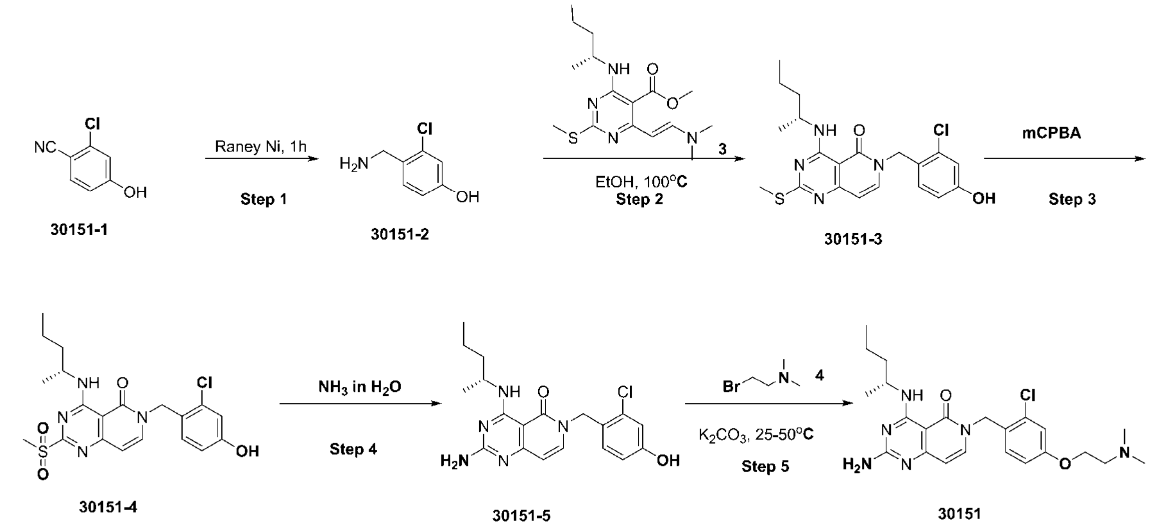


30151-1 30151-2 30151-3 30151-4 30151-5 30151

**Step 1: General Method for Preparation of Compound 30151-2**

At room temperature under a nitrogen atmosphere, compound Raney Ni (191 mg, 3.25 mmol) was added to a MeOH (10 mL) solution of Compound 30151-1 (500 mg, 3.25 mmol). The reaction mixture was stirred at 25°C for 1 hour under a hydrogen atmosphere. The residue was removed by filtration through Celite, and the filtrate was concentrated under reduced pressure to obtain a crude product, Compound 30151-2 (400 mg, 62% yield) as a white solid. $[M+H]^+$ = 158.1.

**Step 2: General Method for Preparation of Compound 30151-3**

At room temperature under a nitrogen atmosphere, Compound 30151-2 (209 mg, 1.32 mmol) was added to an EtOH (5 mL) solution of Compound 3 (300 mg, 0.88 mmol). The reaction mixture was heated and stirred at 100°C for 1 hour. The solvent was removed under reduced pressure to obtain a residue. The crude product was purified by flash column chromatography (0%-10%, ethyl acetate/PE) to obtain Compound 30151-3 (300 mg, 72% yield) as a yellow solid. $[M+H]^+$ = 418.8.

**Step 3: General Method for Preparation of Compound 30151-4**

A solution of Compound 30151-3 (200 mg, 0.47 mmol) and m-CPBA (411 mg, 2.38 mmol) in DMF (5 mL) was stirred at room temperature for 1 hour. The reaction solution was subjected to quenching with an aqueous solution (10 mL) of $Na_2S_2O_3$, followed by extraction with EA (40 mL × 2). Th organic layer was collected, dried over $Na_2SO_4$ and concentrated to obtain the crude product 30151-4 (150 mg, 62% yield) as a yellow solid. $[M+H]^+$ = 451.1.

**Step 4: General Method for Preparation of Compound 30151-5**

Compound 30151-4 (200 mg, 0.44 mmol) was dissolved in 1,4-dioxane (1 mL). Aqueous ammonia (2 mL) was added and the resulting solution was heated to 90°C and stirred for 1 hour. The solvent was removed under reduced pressure. The crude product was purified by flash column chromatography (15 min, 0%-10%, ethyl acetate/PE) to obtain Compound 30151-5 (100 mg, 55% yield) as a yellow solid. $[M+H]^+$ =388.3.

**Step 5: General Method for Preparation of Compound 30151**

A solution of Compound 30151-5 (30 mg, 0.07 mmol) in DMF (2 mL) was added to 4 (16 mg, 0.15 mmol) and $K_2CO_3$ (42 mg, 0.32 mmol). The mixture was stirred at 50°C for 1 hour under a nitrogen atmosphere. The solvent was removed under reduced pressure. Then the crude product was purified by preparative HPLC (column: Phenomenex Luna C18, 250 mm × 100 mm × 10 μm; mobile phase: [A: $H_2O$ (0.1% trifluoroacetic acid), B: acetonitrile]; B%: 0%-80%, 10 min), to obtain Compound 30151 (4 mg, 10% yield) as a white solid.

LCMS: $[M+H]^+$ 459.2; Rt = 0.995 min. $^1H$ NMR (400 MHz, DMSO-$d_6$) δ 9.26(d, *J* = 8 Hz, 1H), 8.18 (s, 1H), 7.57 (d, *J* = 8 Hz, 1H), 7.04 (s, 1H), 6.93 - 6.91 (m, 2H), 6.62 (s, 2H), 6.11 (d, J = 8 Hz, 1H), 5.09 - 4.98 (m, 4H), 4.21 - 4.20 (m, 1H), 4.07 - 4.05 (m, 2H), 2.51 - 2.50 (m, 2H), 2.22 (s, 6H), 1.49 - 1.34 (m, 4H), 1.13 - 1.12.

**Example 28: Preparation of Compound 30155**

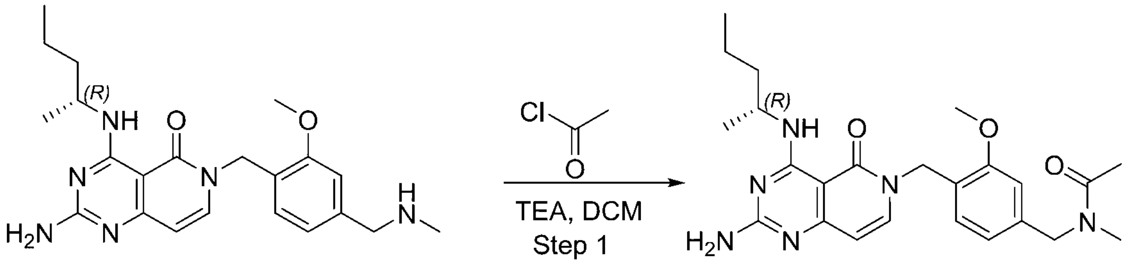


30137 30155

Under a nitrogen atmosphere, a solution of Compound 30137 (50 mg, 0.12 mmol) in 1,2-dichloroethane (1 mL) was added to triethylamine (36 mg, 0.36 mmol) and acetyl chloride (28 mg, 0.36 mmol), and the resulting mixture was stirred at 25°C for 1 hour. The mixture was concentrated under reduced pressure and purified by preparative HPLC (10%-80% acetonitrile/water, 10 min) to obtain Compound 30155 (10 mg, 17% yield) as a white solid.

**LCMS:** $[M+H]^+$ = 453.3.

**Test Example 1: Determination of Agonistic Activity of Compound in This Application Against hTLR7 and hTLR8**

HEK-Blue™ hTLR cell line was used to determine the agonistic activity of the compound in this application against hTLR7 and hTLR8. R848 was used as a positive control compound and has the following structure:

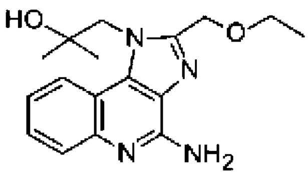

1. The compound was subjected to serial dilution to prepare solutions at 10 concentration points, which were then added to a 96-well plate. 100 nL of DMSO was used for the negative control group to replace the compound in this application, and 28.51 μM of R848 was used for the positive control group to treat hTLR7 and hTLR8 cells.
2. HEK-Blue™ hTLR cells were inoculated into 96-well plates containing the compound, with 100 μL of cells inoculated per well and 50000 cells per well. A concentration of DMSO in the cell was 0.1%.
3. The 96-well plates were incubated overnight at 37°C in a 5% $CO_2$ atmosphere.
4. After overnight incubation, 20 μL of the supernatant from each well was transferred to a well containing 180 μL of QUANTI-Blue™, and the 96-well plates were further incubated at 37°C for 1 hour.
5. After incubation for 1 hour, a microplate reader was used to measure the optical density (OD) at 650 nm ($OD_{65nm}$).
6. Cell viability was determined by using CellTiter-Glo in accordance with the manufacturer's instructions. The microplate reader was used to determine a luminescence signal (RLU) in each well.
7. GraphPad Prism software was used to analyze the $OD_{650nm}$ data, calculate $EC_{50}$ regarding the agonistic activity of the compound against hTLR7 and hTLR8, and plot a fitting curve.
8. GraphPad Prism software was used to analyze cell viability data, calculate $CC_{50}$ regarding cytotoxicity of the compound against hTLR7 and hTLR8, and plot a fitting curve.

$$\text{Cell viability (\%)} = (RLU_{Compound}/RLU_{DMSO\ Control}) \times 100\%.$$

Table 1. Agonistic Activity and Toxicity Data of Compound in This Application Against hTLR7 and hTLR8

| Compound number | hTLR7 EC50 (μM) | hTLR8 EC50 (μM) | CC50 (μM) | Compound number | hTLR7 EC50 (μM) | hTLR8 EC50 (μM) | CC50 (μM) |
|---|---|---|---|---|---|---|---|
| R848 | 0.277 | 3.062 | >28.51 | 30075 | 0.104 | 0.144 | >10 |

(continued)

| Compound number | hTLR7 EC50 (μM) | hTLR8 EC50 (μM) | CC50 (μM) | Compound number | hTLR7 EC50 (μM) | hTLR8 EC50 (μM) | CC50 (μM) |
|---|---|---|---|---|---|---|---|
| 30004 | 2.094 | 0.351 | >10 | 30076 | 0.137 | 0.175 | >10 |
| 30005 | 0.685 | 1.346 | 6.617 | 30077 | >10 | >10 | >10 |
| 30006 | 1.313 | 3.348 | >10 | 30078 | 0.064 | 0.104 | >10 |
| 30007 | 0.702 | 2.72 | 5.552 | 30079 | 0.286 | 0.522 | >10 |
| 30009 | 8.307 | 4.712 | >10 | 30080 | >10 | >10 | >10 |
| 30010 | 8.907 | >10 | >10 | 30081 | 0.057 | 0.193 | >10 |
| 30012 | 0.162 | 0.323 | 3.308 | 30085 | 0.217 | 1.076 | >10 |
| 30013 | 0.449 | 1.999 | 5.496 | 30087 | 0.063 | 0.448 | >10 |
| 30014 | 0.815 | 1.965 | >10 | 30088 | 0.072 | 0.116 | >10 |
| 30016 | 2.422 | 3.689 | >10 | 30089 | 0.033 | 0.016 | >10 |
| 30018 | 2.849 | 0.874 | >10 | 30090 | 1.127 | 2.17 | >10 |
| 30019 | >10 | 6.336 | >10 | 30091 | 0.0335 | 0.0648 | >10 |
| 30027 | >10 | 3.059 | >10 | 30092 | 0.03 | 0.184 | >10 |
| 30039 | 0.676 | 2.9 | >10 | 30095 | 0.4318 | 0.7906 | >10 |
| 30042 | 0.045 | 0.098 | >10 | 30102 | 0.446 | 0.878 | >10 |
| 30043 | 0.068 | 0.118 | >10 | 30103 | 0.0083 | 0.024 | >10 |
| 30046 | 0.063 | 1.002 | 9.554 | 30106 | 0.022 | 0.072 | >10 |
| 30048 | 0.174 | 0.621 | >10 | 30124 | 0.018 | 0.04 | >10 |
| 30049 | 0.101 | 1.431 | 9.903 | 30125 | 0.023 | 0.061 | >10 |
| 30050 | 0.115 | 0.827 | 9.133 | 30126 | 0.016 | 0.043 | >10 |
| 30055 | 0.1 | 0.251 | >10 | 30129 | 0.07 | 0.152 | >10 |
| 30056 | 0.381 | 0.208 | >10 | 30130 | 0.032 | 0.136 | >10 |
| 30057 | 0.114 | 0.268 | >10 | 30134 | 0.034 | 0.142 | >10 |
| 30058 | 0.061 | 0.078 | >10 | 30135 | 0.036 | 0.116 | >10 |
| 30059 | 0.082 | 1.957 | 6.499 | 30137 | 0.246 | 0.334 | >10 |
| 30060 | 0.373 | 0.709 | >10 | 30138 | 0.648 | 1.073 | >10 |
| 30061 | 0.016 | 0.132 | >10 | 30139 | 0.927 | 1.185 | >10 |
| 30062 | 0.043 | 0.191 | >10 | 30140 | 0.035 | 0.126 | >10 |
| 30064 | 0.268 | 0.276 | >10 | 30142 | 0.0081 | 0.063 | >10 |
| 30065 | 0.181 | 0.076 | >10 | 30143 | 0.039 | 0.16 | >10 |
| 30066 | 0.068 | 0.209 | >10 | 30151 | 0.028 | 0.158 | >10 |
| 30068 | 0.098 | 0.167 | >10 | 30154 | 0.098 | 0.705 | >10 |
| 30069 | 0.077 | 0.162 | >10 | 30155 | 0.164 | 1.702 | >10 |

It can be seen from the data in Table 1 that compared with the clinically approved medicament R848 (Resiquimod), the compound in this application exhibits excellent agonistic activity against TLR7 and TLR8, and therefore, can be used for preparation of a medicament for preventing or treating a disease or a disorder associated with TLR activity.

Test Example 2: Investigation of Effect of Compound in This Application on Cytokine Secretion Level of Human Peripheral Blood Mononuclear Cells

The human peripheral blood mononuclear cells were purchased from iXcells Biotechnologies. According to the manufacturer's instructions, the cells were inoculated into 96-well plates at a density of $2 \times 10^6$ cells/mL, and test compounds at various concentrations were added. After 14 hours of incubation, the culture supernatants were collected, and the cytokine levels in the supernatants were detected by using the LEGENDplex™ Human Cytokine Assay Kit (Biolegend, Cat#740390) (see FIG. 1). FIG. 1 shows comparative experimental effects of Compound 89 and R848, with an ordinate representing a mean fluorescence intensity. At a concentration of 1 nM, the compound in the present invention, used as a TLR7/8 agonist, induce human peripheral blood mononuclear cells to secrete significantly higher levels of interferon-$\gamma$ and various other pro-inflammatory cytokines and chemokines (TNF-$\alpha$, IL-10, IL-1$\beta$ and IL-12p70) compared with control R848 (Resiquimod). It is demonstrated that the compound in this application has a high stimulatory effect on immune response of the peripheral blood mononuclear cell system in the immune system.

**Test Example 3: Investigation of Effect of Compound in This Application on Tumor Cell Killing Activity of Human Peripheral Blood Mononuclear Cells In Vitro**

H1568 cells were purchased from ATCC (maintained at 37°C in a 5% $CO_2$ atmosphere, where the maintenance medium contained 10% fetal bovine serum, 100 units/mL penicillin and 100 $\mu$g/mL streptomycin). RPMI 1640 medium containing 2 mM L-glutamine, 10 mM HEPES, 1 mM sodium pyruvate, 4500 mg/mL glucose, and 1500 mg/L sodium bicarbonate was used as the culture medium. H1568 cells were inoculated at a density of 8000 cells per well into 96-well RTCA E-plates (purchased from Agilent, Cat# 300601030; real-time cell analysis electronic plates) and incubated overnight at 37°C. Human peripheral blood mononuclear cells purchased from iXcells (pre-activated with 100 IU/mL IL-2 for 72 hours) were suspended in RPMI 1640 medium containing 10% fetal bovine serum. The suspension and the test compound at certain concentrations were jointly added into the 96-well plates for incubating the H1568 cells, or only the test compound at a certain concentration was added into the 96-well plates for incubating the H1568 cells. DMSO was used as the negative control. An RTCA detection system was used for continuous monitoring of cell culture for 3 - 5 days. The data recorded were cell indices, which correlate with tumor cell viability. The lower the index, the higher the degree of inhibiting or killing tumor cells. Cell index data at 72 hours were collected. GraphPad Prism software was used to analyze the cell index data of the same compound at different concentrations, and calculate $EC_{50}$ regarding tumor cell killing induction activity of the compound in the presence and absence of human peripheral blood mononuclear cells (see Table 2).

The compound in this application exhibited great capability of activating human peripheral blood mononuclear cells to kill cancer cells ($EC_{50}$ ranged from 1 nM to 10 nM).

Table 2

| Inhibitory Activity of Compound Against H1568 Cells | | | |
|---|---|---|---|
| Compound | $IC_{50}$ (nM) without PBMC | $IC_{50}$ (nM) with PBMC | Fold change ($IC_{50}$ without PBMC/$IC_{50}$ with PBMC) |
| 30042 | >2000 | 4.725 | 423 |
| 30043 | >2000 | 15.76 | 127 |
| 30061 | >2000 | 6.949 | 288 |
| 30065 | >10000 | 4.71 | 2123 |
| 30081 | >10000 | 17.39 | 575 |
| 30089 | >10000 | 1.873 | 5339 |
| 30091 | 1803 | 4.267 | 423 |
| 30125 | >2000 | 3.888 | 514 |
| 30126 | >2000 | 0.674 | 2967 |

Test Example 4: Investigation of In Vitro Pharmacokinetic Properties of Compound in This Application

The in vitro pharmacokinetic properties of the compound in this application were entrusted to Pharmaron (Beijing) for testing, which mainly included evaluation of in vitro cell membrane permeability (MDCK-MDR1 ) and metabolic stability of liver microsome (LM).

In vitro cell membrane permeability (i.e., bidirectional transport in the MDCK-MDR1 cell monolayer model): According to an operating procedure, a MDCK-MDR1 cell suspension at a density of 1.56 × $10^6$ cells/mL was prepared and inoculated into the filter wells of 96-well HTS Transwell, with 50 μL of the cell suspension added per well. The cells were incubated continuously for 4 - 8 days in an incubator at 37°C with 5% $CO_2$ and 95% relative humidity, and the medium was changed daily. Integrity and tightness of the MDCK-MDR1 cell monolayer model were evaluated via a trans-epithelial electrical resistance value (greater than 42 $\Omega/cm^2$). After passing of resistance verification, the culture medium in the AP compartment and BL compartment was aspirated completely. Both compartments were washed twice with HBSS solution (10 mM HEPES, pH 7.4) at 37°C, followed by incubation for 30 minutes in a constant-temperature shaking incubator (37°C). Then Ap→Bl absorptive transport experiment was performed: A test solution (1 μmol/L, 125 μL) was added to the AP compartment of the Transwell chamber, and HBSS solution (10 mM HEPES, pH 7.4, 235 μL) was added to the BL compartment. 50 μL of the solution was immediately aspirated from the AP compartment and transferred to a new 96-well plate. Bl→Ap efflux experiment: HBSS solution (10 mM HEPES, pH 7.4, 75 μL) was added to the AP compartment and the test solution (1 μmol/L, 285 μL) was added to the BL compartment of the Transwell chamber. 50 μL of the solution was immediately aspirated from the BL compartment and transferred to a new 96-well plate. After all the 96-well plates were incubated for 2 hours in the constant-temperature shaking incubator (37°C), 50 μL of the solution was collected from both the donor compartment and the receiver compartment and transferred to new 96-well plates. 200 μL of acetonitrile containing internal standard was added to each well of the new 96-well plate, and the plate was centrifugated gently for 5 minutes and then centrifugated at 3220 × g for 40 minutes. The supernatant was collected and treated with ultrapure water, and then the sample concentration was determined by HPLC.

Metabolic stability of liver microsomes (LM): Liver microsomes (human LM, Corning, Cat#452117; Rat LM, Corning, Cat#452501; and Mouse LM, XENOTECH, Cat#M1000) were used. The total volume of each incubation system was 400 μL, the system consisted of 200 μL of 0.2 M phosphate buffer (pH 7.4), 106 μL of ultrapure water, 40 μL of 50 mM magnesium chloride, 10 μL of 20 mg/mL LM, and 40 μL of 10 mM NADPH (if NADPH was excluded, 40 μL of ultrapure water was added instead). Then 4 μL of 100 μM test compound was added, and the mixture was incubated in a 37°C water bath. Each sample was prepared in triplicate, and samples without the NADPH-generating system were used for negative control. At preset reaction time points (i.e., 0, 15, 30, 45, and 60 min), 50 μL of the reaction mixture was sampled separately, and pre-cooled acetonitrile (containing IS: 3% formic acid, 100 nM alprazolam, 200 nM labetalol, 200 nM caffeine, and 2 μM ketoprofen) of 4 volumes were added to quench the reaction. The samples were centrifuged at 3220 × g for 40 minutes. Then 100 μL of the supernatant was mixed with 100 μL of ultrapure water, and content of the analyte was determined by LC-MS/MS. The data analysis was performed by using MS Excel.

Hepatocyte Metabolic Stability Experiment After thawing and resuscitation, the hepatocytes were cultured in the culture medium at 37°C until the concentration reached approximately 1.5 × $10^6$ cells/mL. After viability assay (>75%), the hepatocytes were diluted to a concentration of 0.5 × $10^6$ cells/mL. After 198 μL of hepatocytes were pre-warmed for 10 minutes, 2 μL of the analyte or the control substance at a concentration of 100 μM was added. The metabolic reaction was carried out in the incubation method (37 °C). At different time points (0, 15, 30, 60, 90, and 120 minutes) during the reaction, 25 μL of the solution was sampled from the system, and 150 μL of organic solvent (acetonitrile containing 100 nM alprazolam, 200 nM labetalol, 200 nM caffeine, and 2 μM ketoprofen) was added to quench the reaction. The samples were centrifuged at 3220 × g for 20 minutes. Then 100 μL of the supernatant was mixed with 100 μL of ultrapure water, and content of the analyte was determined by LC-MS/MS. All incubation metabolism experiments were performed twice. The data analysis was performed by using MS Excel.

The results show (see Table 3 below) that compared with R848 (Resiquimod), the compound in the present invention has a shorter half-life period. The shorter half-life of the compound in the present invention can reduce a risk of inducing systemic immune responses by the compound in vivo.

Table 3

| Compound | R848 | 30042 | 30043 | 30061 | 30089 | 300**91** | 30**125** | 30126 |
|---|---|---|---|---|---|---|---|---|
| Mouse Liver Microsome Clearance (mLM Clint, | 12.91/5 6.80 | 77.60/ 341.44 | 92.83/40 8.43 | 333.16/ 1465.93 | 420.89/ 1851.91 | 114.50 / | 198.64/ 874.00 | 472.77/ 2080.23 |
| (μL/min/mg)/(mL/min/ kg)) | | | | | | 503.80 | | |
| Mouse Liver Microsome Half-Life ($T_{1/2}$, min) | **107.37** | **17.86** | **14.93** | **4.16** | **3.29** | **12.1** | **6.98** | **2.93** |
| Rat Liver Microsome Clearance (rLM Clint, (uL/min/$10^6$ cells)/mL/min/kg) | 7.67/35. 89* | 52.96/ 247.85 | 93.19/43 6.13* | 144.06/2 58.15 | 95.21/17 0.82 | 44.52/ 79.78 | 48.62/ 87.13 | 37.94/67 .99 |
| Rat Liver Microsome Half-Life ($T_{1/2}$, min) | **180.75*** | **26.17** | **14.87*** | **9.62** | **14.54** | **31.13** | **28.51** | **36.53** |
| Human Liver Microsome Clearance (hLM Clint, (uL/-min/mg)/(mL/min/ kg)) | 0.32/0.40 | 14.25/ 17.87 | 9.09/11. 40 | 32.32/40 .54 | 41.96/52 .62 | 37.82/ 47.44 | 44.82/56 .22 | 42.23/52 .97 |
| Human Liver Microsome Half-Life ($T_{1/2}$, min) | **4381** | **97.28** | **152.46** | **42.88** | **33.03** | **36.64** | **30.92** | **32.81** |
| Permeability (Papp, (A-B), $10^{-6}$ cm/s) | **13.16** | **5.03** | **3.06** | **0.6** | **0.8** | **0.54** | **0.25** | **0.22** |
| Permeability (Papp, (B-A), $10^{-6}$ cm/s) | 41.85 | 1.90 | 1.82 | 2.27 | 2.25 | 4.13 | 3.83 | 1.18 |
| Ratio (ER, (B-A)/(A-B)) | **3.18** | **0.38** | **0.59** | **3.76** | **2.82** | **7.63** | **15.43** | **5.36** |
| Recovery (%, AP-BL) | 91.83 | 28.37 | 23.17 | 15.64 | 14.46 | 23.13 | 24.63 | 73.71 |
| Recovery (%, BL-AP) | 108.34 | 67.17 | 81.6 | 70.95 | 70.21 | 70.08 | 60.39 | 90.24 |
| Bovine Serum Albumin (BSA, %) | 0 | 0 | 0 | 0 | 0 | 0 | 0 | 3 |

**Test Example 5: Investigation of In Vivo Pharmacokinetic Properties of Compound in This Application in CD1 Mice**

Due to severe side effects associated with the target, the pharmacokinetic properties of TLR7/8 agonists largely determine their druggability. Therefore, an in vivo pharmacokinetic evaluation of the compound in this application was conducted in male CD1 mice aged 6 - 8 weeks and weighing approximately 20g - 30g, with 3 mice per group. The mice were fasted overnight before administration and allowed access to food 4 hours after administration. Every 3-5 analytes with a molecular weight difference of over 4 were grouped into a set. Intraperitoneal administration was performed with the following formulation: 0.4 mg/mL; 10% SBE-β-CD dissolved in 50 mM citrate buffer (pH 5.0); the dosage was 2 mpk; at time points of 5, 15, and 30 minutes, and 1, 2, 4, 8, and 24 hours after administration, approximately 0.03 mL of blood was collected from the saphenous vein. The blood samples were transferred into plastic centrifuge tubes containing EDTA-K2. The residual concentrations of the analytes in processed plasma were determined by LC-MS. At 24 hours after administration, the mice were euthanized via $CO_2$ asphyxiation. Liver tissues were harvested and processed, and then analyzed for residual drug concentrations via LC-MS.

After data processing, parameters such as half-life ($T_{1/2}$), maximum concentration ($C_{max}$), time to maximum concentration ($T_{max}$), $AUC_{last}$, $AUC_{inf}$, $AUC_{_\%Extrap_obs}$, and tissue/plasma distribution ratio were obtained, as shown in Table 4 and FIG. 2 below. Consistent with the in vitro pharmacokinetic evaluation, all compounds in this application except 30078 rapidly reached the maximum concentration in the mouse circulatory system, and then rapidly declined to the range of 10-30 ng/mL.

Table 4

| Compound | 30042 | 30043 | 30061 | 30089 | 30091 | 30125 | 30126 |
|---|---|---|---|---|---|---|---|
| Half-Life ($T_{1/2}$, h) | **15.9** | **8.53** | **11.29** | **12.05** | **14.2** | **11.8** | **14.6** |
| Time to maximum concentration ($T_{max}$, h) | **0.250** | **0.194** | **0.08** | **0.08** | **0.25** | **0.25** | **0.25** |
| Maximum concentration ($C_{max}$, ng/mL) | **85** | **112** | **142.52** | **172.22** | **119** | **65.1** | **106** |
| Area under the concentration-time curve ($AUC_{last}$, h*ng/mL) | **294** | **363** | **238.05** | **316.86** | **526** | **318** | **368** |
| Area under the concentration-time curve ($AUC_{inf}$, h*ng/mL) | **435** | **415** | **290.10** | **401.13** | **779** | **420** | **525** |
| Area under the concentration-time curve ($AUC_{_\%Extrap_obs}$) | **30.5** | **12.5** | **18.28** | **22.17** | **32.3** | **22.9** | **26.6** |
| Mean residence time ($MRT_{inf_obs}$) | **19.8** | **10.1** | **12.03** | **14.05** | **20** | **15.2** | **18.4** |
| Area under the concentration-time curve ($AUC_{last/D}$, h*ng/mL) | **147** | **182** | **119.02** | **158.43** | **263** | **159** | **184** |
| 24-hour liver concentration ($C_{liver}$, ng/mL) | **625** | **150** | 5.57 | 12.16 | **433** | **107** | **80.5** |
| Ratio (liver/plasma) | **115** | **37** | 1.5 | 2.59 | **36.6** | **19.6** | **12.1** |

**Test Example 6: Evaluation of Efficacy of Compound in This Application in Subcutaneous Syngeneic Transplantation Model of Murine Colon Cancer CT-26 Cells in Female Balb/C Mice**

1. CT-26 cells was resuscitated and cultured in vitro in RPMI 1640 medium supplemented with 10% FBS, to obtain $2.5 \times 10^7$ cells.
2. 40 female Balb/C mice aged 6 - 8 weeks were acclimatized for one week, and then weighed and grouped.
3. Inoculation Conditions

Details of cell inoculation are as follows:

| Animal strain | Number of animals (n) | Type of inoculated cells | Inoculation site | Inoculated cell amount (cells) | With or without Matrigel | Volume of inoculated cell suspension | Total amount of desired cells |
|---|---|---|---|---|---|---|---|
| Balb/C | 40 | CT-26 | Subcutaneously in the right shoulder | $5\times10^5$ | No | 0.1 mL | $2.5\times10^7$ |

4. After inoculation, the tumor volumes and body weight were measured once a week. When a mean tumor volume reached 79.6 $mm^3$, the mice were randomly divided into 6 groups according to tumor volumes and body weight, with 4 mice per group. The mice were administered immediately after grouping. A start date of drug administration was designated as Day 0. Details of drug administration and grouping are as follows:
Details of grouping and drug administration are as follows:

| Group | Number of animals | Treatment | Dosage | Route of administration | Dosage | Administration cycle |
|---|---|---|---|---|---|---|
| Group number | 4 | Drug number | 1 mg/kg | i.p. | 10 μL/g | QW, Day 0, Day 7 |

5. After the start of drug administration, the body weight and tumor volumes of the mice were measured three times a week. The drug was administered twice during the experimental period. Experimental observation was ended on Day 12, and at the end of the experiment, tumors were collected and residual concentrations of the administered drug in tumor tissues were measured.

Referring to FIG. 3, the results demonstrate that the compound in this application has high relative safety and significant tumor-inhibitory efficacy.

The preferred embodiments of the present invention have been described in detail above. However, the present invention is not limited to the specific details in the above embodiments. Within the scope of the technical concept of the present invention, many simple modifications can be made to the technical solution of the present invention, all of which fall within the claimed scope of the present invention.

Furthermore, it should be noted that various specific technical features described in the above specific embodiments can be combined in any suitable manner without contradiction. In order to avoid unnecessary repetition, the present invention does not explain various possible combinations separately. As long as the combination does not violate the idea of the present invention, it should also be regarded as the disclosed content of the present invention.

## Claims

**1.** A pyrimidine derivative, which is a compound of Formula (I-1) or (I-2), or a stereoisomer, a tautomer, an isotopic derivative, a hydrate, a solvate, a prodrug, or a pharmaceutically acceptable salt thereof;

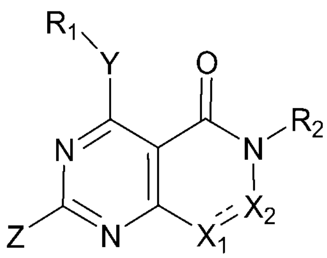

Formula (I-1)

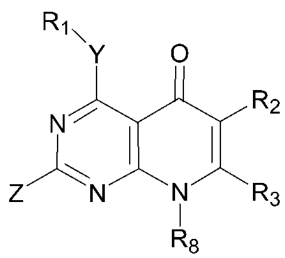

Formula (I-2)

wherein:

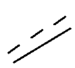

represents a single bond or a double bond;

$X_1$ and $X_2$ are both C-$R_3$, or one of them is C-$R_3$ and the other is N;

Z is -$NH_2$, -OH, -NH-alkyl, -O-alkyl, -NH-C(O)-alkyl, -O-C(O)-alkyl, -NH-C(O)-OH, or -O-C(O)-OH;

Y is -O-, -S-, or -$NR_4$-, wherein $R_4$ is H or an optionally substituted alkyl group;

$R_1$ is an alkyl group or an aryl group optionally substituted with one or more substituents selected from oxo, halogen, an amino group, a hydroxyl group, a nitro group, a cyano group, a thiol group, -C(O)-$NH_2$, -C(O)-OH, a heteroaryl group (optionally substituted with one or more of halogen, an amino group, a hydroxyl group, a nitro group, a cyano group, a thiol group, an alkoxyl group, and an alkyl group), an aryl group (optionally substituted with one or more of halogen, an amino group, a hydroxyl group, a nitro group, a cyano group, a thiol group, an alkoxyl group, and an alkyl group), a heterocycloalkyl group (optionally substituted with one or more of halogen, an amino group, a hydroxyl group, a nitro group, a cyano group, a thiol group, an alkoxyl group, and an alkyl group), a cycloalkyl group (optionally substituted with one or more of halogen, an amino group, a hydroxyl group, a nitro group, a cyano group, a thiol group, an alkoxyl group, and an alkyl group), an optionally substituted alkyl group, an optionally substituted alkyloxy group, an optionally substituted alkylthio group, an optionally substituted alkylamino group, an optionally substituted alkylacyl group, an optionally substituted alkylsulfinyl group, an optionally substituted alkylsulfonyl group, an optionally substituted -C(O)-O-alkyl, optionally substituted -O-C(O)-alkyl, optionally substituted -C(O)-NH-alkyl, optionally substituted -NH-C(O)-alkyl, optionally substituted -S(O)-O-alkyl, optionally substituted -O-S(O)-alkyl, optionally substituted -$S(O)_2$-O-alkyl, optionally substituted -O-$S(O)_2$-alkyl, optionally substituted -S(O)-NH-alkyl, optionally substituted -NH-S(O)-alkyl, optionally substituted -$S(O)_2$-NH-alkyl, and optionally substituted -NH-$S(O)_2$-alkyl;

$R_2$ is

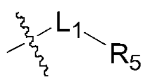 or 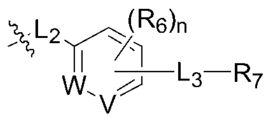 ;

and -$L_3$-$R_7$ may be substituted at a para-, meta- or ortho-position of $L_2$;

$L_1$, $L_2$ and $L_3$ are each a bond or an optionally substituted linear alkylene group, wherein one or more carbon atoms in the linear alkylene group may be substituted with oxygen, sulfur and nitrogen heteroatoms, and the substituent is selected from one or more of an optionally substituted alkyl group, halogen, an amino group, a hydroxyl group, a nitro group, a cyano group, a thiol group, an optionally substituted alkyloxy group, an optionally substituted alkylthio group and an optionally substituted alkylamino group;

$R_5$ is a cycloalkyl group or a heterocycloalkyl group optionally substituted with one or more substituents selected from oxo, halogen, an amino group, a hydroxyl group, a nitro group, a cyano group, a thiol group, an optionally substituted alkyl group, an optionally substituted alkyloxy group, an optionally substituted alkylthio group, and an optionally substituted alkylamino group;

each of W and V may be N or CH; and W or V may be substituted with $R_6$ or -$L_3$-$R_7$ when being CH;

$R_3$ and $R_6$ are each independently H, an optionally substituted alkyl group, an optionally substituted alkyloxy group, an optionally substituted alkylthio group, an optionally substituted alkylamino group, halogen, an amino group, a hydroxyl group, a nitro group, a cyano group, and a thiol group;

$R_7$ is H, -OH, -N($R_9R_{10}$), -N($R_9$)$NH_2$, -NO($R_9R_{10}$), an optionally substituted heterocycloalkyl group, an optionally substituted cycloalkyl group, an optionally substituted aryl group, an optionally substituted heteroaryl group, optionally substituted heterocycloalkyl-$NR_9$-, optionally substituted cycloalkyl-$NR_9$-, optionally substituted aryl-$NR_9$-, optionally substituted heteroaryl-$NR_9$-, -OC(O)-$R_9$, -C(O)O-$R_9$, -N($R_9$)-C(O)-$R_{10}$ or -C(O)-N($R_9R_{10}$); and the substituent is selected from one or more of an optionally substituted alkyl group, an optionally substituted alkylamino group, an optionally substituted dialkylamino group and oxo;

$R_8$ is H or an optionally substituted alkyl group;

$R_9$ and $R_{10}$ are each independently H, an optionally substituted alkyl group, an optionally substituted cycloalkyl group, an optionally substituted heterocycloalkyl group, an optionally substituted aryl group, and an optionally substituted heteroaryl group; and

the substituent of the optionally substituted group is selected from one or more of halogen, an amino group, a hydroxyl group, a nitro group, a cyano group, a thiol group, a carboxyl group, an ester group, an amide group and the like;

wherein n = 0, 1, 2, 3 or 4.

**2.** The pyrimidine derivative of claim 1, wherein one of $X_1$ and $X_2$ is C-$R_3$, and the other is N.

**3.** The pyrimidine derivative of claim 1, wherein $R_8$ is H or a C1-8 alkyl group.

**4.** The pyrimidine derivative of any one of claims 1 to 3, wherein $R_1$ is a C1-8 alkyl group optionally substituted with one or more substituents selected from an optionally substituted C1-8 alkyloxy group, an optionally substituted C1-8 alkylthio group, an optionally substituted C1-8 alkylamino group, an optionally substituted C1-8 alkylacyl group, an optionally substituted C1-8 alkylsulfinyl group, and an optionally substituted C1-8 alkylsulfonyl group;

preferably, $R_1$ is a C2-6 alkyl group optionally substituted with an optionally substituted C1-6 alkyloxy group, an optionally substituted C1-6 alkylthio group, and an optionally substituted C1-6 alkylsulfonyl group;
preferably, $R_1$ is an optionally substituted methyl group, ethyl group, n-propyl group, isopropyl group, n-butyl group, isobutyl group, tert-butyl group, n-pentyl group, 2-pentyl group, n-hexyl group, 2-hexyl group or 3-hexyl group;
more preferably, $R_1$ is an n-butyl group, an isobutyl group, a tert-butyl group, an n-pentyl group, a 2-pentyl group, a 3-hexyl group, a methoxyethyl group, a methylthioethyl group, a methylsulfonylethyl group, a methoxypropyl group, a methylthiopropyl group, a methylsulfonylpropyl group,

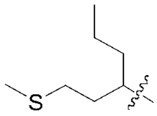 or 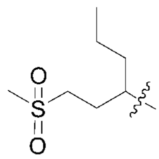 ;

or
more preferably, $R_1$ is an n-butyl group,

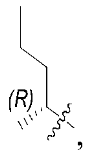 , 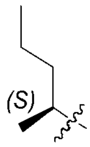 ,

a methylthiopropyl group, a methylsulfonylpropyl group,

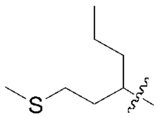 or 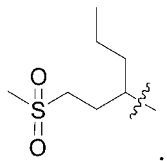 .

**5.** The pyrimidine derivative of any one of claims 1 to 4, wherein W and V are both N, W and V are both C, or one of W and V is N.

**6.** The pyrimidine derivative of any one of claims 1 to 5, wherein the heterocycloalkyl group is a 3- to 12-membered monocyclic, bicyclic or polycyclic heterocycloalkyl group; preferably, the heterocycloalkyl group is a 3- to 12-membered monocyclic, bicyclic or polycyclic heterocycloalkyl group containing 1, 2, 3 or 4 heteroatoms selected from N, O, and S; more preferably, the heteroatom in the heterocycloalkyl group is linked to $L_1$ or $L_3$; and more preferably, N atom in the heterocycloalkyl group is linked to $L_1$ or $L_3$.

**7.** The pyrimidine derivative of claim 6, wherein the heterocycloalkyl group is a 3- to 7-membered monocyclic heterocycloalkyl group containing 1 or 2 heteroatoms selected from N, O, and S, or is a 7- to 12-membered fused-ring or spiro heterocycloalkyl group containing 1, 2, 3 or 4 heteroatoms selected from N, O, and S;

preferably, the heterocycloalkyl group is a 4- to 6-membered monocyclic heterocycloalkyl group containing 1 or 2 heteroatoms selected from N and O, or is a 7- to 11-membered spiro heterocycloalkyl group containing 2, 3 or 4 heteroatoms selected from N and O; or
more preferably, the heterocycloalkyl group is one of the following groups:

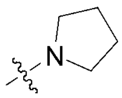, 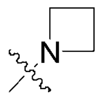, 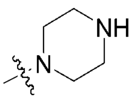, 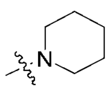, 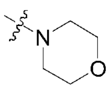, 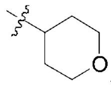, 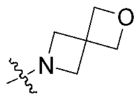 and 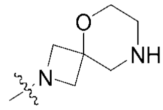.

**8.** The pyrimidine derivative of any one of claims 1 to 7, wherein the aryl group is a C6-10 aryl group, preferably a phenyl group.

**9.** The pyrimidine derivative of any one of claims 1 to 8, wherein $L_1$, $L_2$ and $L_3$ are each a bond or an optionally substituted linear alkylene group containing 1 to 6 chain atoms, 1 or 2 carbon atoms in the linear alkylene group may be substituted with oxygen, sulfur and nitrogen heteroatoms, and the substituent is selected from the C1-8 alkyl group, halogen, an amino group, a hydroxyl group, a nitro group, a cyano group and a thiol group;

preferably, $L_1$ is an optionally substituted linear alkylene group containing 1 to 4 chain atoms, more preferably is an optionally substituted linear alkylene group containing 2 or 3 chain atoms, and more preferably, is -O-propylene or $-CH_2CH_2CH_2-$;
preferably, $L_2$ is an optionally substituted linear alkylene group containing 1 to 4 chain atoms, more preferably is an optionally substituted linear alkylene group containing 2 or 3 chain atoms, and more preferably, is $-CH_2-$, $-CH_2CH_2-$, -O-, $-O-CH_2-CH_2-$ and $-O-CH_2-$; and
preferably, $L_3$ is a bond or an optionally substituted linear alkylene group containing 1 to 4 chain atoms, preferably is a bond or an optionally substituted linear alkylene group containing 2 or 3 chain atoms, more preferably is a bond, $-CH_2-$, $-CH_2CH_2-$, $-CH_2CH_2CH_2-$, $-O-CH_2-CH_2-$, $-O-CH_2-$ and $-CH(CH_3)CH_2-$, and more preferably, is

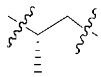 or 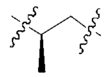.

**10.** The pyrimidine derivative of any one of claims 1 to 9, wherein $R_9$ and $R_{10}$ are each independently H or a C1-8 alkyl group.

**11.** The pyrimidine derivative of any one of claims 1 to 10, wherein $R_7$ is -OH, $-N(R_9R_{10})$, $-N(R_9)NH_2$, $-NO(R_9R_{10})$, an optionally substituted 3- to 12-membered heterocycloalkyl group containing 1, 2, 3 or 4 heteroatoms selected from N, O, and S, an optionally substituted C6-10 aryl group, optionally substituted 3- to 12-membered heterocycloalkyl-$NR_9$- containing 1, 2, 3 or 4 heteroatoms selected from N, O, and S, or optionally substituted C6-10 aryl-$NR_9$-; the substituent is selected from one or more of an optionally substituted C1-8 alkyl group, an optionally substituted C1-8 alkylamino group, an optionally substituted di(C1-8)alkylamino group and oxo; and $R_9$ and $R_{10}$ are each independently H or a C1-8 alkyl group; and
preferably, the heterocycloalkyl group is a 4- to 6-membered monocyclic heterocycloalkyl group containing 1 or 2 heteroatoms selected from N and O, or is a 7- to 11-membered spiro heterocycloalkyl group containing 2, 3 or 4 heteroatoms selected from N and O; more preferably, the heteroatom in the heterocycloalkyl group is linked to $L_3$; and more preferably, the N atom in the heterocycloalkyl group is linked to $L_3$.

**12.** The pyrimidine derivative of any one of claims 1 to 10, wherein $R_2$ is selected from one of the following optionally substituted groups:

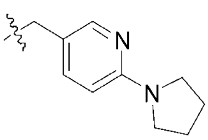, 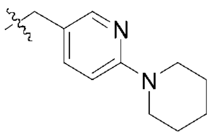, 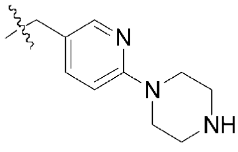,

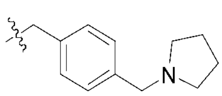, 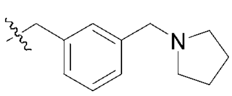, 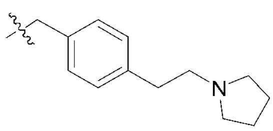, 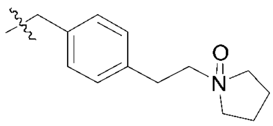,

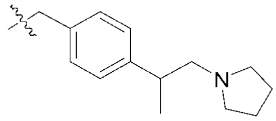, 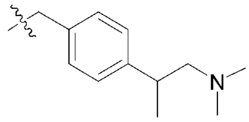, 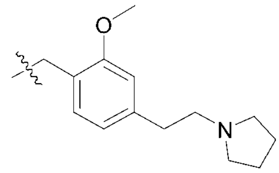, 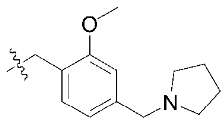,

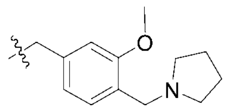, 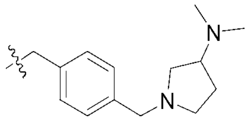, 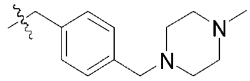, 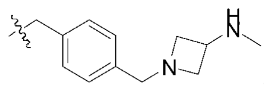,

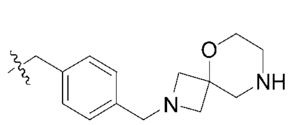, 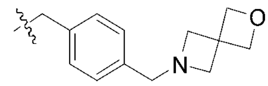, 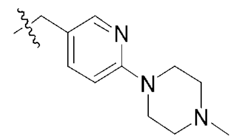, 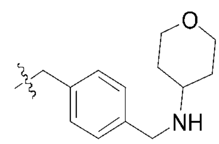,

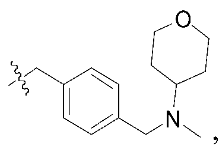, 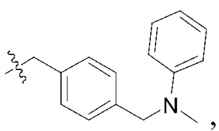, 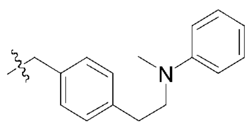, 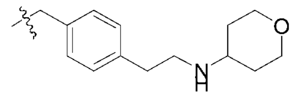,

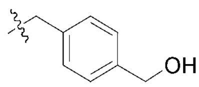, 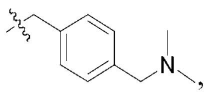, 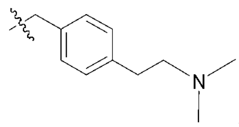, 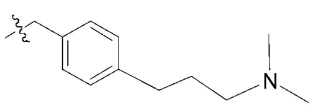,

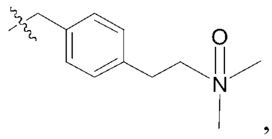, 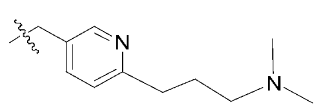, 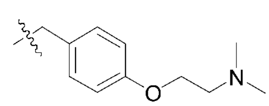, 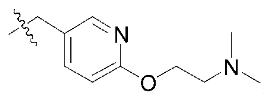,

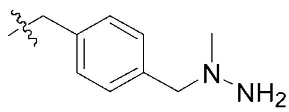, 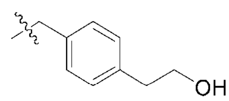, 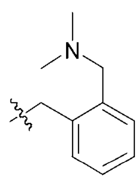, 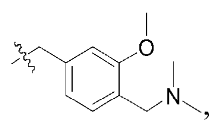, 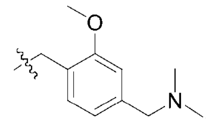,

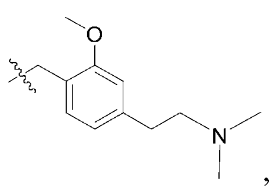, 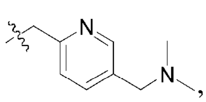, 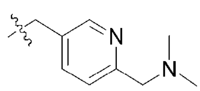, 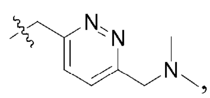, 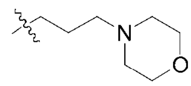,

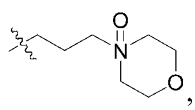, 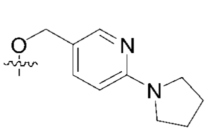, 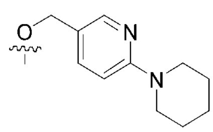, 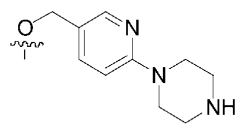, 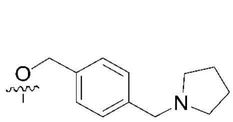,

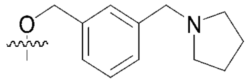, 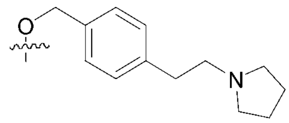, 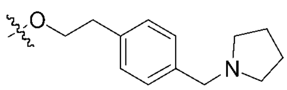, 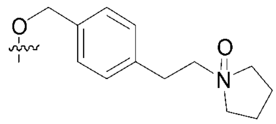,

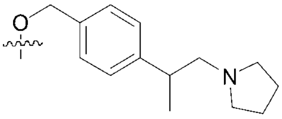, 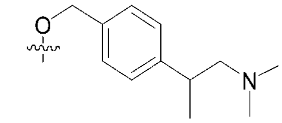, 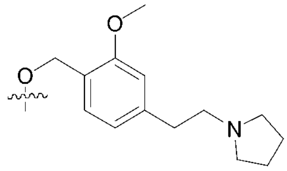, 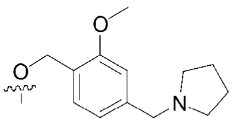,

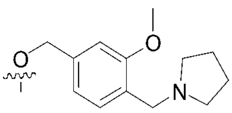, 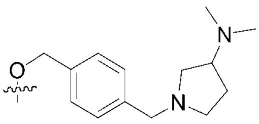, 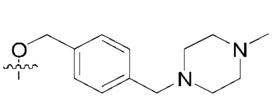, 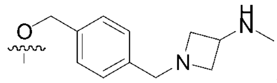,

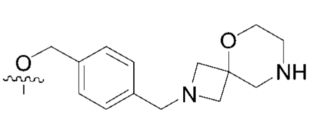, 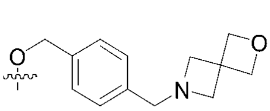, 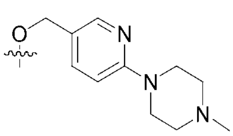, 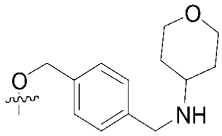,

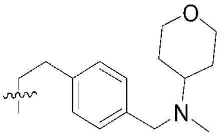, 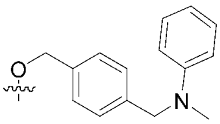, 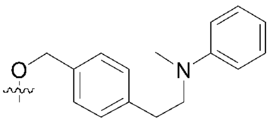, 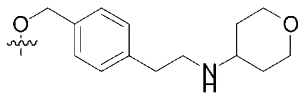,

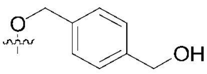, 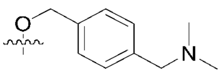, 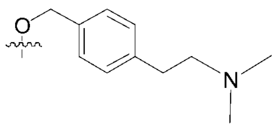, 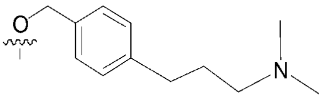,

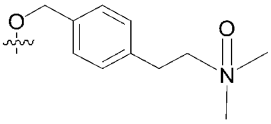, 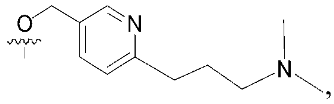, 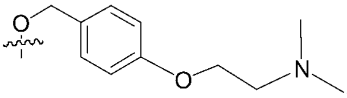,

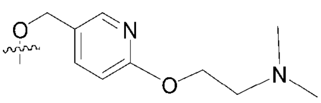, 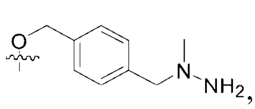, 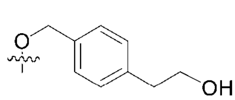, 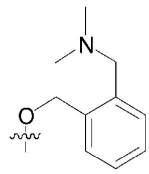,

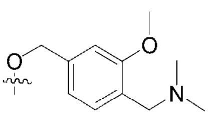, 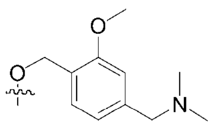, 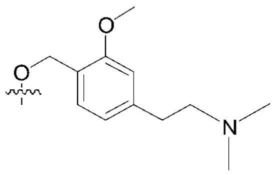, 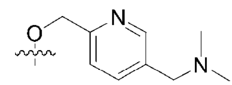,

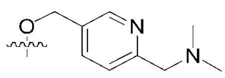,
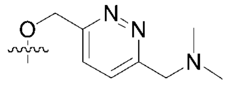,
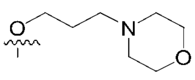,
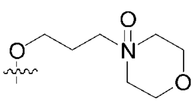,
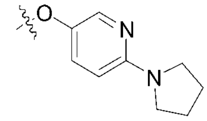,
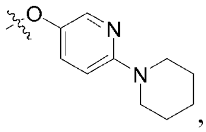,
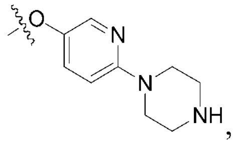,
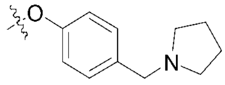,
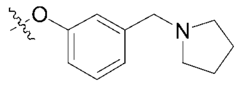,
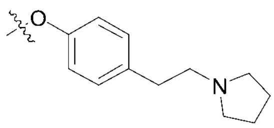,
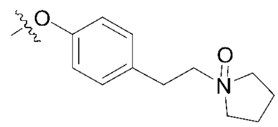,
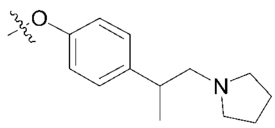,
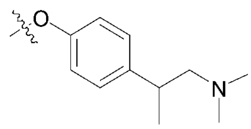,
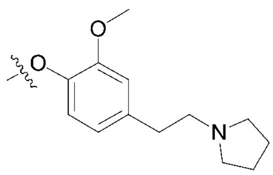,
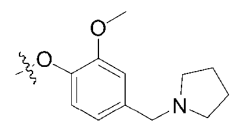,
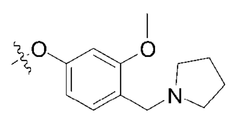,
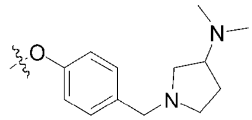,
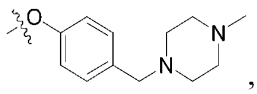,
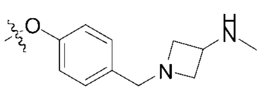,
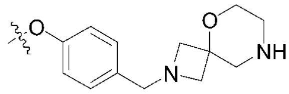,
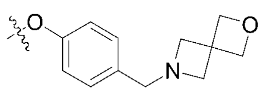,
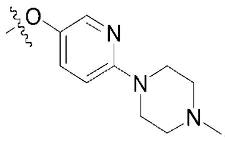,
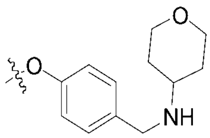,
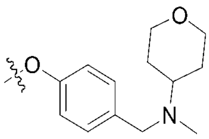,
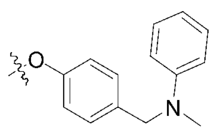,
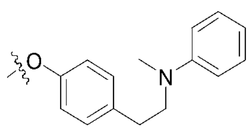,
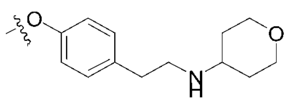,
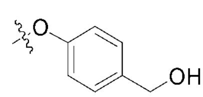,
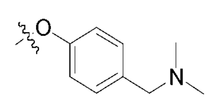,
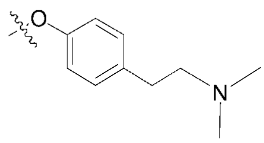,
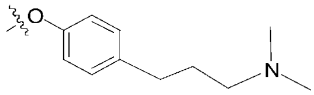,
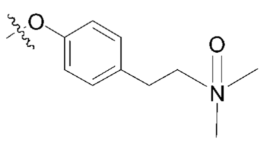,
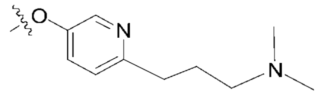,
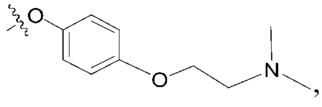,
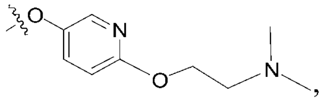,
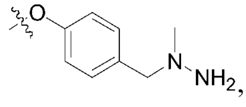,
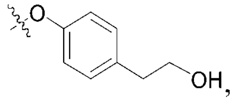,
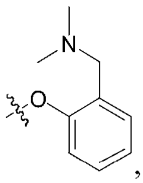,
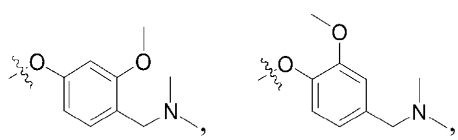
, ,
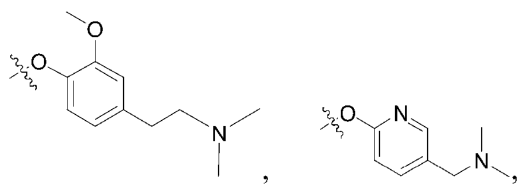
, ,

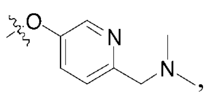, 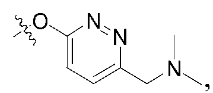, 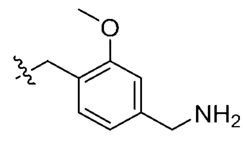, 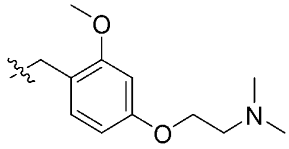,

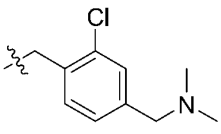, 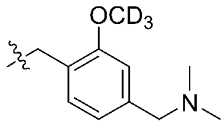, 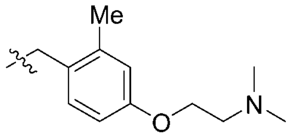, 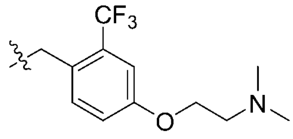,

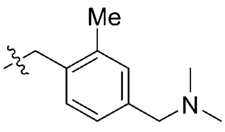, 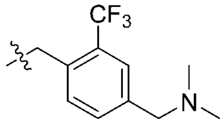, 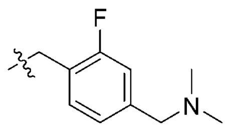, 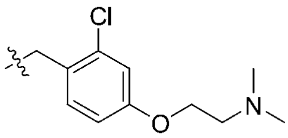,

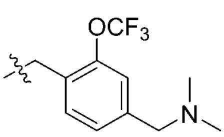, 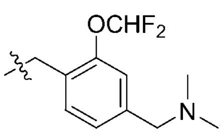, 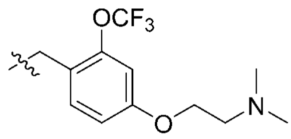, 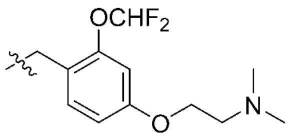,

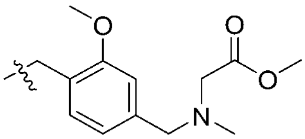, 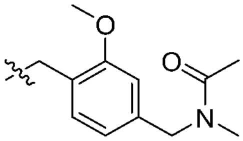, 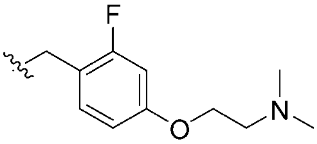; or

further, $R_2$ is optionally substituted

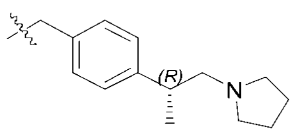, 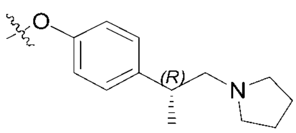,

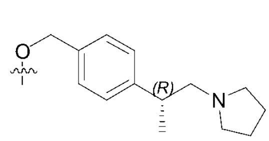, 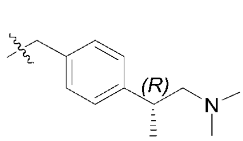, 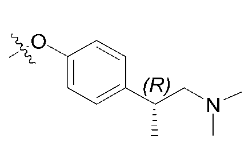, 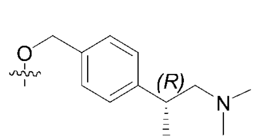,

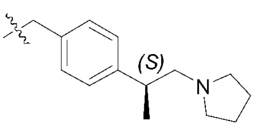, 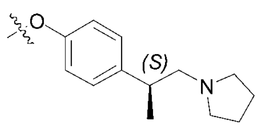, 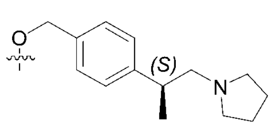, 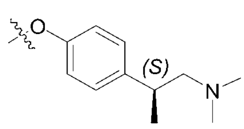,

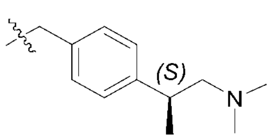, 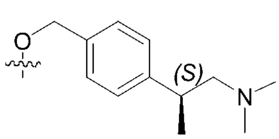;

and

the substituent is selected from one or more of an alkyl group, an alkyloxy group, an alkylthio group, an alkylamino group, halogen, an amino group, a hydroxyl group, a nitro group, a cyano group and a sulfhydryl group.

**13.** The pyrimidine derivative of any one of claims 1 to 12, wherein the compound of Formula (I-1) or (I-2) specifically has one of the following formulas:

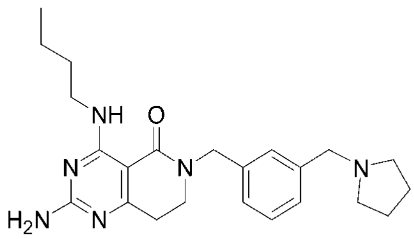

18 ,

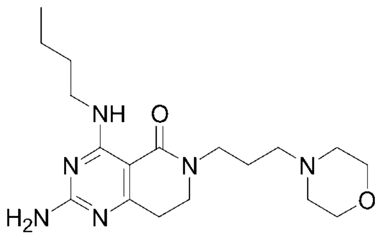

19 ,

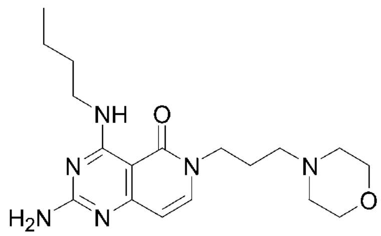

27 ,

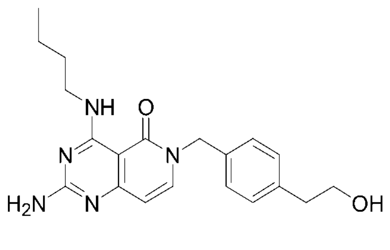

39 ,

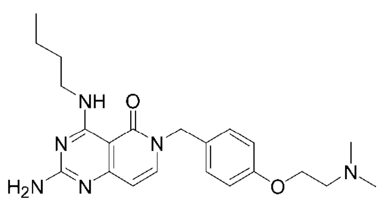

42 ,

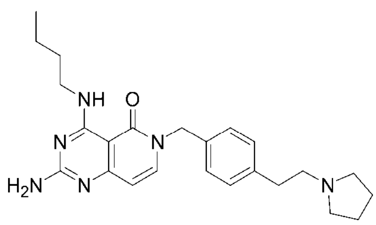

43 ,

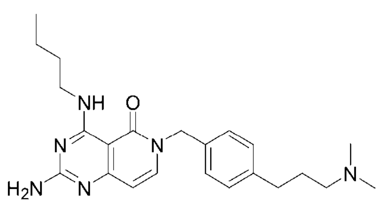

55 ,

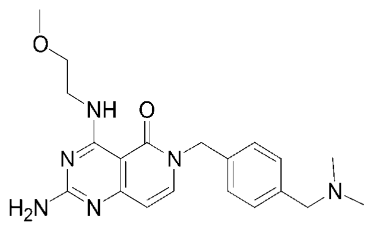

56 ,

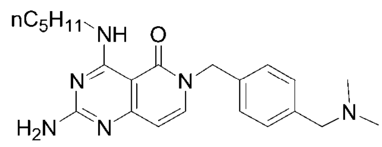

57 ,

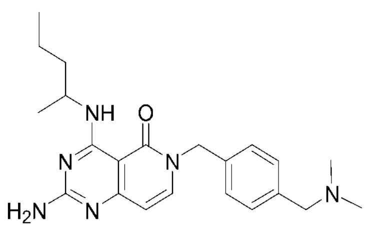

58 ,

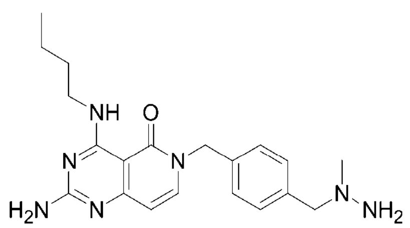

60 ,

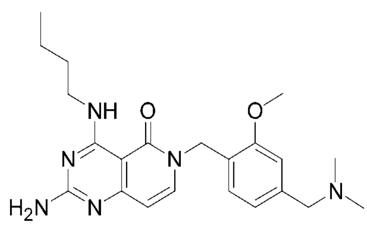

61 ,

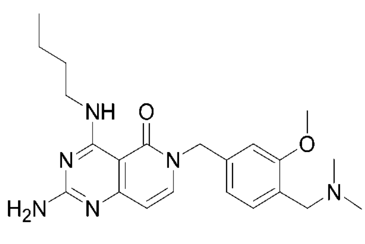

62 ,

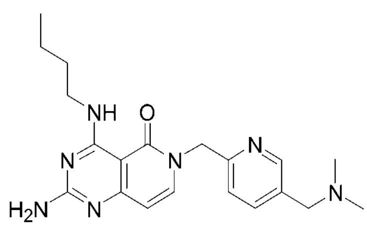

64 ,

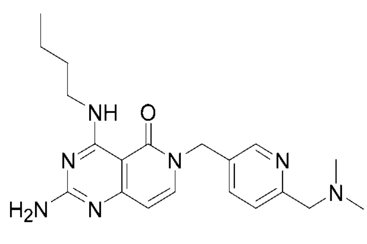

65 ,

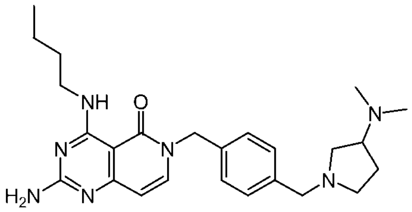

66 ,

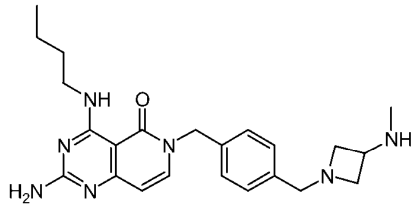

68 ,

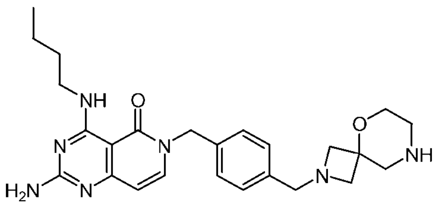

69 ,

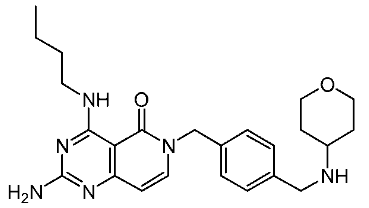

75 ,

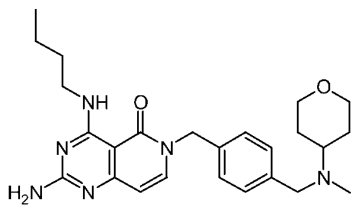

76 ,

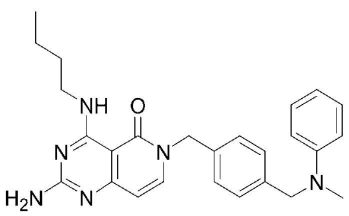

77 ,

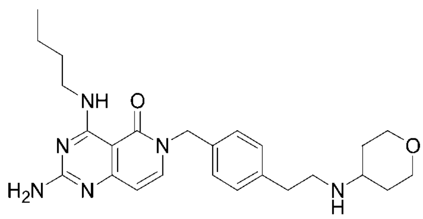

78 ,

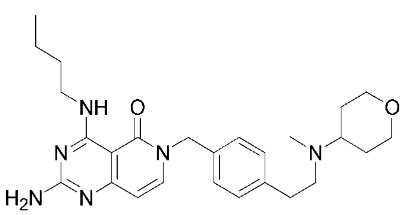

79 ,

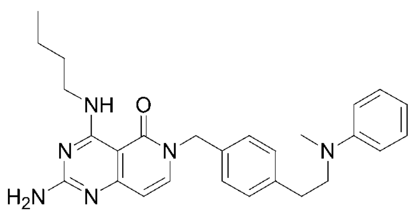

80 ,

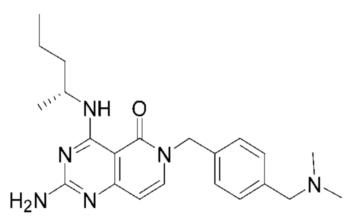

89 ,

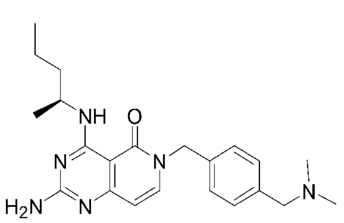

90 ,

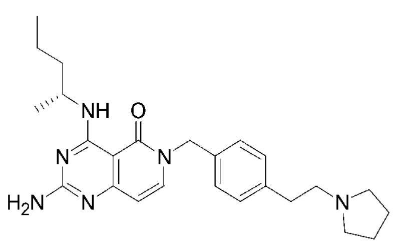

91 ,

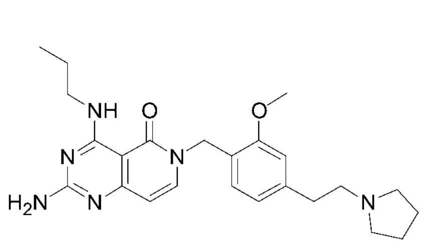

92 ,

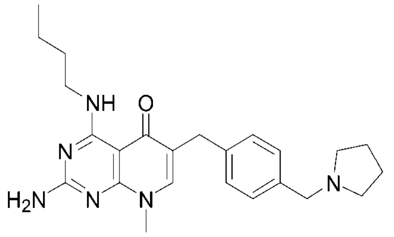

94 ,

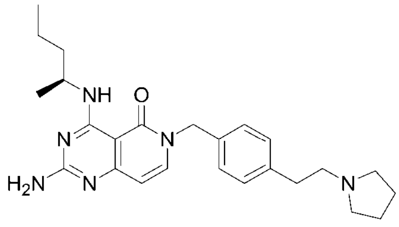

95 ,

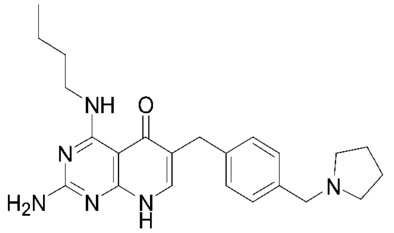

96 ,

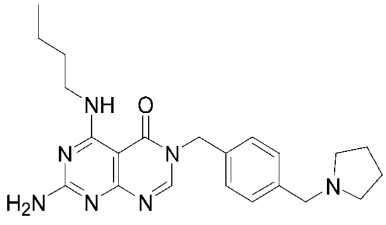

102 ,

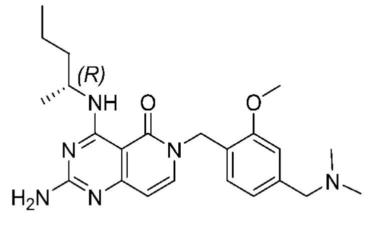

103 ,

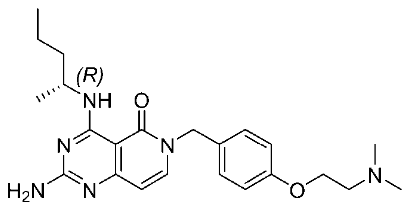

106 ,

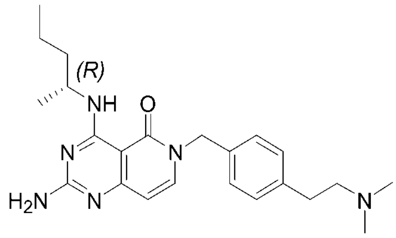

124 ,

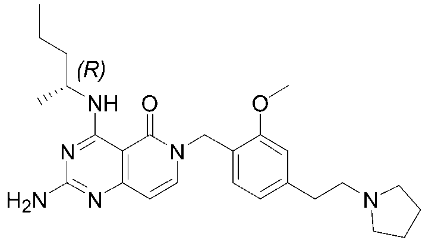

125 ,

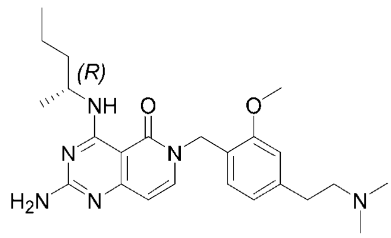

126 ,

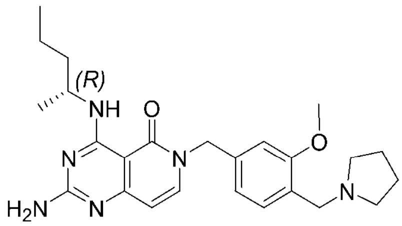

134 ,

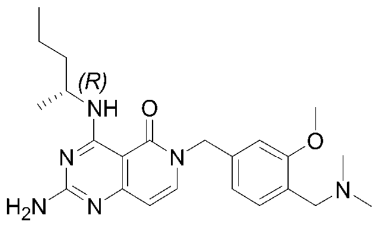

135 ,

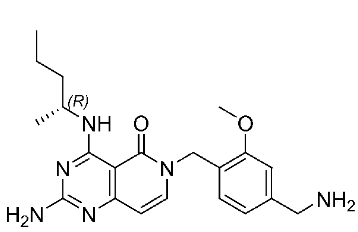

137 ,

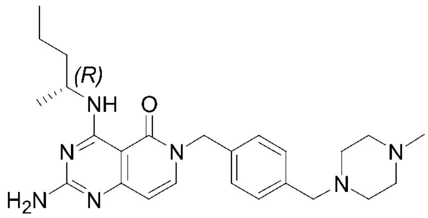

140 ,

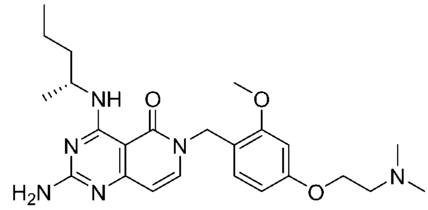

142 ,

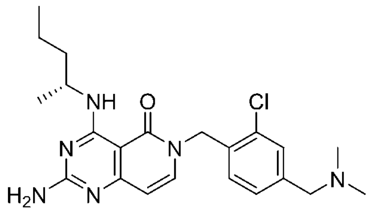

143 ,

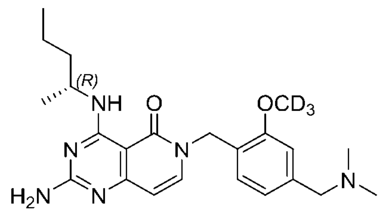

144 ,

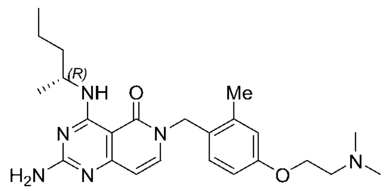

145 ,

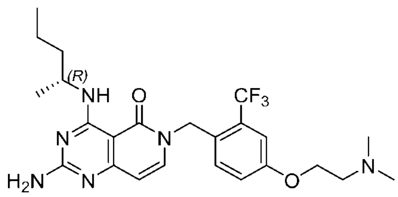

146 ,

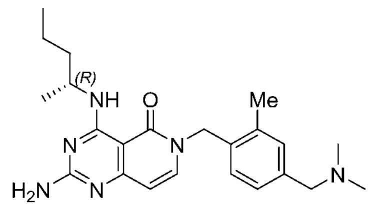

147 ,

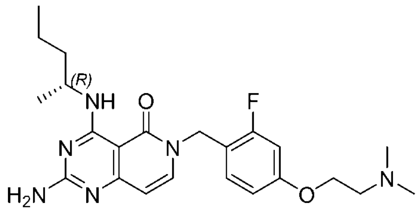

148 ,

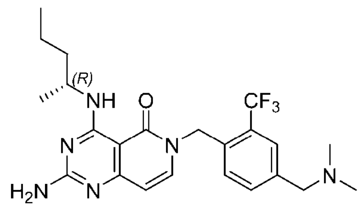

149 ,

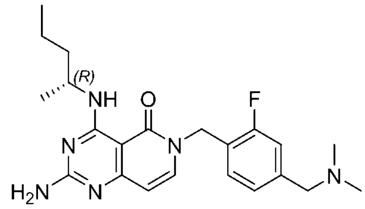

150 ,

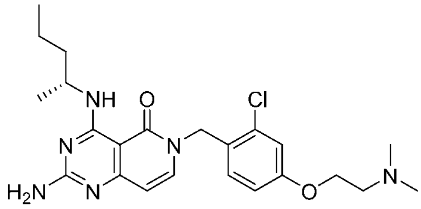

151 ,

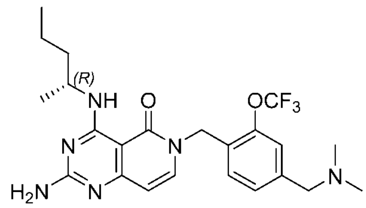

152 ,

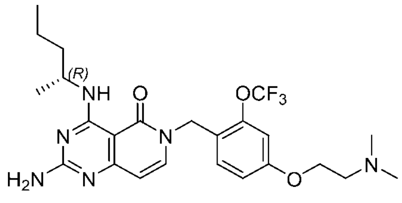

153,

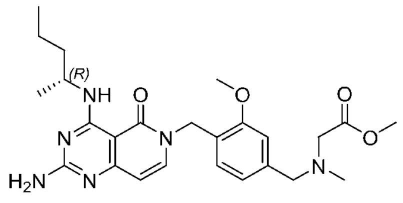

154,

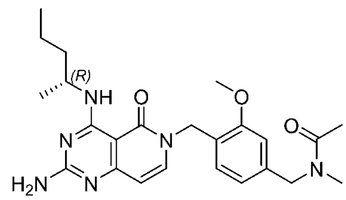

155,

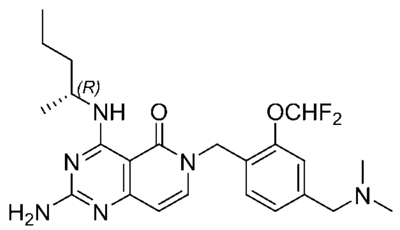

156,

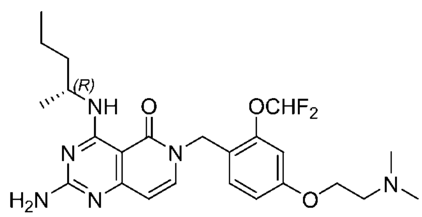

157.

**14.** The pyrimidine derivative of claim 1, wherein $X_1$ and $X_2$ are both selected from C-$R_3$.

**15.** The pyrimidine derivative of claim 14, wherein

Z is $NH_2$- or -OH;
Y is -O-, -S-, or -$NR_4$-, wherein $R_4$ is H or an optionally substituted alkyl group;
$R_1$ is an alkyl group optionally substituted with one or more substituents selected from oxo, halogen, an amino group, a hydroxyl group, a nitro group, a cyano group, a thiol group, an optionally substituted alkyl group, an optionally substituted alkyloxy group, an optionally substituted alkylthio group, an optionally substituted alkylamino group, an optionally substituted alkylacyl group, an optionally substituted alkylsulfinyl group, and an optionally substituted alkylsulfonyl group;
$R_2$ is

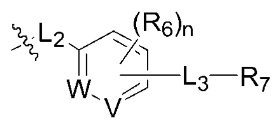

;

and -$L_3$-$R_7$ may be substituted at a para-, meta- or ortho-position of $L_2$;
$L_2$ and $L_3$ are each a bond or an optionally substituted linear alkylene group, wherein one or more carbon atoms in the linear alkylene group may be substituted with oxygen, sulfur and nitrogen heteroatoms, and the substituent is selected from one or more of an optionally substituted alkyl group, halogen, an amino group, a hydroxyl group, a nitro group, a cyano group, a thiol group, an optionally substituted alkyloxy group, an optionally substituted alkylthio group and an optionally substituted alkylamino group;
each of W and V may be N or CH; and W or V may be substituted with $R_6$ or -$L_3$-$R_7$ when being CH;
$R_3$ and $R_6$ are each independently H, an optionally substituted alkyl group, an optionally substituted alkyloxy group, an optionally substituted alkylthio group, an optionally substituted alkylamino group, halogen, an amino group, a hydroxyl group, a nitro group, a cyano group, and a thiol group;
$R_7$ is H, -OH, -N($R_9R_{10}$), -N($R_9$)$NH_2$, -NO($R_9R_{10}$), an optionally substituted heterocycloalkyl group, an optionally substituted cycloalkyl group, an optionally substituted aryl group, an optionally substituted heteroaryl group, optionally substituted heterocycloalkyl-$NR_9$-, optionally substituted cycloalkyl-$NR_9$-, optionally substituted aryl-$NR_9$-, optionally substituted heteroaryl-$NR_9$-, -OC(O)-$R_9$, -C(O)O-$R_9$, -N($R_9$)-C(O)-$R_{10}$ or -C(O)-N($R_9R_{10}$); and the substituent is selected from one or more of an optionally substituted alkyl group, an optionally substituted alkylamino group and oxo;
$R_9$ and $R_{10}$ are each independently H, an optionally substituted alkyl group, an optionally substituted cycloalkyl group, an optionally substituted heterocycloalkyl group, an optionally substituted aryl group, and an optionally substituted heteroaryl group; and
the substituent of the optionally substituted alkyl group is selected from one or more of halogen, an amino group, a

hydroxyl group, a nitro group, a cyano group, a thiol group, a carboxyl group, an ester group and an amide group; wherein n = 0, 1, 2, 3 or 4.

**16.** The pyrimidine derivative of claim 15, wherein $R_1$ is a C1-8 alkyl group optionally substituted with one or more substituents selected from an optionally substituted C1-8 alkylthio group and an optionally substituted C1-8 alkylsulfonyl group;

preferably, $R_1$ is a C2-6 alkyl group optionally substituted with a substituent selected from an optionally substituted C1-6 alkylthio group and an optionally substituted C1-6 alkylsulfonyl group;
preferably, $R_1$ is an n-butyl group, an n-pentyl group, a 2-pentyl group, an n-hexyl group, a 2-hexyl group, a 3-hexyl group, a methoxyethyl group, a methylthioethyl group, a methylsulfonylethyl group, a methoxypropyl group, a methylthiopropyl group, a methylsulfonylpropyl group,

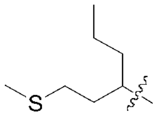 or 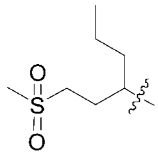 ;

or
more preferably, $R_1$ is an n-butyl group,

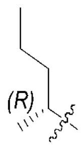, 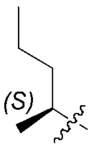,

a methylthiopropyl group, a methylsulfonylpropyl group,

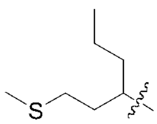 or 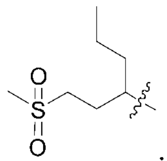 .

**17.** The pyrimidine derivative of any one of claim 15 or 16, wherein both W and V are N, both W and V are C, or one of W and V is N.

**18.** The pyrimidine derivative of any one of claims 15 to 17, wherein the heterocycloalkyl group is a 3- to 12-membered monocyclic, bicyclic or polycyclic heterocycloalkyl group; preferably, the heterocycloalkyl group is a 3- to 12-membered monocyclic, bicyclic or polycyclic heterocycloalkyl group containing 1, 2, 3 or 4 heteroatoms selected from N, O, and S; more preferably, the heteroatom in the heterocycloalkyl group is linked to $L_1$ or $L_3$; and more preferably, N atom in the heterocycloalkyl group is linked to $L_1$ or $L_3$.

**19.** The pyrimidine derivative of claim 18, wherein the heterocycloalkyl group is a 3- to 7-membered monocyclic heterocycloalkyl group containing 1 or 2 heteroatoms selected from N, O, and S, or is a 7- to 12-membered fused-ring or spiro heterocycloalkyl group containing 1, 2, 3 or 4 heteroatoms selected from N, O, and S;

preferably, the heterocycloalkyl group is a 4- to 6-membered monocyclic heterocycloalkyl group containing 1 or 2 heteroatoms selected from N and O, or is a 7- to 11-membered spiro heterocycloalkyl group containing 2, 3 or 4 heteroatoms selected from N and O; or
more preferably, the heterocycloalkyl group is one of the following groups:

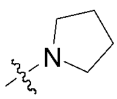, 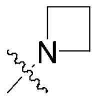, 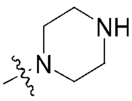, 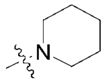, 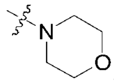, 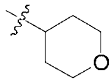, 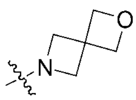 and 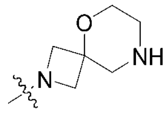.

**20.** The pyrimidine derivative of any one of claims 15 to 19, wherein $L_2$ and $L_3$ are each a bond or an optionally substituted linear alkylene group containing 1 to 6 chain atoms, 1 or 2 carbon atoms in the linear alkylene group may be substituted with a heteroatom of oxygen, and the substituent is selected from the C1-8 alkyl group, halogen, an amino group, a hydroxyl group, a nitro group, a cyano group and a thiol group;

preferably, $L_2$ is an optionally substituted linear alkylene group containing 1 to 4 chain atoms, more preferably is an optionally substituted linear alkylene group containing 2 or 3 chain atoms, and more preferably, is $-CH_2-$, $-CH_2CH_2-$, -O-, $-O-CH_2-CH_2-$ and $-O-CH_2-$; and
preferably, $L_3$ is a bond or an optionally substituted linear alkylene group containing 1 to 4 chain atoms, preferably is a bond or an optionally substituted linear alkylene group containing 2 or 3 chain atoms, more preferably is a bond, $-CH_2-$, $-CH_2CH_2-$, $-CH_2CH_2CH_2-$, $-O-CH_2-CH_2-$, $-O-CH_2-$ and $-CH(CH_3)CH_2-$, and more preferably, is

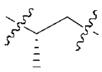 or 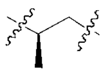.

**21.** The pyrimidine derivative of any one of claims 15 to 20, wherein $R_7$ is -OH, $-N(R_9R_{10})$, $-N(R_9)NH_2$, $-NO(R_9R_{10})$, an optionally substituted 3- to 12-membered heterocycloalkyl group containing 1, 2, 3 or 4 heteroatoms selected from N, O, and S, an optionally substituted C6-10 aryl group, optionally substituted 3- to 12-membered heterocycloalkyl-$NR_9$- containing 1, 2, 3 or 4 heteroatoms selected from N, O, and S, or optionally substituted C6-10 aryl-$NR_9$-; the substituent is selected from one or more of an optionally substituted C1-8 alkyl group, an optionally substituted C1-8 alkylamino group, an optionally substituted di(C1-8)alkylamino group and oxo; and $R_9$ and $R_{10}$ are each independently H or a C1-8 alkyl group; and
preferably, the heterocycloalkyl group is a 4- to 6-membered monocyclic heterocycloalkyl group containing 1 or 2 heteroatoms selected from N and O, or is a 7- to 11-membered spiro heterocycloalkyl group containing 2, 3 or 4 heteroatoms selected from N and O; more preferably, the heteroatom in the heterocycloalkyl group is linked to $L_3$; and more preferably, the N atom in the heterocycloalkyl group is linked to $L_3$.

**22.** The pyrimidine derivative of claim 21, wherein $R_7$ is $-NO(R_9R_{10})$ or an optionally oxo-substituted 3- to 12-membered heterocycloalkyl group containing 1, 2, 3 or 4 heteroatoms selected from N, O, and S.

**23.** The pyrimidine derivative of any one of claims 15 to 22, wherein $R_2$ is selected from one of the following optionally substituted groups:

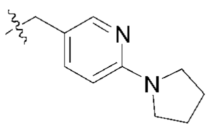, 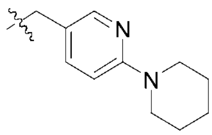,

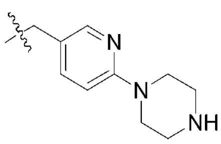, 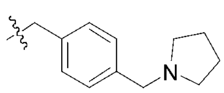, 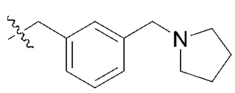, 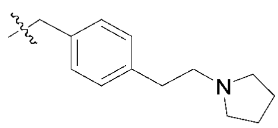,

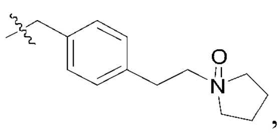, 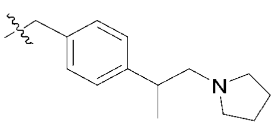, 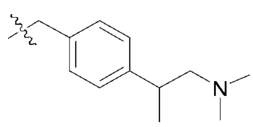, 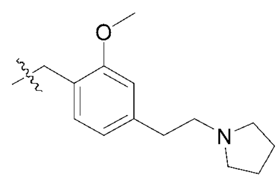,

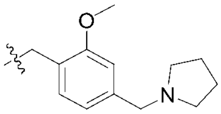, 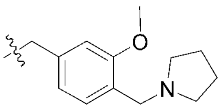, 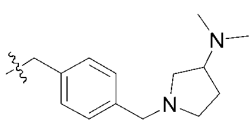, 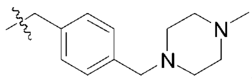,

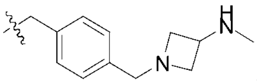, 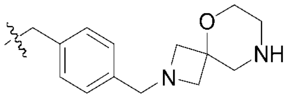, 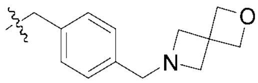, 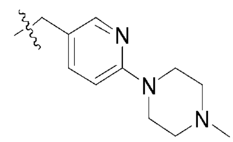,

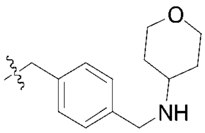, 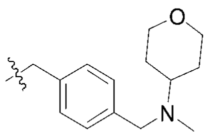, 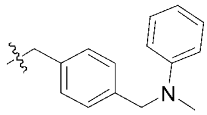, 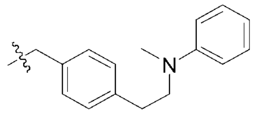,

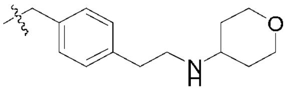, 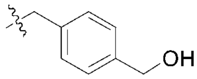, 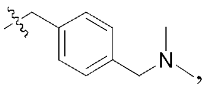, 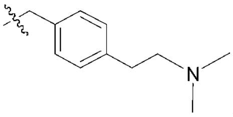,

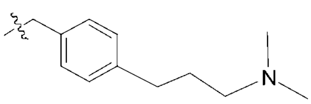, 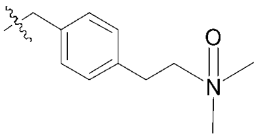, 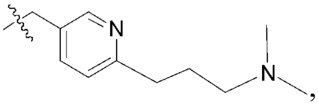, 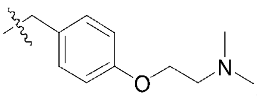,

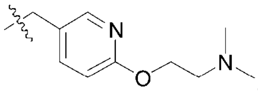, 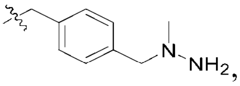, 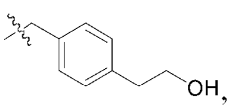, 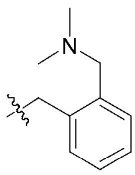, 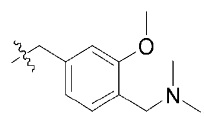,

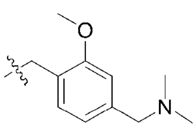, 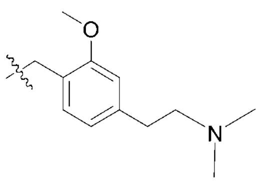, 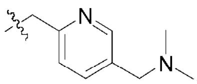, 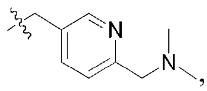, 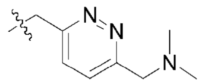,

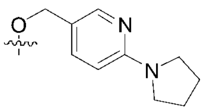, 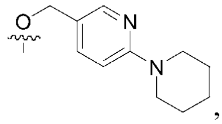, 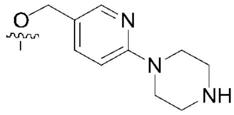, 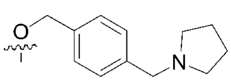,

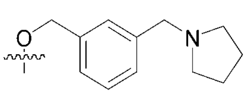, 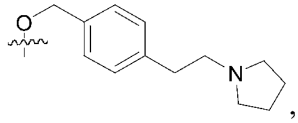, 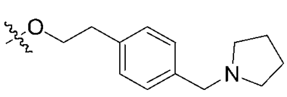, 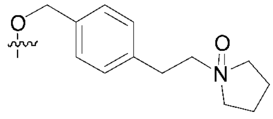,

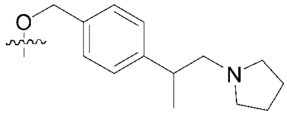, 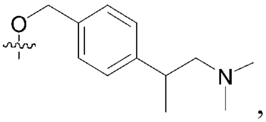, 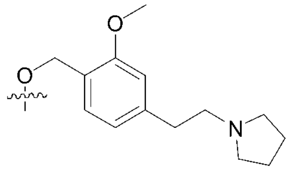, 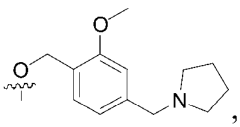,

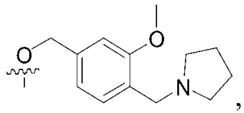, 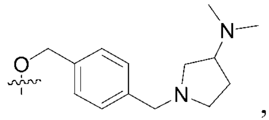, 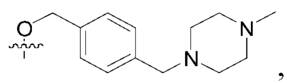, 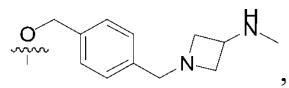,

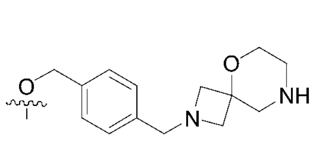, 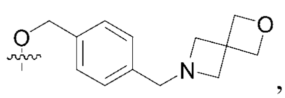, 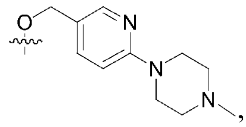, 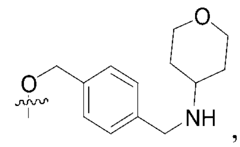,

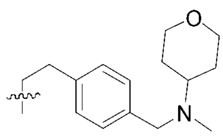, 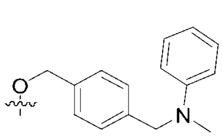, 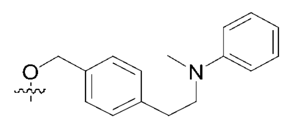, 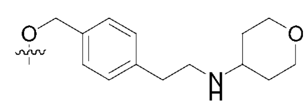,

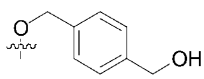, 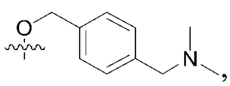, 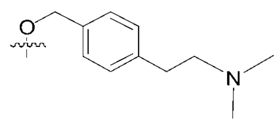, 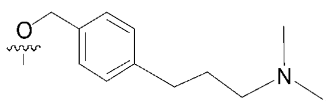,

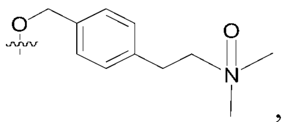, 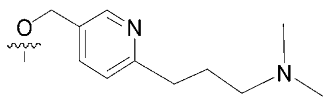, 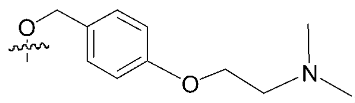,

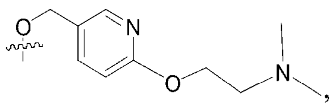, 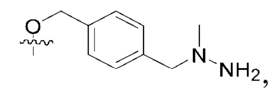, 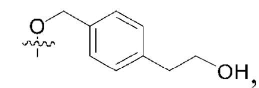, 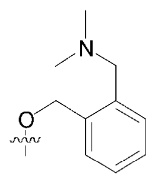,

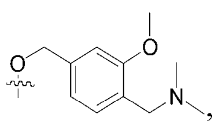, 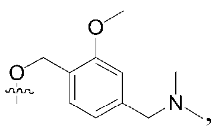, 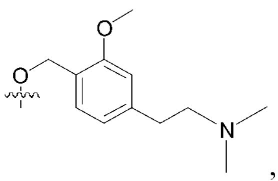, 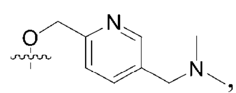,

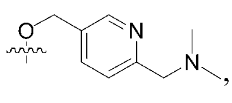, 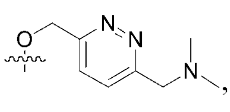, 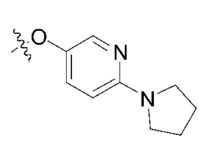, 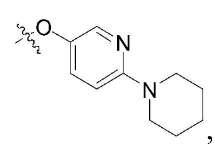, 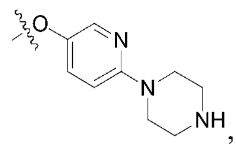,

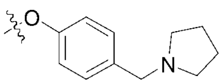, 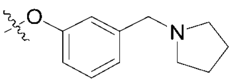, 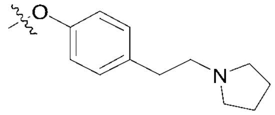, 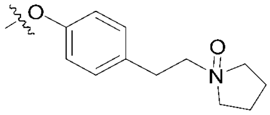,

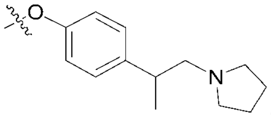, 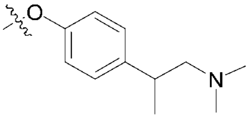, 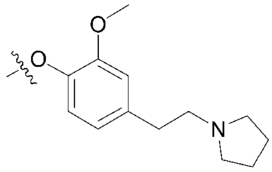, 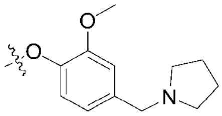,

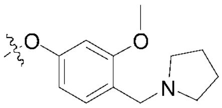, 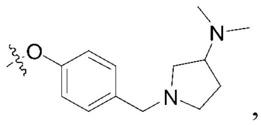, 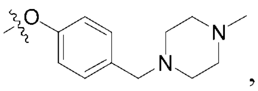, 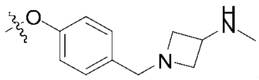,

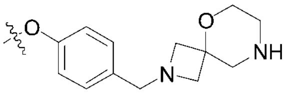, 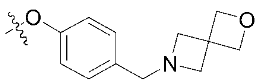, 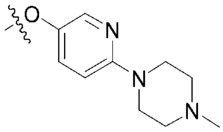, 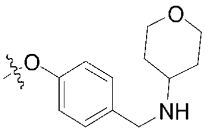,

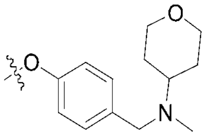, 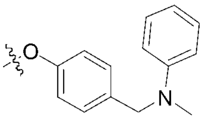, 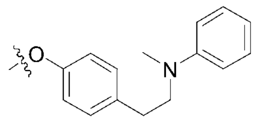, 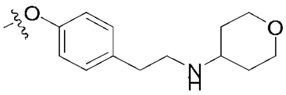,

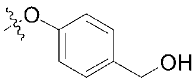, 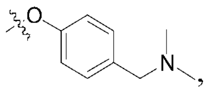, 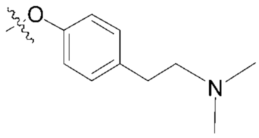, 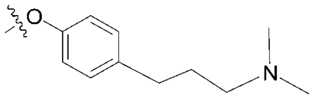,

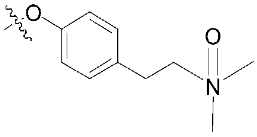, 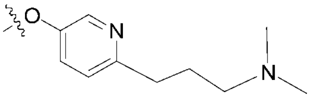, 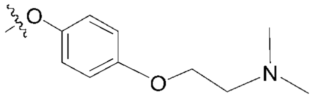,

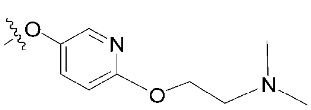, 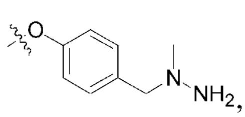, 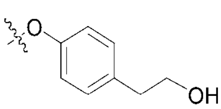, 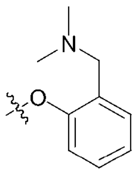, 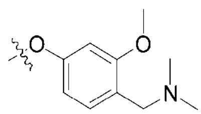,

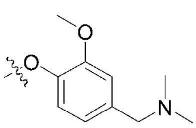, 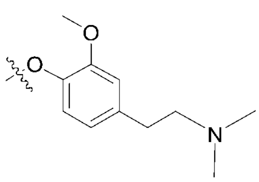, 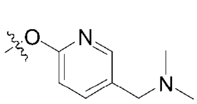, 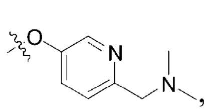, 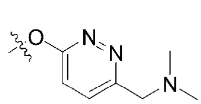,

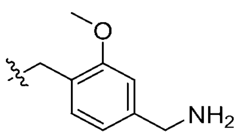, 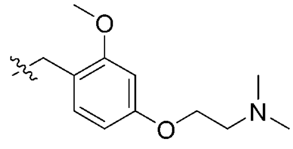, 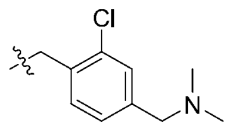, 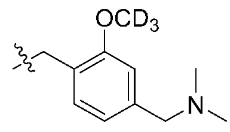,

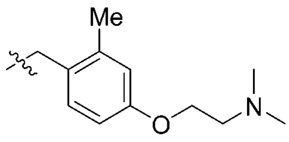, 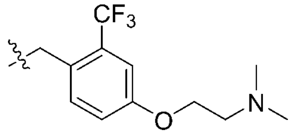, 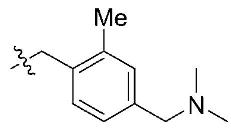, 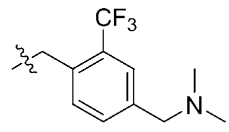,

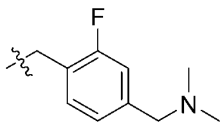, 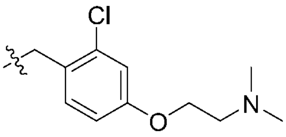, 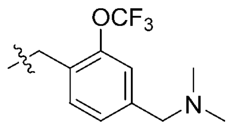, 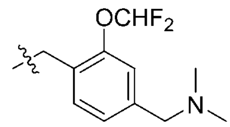,

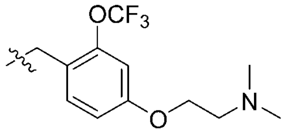, 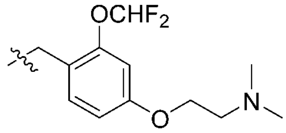, 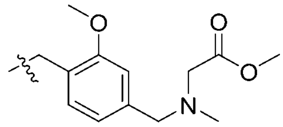, 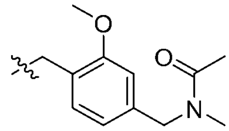,

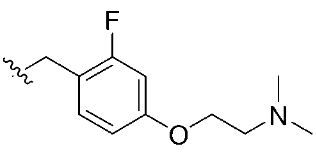;

or

further, $R_2$ is optionally substituted

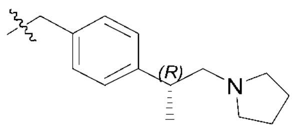, 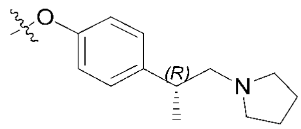,

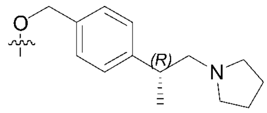, 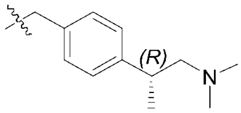, 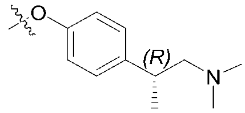, 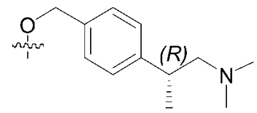,

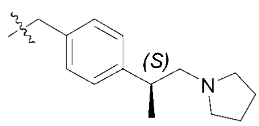, 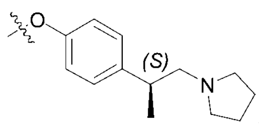, 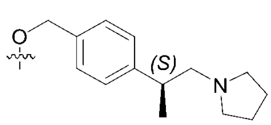, 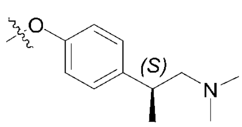,

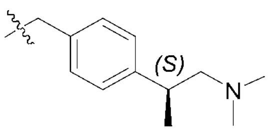, 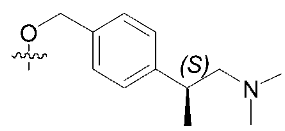;

and

the substituent is selected from one or more of an alkyl group, an alkyloxy group, an alkylthio group, an alkylamino

group, halogen, an amino group, a hydroxyl group, a nitro group, a cyano group and a sulfhydryl group.

**24.** The pyrimidine derivative of any one of claims 15 to 23, wherein the compound of Formula (I-1) specifically has one of the following formulas:

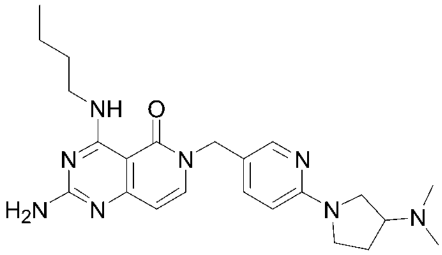
71 ,

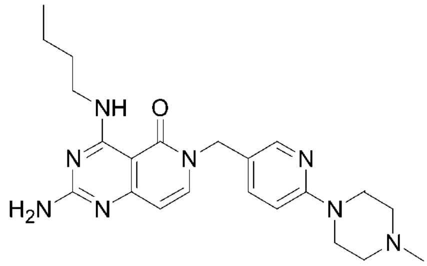
72 ,

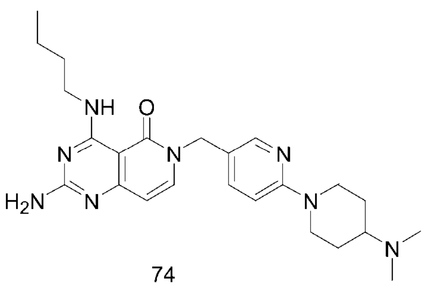
74 ,

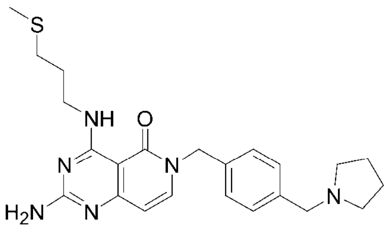
85 ,

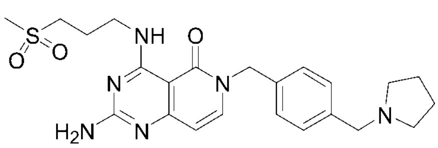
86 ,

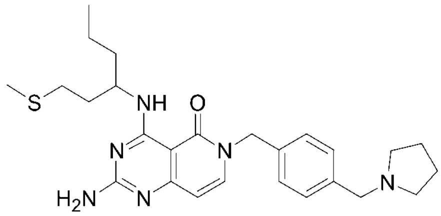
87 ,

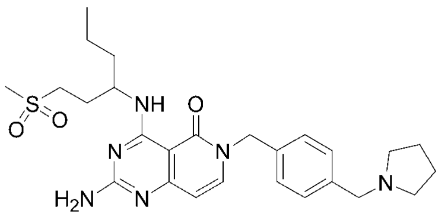
88 ,

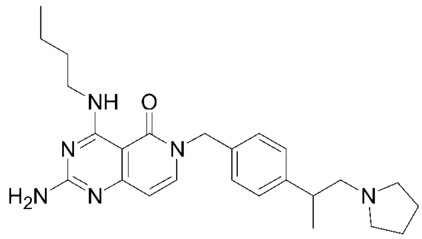
81 ,

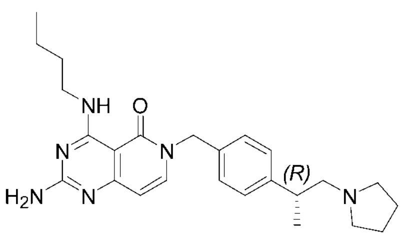
127 ,

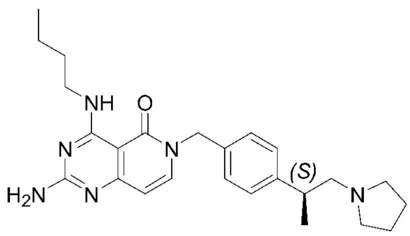
128 ,

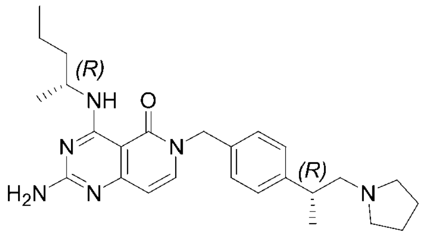

129 ,

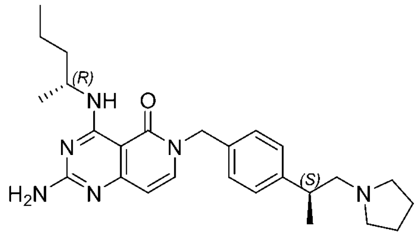

130 ,

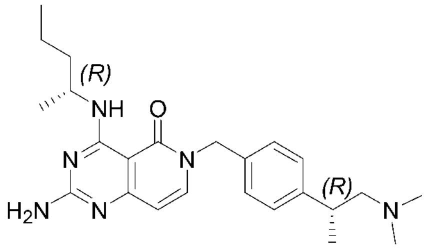

132 ,

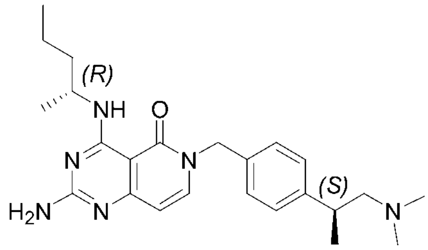

133 ,

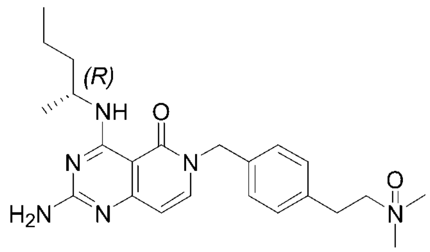

138 ,

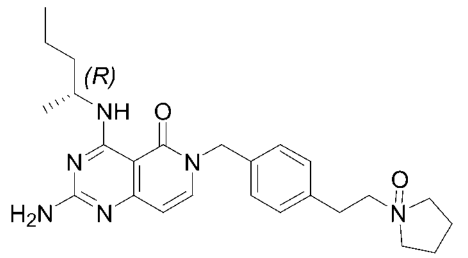

139 .

**25.** A pyrimidine derivative, which is a compound of Formula (II-1) or (II-2), or a stereoisomer, a tautomer, an isotopic derivative, a halogenated derivative, a hydrate, a solvate, a prodrug, or a pharmaceutically acceptable salt thereof;

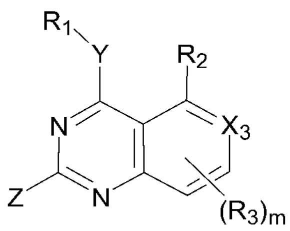

Formula (II-1)

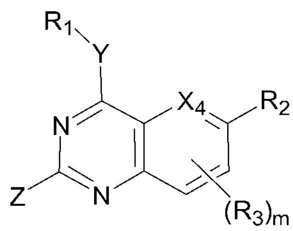

Formula (II-2)

wherein:

$X_3$ is N or CH, $X_4$ is CH, N or NO, and $X_3$ or $X_4$ may be substituted with $R_3$ when being CH;
Z is $-NH_2$, -OH, -NH-alkyl, -O-alkyl, -NH-C(O)-alkyl, -O-C(O)-alkyl, -NH-C(O)-OH, or -O-C(O)-OH;
Y is -O-, -S-, or $-NR_4-$, wherein $R_4$ is H or an optionally substituted alkyl group;
$R_1$ is an alkyl group or an aryl group optionally substituted with one or more substituents selected from oxo, halogen, an amino group, a hydroxyl group, a nitro group, a cyano group, a thiol group, $-C(O)-NH_2$, -C(O)-OH, a heteroaryl group (optionally substituted with one or more of halogen, an amino group, a hydroxyl group, a nitro group, a cyano group, a thiol group, an alkoxyl group, and an alkyl group), an aryl group (optionally substituted with one or more of halogen, an amino group, a hydroxyl group, a nitro group, a cyano group, a thiol group, an alkoxyl group, and an alkyl group), a heterocycloalkyl group (optionally substituted with one or more of halogen,

an amino group, a hydroxyl group, a nitro group, a cyano group, a thiol group, an alkoxyl group, and an alkyl group), a cycloalkyl group (optionally substituted with one or more of halogen, an amino group, a hydroxyl group, a nitro group, a cyano group, a thiol group, an alkoxyl group, and an alkyl group), an optionally substituted alkyl group, an optionally substituted alkyloxy group, an optionally substituted alkylthio group, an optionally substituted alkylamino group, an optionally substituted alkylacyl group, an optionally substituted alkylsulfinyl group, an optionally substituted alkylsulfonyl group, an optionally substituted -C(O)-O-alkyl, optionally substituted -O-C(O)-alkyl, optionally substituted -C(O)-NH-alkyl, optionally substituted -NH-C(O)-alkyl, optionally substituted -S(O)-O-alkyl, optionally substituted -O-S(O)-alkyl, optionally substituted $-S(O)_2$-O-alkyl, optionally substituted $-O-S(O)_2$-alkyl, optionally substituted -S(O)-NH-alkyl, optionally substituted -NH-S(O)-alkyl, optionally substituted $-S(O)_2$-NH-alkyl, and optionally substituted -NH-$S(O)_2$-alkyl;

$R_2$ is

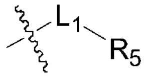 or 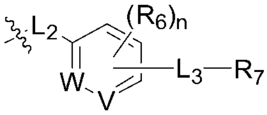 ;

and $-L_3-R_7$ may be substituted at a para-, meta- or ortho-position of $L_2$;

$L_1$, $L_2$ and $L_3$ are each a bond or an optionally substituted linear alkylene group, wherein one or more carbon atoms in the linear alkylene group may be substituted with oxygen, sulfur and nitrogen heteroatoms, and the substituent is selected from one or more of an optionally substituted alkyl group, halogen, an amino group, a hydroxyl group, a nitro group, a cyano group, a thiol group, an optionally substituted alkyloxy group, an optionally substituted alkylthio group and an optionally substituted alkylamino group;

$R_5$ is a cycloalkyl group or a heterocycloalkyl group optionally substituted with one or more substituents selected from oxo, halogen, an amino group, a hydroxyl group, a nitro group, a cyano group, a thiol group, an optionally substituted alkyl group, an optionally substituted alkyloxy group, an optionally substituted alkylthio group, and an optionally substituted alkylamino group;

each of W and V may be N or CH; and W or V may be substituted with $R_6$ or $-L_3-R_7$ when being CH;

$R_3$ and $R_6$ are independently H, an optionally substituted alkyl group, an optionally substituted alkyloxy group, an optionally substituted alkylthio group, an optionally substituted alkylamino group, halogen, an amino group, a hydroxyl group, a nitro group, a cyano group, and a thiol group;

$R_7$ is H, -OH, $-N(R_9R_{10})$, $-N(R_9)NH_2$, $-NO(R_9R_{10})$, an optionally substituted heterocycloalkyl group, an optionally substituted cycloalkyl group, an optionally substituted aryl group, an optionally substituted heteroaryl group, optionally substituted heterocycloalkyl-$NR_9$-, optionally substituted cycloalkyl-$NR_9$-, optionally substituted aryl-$NR_9$-, optionally substituted heteroaryl-$NR_9$-, $-OC(O)-R_9$, $-C(O)O-R_9$, $-N(R_9)-C(O)-R_{10}$ or $-C(O)-N(R_9R_{10})$; and the substituent is selected from one or more of an optionally substituted alkyl group, an optionally substituted alkylamino group and oxo;

$R_9$ and $R_{10}$ are each independently H, an optionally substituted alkyl group, an optionally substituted cycloalkyl group, an optionally substituted heterocycloalkyl group, an optionally substituted aryl group, and an optionally substituted heteroaryl group; and

the substituent of the optionally substituted group is selected from one or more of halogen, an amino group, a hydroxyl group, a nitro group, a cyano group, a thiol group, a carboxyl group, an ester group and an amide group; wherein m = 0, 1, 2 or 3; and n = 0, 1, 2, 3 or 4.

**26.** The pyrimidine derivative of claim 25, wherein $R_1$ is a C1-8 alkyl group optionally substituted with one or more substituents selected from an optionally substituted C1-8 alkyloxy group, an optionally substituted C1-8 alkylthio group, an optionally substituted C1-8 alkylamino group, an optionally substituted C1-8 alkylacyl group, an optionally substituted C1-8 alkylsulfinyl group, and an optionally substituted C1-8 alkylsulfonyl group;

preferably, $R_1$ is a C2-6 alkyl group optionally substituted with an optionally substituted C1-6 alkyloxy group, an optionally substituted C1-6 alkylthio group, and an optionally substituted C1-6 alkylsulfonyl group;

preferably, $R_1$ is an optionally substituted methyl group, ethyl group, n-propyl group, isopropyl group, n-butyl group, isobutyl group, tert-butyl group, n-pentyl group, 2-pentyl group, n-hexyl group, 2-hexyl group or 3-hexyl group;

more preferably, $R_1$ is an n-butyl group, an isobutyl group, a tert-butyl group, an n-pentyl group, a 2-pentyl group, a 3-hexyl group, a methoxyethyl group, a methylthioethyl group, a methylsulfonylethyl group, a methoxypropyl group, a methylthiopropyl group, a methylsulfonylpropyl group,

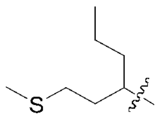 or 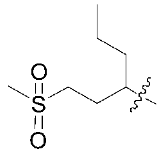 ;

or
more preferably, $R_1$ is an n-butyl group,

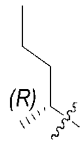 , 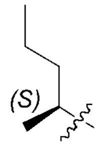 ,

a methylthiopropyl group, a methylsulfonylpropyl group,

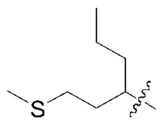 or 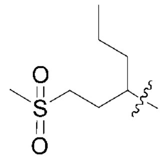 .

**27.** The pyrimidine derivative of any one of claim 25 or 26, wherein W and V are both N, W and V are both C, or one of W and V is N.

**28.** The pyrimidine derivative of any one of claims 25 to 27, wherein the heterocycloalkyl group is a 3- to 12-membered monocyclic, bicyclic or polycyclic heterocycloalkyl group; preferably, the heterocycloalkyl group is a 3- to 12-membered monocyclic, bicyclic or polycyclic heterocycloalkyl group containing 1, 2, 3 or 4 heteroatoms selected from N, O, and S; more preferably, the heteroatom in the heterocycloalkyl group is linked to $L_1$ or $L_3$; and more preferably, N atom in the heterocycloalkyl group is linked to $L_1$ or $L_3$.

**29.** The pyrimidine derivative of claim 28, wherein the heterocycloalkyl group is a 3- to 7-membered monocyclic heterocycloalkyl group containing 1 or 2 heteroatoms selected from N, O, and S, or is a 7- to 12-membered fused-ring or spiro heterocycloalkyl group containing 1, 2, 3 or 4 heteroatoms selected from N, O, and S;

preferably, the heterocycloalkyl group is a 4- to 6-membered monocyclic heterocycloalkyl group containing 1 or 2 heteroatoms selected from N and O, or is a 7- to 11-membered spiro heterocycloalkyl group containing 2, 3 or 4 heteroatoms selected from N and O; or
more preferably, the heterocycloalkyl group is one of the following groups:

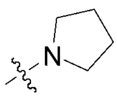 , 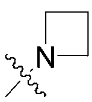 , 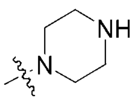 , 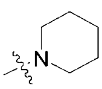 , 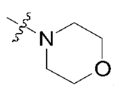 , 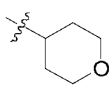 , 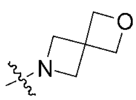 and 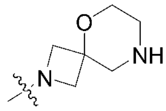 .

**30.** The pyrimidine derivative of any one of claims 25 to 29, wherein the aryl group is a C6-10 aryl group, preferably a phenyl group.

**31.** The pyrimidine derivative of any one of claims 25 to 30, wherein $L_1$, $L_2$ and $L_3$ are each a bond or an optionally substituted linear alkylene group containing 1 to 6 chain atoms, 1 or 2 carbon atoms in the alkylene group may be substituted with oxygen and sulfur heteroatoms, and the substituent is selected from the C1-8 alkyl group, halogen, an amino group, a hydroxyl group, a nitro group, a cyano group and a thiol group;

preferably, $L_1$ is an optionally substituted linear alkylene group containing 1 to 4 chain atoms, more preferably is an optionally substituted linear alkylene group containing 2 or 3 chain atoms, and more preferably, is -O-propylene or $-CH_2CH_2CH_2-$;

preferably, $L_2$ is an optionally substituted linear alkylene group containing 1 to 4 chain atoms, more preferably is an optionally substituted linear alkylene group containing 2 or 3 chain atoms, and more preferably, is $-CH_2-$, $-CH_2CH_2-$, -O-, $-O-CH_2-CH_2-$ and $-O-CH_2-$; and

preferably, $L_3$ is a bond or an optionally substituted linear alkylene group containing 1 to 4 chain atoms, preferably is a bond or an optionally substituted linear alkylene group containing 2 or 3 chain atoms, more preferably is a bond, $-CH_2-$, $-CH_2CH_2-$, $-CH_2CH_2CH_2-$, $-O-CH_2-CH_2-$, $-O-CH_2-$ and $-CH(CH_3)CH_2-$, and more preferably, is

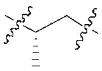 or 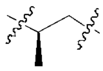.

**32.** The pyrimidine derivative of any one of claims 25 to 31, wherein $R_7$ is -OH, $-N(R_9R_{10})$, $-N(R_9)NH_2$, $-NO(R_9R_{10})$, an optionally substituted 3- to 12-membered heterocycloalkyl group containing 1, 2, 3 or 4 heteroatoms selected from N, O, and S, an optionally substituted C6-10 aryl group, optionally substituted 3- to 12-membered heterocycloalkyl-$NR_9$- containing 1, 2, 3 or 4 heteroatoms selected from N, O, and S, or optionally substituted C6-10 aryl-$NR_9$-; the substituent is selected from one or more of an optionally substituted C1-8 alkyl group, an optionally substituted C1-8 alkylamino group, an optionally substituted di(C1-8)alkylamino group and oxo; and $R_9$ and $R_{10}$ are each independently H or a C1-8 alkyl group; and

preferably, the heterocycloalkyl group is a 4- to 6-membered monocyclic heterocycloalkyl group containing 1 or 2 heteroatoms selected from N and O, or is a 7- to 11-membered spiro heterocycloalkyl group containing 2, 3 or 4 heteroatoms selected from N and O; more preferably, the heteroatom in the heterocycloalkyl group is linked to $L_3$; and more preferably, the N atom in the heterocycloalkyl group is linked to $L_3$.

**33.** The pyrimidine derivative of any one of claims 25 to 32, wherein $R_2$ is selected from one of the following optionally substituted groups:

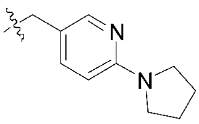, 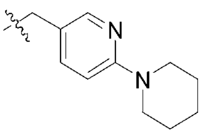, 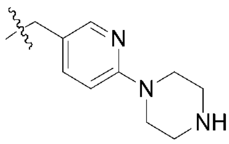, 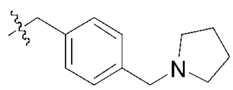, 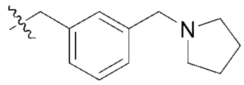,

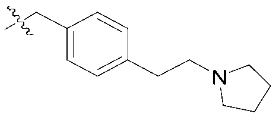, 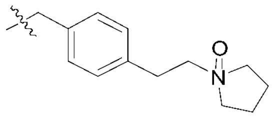, 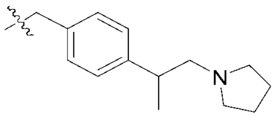, 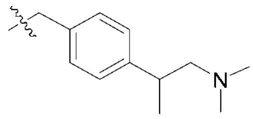,

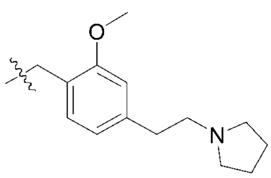, 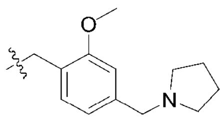, 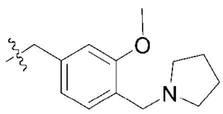, 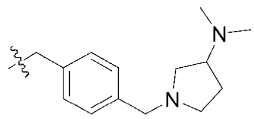,

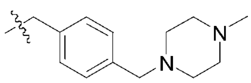, 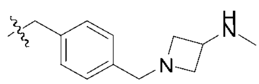, 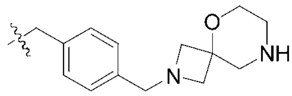, 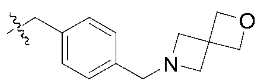,

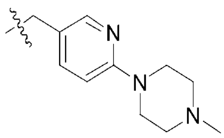, 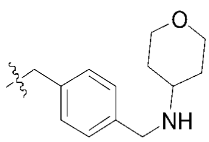, 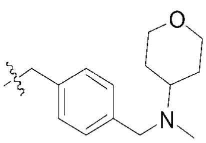, 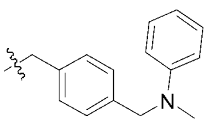, 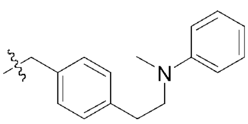,

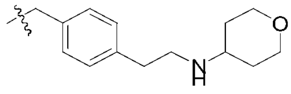, 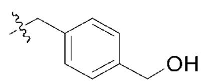, 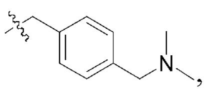, 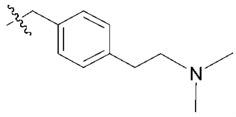,

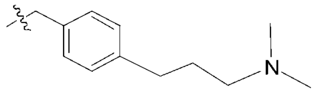, 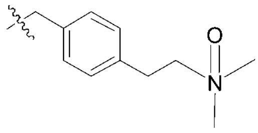, 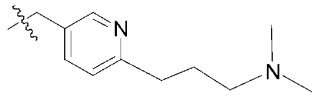, 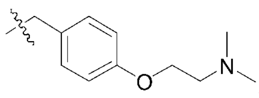,

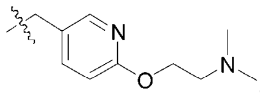, 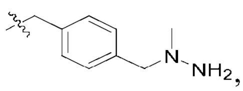, 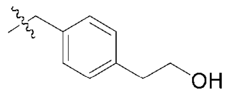, 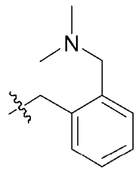, 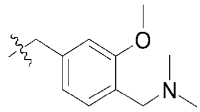,

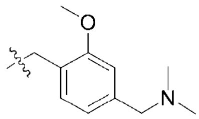, 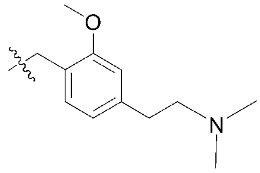, 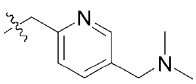, 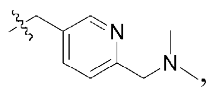, 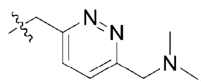,

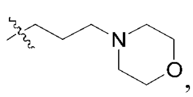, 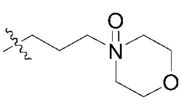, 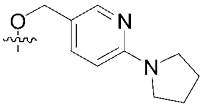, 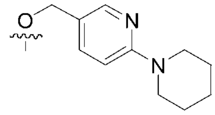, 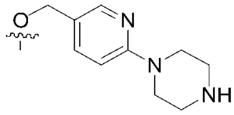,

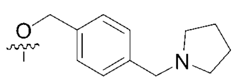, 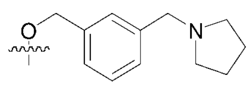, 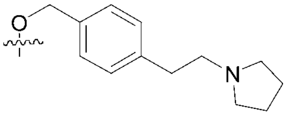, 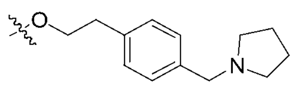,

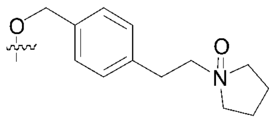, 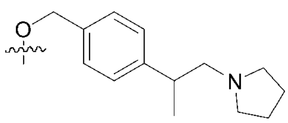, 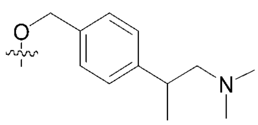, 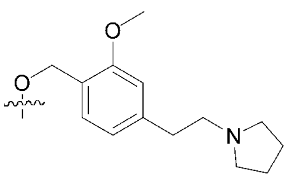,

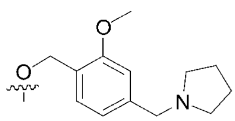, 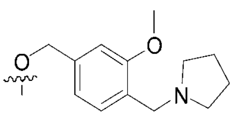, 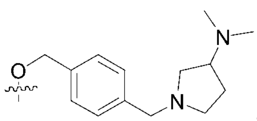, 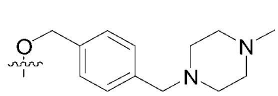,

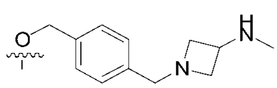, 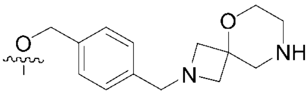, 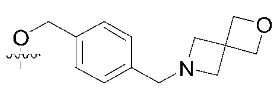, 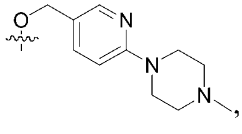,

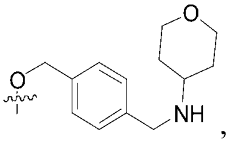, 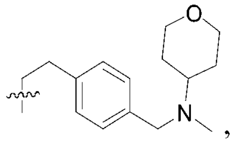, 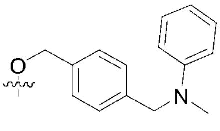, 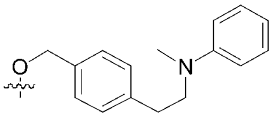,

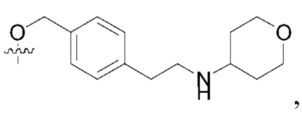, 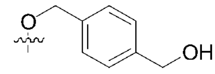, 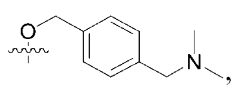, 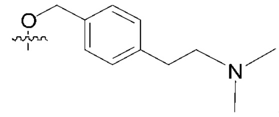,

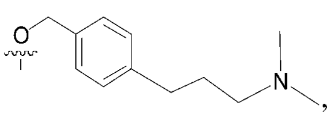, 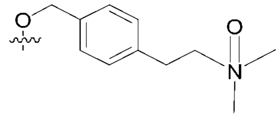, 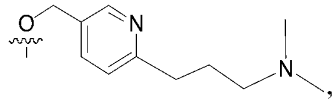,

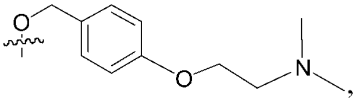, 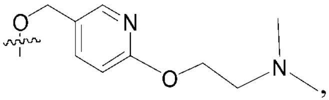, 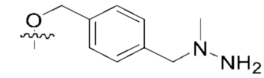,

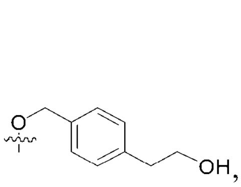, 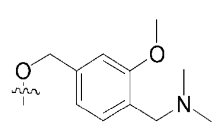, 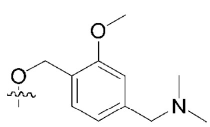, 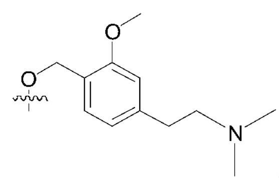,

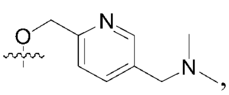, 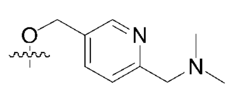, 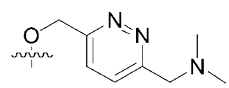, 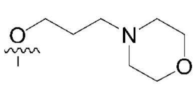, 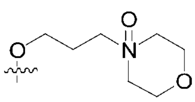,

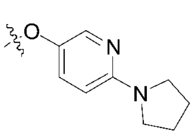, 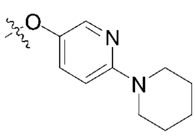, 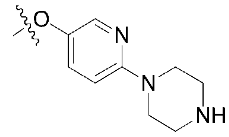, 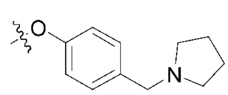, 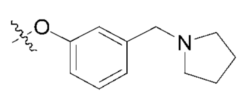,

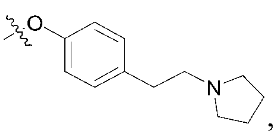, 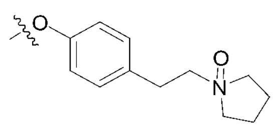, 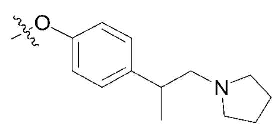, 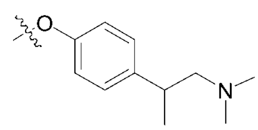,

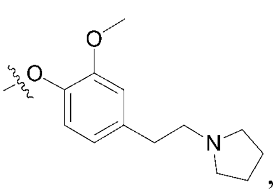, 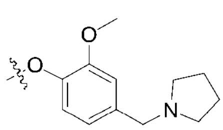, 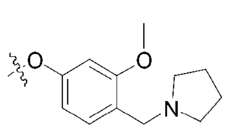, 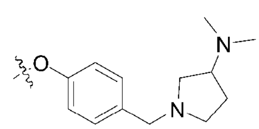,

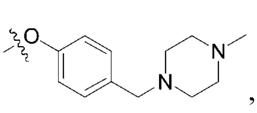, 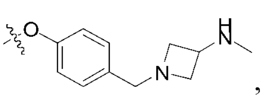, 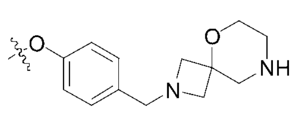, 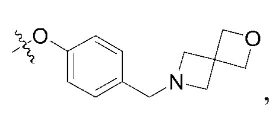,

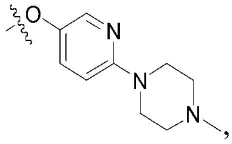, 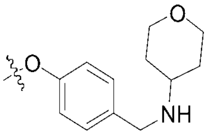, 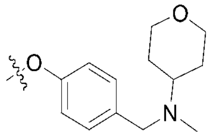, 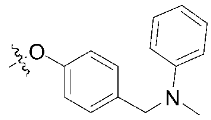, 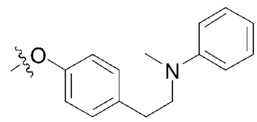,

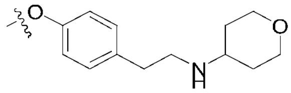, 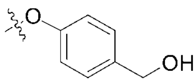, 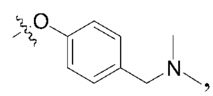, 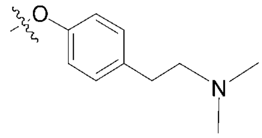,

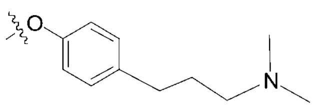, 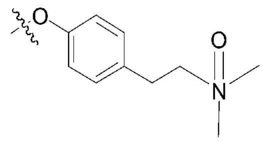, 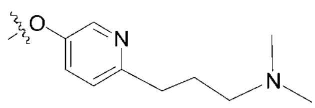,

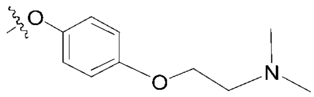, 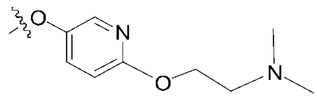, 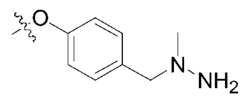, 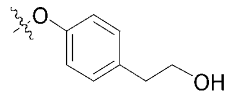,

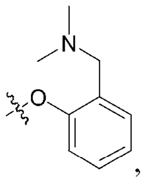, 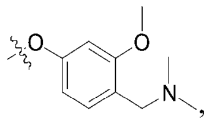, 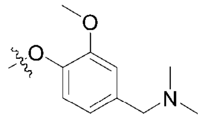, 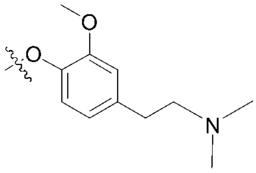, 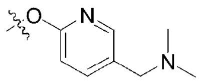,

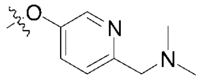, 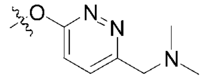, 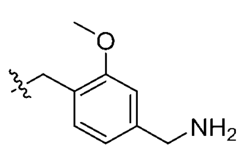, 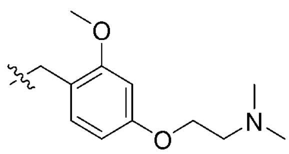,

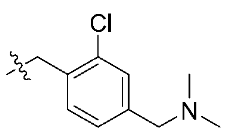, 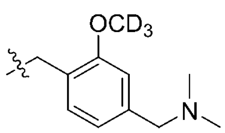, 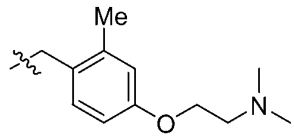, 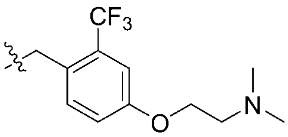,

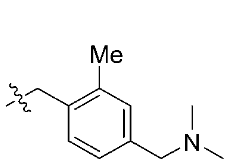, 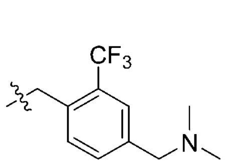, 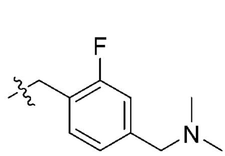, 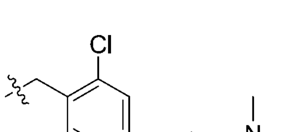,

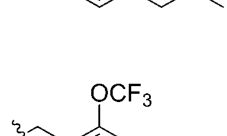, 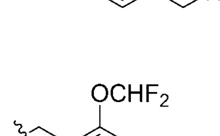, 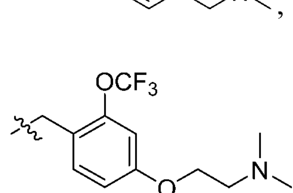, 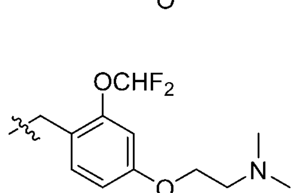,

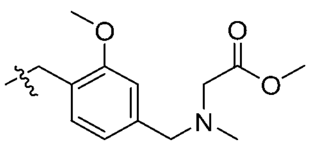 , 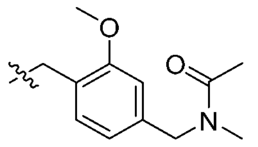 , 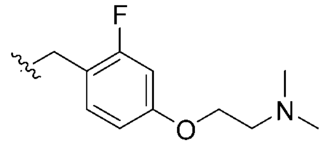 ;

further, $R_2$ is optionally substituted

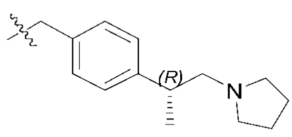 , 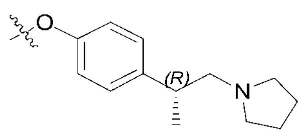 ,

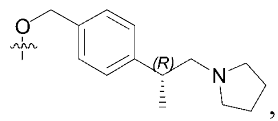 , 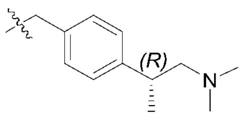 , 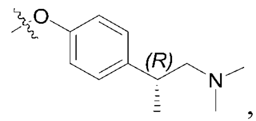 , 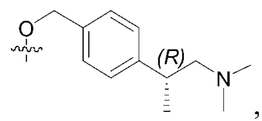 ,

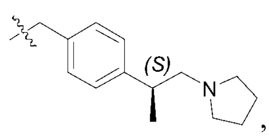 , 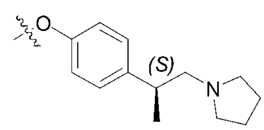 , 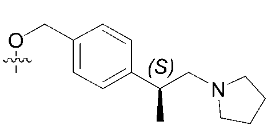 , 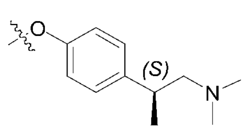 ,

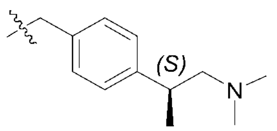 , 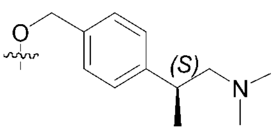 ;

and
the substituent is selected from one or more of an alkyl group, an alkyloxy group, an alkylthio group, an alkylamino group, halogen, an amino group, a hydroxyl group, a nitro group, a cyano group and a sulfhydryl group.

**34.** The pyrimidine derivative of any one of claims 25 to 33, wherein the compound of Formula (II-1) or (II-2) may specifically have the following formulas:

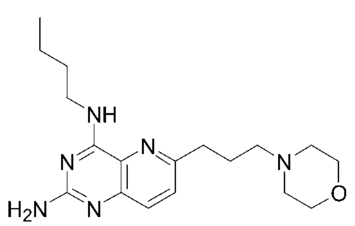

4 ,

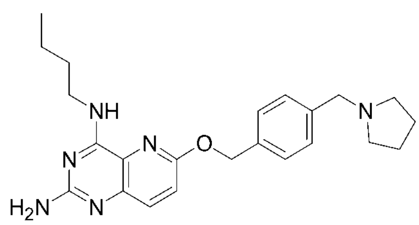

5 ,

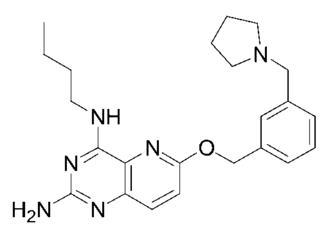

6 ,

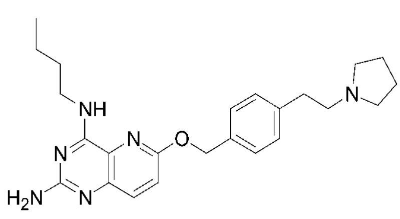

7 ,

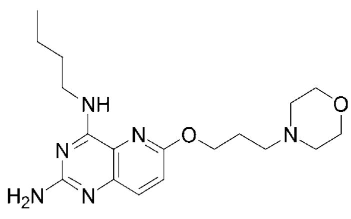

8 ,

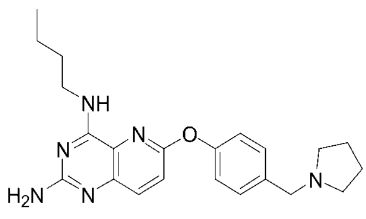

9 ,

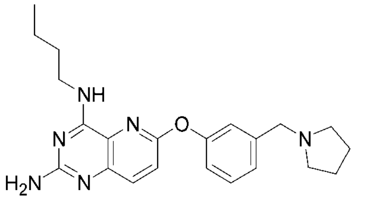
10 ,

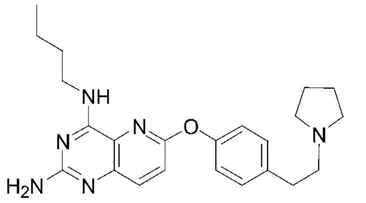
11 ,

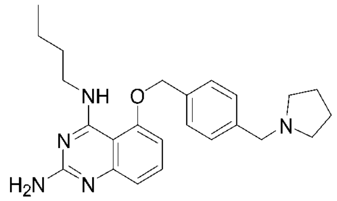
12 ,

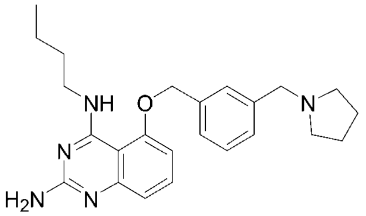
13 ,

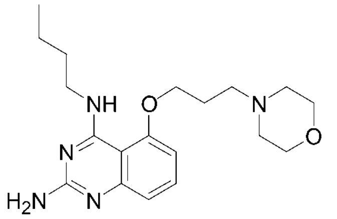
14 ,

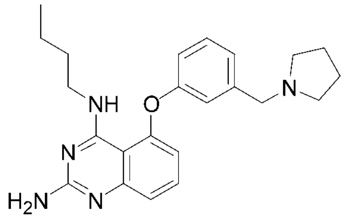
16 ,

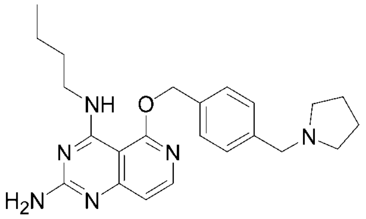
20 ,

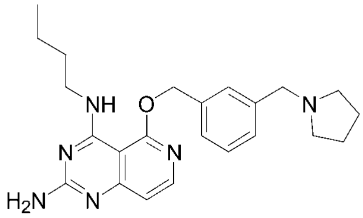
21 ,

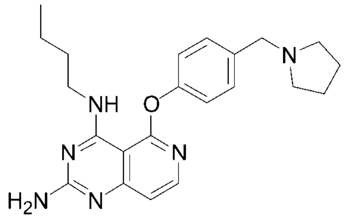
23 ,

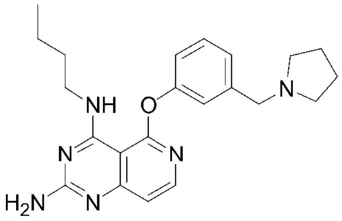
24 ,

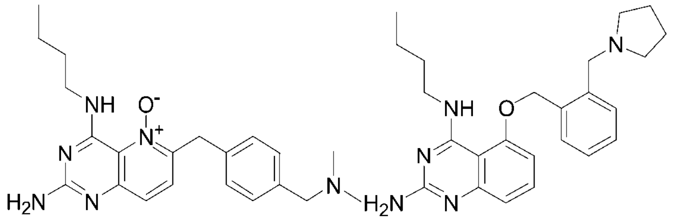
28 44 ,

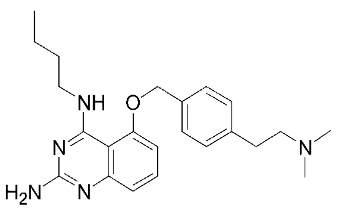
46 ,

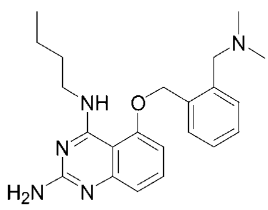
47 ,

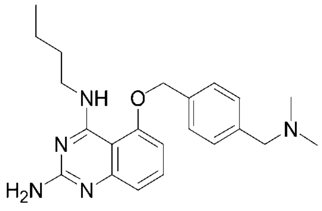
48 ,

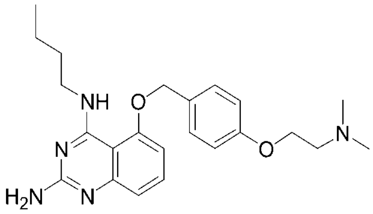
49 ,
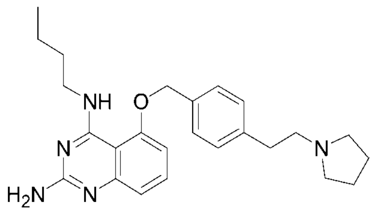
50 ,
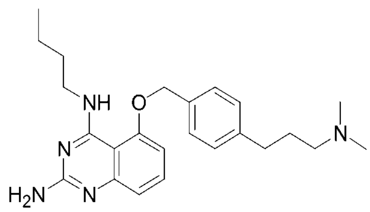
59 ,

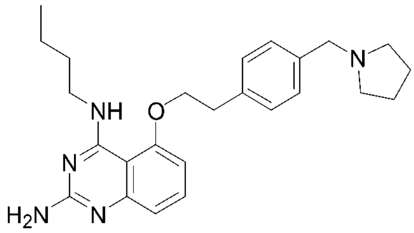
82 .

**35.** A pharmaceutical composition, comprising the pyrimidine derivative of any one of claims 1 to 34, preferably, wherein the pharmaceutical composition further comprises a pharmaceutically acceptable carrier or excipient.

**36.** The pharmaceutical composition of claim 35, further comprising at least one additional therapeutic agent, preferably, wherein the therapeutic agent is selected from a chemotherapeutic agent, immunotherapeutics, an anti-angiogenic agent, a cytokine, a hormone, a polynucleotide, an antibody, and an immunologically active fragment; and when such medicament contains multiple active ingredients, the active ingredients may be administered simultaneously, sequentially or separately at discretion of a physician.

**37.** Use of the pyrimidine derivative of any one of claims 1 to 34 or the pharmaceutical composition of any one of claims 35 and 36 in preparation of a medicament for preventing or treating a disease or a disorder associated with activity of TLRs.

**38.** Use of the pyrimidine derivative of any one of claims 1 to 34 or the pharmaceutical composition of any one of claims 35 and 36 in preparation of a medicament for preventing or treating an infectious disease, a respiratory disease, an immune-related disease, a viral disease or a cell proliferative disease.

**39.** The use of claim 38, wherein the disease is asthma, a tumor, HIV or HBV.

**40.** The use of claim 38, wherein the respiratory disease is asthma, a chronic obstructive pulmonary disease or an acute respiratory distress syndrome;

the immune-related disease is systemic lupus erythematosus, Sjögren's syndrome, Wegener's granulomatosis, sarcoidosis, Reiter's syndrome, Behçet's syndrome, rheumatoid arthritis, inflammatory bowel disease, polymyositis, vasculitis, ankylosing spondylitis or psoriatic arthritis;
the viral disease is Ebola virus disease, anthrax, condyloma acuminatum, verruca vulgaris, plantar wart, respiratory syncytial virus, hepatitis B, hepatitis C, dengue virus, herpes simplex virus (e.g., HSV-1 and HSV-2), molluscum contagiosum, cowpox, smallpox, lentivirus, human immunodeficiency virus (HIV), human papillomavirus (HPV), cytomegalovirus, varicella-zoster virus, rhinovirus, enterovirus, adenovirus, influenza, parainfluenza, mumps virus, measles virus, papovavirus, flavivirus, retrovirus, arenavirus (e.g., LCM, Junín virus, Machupo virus, Guanarito virus and Lassa fever) or filovirus (e.g., Ebola virus or Marburg virus); and
the cell proliferative disease is a tumor, comprising, but not limited to, a human sarcoma and carcinoma, for example, lymphoma, osteosarcoma, fibrosarcoma, myosarcoma, liposarcoma, chondrosarcoma, osteogenic sarcoma, chordoma, angiosarcoma, endothelial sarcoma, lymphangiosarcoma, lymphangioendotheliosarcoma, synovioma, mesothelioma, Ewing's tumor, leiomyosarcoma, rhabdomyosarcoma, uterine tumor, neuroma, gastrointestinal carcinoma, colon cancer, rectal cancer, squamous cell carcinoma, basal cell carcinoma,

adenocarcinoma, papillary carcinoma, medullary carcinoma, bronchogenic carcinoma, hepatoma, liver cancer, cholangiocarcinoma, choriocarcinoma, seminoma, embryonal carcinoma, nephroblastoma, cervical cancer, ovarian cancer, testicular tumor, renal cancer, lung cancer, epithelial carcinoma, glioma, astrocytoma, medulloblastoma, craniopharyngioma, ependymoma, pinealoma, hemangioblastoma, acoustic neuroma, oligodendroglioma, meningioma, melanoma, neuroblastoma, retinoblastoma, leukemia, polycythemia vera, multiple myeloma and a heavy chain disease; wherein the adenocarcinoma may be thyroid carcinoma, pancreatic carcinoma, sweat gland carcinoma, sebaceous gland carcinoma, breast carcinoma, papillary adenocarcinoma, cystadenocarcinoma or prostate carcinoma; and the lung cancer may be small cell lung cancer; the leukemia is acute lymphoblastic leukemia, acute myeloblastic leukemia, acute promyelocytic leukemia, acute myelomonocytic leukemia, acute monocytic leukemia, acute erythroleukemia, chronic myelogenous leukemia, chronic lymphocytic leukemia or chronic granulocytic leukemia; the lymphoma is Hodgkin's disease or non-Hodgkin's disease; and

preferably, the tumor is a cold tumor; or more preferably, the tumor is colon cancer, bladder cancer, melanoma, meningioma, lung cancer, liver cancer or pancreatic cancer.

**41.** Use of the pyrimidine derivative of any one of claims 1 to 34 or the pharmaceutical composition of any one of claims 35 and 36 in preparation of a medicament for preventing tumor recurrence or preferably a medicament capable of inducing durable systemic immune memory to prevent recurrence.

**42.** Use of the pyrimidine derivative of any one of claims 1 to 34 or the pharmaceutical composition of any one of claims 35 and 36 in preparation of a payload of a conjugated drug.

**43.** Use of the pyrimidine derivative of any one of claims 1 to 34 or the pharmaceutical composition of any one of claims 35 and 36 in preparation of a pharmaceutical preparation, wherein the pharmaceutical preparation is used to increase the number of genes beneficial to immunotherapy in each of inflammation- and cytokine-mediated pathways, T cell activation/apoptosis signaling pathways, and a B cell activation pathway; and preferably, the pharmaceutical preparation is used to increase the number of genes beneficial to tumor immunotherapy in the pathways.

**44.** The use of claim 43, wherein the pharmaceutical preparation is used to increase the number of CD8+ T cells, increase a ratio of CD8+ T cells to Treg cells, upregulate expression of PDL-1, increase a level of interleukin-2, increase a level of interferon-y, and/or decrease a level of interleukin-10.

**45.** Use of the pyrimidine derivative of any one of claims 1 to 34 or the pharmaceutical composition of any one of claims 35 and 36 in preparation of an agent for downregulating a Wnt signaling pathway, or preferably downregulating an expression level of β-catenin protein.

**46.** Use of the pyrimidine derivative of any one of claims 1 to 34 or the pharmaceutical composition of any one of claims 35 and 36 in preparation of a pharmaceutical preparation for modulating secretion levels of various cytokines in macrophages or preferably modulating mRNA expression levels of IL-1β, IL-6, IL-12β, TNF, IFN-β1, CXCL1, CXCL10 and IL-10 genes.

**47.** Use of the pyrimidine derivative of any one of claims 1 to 34 or the pharmaceutical composition of any one of claims 35 and 36 in preparation of a vaccine adjuvant.

**48.** A method for enhancing a positive immune response of an organism, comprising administering a therapeutically effective dose of the pyrimidine derivative of any one of claims 1 to 34 or the pharmaceutical composition of any one of claims 35 and 36, to modulate TLR7 and/or TLR8 to a system or an individual in need.

**49.** A method for enhancing chemotherapeutic efficacy, comprising administering a therapeutically effective dose of a chemotherapeutic agent, and simultaneously or subsequently administering a therapeutically effective dose of the pyrimidine derivative of any one of claims 1 to 34 or the pharmaceutical composition of any one of claims 35 and 36 to a system or an individual in need.

**50.** A method for improving immunotherapy, comprising administering a therapeutically effective dose of the pyrimidine derivative of any one of claims 1 to 34 or the pharmaceutical composition of any one of claims 35 and 36 to a system or an individual in need, and simultaneously or subsequently injecting chimeric antigen receptor T cells (CAR-T) into the system or the individual.

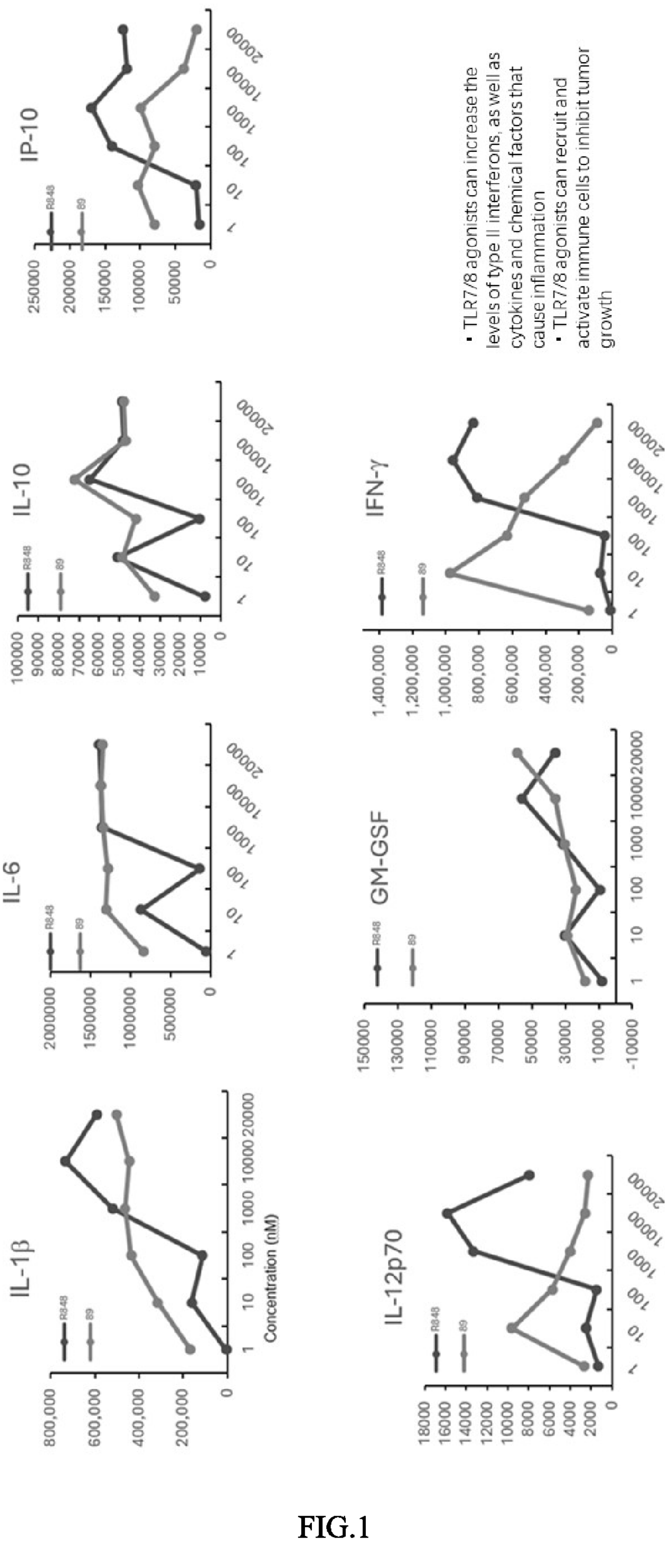
IL-1β
IL-6
IL-10
IP-10
IL-12p70
GM-GSF
IFN-γ
R848
89
Concentration (nM)
▪ TLR7/8 agonists can increase the levels of type II interferons, as well as cytokines and chemical factors that cause inflammation
▪ TLR7/8 agonists can recruit and activate immune cells to inhibit tumor growth

FIG.1

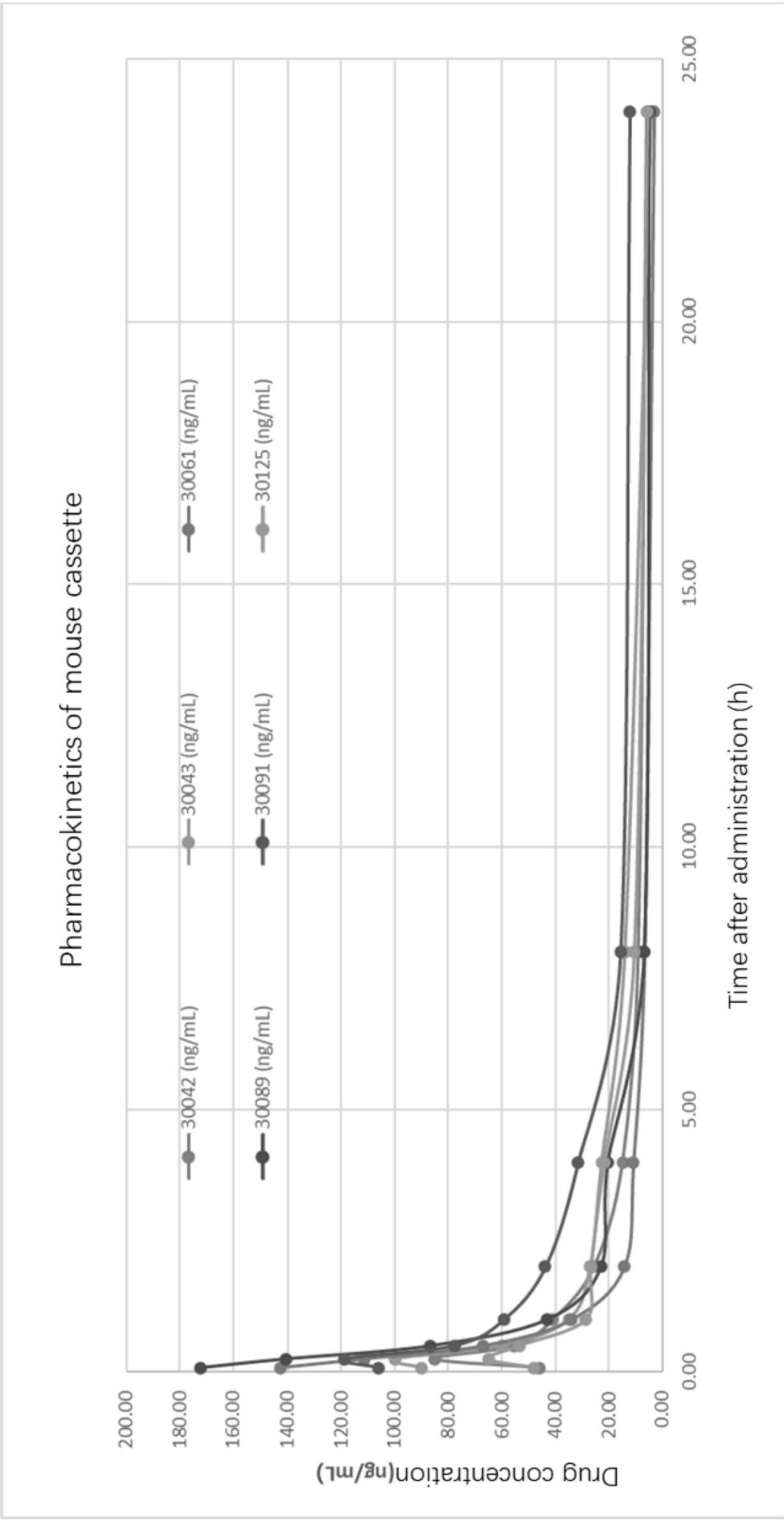
Pharmacokinetics of mouse cassette
30042 (ng/mL)
30043 (ng/mL)
30061 (ng/mL)
30089 (ng/mL)
30091 (ng/mL)
30125 (ng/mL)
Drug concentration(ng/mL)
Time after administration (h)
0.00
20.00
40.00
60.00
80.00
100.00
120.00
140.00
160.00
180.00
200.00
0.00
5.00
10.00
15.00
20.00
25.00

FIG.2

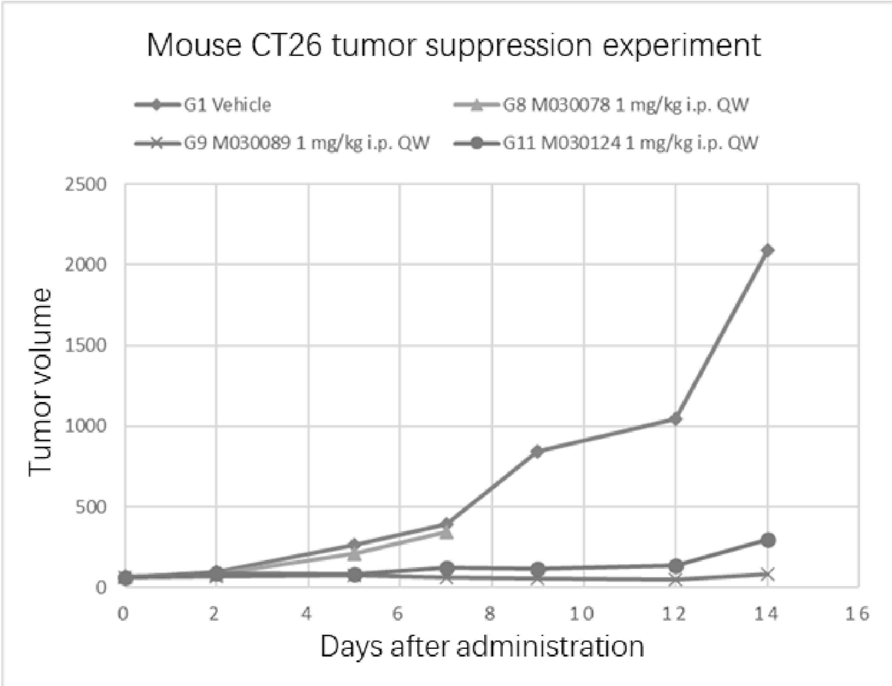
Mouse CT26 tumor suppression experiment
G1 Vehicle
G8 M030078 1 mg/kg i.p. QW
G9 M030089 1 mg/kg i.p. QW
G11 M030124 1 mg/kg i.p. QW
Tumor volume
0
500
1000
1500
2000
2500
0
2
4
6
8
10
12
14
16
Days after administration

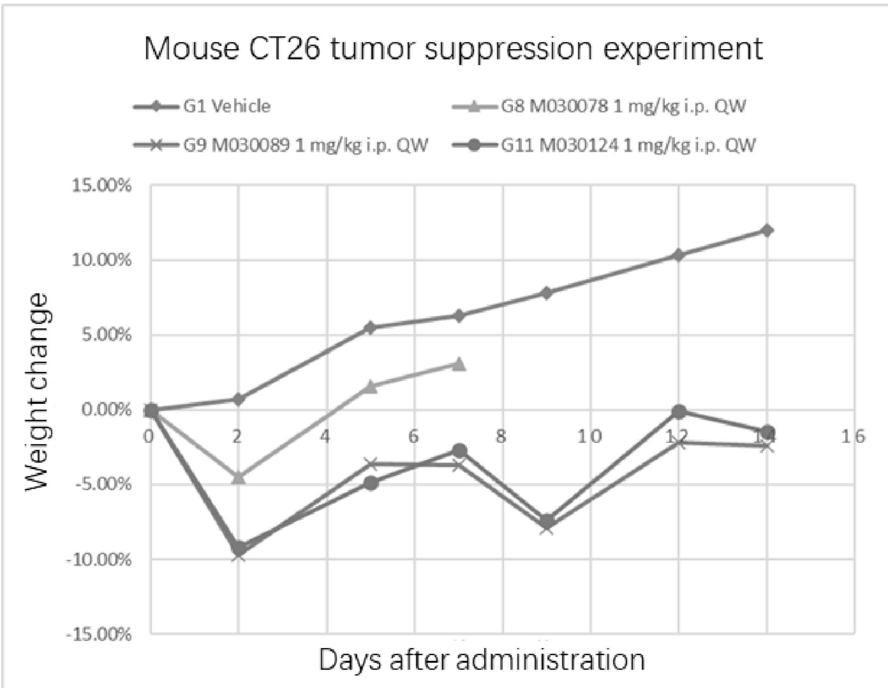
Mouse CT26 tumor suppression experiment
G1 Vehicle
G8 M030078 1 mg/kg i.p. QW
G9 M030089 1 mg/kg i.p. QW
G11 M030124 1 mg/kg i.p. QW
Weight change
15.00%
10.00%
5.00%
0.00%
-5.00%
-10.00%
-15.00%
0
2
4
6
8
10
12
14
16
Days after administration

FIG.3

## INTERNATIONAL SEARCH REPORT

International application No.
**PCT/CN2024/112501**

**A. CLASSIFICATION OF SUBJECT MATTER**

C07D471/04(2006.01)i; C07D487/02(2006.01)i; C07D519/00(2006.01)i; A61K31/519(2006.01)i; A61P31/00(2006.01)i; A61P35/00(2006.01)i

According to International Patent Classification (IPC) or to both national classification and IPC

**B. FIELDS SEARCHED**

Minimum documentation searched (classification system followed by classification symbols)

IPC:C07D,A61K,A61P

Documentation searched other than minimum documentation to the extent that such documents are included in the fields searched

Electronic data base consulted during the international search (name of data base and, where practicable, search terms used)

CNTXT, ENTXTC, REGISTRY (STN), CAPLUS (STN), CNKI: 稠环, 嘧啶, 激动剂, TLR, fused ring, pyrimidine, agonist, toll-like receptor, 结构检索, structure search

**C. DOCUMENTS CONSIDERED TO BE RELEVANT**

| Category* | Citation of document, with indication, where appropriate, of the relevant passages | Relevant to claim No. |
|---|---|---|
| X | WO 2010089686 A1 (PFIZER INC. et al.) 12 August 2010 (2010-08-12) description, page 108, line 5 to page 110, line 15 | 1, 5-10, 14 |
| X | Registry. "STN Columbus" *STN Columbus*, 09 July 2023 (2023-07-09), pp. 1-106 | 1, 4-11, 14-22, 25-33 |
| PX | CN 118382625 A (SHANGHAI WEISHEN PHARMACEUTICAL CO., LTD.) 23 July 2024 (2024-07-23) claims 12-15 | 12-13, 23, 35-50 |
| PX | CN 116789671 A (SHANGHAI WEISHEN PHARMACEUTICAL CO., LTD.) 22 September 2023 (2023-09-22) claims 9-11 | 12-13, 23, 35-50 |
| PX | WO 2024055879 A1 (SICHUAN KELUN-BIOTECH BIOPHARMACEUTICAL CO., LTD.) 21 March 2024 (2024-03-21) claims 9 and 17-20 | 12-13, 23, 35-50 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **18 November 2024** | **27 November 2024** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | Telephone No. |

Form PCT/ISA/210 (second sheet) (July 2022)

## INTERNATIONAL SEARCH REPORT

International application No.
**PCT/CN2024/112501**

### C. DOCUMENTS CONSIDERED TO BE RELEVANT

| Category* | Citation of document, with indication, where appropriate, of the relevant passages | Relevant to claim No. |
|---|---|---|
| X | CN 103748081 A (JANSSEN R&D IRELAND) 23 April 2014 (2014-04-23) description, paragraph 0229, and claims 7-8 | 25-31, 35-50 |
| X | CN 108069963 A (TSINGHUA UNIVERSITY) 25 May 2018 (2018-05-25) claims 5 and 9-20, and description, paragraphs 0060-0064 | 25-31, 35-50 |
| X | PIETERS, Serge et al. "Discovery of Selective 2,4-diaminoquinazoline Toll-Like Receptor 7 (TLR 7) Agonists" *Bioorganic and Medicinal Chemistry Letters,* Vol. 28, No. (4), 16 January 2018 (2018-01-16), pp. 711-719 | 25-32, 35-50 |
| A | CN 115337406 A (TSINGHUA UNIVERSITY et al.) 15 November 2022 (2022-11-15) claims 1-52 | 1-50 |

Form PCT/ISA/210 (second sheet) (July 2022)

**INTERNATIONAL SEARCH REPORT**

International application No.
**PCT/CN2024/112501**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **48-50**
because they relate to subject matter not required to be searched by this Authority, namely:

The subject matter of claims 48-50 falls within methods for treatment of diseases in the human body (PCT Rule 39.1(iv)). The present search is carried out on the basis of the use of the compound or the pharmaceutical composition, etc., in the preparation of a drug for treating corresponding diseases.

2. ☑ Claims Nos.: **25-33**
because they relate to parts of the international application that do not comply with the prescribed requirements to such an extent that no meaningful international search can be carried out, specifically:

The general formulae in claims 25-33 cover a large number of known compounds, and therefore no effective search can be carried out. The present search report is provided with reference to the specific compound in claim 34 of the present application, with Y being defined as -NH-, R1 being defined as n-butyl, and Z being defined as an amino.

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2024/112501**

| Patent document cited in search report | Publication date (day/month/year) | Patent family member(s) | Publication date (day/month/year) |
|---|---|---|---|
| WO 2010089686 A1 | 12 August 2010 | UY 32411 A | 30 September 2010 |
| | | AR 075207 A1 | 16 March 2011 |
| | | TW 201040175 A | 16 November 2010 |
| | | US 2010197591 A1 | 05 August 2010 |
| | | JP 2012516885 A | 26 July 2012 |
| | | EP 2393811 A1 | 14 December 2011 |
| | | CA 2749893 A1 | 12 August 2010 |
| CN 118382625 A | 23 July 2024 | WO 2023104165 A1 | 15 June 2023 |
| | | EP 4446323 A1 | 16 October 2024 |
| CN 116789671 A | 22 September 2023 | WO 2023179567 A1 | 28 September 2023 |
| WO 2024055879 A1 | 21 March 2024 | None | |
| CN 103748081 A | 23 April 2014 | CL 2013003313 A1 | 04 July 2014 |
| | | PT 2709989 T | 27 March 2018 |
| | | SI 2709989 T1 | 30 April 2018 |
| | | LT 2709989 T | 10 April 2018 |
| | | US 2014073642 A1 | 13 March 2014 |
| | | US 8916575 B2 | 23 December 2014 |
| | | HUE 036220 T2 | 28 June 2018 |
| | | UA 114476 C2 | 26 June 2017 |
| | | EA 201391719 A1 | 31 March 2014 |
| | | EA 028254 B1 | 31 October 2017 |
| | | ME 02986 B | 20 October 2018 |
| | | NZ 616642 A | 26 June 2015 |
| | | PH 12019502269 A1 | 07 December 2020 |
| | | KR 20190124337 A | 04 November 2019 |
| | | KR 102119700 B1 | 08 June 2020 |
| | | ES 2663601 T3 | 16 April 2018 |
| | | NO 2709989 T3 | 19 May 2018 |
| | | EP 2709989 A1 | 26 March 2014 |
| | | EP 2709989 B1 | 20 December 2017 |
| | | EP 2709989 B8 | 18 April 2018 |
| | | JP 2014516958 A | 17 July 2014 |
| | | JP 6050329 B2 | 21 December 2016 |
| | | WO 2012156498 A1 | 22 November 2012 |
| | | BR 112013029537 A2 | 24 January 2017 |
| | | BR 112013029537 B1 | 30 June 2020 |
| | | CA 2835229 A1 | 22 November 2012 |
| | | CA 2835229 C | 05 May 2020 |
| | | DK 2709989 T3 | 09 April 2018 |
| | | PL 2709989 T3 | 29 June 2018 |
| | | KR 20140051849 A | 02 May 2014 |
| | | KR 102038895 B1 | 31 October 2019 |
| | | MX 2013013448 A | 27 February 2014 |
| | | MX 347966 B | 18 May 2017 |
| | | IL 228906 A0 | 31 December 2013 |
| | | IL 228906 A | 31 August 2017 |
| | | AU 2012258220 A1 | 31 October 2013 |
| | | AU 2012258220 B2 | 19 January 2017 |
| | | SG 194852 A1 | 30 December 2013 |
| | | RS 57023 B1 | 31 May 2018 |

Form PCT/ISA/210 (patent family annex) (July 2022)

**INTERNATIONAL SEARCH REPORT**
**Information on patent family members**

International application No.
**PCT/CN2024/112501**

| Patent document cited in search report | | | Publication date (day/month/year) | Patent family member(s) | | | Publication date (day/month/year) |
|---|---|---|---|---|---|---|---|
| | | | | MY | 166557 | A | 16 July 2018 |
| | | | | HRP | 20180447 | T1 | 20 April 2018 |
| | | | | CY | 1120429 | T1 | 10 July 2019 |
| | | | | ZA | 201308746 | B | 27 January 2016 |
| CN | 108069963 | A | 25 May 2018 | WO | 2019095455 | A1 | 23 May 2019 |
| CN | 115337406 | A | 15 November 2022 | US | 2024252670 | A1 | 01 August 2024 |
| | | | | JP | 2024517341 | A | 19 April 2024 |
| | | | | EP | 4331616 | A1 | 06 March 2024 |
| | | | | WO | 2022237884 | A1 | 17 November 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 202311048960 A


### Non-patent literature cited in the description


- *RSC Med. Chem.*, 2021, vol. 12, 1065-1120
- *Advanced Drug Delivery Reviews*, 2021, vol. 175, 113803
- IUPAC 1974 Recommendations for Section E, Fundamental Stereochemistry. *Pure Appl. Chem.*, 1976, vol. 45, 13-10
- *J. Med. Chem.*, 2021, vol. 64, 7507-7532